# EUROPÄISCHE PATENTSCHRIFT

(11) **EP 2 900 672 B1**
(45) Veröffentlichungstag und Bekanntmachung des Hinweises auf die Patenterteilung: **22.02.2017**
(21) Anmeldenummer: 13766962.8
(22) Anmeldetag: 25.09.2013
(51) Int. Cl.: C07D 487/04, C07D 519/00, A61K 31/55, A61P 35/00, A61P 31/12, A61P 29/00, A61P 25/28, A61P 9/10

(54) **BET-PROTEININHIBITORISCHE 5-ARYL-TRIAZOLO-AZEPINE**
BET-PROTEIN INHIBITORY 5-ARYL-TRIAZOLO-AZEPINES
5-ARYL-TRIAZOLO-AZÉPINES INHIBITRICE DE LA PROTÉINE BET

(30) Priorität: 28.09.2012 EP 12186658
(43) Veröffentlichungstag der Anmeldung: 05.08.2015
(73) Patentinhaber: Bayer Pharma Aktiengesellschaft, 13353 Berlin (DE)
(72) Erfinder: SCHMEES, Norbert, Dr., 13469 Berlin (DE); KUHNKE, Joachim, Dr., 14482 Potsdam (DE); HAENDLER, Bernard, Dr., 13465 Berlin (DE); NEUHAUS, Roland, Dr., 12157 Berlin (DE); LEJEUNE, Pascale, Dr., 13469 Berlin (DE); SIEGEL, Stephan, Dr., 10779 Berlin (DE); KRÜGER, Martin, Dr., 13465 Berlin (DE); FERNANDEZ-MONTALVAN, Amaury Ernesto, Dr., 10437 Berlin (DE); KÜNZER, Hermann, Dr., verstorben (DE); GALLENKAMP, Daniel, Dr., 42109 Wuppertal (DE)
(74) Vertreter: BIP Patents
(86) Internationale Anmeldenummer: PCT/EP2013/069902
(87) Internationale Veröffentlichungsnummer: WO 2014/048945

(56) Entgegenhaltungen:
- WO-A1-2011/054845

## Beschreibung

Die vorliegende Erfindung betrifft BET-proteininhibitorische, insbesondere BRD4-inhibitorische 5-Aryl-triazolo-azepine, pharmazeutische Mittel enthaltend die erfindungsgemäßen Verbindungen sowie deren prophylaktische und therapeutische Verwendung bei hyper-proliferativen Erkrankungen, insbesondere bei Tumorerkrankungen . Desweiteren betrifft diese Erfindung die Verwendung von BET-Proteininhibitoren in viralen Infektionen, in neurodegenerativen Erkrankungen, in inflammatorischen Krankheiten, in atherosklerotischen Erkrankungen und in der männlichen Fertilitätskontrolle.

Die humane BET-Familie (bromodomain and extra C-terminal domain family) hat vier Mitglieder (BRD2, BRD3, BRD4 und BRDT), die zwei verwandte Bromodomänen und eine extraterminale Domäne enthalten (Wu und Chiang, J. Biol. Chem., 2007, 282:13141-13145). Die Bromodomänen sind Proteinregionen die azetylierte Lysinreste erkennen. Solche azetylierte Lysine findet man oft am N-terminalen Ende von Histonen (z. B. Histon 3 oder Histon 4) und sind Merkmale für eine offene Chromatin-Struktur und aktive Gentranskription (Kuo und Allis, Bioessays, 1998, 20:615-626). Zusätzlich können Bromodomänen weitere azetylierte Proteine erkennen. Zum Beispiel bindet BRD4 an RelA, was zur Stimulierung von NF-κB und transkriptioneller Aktivität von inflammatorischen Genen führt (Huang et al., Mol. Cell. Biol., 2009, 29:1375-1387). Die extraterminale Domäne von BRD2, BRD3 und BRD4 interagiert mit mehreren Proteinen, die eine Rolle in der Chromatinmodulierung und der Regulation der Genexpression haben (Rahman et al., Mol. Cell. Biol., 2011, 31:2641-2652).

Mechanistisch spielen BET-Proteine eine wichtige Rolle im Zellwachstum und im Zellzyklus. Sie sind mit mitotischen Chromosomen assoziiert, was eine Rolle im epigenetischen Gedächtnis nahelegt (Dey et al., Mol. Biol. Cell, 2009, 20:4899-4909; Yang et al., Mol. Cell. Biol., 2008, 28:967-976). BRD4 ist essentiell für die Transkriptionselongation und rekrutiert den Elongationskomplex P-TEFb, der aus CDK9 und Cyclin T1 besteht, was zur Aktivierung der RNA Polymerase II führt (Yang et al., Mol. Cell, 2005, 19:535-545). Folglich wird die Expression von Genen stimuliert, die in der Zellproliferation involviert sind, wie zum Beispiel c-Myc und Aurora B (You et al., Mol. Cell. Biol., 2009, 29:5094-5103; Zuber et al., Nature, 2011, doi:10.1038). BRD2 und BRD3 binden an transkribierte Gene in hyperazetylierten Chromatinbereichen und fördern die Transkription durch RNA Polymerase II (LeRoy et al., Mol. Cell, 2008, 30:51-60).

Der Knock-down von BRD4 in verschiedenen Zelllinien führt zu einem G1-Arrest (Mochizuki et al., J. Biol. Chem., 2008, 283:9040-9048). Es wurde auch gezeigt, dass BRD4 an Promotorregionen von mehreren Genen, die in der G1-Phase aktiviert werden wie zum Beispiel Cyclin D1 und D2, bindet (Mochizuki et al., J. Biol. Chem., 2008, 283:9040-9048).

BRD2 und BRD4 Knockout-Mäuse sterben früh während der Embryogenese (Gyuris et al., Biochim. Biophys. Acta, 2009, 1789:413-421; Houzelstein et al., Mol. Cell. Biol., 2002, 22:3794-3802). Heterozygote BRD4 Mäuse haben verschiedene Wachstumsdefekte, die auf eine reduzierte Zellproliferation zurückzuführen sind (Houzelstein et al., Mol. Cell. Biol., 2002, 22:3794-3802). BET-Proteine spielen eine wichtige Rolle in verschiedenen Tumorarten. Die Fusion zwischen den BET-Proteinen BRD3 oder BRD4 und NUT, einem Protein das normalerweise nur im Hoden exprimiert wird, führt zu einer aggressiven Form des Plattenepithelkarzinoms, genannt NUT midline carcinoma (French, Cancer Genet. Cytogenet., 2010, 203:16-20). Das Fusionsprotein verhindert Zelldifferenzierung und fördert Proliferation (Yan et al., J. Biol. Chem., 2011, 286:27663-27675). Das Wachstum von davon abgeleiteten in vivo Modellen wird durch einen BRD4-Inhibitor gehemmt (Filippakopoulos et al., Nature, 2010, 468:1067-1073). Ein Screening für therapeutische Targets in einer akuten myeloiden Leukämiezelllinie (AML) zeigte, dass BRD4 eine wichtige Rolle in diesem Tumor spielt (Zuber et al., Nature, 2011, doi:10.1038). Die Reduktion der BRD4-Expression führt zu einem selektiven Arrest des Zellzyklus und zur Apoptose. Die Behandlung mit einem BRD4-Hemmer verhindert die Proliferation eines AML-Xenografts in vivo. Eine Amplifizierung der DNA-Region die das BRD4-Gen enthält wurde in primären Brusttumoren nachgewiesen (Kadota et al., Cancer Res, 2009, 69:7357-7365). Auch für BRD2 gibt es Daten bezüglich einer Rolle in Tumoren. Eine transgene Maus die BRD2 selektiv in B-Zellen hochexprimiert entwickelt B-Zell Lymphome und Leukemien (Greenwall et al., Blood, 2005, 103:1475-1484).

BET-Proteine sind auch an viralen Infektionen beteiligt. BRD4 bindet an das E2 Protein von verschiedenen Papillomaviren und ist wichtig für das Überleben der Viren in latent infizierten Zellen (Wu et al., Genes Dev., 2006, 20:2383-2396). Auch das Herpesvirus, das für das Kaposi-Sarkom verantwortlich ist, interagiert mit verschiedenen BET-Proteinen, was für die Krankheitsbeständigkeit wichtig ist (Viejo-Borbolla et al., J. Virol., 2005, 79:13618-13629; You et al., J. Virol., 2006, 80:8909-8919). Durch Bindung an P-TEFb spielt BRD4 auch eine wichtige Rolle in der Replikation von HIV (Bisgrove et al., Proc. Natl Acad. Sei. USA, 2007, 104:13690-13695).

BET-Proteine sind zusätzlich an Inflammationsprozessen beteiligt. BRD2-hypomorphe Mäuse zeigen eine reduzierte Inflammation im Fettgewebe (Wang et al., Biochem. J., 2009, 425:71-83). Auch die Infiltration von Makrophagen in weißem Fettgewebe ist in BRD2-defizienten Mäusen reduziert (Wang et al., Biochem. J., 2009, 425:71-83). Es wurde auch gezeigt, dass BRD4 eine Reihe von Genen reguliert, die in der Inflammation involviert sind. In LPS-stimulierten Makrophagen verhindert ein BRD4-Inhibitor die Expression von inflammatorischen Genen, wie zum Beispiel IL-1 oder IL-6 (Nicodeme et al., Nature, 2010, 468:1119-1123). BET-Proteine sind auch in der Regulierung des ApoA1-Gens involviert (Mirguet el al., Bioorg. Med. Chem. Lett., 2012, 22:2963-2967). Das entsprechende Protein ist Bestandteil des Lipoproteins höherer Dichte (HDL), das bei Atherosklerose eine wichtige Rolle spielt (Smith, Arterioscler. Thromb. Vasc. Biol., 2010, 30:151-155). Durch die Stimulierung der ApoA1 Expression, können BET-Proteininhibitoren die Konzentrationen an Cholesterol HDL erhöhen und somit für die Behandlung von Atherosklerose potentiell nützlich sein (Mirguet el al., Bioorg. Med. Chem. Lett., 2012, 22:2963-2967).

Das BET-Protein BRDT spielt eine wesentliche Rolle in der Spermatogenese durch die Regulierung der Expression mehreren Genen, die während und nach der Meiose wichtig sind (Shang et al., Development, 2007, 134:3507-3515; Matzuk et al., Cell, 2012, 150:673-684). Desweiteren ist BRDT in der post-meiotischen Organisation des Chromatins involviert (Dhar et al., J. Biol. Chem., 2012, 287:6387-6405). *In vivo* Versuche in der Maus zeigen, dass die Behandlung mit einem BET-Hemmer, der auch BRDT inhibiert, zu einer Abnahme der Spermienproduktion und Infertilität führt (Matzuk et al., Cell, 2012, 150:673-684).

Alle diese Untersuchungen zeigen, dass die BET-Proteine eine essentielle Rolle in verschiedenen Pathologien und auch in der männlichen Fertilität spielen. Es wäre deshalb wünschenswert potente und selektive Inhibitoren zu finden, die die Interaktion zwischen den BET-Proteinen und azetylierten Proteinen verhindern. Diese neuen Inhibitoren sollten auch geeignete pharmakokinetische Eigenschaften haben, die es erlauben *in vivo,* also im Patienten diese Interaktionen zu hemmen.

Es wurde nun gefunden, dass substituierte 4-Alkyl-6-aryl-4H-[1,2,4]triazolo[4,3-a][1]benzazepine die erwünschten Eigenschaften aufweisen, d.h. eine BRD4 inhibitorische Wirkung zeigen.

### Stand der Technik

Die bei der Betrachtung des strukturellen Standes der Technik angewendete Nomenklatur wird durch die nachfolgende Abbildung verdeutlicht:

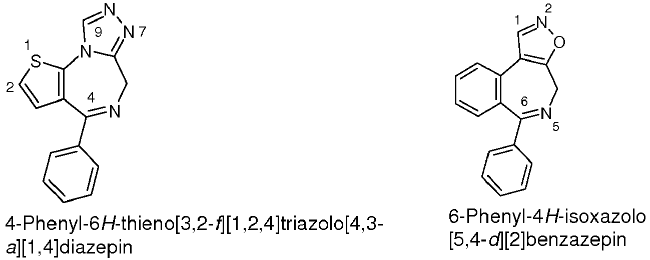

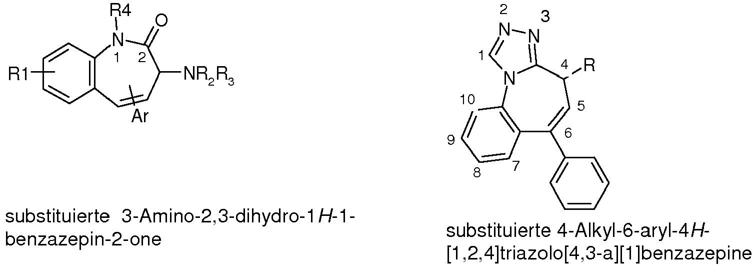

Bezogen auf die chemische Struktur wurden bisher nur sehr wenige Typen von BRD4-Inhibitoren beschrieben (Chun-Wa Chung et al., Progress in Medicinal Chemistry 2012, 51, 1-55).

Die ersten publizierten BRD4-Inhibitoren waren Diazepine. So werden z. B. Phenyl-thieno-triazolo-1,4-diazepine (4-Phenyl-6*H*-thieno[3,2-*f*][1,2,4]triazolo[4,3-*a*][1,4]diazepine) in der WO2009/084693 (Mitsubishi Tanabe Pharma Corporation) und als Verbindung JQ1 in der WO2011/143669 (Dana Farber Cancer Institute) beschrieben. Der Ersatz der Thieno- durch eine Benzo-Einheit führte auch zu aktiven Inhibitoren (J. Med. Chem. 2011, 54, 3827 - 3838; E. Nicodeme et al., Nature 2010, 468, 1119). Weitere 4-Phenyl-6*H*-thieno[3,2-*f*]1[1,2,4]triazolo[4,3-*a*][1,4]diazepine und verwandte Verbindungen mit alternativen Ringen als Fusionspartner anstelle der Benzo-Einheit wurden generisch adressiert oder direkt beschrieben in der WO2012/075456 (Constellation Pharmaceuticals).

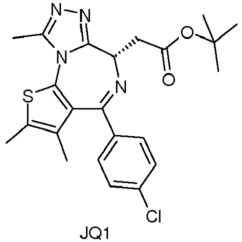

Azepine als BRD-4 Inhibitoren wurden kürzlich in der WO2012/075383 (Constellation Pharmaceuticals) beschrieben. Diese Anmeldung umfasst 6-substituierte 4*H*-Isoxazolo[5,4-*d*][2]benzazepine und 4*H*-Isoxazolo[3,4-*d*][2]benzazepine einschließlich solcher Verbindungen, die an Position 6 optional substituiertes Phenyl aufweisen und auch Analoga mit alternativen heterozyklischen Fusionspartnern anstelle der Benzo-Einheit, wie z.B. Thieno- oder Pyridoazepine.

Die erfindungsgemässen Verbindungen sind hingegen substituierte 4-Alkyl-6-aryl-4H-[1,2,4]triazolo[4,3-a][1]benzazepine, die im Unterschied zu den weiter oben genannten 4H-Isoxazolo-[5,4-*d*][2]-benzazepinen den Ringstickstoff an einer völlig anderen Position, nämlich an Position 10b, aufweisen. Aufgrund der wesentlichen Strukturunterschiede war nicht davon auszugehen, dass die hier beanspruchten Verbindungen auch BRD4 inhibitorisch wirksam sind. Es ist deshalb überrraschend, dass die erfindungsgemäßen Verbindungen trotz der Strukturunterschiede eine gute inhibitorische Wirkung aufweisen. Als eine andere strukturelle Klasse von BRD4 Inhibitoren werden 7-Isoxazolochinoline und verwandte Chinolon-Derivate beschrieben (Bioorganic & Medicinal Chemistry Letters 22 (2012) 2963-2967). In der WO2011/054845 (GlaxoSmithKline) werden weitere Benzodiazepine als BRD4 Inhibitoren beschrieben.

Einige Schriften beinhalten strukturell ähnliche, aber auf völlig andere Targets bzw. Indikationen zielende Verbindungen. So werden in der WO94/26718 bzw. EP0703222A1 (Yoshitomi Pharmaceutical Industries) substituierte 3-Amino-2,3-dihydro-1*H*-1-benzazepin-2-one oder die entsprechenden 2-Thione und Analoga, in denen die Benzo-Einheit durch alternative monocyclische Systeme ersetzt ist und in denen, das 2-Keton oder das 2-Thion zusammen mit dem substituierten Stickstoffatom des Azepinringes einen Heterozyklus bilden kann, als CCK- und Gastrin-Antagonisten für die Therapie von Erkrankungen des ZNS, wie Angstzustände und Depressionen, sowie von Erkrankungen der Bauchspeicheldrüse und von gastrointestinalen Geschwüren, beschrieben. Diese Verbindungen unterscheiden sich jedoch von den erfindungsgemäßen Verbindungen durch die obligatorische 2-Oxogruppe und die Stickstoffgruppe an Position 4 anstelle einer Alkylgruppe.

In der WO2012/075456A1 bzw. EP1939205A1 (Daiichi Sankyo) werden substituierte 4-Alkyl-6-aryl-5,6-dihydro-4H-[1,2,4]triazolo[4,3-a][1]benzazepine für die Indikationen Hypercholesterolämie, Hyperlipidämie und Atherosklerose beschrieben. Im Unterschied zu den erfindungsgemäßen Verbindungen fehlt bei diesen Verbindungen die 5,6-Doppelbindung im Azepinring.

Dennoch besteht nach wie vor ein großes Bedürfnis nach selektiv wirksamen Verbindungen zur Prophylaxe und Therapie von Krebs- und insbesondere Tumorerkrankungen.

Es wurde nun gefunden, dass Verbindungen der allgemeinen Formel (I) 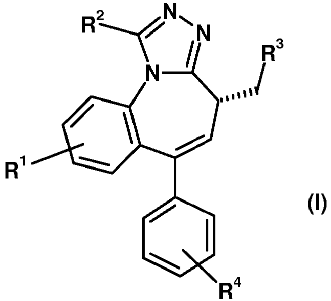 in der
- R¹: für Wasserstoff, Hydroxy, C₁-C₆-Alkyl, Halogen-C₁-C₆-Alkyl, C₁-C₆-Alkoxy, Halogen-C₁-C₆-Alkoxy, Halogen oder Cyano steht,
oder
für C₃-C₈-Zykloalkyl, C₃-C₈-Zykloalkoxy, C₃-C₈-Heterozykloalkyl, Aryl oder Heteroaryl mit 5 oder 6 Ringatomen steht, die gegebenenfalls ein- oder mehrfach, gleich oder verschieden substituiert sein können mit C₁-C₆-Alkyl, Halogen-C₁-C₆-Alkyl, C₁-C₆-Alkoxy, Halogen-C₁-C₆-Alkoxy, Hydroxy, Oxo, Halogen, Cyano, Heteroaryl oder Aryl, oder für -C(=O)-OR⁸, -C(=O)-NR¹²R¹³, -C(=O)-R¹⁴, -S(=O)₂-C₁-C₆-Alkyl,-S(=O)₂-OR⁸ oder -S(=O)ₐ-NR¹²R¹³ steht,
- R²: für Wasserstoff, C₁-C₆-Alkyl oder für -NR⁶R⁷ steht,
- R³: für Cyano, -C(=O)-OR⁸, -C(=O)-R⁹ oder -C(=O)-NR⁶R⁷ steht,
oder
für ein 5- oder 6- gliedriges Ringsystem steht, enthaltend 0, 1, 2, 3 oder 4 Heteroatome unabhängig voneinander ausgewählt aus der Gruppe O, N oder S, welches wahlweise aromatisch oder auch nicht-aromatisch sein kann und gegebenenfalls ein- oder mehrfach, gleich oder verschieden mit R⁵ substituiert sein kann,
- R⁴: für Wasserstoff, Fluor, Chlor, Brom oder Cyano steht,
- R⁵: für Wasserstoff, C₁-C₆-Alkyl-, C₃-C₈-Zykloalkyl, C₃-C₈-Heterozykloalkyl, Halogen-C₁-C₆-Alkyl, Aryl, Heteroaryl, Hydroxy, C₁-C₆-Alkoxy, C₁-C₃-Alkoxy-C₁-C₃-Alkyl-, Aryloxy-C₁-C₃-Alkyl, Aryl-C₁-C₃-Alkoxy-C₁-C₃-Alkyl-, Halogen-C₁-C₆-Alkoxy, Halogen, Cyano oder Oxo steht,
- R⁶ und R⁷: unabhängig voneinander für Wasserstoff oder für -NH-C(=O)-R¹⁵ stehen,
oder
für C₁-C₆-Alkyl, C₁-C₆-Alkoxy, C₃-C₈-Heterozykloalkyl, Aryl, Heteroaryl, C₃-C₈-Zykloalkyl, C₆-C₁₂-Bizykloalkyl, C₅-C₁₁-Spirozykloalkyl, C₆-C₁₂-Heterobizycloalkyl oder C₅-C₁₁-Heterospirozykloalkyl stehen, die gegebenenfalls ein- oder mehrfach, gleich oder verschieden substituiert sein können mit C₁-C₆-Alkyl, Halogen-C₁-C₆-Alkyl, C₁-C₆-Alkoxy, Halogen-C₁-C₆-Alkoxy, Phenoxy, Hydroxy, Oxo, Halogen, Cyano, C₃-C₈-Zykloalkyl, C₃-C₈-Heterozykloalkyl, Heteroaryl oder Aryl, oder für die Gruppe 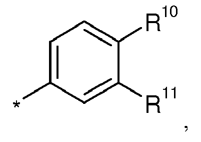 stehen, in welcher
- R¹⁰ und R¹¹: gemeinsam für C₃-C₆-Alkylen oder C₃-C₆-Heteroalkylen stehen, die gegebenenfalls ein- oder mehrfach, gleich oder verschieden substituiert sein können mit Halogen, Oxo, Hydroxy, Cyano, Nitro, C₁-C₃-Alkyl, Halogen-C₁-C₆-Alkyl, C₁-C₆-Alkoxy, Halogen- C₁-C₆-Alkoxy, C₁-C₆-Alkoxy- C1-C₆-Alkyl, C₁-C₆-Alkylcarbonyl, und worin "*" den Anknüpfungspunkt an den Rest des Moleküls kennzeichnet,
- R⁸: für Wasserstoff, C₁-C₆-Alkyl, C₃-C₃-Zykloalkyl, C₃-C₈-Heterozykloalkyl, Aryl oder Heteroaryl steht, die gegebenenfalls ein- oder mehrfach, gleich oder verschieden substituiert sein können mit C₁-C₆-Alkyl, Halogen-C₁-C₆-Alkyl, C₁-C₆-Alkoxy, Halogen-C₁-C₆-Alkoxy, Hydroxy, Oxo, Halogen, Cyano, Nitro, Heteroaryl oder Aryl,
- R⁹: für C₃-C₈-Heterozykloalkyl, C₅-C₁₁-Spiroheterozykloalkyl, C₆-C₁₂-Heterobizykloalkyl oder für einen überbrückten Heterozyklus, bestehend aus 7 bis 15 Ringatomen, steht, die gegebenfalls ein- oder mehrfach, gleich oder verschieden substituiert sein können mit C₁-C₄-Alkyl, C₃-C₈-Zykloalkyl, C₃-C₈-Heterozykloalkyl, Halogen-C₁-C₆-Alkyl-, Aryl, Aryloxy, Aryl-C₁-C₂-Alkyl,-C(=O)-O-C₁-C₆-Alkyl, Heteroaryl, Hydroxy, C₁-C₆-Alkoxy, Halogen-C₁-C₆-Alkoxy, Halogen, Cyano oder Oxo,
- R¹² und R¹³: unabhängig voneinander für Wasserstoff, C₁-C₆-Alkyl stehen,
- R¹⁴: für Wasserstoff, C₁-C₆-Alkyl, Amino, C₁-C₆-Alkoxy, C₃-C₈-Zykloalkyl, C₃-C₈-Heterozykloalkyl, Aryl oder Heteroaryl steht, die gegebenenfalls ein- oder mehrfach, gleich oder verschieden substituiert sein können mit C₁-C₆-Alkyl, Halogen-C₁-C₆-Alkyl, C₁-C₆-Alkoxy, Halogen-C₁-C₆-Alkoxy, Hydroxy, Oxo, Halogen, Cyano, Nitro, Heteroaryl oder Aryl, und
- R¹⁵: für Wasserstoff, C₁-C₆-Alkyl, Halogen-C₁-C₆-Alkyl, Aryl oder Aryl-C₁-C₂-Alkyl steht,
worin das Aryl und das in Aryl-C₁-C₂-Alkyl enthaltene Aryl ihrerseits gegebenenfalls ein- oder mehrfach, gleich oder verschieden substituiert sein können mit Halogen, Cyano, C₁-C₃-Alkyl, C₁-C₃-Alkoxy oder Trifluormethyl,
sowie deren Diastereomere, Razemate, Tautomere, Polymorphe, Solvate und physiologisch verträglichen Salze, zur Prophylaxe und Therapie von hyperproliferativen Erkrankungen und insbesondere von Tumorerkrankungen geeignet sind.

So verhindern die erfindungsgemäßen Verbindungen überraschenderweise die Interaktion zwischen zwischen BET-Proteinen, insbesondere BRD4, und einem azetylierten Histon 4 Peptid und inhibieren somit das Wachstum von Krebs- und Tumorzellen.

Bevorzugt sind solche Verbindungen der allgemeinen Formel (I), in der
- R¹: für Wasserstoff, Hydroxy, C₁-C₆-Alkyl, Fluor-C₁-C₆-Alkyl, C₁-C₆-Alkoxy, Fluor-C₁-C₆-Alkoxy, Halogen oder Cyano steht,
oder
für C₃-C₈-Zykloalkyl, C₃-C₈-Zykloalkoxy, C₃-C₈-Heterozykloalkyl, Aryl oder Heteroaryl mit 5 oder 6 Ringatomen steht, die gegebenenfalls ein- oder mehrfach, gleich oder verschieden substituiert sein können mit C₁-C₆-Alkyl, Fluor-C₁-C₆-Alkyl, C₁-C₆-Alkoxy, Fluor-C₁-C₆-Alkoxy, Hydroxy, Oxo, Halogen, Cyano, Heteroaryl oder Aryl,
- R²: für Methyl oder Methylamino- steht,
- R³: für Cyano, -C(=O)-OR⁸, -C(=O)-R⁹ oder -C(=O)-NR⁶R⁷ steht,
oder
für ein 5- oder 6- gliedriges Ringsystem steht, enthaltend 0, 1, 2 oder 3 Heteroatome unabhängig voneinander ausgewählt aus der Gruppe O, N oder S, welches wahlweise aromatisch oder auch nicht-aromatisch sein kann und gegebenenfalls ein- oder mehrfach, gleich oder verschieden mit R⁵ substituiert sein kann,
- R⁴: für Wasserstoff, Fluor, Chlor, Brom oder Cyano steht,
- R⁵: für Wasserstoff, C₁-C₆-Alkyl-, C₃-C₈-Zykloalkyl, C₃-C₈-Heterozykloalkyl, Fluor-C₁-C₆-Alkyl, Aryl, Heteroaryl, Hydroxy, C₁-C₆-Alkoxy, C₁-C₃-Alkoxy-C₁-C₃-Alkyl-, Aryloxy-C₁-C₃-Alkyl, Aryl-C₁-C₃-Alkoxy-C₁-C₃-Alkyl-, Fluor-C₁-C₆-Alkoxy, Halogen, Cyano oder Oxo steht,
- R⁶ und R⁷: unabhängig voneinander für Wasserstoff oder für -NH-C(=O)-R¹⁵ stehen,
oder
für C₁-C₆-Alkyl, C₁-C₆-Alkoxy, C₃-C₈-Heterozykloalkyl, Aryl, Heteroaryl, C₃-C₈-Zykloalkyl, C₆-C₁₂-Bizykloalkyl, C₅-C₁₁-Spirozykloalkyl, C₆-C₁₂-Heterobizycloalkyl oder C₅-C₁₁-Heterospirozykloalkyl stehen, die gegebenenfalls ein- oder mehrfach, gleich oder verschieden substituiert sein können mit C₁-C₃-Alkyl, Fluor-C₁-C₃-Alkyl, C₁-C₃-Alkoxy, Fluor-C₁-C₃-Alkoxy, Phenoxy, Hydroxy, Oxo, Halogen, Cyano, C₃-C₈-Zykloalkyl, C₃-C₈-Heterozykloalkyl, Heteroaryl oder Aryl, oder für die Gruppe 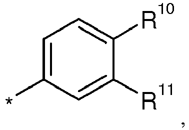 stehen, in welcher
- R¹⁰ und R¹¹: gemeinsam für C₃-C₆-Alkylen oder C₃-C₆-Heteroalkylen stehen, die gegebenenfalls ein- oder mehrfach, gleich oder verschieden substituiert sein können mit Halogen, Oxo, Hydroxy, Cyano, Nitro, C₁-C₃-Alkyl, Fluor-C₁-C₃-Alkyl, C₁-C₃-Alkoxy, Fluor-C₁-C₃-Alkoxy, C₁-C₃-Alkoxy-C₁-C₃-Alkyl, C₁-C₃-Alkylcarbonyl,
und worin "*" den Anknüpfungspunkt an den Rest des Moleküls kennzeichnet,
- R⁸: für Wasserstoff, C₁-C₆-Alkyl, C₃-C₈-Zykloalkyl, Aryl oder Heteroaryl steht, die gegebenenfalls ein- oder mehrfach, gleich oder verschieden substituiert sein können mit C₁-C₆-Alkyl, Fluor-C₁-C₆-Alkyl, C₁-C₆-Alkoxy, Fluor-C₁-C₆-Alkoxy, Hydroxy, Oxo, Halogen, Cyano, Nitro, Heteroaryl oder Aryl,
- R⁹: für C₃-C₈-Heterozykloalkyl, C₅-C₁₁-Spiroheterozykloalkyl, C₆-C₁₂-Heterobizykloalkyl oder für einen überbrückten Heterozyklus, bestehend aus 7 bis 15 Ringatomen, steht, die gegebenfalls ein- oder mehrfach, gleich oder verschieden substituiert sein können mit C₁-C₄-Alkyl, C₃-C₈-Zykloalkyl, C₃-C₈-Heterozykloalkyl, Fluor-C₁-C₆-Alkyl-, Aryl, Aryloxy, Aryl-C₁-C₂-Alkyl,-C(=O)-O-C₁-C₆-Alkyl, Heteroaryl, Hydroxy, C₁-C₆-Alkoxy, Fluor-C₁-C₆-Alkoxy, Halogen, Cyano oder Oxo, und
- R¹⁵: für C₁-C₆-Alkyl, Fluor-C₁-C₃-Alkyl, Phenyl oder Phenyl-C₁-C₂-Alkyl steht, worin das Phenyl und das in Phenyl-C₁-C₂-Alkyl enthaltene Phenyl ihrerseits gegebenenfalls ein- oder mehrfach, gleich oder verschieden substituiert sein können mit Halogen, Cyano, C₁-C₃-Alkyl, C₁-C₃-Alkoxy oder Trifluormethyl,
sowie deren Diastereomere, Razemate, Tautomere, Polymorphe, Solvate und physiologisch verträglichen Salze.

Besonders bevorzugt sind solche Verbindungen der allgemeinen Formel (I), in der
- R¹: für Wasserstoff, Hydroxy, C₁-C₃-Alkyl, Fluor-C₁-C₃-Alkyl, C₁-C₃-Alkoxy oder Fluor-C₁-C₃-Alkoxy steht,
oder
für Heteroaryl mit 5 oder 6 Ringatomen steht, das gegebenenfalls ein- oder mehrfach, gleich oder verschieden substituiert sein kann mit C₁-C₃-Alkyl, Fluor-C₁-C₃-Alkyl, C₁-C₃-Alkoxy, Fluor-C₁-C₃-Alkoxy, Halogen oder Cyano,
- R²: für Methyl steht,
- R³: für -C(=O)-OR⁸, -C(=O)-R⁹ oder -C(=O)-NR⁶R⁷ steht,
oder
für ein 5- gliedriges aromatisches Ringsystem steht, enthaltend 1, 2 oder 3 Heteroatome unabhängig voneinander ausgewählt aus der Gruppe O, N oder S, welches gegebenenfalls ein- oder mehrfach, gleich oder verschieden mit R⁵ substituiert sein kann,
- R⁴: für Chlor steht,
- R⁵: für Wasserstoff, C₁-C₃-Alkyl-, C₃-C₆-Zykloalkyl, C₃-C₆-Heterozykloalkyl, Fluor-C₁-C₃-Alkyl, Aryl, Heteroaryl, Hydroxy, C₁-C₃-Alkoxy, C₁-C₃-Alkoxy-C₁-C₃-Alkyl-, Aryloxy-C₁-C₃-Alkyl, Aryl-C₁-C₃-Alkoxy-C₁-C₃-Alkyl-, Fluor-C₁-C₃-Alkoxy, Halogen, oder Cyano steht,
- R⁶ und R⁷: unabhängig voneinander für Wasserstoff oder für -NH-C(=O)-R¹⁵ stehen,
oder
für C₁-C₆-Alkyl, C₁-C₆-Alkoxy, C₃-C₈-Heterozykloalkyl, Aryl, Heteroaryl, C₃-C₈-Zykloalkyl, C₆-C₁₂-Bizykloalkyl, C₅-C₁₁-Spirozykloalkyl, C₆-C₁₂-Heterobizycloalkyl oder C₅-C₁₁-Heterospirozykloalkyl stehen, die gegebenenfalls ein- oder mehrfach, gleich oder verschieden substituiert sein können mit C₁-C₃-Alkyl, Fluor-C₁-C₃-Alkyl, C₁-C₃-Alkoxy, Fluor-C₁-C₃-Alkoxy, Phenoxy, Hydroxy, Oxo, Halogen, Cyano, C₃-C₈-Zykloalkyl, C₃-C₈-Heterozykloalkyl, Heteroaryl oder Aryl, oder für die Gruppe 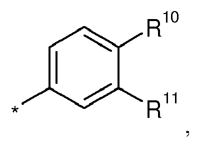 stehen, in welcher
- R¹⁰ und R¹¹: gemeinsam für C₃-C₆-Alkylen oder C₃-C₆-Heteroalkylen stehen, die gegebenenfalls ein- oder mehrfach, gleich oder verschieden substituiert sein können mit Halogen, Oxo, Hydroxy, Cyano, Nitro, C₁-C₃-Alkyl, Fluor-C₁-C₃-Alkyl, C₁-C₃-Alkoxy, Fluor-C₁-C₃-Alkoxy, C₁-C₃-Alkoxy-C₁-C₃-Alkyl, C₁-C₃-Alkylcarbonyl, und worin "*" den Anknüpfungspunkt an den Rest des Moleküls kennzeichnet,
- R⁸: für C₁-C₆-Alkyl steht,
- R⁹: für eine der folgenden Gruppen 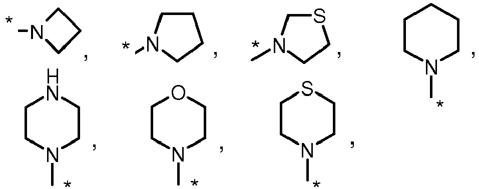 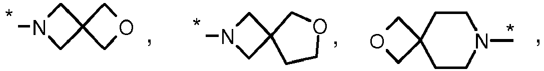 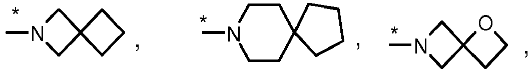 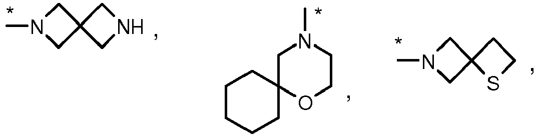 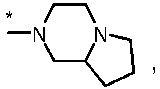 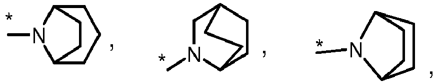 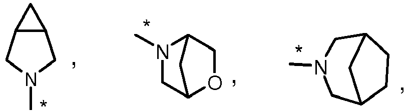 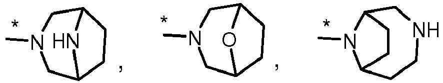 steht, die gegebenfalls ein- oder mehrfach, gleich oder verschieden substituiert sein können mit C₁-C₄-Alkyl, C₃-C₈-Zykloalkyl, C₃-C₈-Heterozykloalkyl, Fluor-C₁-C₆-Alkyl-, Aryl, Aryloxy, Aryl-C₁-C₂-Alkyl, -C(=O)-O-C₁-C₆-Alkyl, Heteroaryl, Hydroxy, C₁-C₆-Alkoxy, Fluor-C₁-C₆-Alkoxy, Halogen, Cyano oder Oxo,
und worin "*" den Anknüpfungspunkt an den Rest des Moleküls kennzeichnet, und
- R¹⁵: für C₁-C₃-Alkyl, Trifluormethyl, Phenyl oder Benzyl steht, worin das Phenyl und das in Benzyl enthaltene Phenyl ihrerseits gegebenenfalls ein- oder mehrfach, gleich oder verschieden substituiert sein können mit Fluor, Chlor, Brom, Methyl oder Methoxy,
sowie deren Diastereomere, Razemate, Tautomere, Polymorphe,Solvate und physiologisch verträglichen Salze.

Ganz besonders bevorzugt sind solche Verbindungen der allgemeinen Formel (I), in der
- R¹: für Wasserstoff, Hydroxy, C₁-C₃-Alkyl, Fluor-C₁-C₃-Alkyl, C₁-C₃-Alkoxy oder Fluor-C₁-C₃-Alkoxy steht,
oder
für Heteroaryl mit 5 Ringatomen steht, das gegebenenfalls ein- oder mehrfach, gleich oder verschieden substituiert sein kann mit C₁-C₃-Alkyl oder C₁-C₃-Alkoxy,
- R²: für Methyl steht,
- R³: für -C(=O)-OR⁸, -C(=O)-R⁹ oder -C(=O)-NR⁶R⁷ steht,
oder
für eines der folgenden Ringsysteme 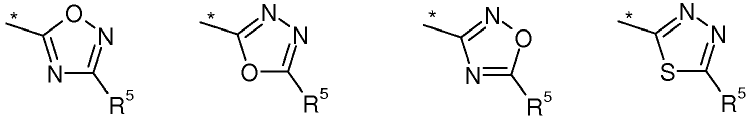 worin "*" den Anknüpfungspunkt an den Rest des Moleküls kennzeichnet, steht,
- R⁴: für Chlor steht,
- R⁵: für Wasserstoff, C₁-C₃-Alkyl-, C₃-C₆-Zykloalkyl, C₃-C₆-Heterozykloalkyl, Phenyl, 5- oder 6-gliedriges Heteroaryl, C₁-C₃-Alkoxy-C₁-C₃-Alkyl-, Phenoxy-C₁-C₃-Alkyl- oder Benzyloxy-C₁-C₃-Alkyl- steht,
- R⁶ und R⁷: unabhängig voneinander für Wasserstoff oder für -NH-C(=O)-R¹⁵ stehen,
oder
für C₁-C₆-Alkyl, C₃-C₈-Heterozykloalkyl, Phenyl, Heteroaryl mit 5 oder 6 Ringatomen, oder C₃-C₈-Zykloalkyl stehen, die gegebenenfalls ein- oder mehrfach, gleich oder verschieden substituiert sein können mit C₁-C₃-Alkyl, Fluor-C₁-C₃-Alkyl, C₁-C₃-Alkoxy, Phenoxy, Hydroxy, Oxo, Halogen, Cyano, C₃-C₈-Zykloalkyl, C₃-C₈-Heterozykloalkyl, 5- oder 6-gliedriges Heteroaryl oder Phenyl, oder für die Gruppe 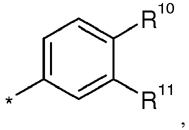 stehen, in welcher
- R¹⁰ und R¹¹: gemeinsam für C₃-C₄-Alkylen oder C₃-C₄-Heteroalkylen stehen, die gegebenenfalls ein- oder mehrfach, gleich oder verschieden substituiert sein können mit Halogen, Oxo, Hydroxy, C₁-C₃-Alkyl, C₁-C₃-Alkoxy, C₁-C₃-Alkylcarbonyl,
und worin "*" den Anknüpfungspunkt an den Rest des Moleküls kennzeichnet,
- R⁸: für C₁-C₄-Alkyl steht,
- R⁹: für eine der folgenden Gruppen 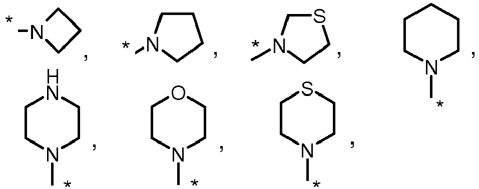 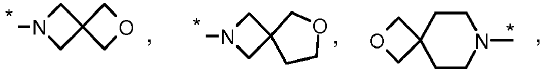 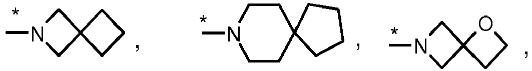 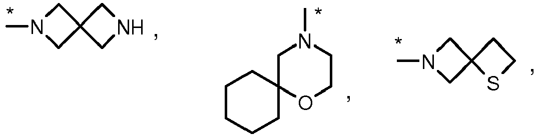 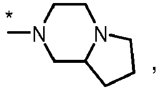 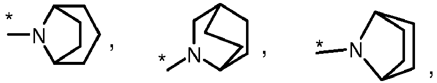 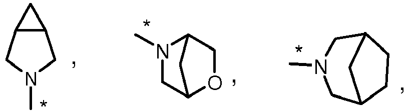 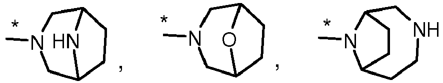 steht, die gegebenfalls ein- oder mehrfach, gleich oder verschieden substituiert sein können mit C₁-C₄-Alkyl, Phenyl, Phenoxy, Benzyl, -C(=O)-O-C₁-C₄-Alkyl, 5- oder 6-gliedriges Heteroaryl, Hydroxy, C₁-C₄-Alkoxy, Fluor, Cyano oder Oxo, und worin "*" den Anknüpfungspunkt an den Rest des Moleküls kennzeichnet, und
- R¹⁵: für C₁-C₃-Alkyl steht,
sowie deren Diastereomere, Razemate, Tautomere, Polymorphe, Solvate und physiologisch verträglichen Salze.

Noch mehr bevorzugt sind solche Verbindungen der allgemeinen Formel (I), in der
- R¹: für Wasserstoff, C₁-C₃-Alkoxy oder Fluor-C₁-C₃-Alkoxy steht,
oder
für Oxazolyl oder Isoxazolyl steht, die gegebenenfalls ein- oder mehrfach, gleich oder verschieden substituiert sein können mit C₁-C₃-Alkyl oder C₁-C₃-Alkoxy,
- R²: für Methyl steht,
- R³: für -C(=O)-OR⁸, -C(=O)-R⁹ oder -C(=O)-NR⁶R⁷ steht,
oder für eines der folgenden Ringsysteme 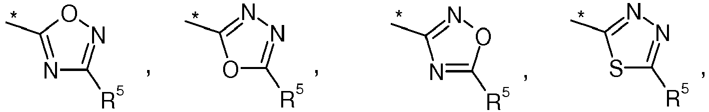 worin "*" den Anknüpfungspunkt an den Rest des Moleküls kennzeichnet, steht,
- R⁴: für Chlor steht,
- R⁵: für Wasserstoff, C₁-C₃-Alkyl-, C₃-C₆-Zykloalkyl, Pyridinyl oder Benzyloxy-C₁-C₃-Alkyl- steht,
- R⁶ und R⁷: unabhängig voneinander für Wasserstoff oder für -NH-C(=O)-R¹⁵ stehen,
oder
für C₁-C₆-Alkyl stehen, das gegebenenfalls ein- oder mehrfach, gleich oder verschieden substituiert sein kann mit C₃-C₈-Heterozykloalkyl, oder für die Gruppe 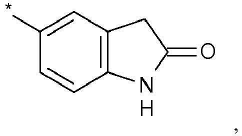 stehen, worin "*" den Anknüpfungspunkt an den Rest des Moleküls kennzeichnet,
- R⁸: für Ethyl oder *tert*-Butyl steht,
- R⁹: für eine der folgenden Gruppen 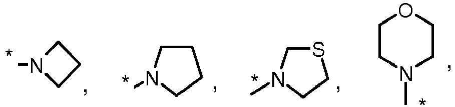 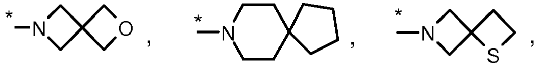 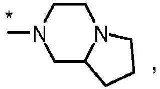 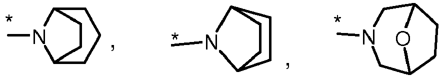 steht, die gegebenfalls ein- oder mehrfach, gleich oder verschieden substituiert sein können mit C₁-C₄-Alkyl, Phenoxy, Benzyl, -C(=O)-O-C₁-C₄-Alkyl, Fluor oder Oxo, und worin "*" den Anknüpfungspunkt an den Rest des Moleküls kennzeichnet, und
- R¹⁵: für C₁-C₃-Alkyl steht,
sowie deren Diastereomere, Razemate, Tautomere, Polymorphe, Solvate und physiologisch verträglichen Salze.

Überaus bevorzugt sind solche Verbindungen der allgemeinen Formel (I), in der
- R¹: für Wasserstoff, Methoxy, Trifluormethoxy oder für 3,5-Dimethylisoxazol-4-yl steht,
- R²: für Methyl steht,
- R³: für -C(=O)-OR⁸, -C(=O)-R⁹ oder -C(=O)-NR⁶R⁷ steht, oder
für eines der folgenden Ringsysteme 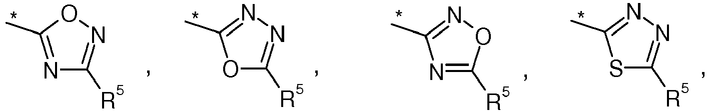 worin "*" den Anknüpfungspunkt an den Rest des Moleküls kennzeichnet, steht,
- R⁴: für Chlor steht,
- R⁵: für Methyl, *iso*-Propyl, Zyklopropyl, Pyridin-3-yl oder Benzyloxymethyl steht,
- R⁶ und R⁷: unabhängig voneinander für Wasserstoff oder für -NH-C(=O)-R¹⁵ stehen,
oder
für Ethyl stehen das gegebenenfalls einfach substituiert sein kann mit Morpholinyl, oder für die Gruppe 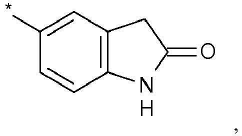 stehen, worin "*" den Anknüpfungspunkt an den Rest des Moleküls kennzeichnet,
- R⁸: für Ethyl oder *tert*-Butyl steht,
- R⁹: für eine der folgenden Gruppen 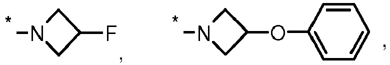 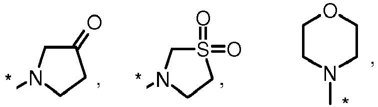 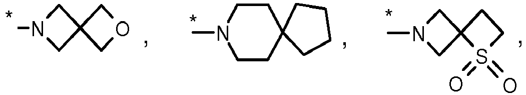 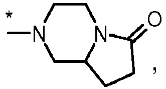 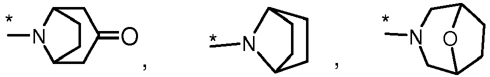 steht,
worin "*" den Anknüpfungspunkt an den Rest des Moleküls kennzeichnet, und
- R¹⁵: für Methyl steht,
sowie deren Diastereomere, Razemate, Tautomere, Polymorphe, Solvate und physiologisch verträglichen Salze.

Weiterhin interessant sind solche Verbindungen der allgemeinen Formel I, in der
- R¹: für Wasserstoff, Hydroxy, C₁-C₆-Alkyl, Halogen-C₁-C₆-Alkyl, C₁-C₆-Alkoxy, Halogen-C₁-C₆-Alkoxy, Halogen, Cyano, oder für Aryl, welches ein- oder mehrfach, gleich oder verschieden mit C₁-C₆-Alkyl, Halogen-C₁-C₆-Alkyl, C₁-C₆-Alkoxy, Halogen-C₁-C₆-Alkoxy, Hydroxy, Oxo, Halogen, Cyano, Heteroaryl oder Aryl substituiert sein kann, oder für C₃-C₈-Zykloalkyl, C₃-C₈-Zykloalkoxy, C₃-C₈-Heterozykloalkyl, Aryl oder Heteroaryl mit 5-6 Ringatomen und 1-3 Heteroatomen aus der Gruppe N, O und S, welches ein- oder mehrfach, gleich oder verschieden mit C₁-C₆-Alkyl, Halogen-C₁-C₆-Alkyl, C₁-C₆-Alkoxy, Halogen-C₁-C₆-Alkoxy, Hydroxy, Oxo, Halogen, Cyano, Heteroaryl oder Aryl substituiert sein kann, oder für die Gruppe -C(O)OR⁸, -C(O)NR⁶R⁷, -C(O)R¹⁴, -S(O)₂C₁-C₆-alkyl, -S(O)₂OR⁸ oder -S(O)₂NR⁶R⁷ steht,
- R²: für Wasserstoff, eine C₁-C₆-Alkyl-Gruppe oder für -NR⁶R⁷ steht,
- R³: für eine -C(O)OR⁸, -C(O)R⁹, -C(O)NR⁶R⁷ Gruppe oder für Cyano, oder für ein 5-6 gliedriges Ringsystem, enthaltend 0-4 Heteroatome ausgewählt aus der Gruppe O, N oder S steht, welches wahlweise aromatisch oder auch nicht-aromatisch sein kann und weitere Reste R⁵ tragen kann,
- R⁴: für Wasserstoff, Fluor, Chlor, Brom oder Cyano steht,
- R⁵: für Wasserstoff, C₁-C₆-Alkyl-, C₃-C₈-Zykloalkyl, C₃-C₈-Heterozykloalkyl, Halogen-C₁-C₆-Alkyl, Aryl, Heteroaryl, Hydroxy, C₁-C₆-Alkoxy, Halogen-C₁-C₆-Alkoxy, Halogen, Cyano oder Oxo steht,
- R⁶ und R⁷: unabhängig voneinander für Wasserstoff, C₁-C₆-Alkyl, C₁-C₆-Alkoxy, C₃-C₈-Heterozykloalkyl, Aryl, Heteroaryl, C₃-C₈-Zykloalkyl, C₆-C₁₂- Bizykloalkyl, C₅-C₁₁-Spirozykloalkyl, C₆-C₁₂-Heterobizycloalkyl oder C₅-C₁₁-Heterospirozykloalkyl steht, welche weitere Substituenten aus der Gruppe C₁-C₆-Alkyl, Halogen-C₁-C₆-Alkyl, C₁-C₆-Alkoxy, Halogen-C₁-C₆-Alkoxy, Phenoxy, Hydroxy, Oxo, Halogen, Cyano, C₃-C₈-Zykloalkyl, C₃-C₈-Heterozykloalkyl, Heteroaryl oder Aryl tragen kann, oder für die Gruppe 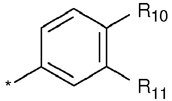 steht, in der
- R¹⁰ und R¹¹: gemeinsam einen 5- bis 8-gliedrigen Zykloalkyl- oder 5- bis-8-gliedrigen Heterozykloalkyl-Ring mit 1-3 Heteroatomen aus der Gruppe N, O und S bilden, der gegebenenfalls mit Halogen, Hydroxy, Cyano, Nitro und/oder mit einem C₁-C₃-Alkyl, Halogen-C₁-C₆-Alkyl, C₁-C₆-Alkoxy, Halogen- C₁-C₆-Alkoxy, C₁-C₆-Alkoxy- C₁-C₆-Alkyl und/oder C₁-C₆-Alkylcarbonylrest ein- oder mehrfach, gleich oder verschieden substituiert sein kann und im Ring eine -C(O)- oder -S(O)₂ Gruppe enthalten sein kann
- R⁸: für Wasserstoff, C₁-C₆-Alkyl, C₃-C₈-Zykloalkyl, C₃-C₈-Heterozykloalkyl, Aryl oder Heteroaryl steht, welches ein- oder mehrfach, gleich oder verschieden mit C₁-C₆-Alkyl, Halogen-C₁-C₆-Alkyl, C₁-C₆-Alkoxy, Halogen-C₁-C₆-Alkoxy, Hydroxy, Oxo, Halogen, Cyano, Nitro, Heteroaryl oder Aryl substituiert sein kann,
- R⁹: für die Gruppe eines mono- oder bizyklischen Heterozyklus mit 3-14 Ringatomen, einen Spiroheterozyklus bestehend aus 5-12 Ringatomen oder einen überbrückten Heterozyklus bestehend aus 7-15 Ringatomen steht, der jeweils 0,1,2,3,4 oder 5 weitere Heteroatome aus der Gruppe N, O und S beinhalten kann und und die gegebenfalls ein- oder mehrfach mit C₁-C₄-Alkyl, C₃-C₈-Zykloalkyl, C₃-C₈-Heterozykloalkyl, Halogen- C₁-C₆-Alkyl-, Aryl, Aryloxy, -C₁-C₂-Alkylaryl, -C(O)-O-C₁-C₆-Alkyl, Heteroaryl, Hydroxy, C₁-C₆-Alkoxy, Halogen-C₁-C₆-Alkoxy, Halogen, Cyano oder Oxo substituiert sein kann und
- R¹² und R¹³: unabhänging voneinander für Wasserstoff, C₁-C₆-Alkyl stehen,
- R¹⁴: für Wasserstoff, C₁-C₆-Alkyl, Amino, C₁-C₆-Alkoxy, C₃-C₈-Zykloalkyl, C₃-C₈-Heterozyklyl, Aryl oder Heteroaryl steht, welches ein- oder mehrfach, gleich oder verschieden mit C₁-C₆-Alkyl, Halogen-C₁-C₆-Alkyl, C₁-C₆-Alkoxy, Halogen-C₁-C₆-Alkoxy, Hydroxy, Oxo, Halogen, Cyano, Nitro, Heteroaryl oder Aryl substituiert sein kann,
sowie deren Diastereomere, Razemate, Metaboliten und physiologisch verträglichen Salze.

Ferner sind solche Verbindungen der allgemeinen Formel I interessant, in der
- R¹: für Wasserstoff, Hydroxy, C₁-C₆-Alkyl, Halogen-C₁-C₆-Alkyl, C₁-C₆-Alkoxy, Halogen-C₁-C₆-Alkoxy, Halogen oder Cyano steht, oder für Aryl-, welches ein- oder mehrfach, gleich oder verschieden mit C₁-C₆-Alkyl, Halogen-C₁-C₆-Alkyl, C₁-C₆-Alkoxy, Halogen-C₁-C₆-Alkoxy, Hydroxy, Oxo, Halogen, Cyano, Heteroaryl oder Aryl substituiert sein kann, oder für C₃-C₈-Zykloalkyl, C₃-C₈-Zykloalkoxy, Heterozykloalkyl, Aryl oder Heteroaryl mit 1-3 Heteroatomen aus der Gruppe N, O und S, welches ein- oder mehrfach, gleich oder verschieden mit C₁-C₆-Alkyl, Halogen-C₁-C₆-Alkyl, C₁-C₆-Alkoxy, Halogen-C₁-C₆-Alkoxy, Hydroxy, Oxo, Halogen oder Cyano substituiert sein kann, steht,
- R²: für Methyl oder für Methylamino steht,
- R³: für eine -C(O)OR⁸, -C(O)R⁹, -C(O)NR⁶R⁷ Gruppe oder für Cyano, oder für ein 5 bis 6 gliedriges Ringsystem, enthaltend 0-3 Heteroatome ausgewählt aus der Gruppe O, N oder S steht, welches wahlweise aromatisch oder auch nichtaromatisch sein kann und weitere Reste R⁵ tragen kann,
- R⁴: für Wasserstoff, Fluor, Chlor, Brom oder Cyano steht,
- R⁵: für Wasserstoff, C₁-C₆-Alkyl, C₃-C₈-Zykloalkyl, C₃-C₈-Heterozykloalkyl, Halogen-C₁-C₆-Alkyl, Aryl, Heteroaryl, Hydroxy, C₁-C₆-Alkoxy, Halogen-C₁-C₆-Alkoxy, Halogen, Cyano oder Oxo steht,
- R⁶ und R⁷: unabhängig voneinander für Wasserstoff, C₁-C₃-Alkyl, C₁-C₃-Alkoxy, C₃-C₈-Heterozykloalkyl, Aryl, Heteroaryl, C₃-C₈-Zykloalkyl, C₆-C₁₂- Bizykloalkyl, C₅-C₁₁-Spirozykloalkyl, C₆-C₁₂-Heterobizykloalkyl oder C₅-C₁₁-Heterospirozykloalkyl steht, welche weitere Susbtituenten aus der Gruppe C₁-C₃-Alkyl, Halogen-C₁-C₃-Alkyl, C₁-C₃-Alkoxy, Halogen-C₁-C₃-Alkoxy, Phenoxy, Hydroxy, Oxo, Halogen, Cyano, C₃-C₈-Zykloalkyl, C₃-C₈-Heterozykloalkyl, Heteroaryl oder Aryl tragen kann, oder für die Gruppe 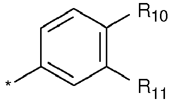 steht, in der
- R¹⁰ und R¹¹: gemeinsam einen 5- bis 8-gliedrigen Zykloalkyl- oder 5- bis-8-gliedrigen Heterozykloalkyl-Ring mit 1-3 Heteroatomen aus der Gruppe N, O und S bilden, der gegebenenfalls mit Halogen, Hydroxy, Cyano, Oxo, Nitro und/oder mit einem C₁-C₃-Alkyl, Halogen- C₁-C₆-Alkyl, C₁-C₆-Alkoxy, Halogen- C₁-C₆-Alkoxy, C₁-C₆-Alkoxy- C₁-C₆-Alkyl, und/oder C₁-C₆-Alkylcarbonylrest ein- oder mehrfach, gleich oder verschieden substituiert sein kann und im Ring eine -C(O) oder-S(O)₂ Gruppe enthalten sein kann,
- R⁸: für Wasserstoff, C₁-C₆-Alkyl, C₃-C₈- Zykloalkyl, Aryl oder Heteroaryl steht, welches ein- oder mehrfach, gleich oder verschieden mit Sustituenten aus der Gruppe C₁-C₆-Alkyl, Halogen-C₁-C₆-Alkyl, C₁-C₆-Alkoxy, Halogen-C₁-C₆-Alkoxy, Hydroxy, Oxo, Halogen, Cyano, Nitro, Heteroaryl oder Aryl substituiert sein kann,
- R⁹: für einen mono- oder bizyklischen Heterozyklus, einen Spiroheterozyklus oder einen überbrückten Heterozyklus der Gruppe 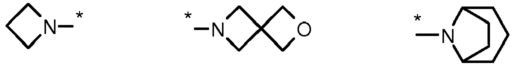 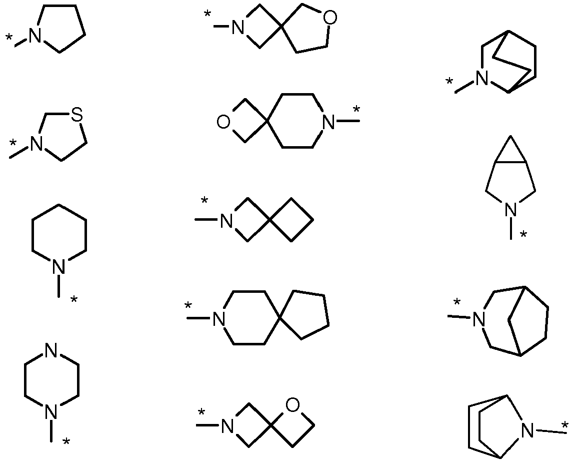 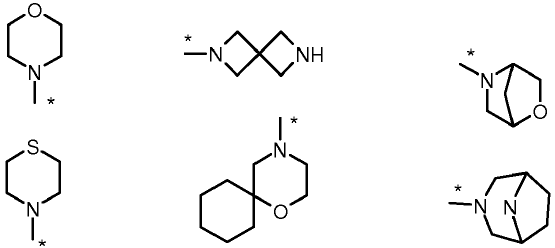 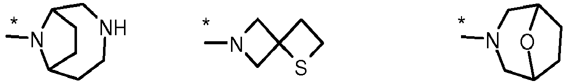
steht, und die gegebenenfalls ein- oder mehrfach mit C₁-C₄-Alkyl, C₃-C₈-Zykloalkyl, C₃-C₈-Heterozykloalkyl, Halogen- C₁-C₆-Alkyl, Aryl, Aryloxy, C₁-C₂-Alkylaryl, -C(O)-O-C₁-C₆-Alkyl, Heteroaryl, Hydroxy, C₁-C₆-Alkoxy, Halogen-C₁-C₆-Alkoxy, Halogen, Cyano oder Oxo substituiert sein kann,
sowie deren Diastereomere, Razemate, Metaboliten und physiologisch verträglichen Salze.

Besonders interessant sind auch solche Verbindungen der allgemeinen Formel I, in der
- R¹: für Wasserstoff,
- R²: für Methyl,
- R³: für eine Gruppe -C(O)OR⁸, -C(O)R⁹ oder -C(O)NR⁶R⁷, oder steht oder für ein 5 gliedriges aromatisches Ringsystem, enthaltend 1-3 Heteroatome ausgewählt aus der Gruppe O, N oder S steht, welches wahlweise weitere Reste R⁵ tragen kann,
- R⁴: für Chlor steht,
- R⁵: für Wasserstoff, C₁-C₆-Alkyl-, C₃-C₈-Zykloalkyl, C₃-C₈-Heterozykloalkyl, Halogen-C₁-C₆-Alkyl, Aryl, Heteroaryl, Hydroxy, C₁-C₆-Alkoxy, Halogen-C₁-C₆-Alkoxy, Halogen, Cyano oder Oxo steht,
- R⁶ und R⁷: unabhängig voneinander für Wasserstoff, C₁-C₃-Alkyl, C₁-C₃-Alkoxy, C₃-C₈-Heterozykloalkyl, Aryl, Heteroaryl, C₃-C₈-Zykloalkyl, C₆-C₁₂-Bizykloalkyl oder C₅-C₁₁- Spirozykloalkyl steht, welche weitere Susbtituenten aus der Gruppe C₁-C₃-Alkyl, Halogen-C₁-C₃-Alkyl, C₁-C₃-Alkoxy, Halogen-C₁-C₃-Alkoxy, Phenoxy, Hydroxy, Oxo, Halogen, Cyano, C₃-C₈-Zykloakyl, C₃-C₈-Heterozykloalkyl, Heteroaryl oder Aryl tragen kann, oder für die Gruppe 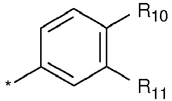 steht, in der
- R¹⁰ und R¹¹: gemeinsam einen 5- bis 6-gliedrigen Zykloalkyl- oder 5- bis-6-gliedrigen Heterozykloalkyl-Ring mit 1-3 Heteroatomen aus der Gruppe N, O und S bilden, der gegebenenfalls mit Halogen, Hydroxy, Cyano, Oxo, Nitro und/oder mit einem C₁-C₃-Alkyl, Halogen- C₁-C₆-Alkyl, C₁-C₆-Alkoxy, Halogen- C₁-C₆-Alkoxy, C₁-C₆-Alkoxy- C₁-C₆-Alkyl, und/oder C₁-C₆-Alkylcarbonylrest ein- oder mehrfach, gleich oder verschieden substituiert sein kann und im Ring eine -C(O) oder-S(O)₂ Gruppe enthalten sein kann,
- R⁸: für Ethyl oder tert.-Butyl steht,
- R⁹: für einen mono- oder bizyklischen Heterozyklus, einen Spiroheterozyklus oder einen überbrückten Heterozyklus der Gruppe 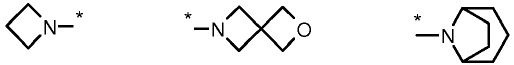 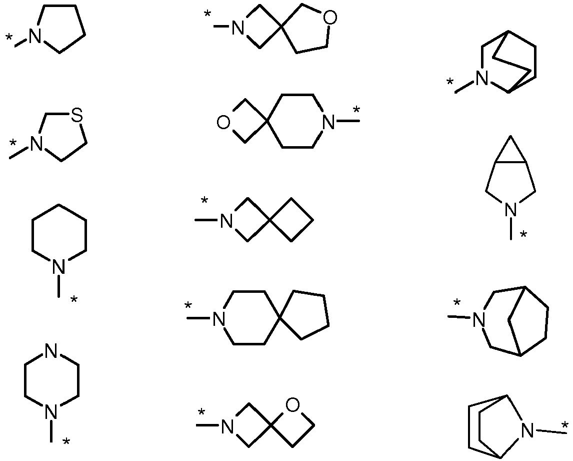 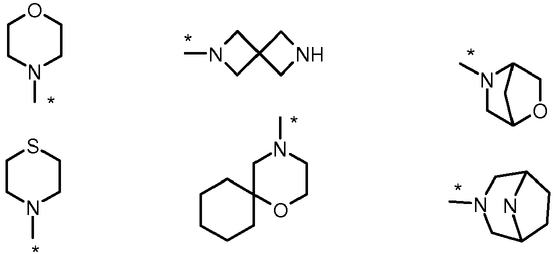 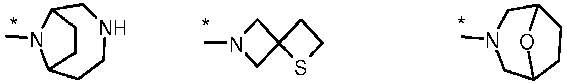
steht,
und die gegebenfalls ein- oder mehrfach mit C₁-C₄-Alkyl, C₃-C₈-Zykloalkyl, C₃-C₈-Heterozykloalkyl, Halogen- C₁-C₆-Alkyl-, Aryl, Aryloxy, -C₁-C₂-Alkylaryl, -C(O)-O-C₁-C₆-Alkyl, Heteroaryl, Hydroxy, C₁-C₆-Alkoxy, Halogen-C₁-C₆-Alkoxy, Halogen, Cyano oder Oxo substituiert sein kann,
sowie deren Diastereomere, Razemate, Metaboliten und physiologisch verträglichen Salze.

Noch interessanter sind solche Verbindungen der allgemeinen Formel I, in der
- R¹: für Wasserstoff,
- R²: für Methyl,
- R³: für eine Gruppe -C(O)OR⁸, -C(O)R⁹ oder -C(O)NR⁶R⁷, oder steht oder für ein 5 gliedriges aromatisches Ringsystem, enthaltend 1-3 Heteroatome ausgewählt aus der Gruppe O, N oder S steht, welches wahlweise weitere Reste R⁵ tragen kann,
- R⁴: für Chlor steht,
- R⁵: für Wasserstoff, C₁-C₆-Alkyl-, C₃-C₈-Zykloalkyl, C₃-C₈-Heterozykloalkyl, Halogen-C₁-C₆-Alkyl, Aryl, Heteroaryl, Hydroxy, C₁-C₆-Alkoxy, Halogen-C₁-C₆-Alkoxy, Halogen, Cyano oder Oxo steht,
- R⁶ und R⁷: unabhängig voneinander für Wasserstoff, Ethyl oder die Gruppe 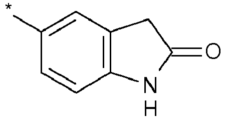 stehen,
- R⁸: für Ethyl oder tert.-Butyl steht,
- R⁹: für einen mono- oder bizyklischen Heterozyklus, einen Spiroheterozyklus oder einen überbrückten Heterozyklus der Gruppe 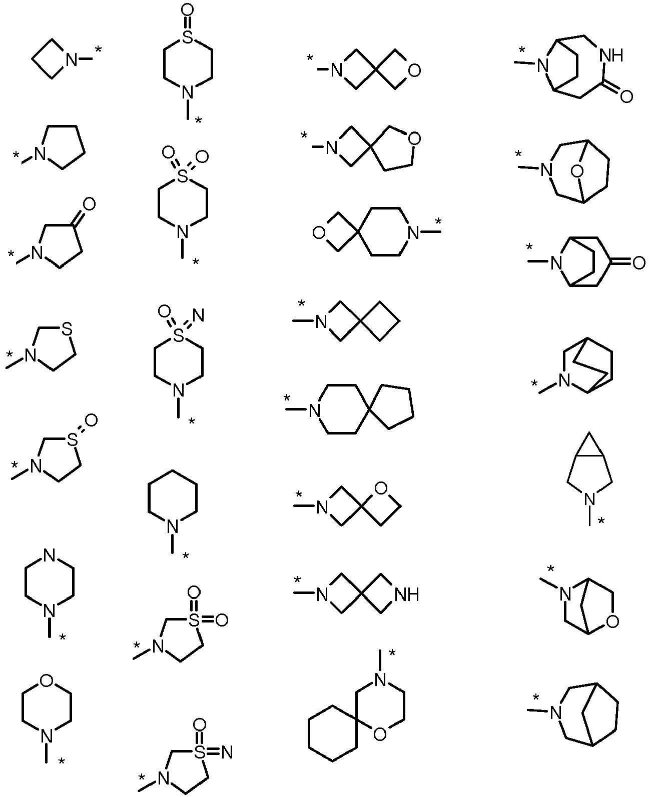 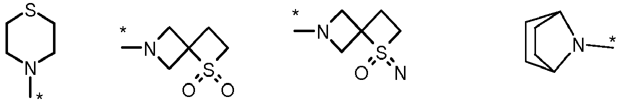
steht, und die gegebenfalls ein- oder mehrfach mit C₁-C₄-Alkyl, Cs-Cs-Zykloalkyl, C₃-C₈-Heterozykloalkyl, Halogen- C₁-C₆-Alkyl-, Aryl, Phenoxy, -C₁-C₂-Alkylaryl, -C(O)-O-C₁-C₆-Alkyl, Heteroaryl, Hydroxy, C₁-C₆-Alkoxy, Halogen-C₁-C₆-Alkoxy, Halogen, Cyano oder Oxo substituiert sein kann,
sowie deren Diastereomere, Razemate, Metaboliten und physiologisch verträglichen Salze.

Auch sind solche Verbindungen der allgemeinen Formel I besonders interessant, in der
- R¹: für Wasserstoff,
- R²: für Methyl,
- R³: für eine Gruppe -C(O)OR⁸, -C(O)R⁹ oder -C(O)NR⁶R⁷ steht oder für ein 5 gliedriges Ringsystem, enthaltend 2- 3 Heteroatome und einem weiteren Rest R⁵ der Struktur 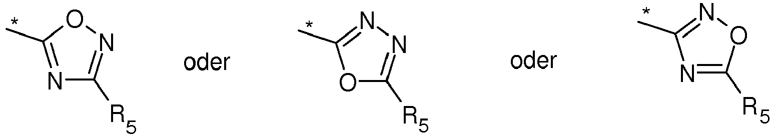
steht,
- R⁴: für Chlor steht,
- R⁵: für Wasserstoff, Methyl, Ethyl, Propyl, iso-Propyl, Zyklopropyl oder tert-Butyl steht,
- R⁶ und R⁷: unabhängig voneinander für Wasserstoff, Ethyl oder die Gruppe 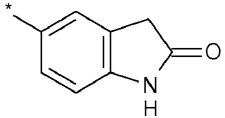
stehen,
- R⁸: für Ethyl oder tert.-Butyl steht,
- R⁹: für die Gruppe 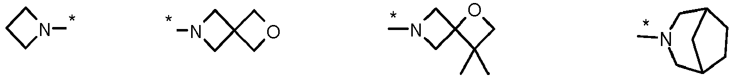 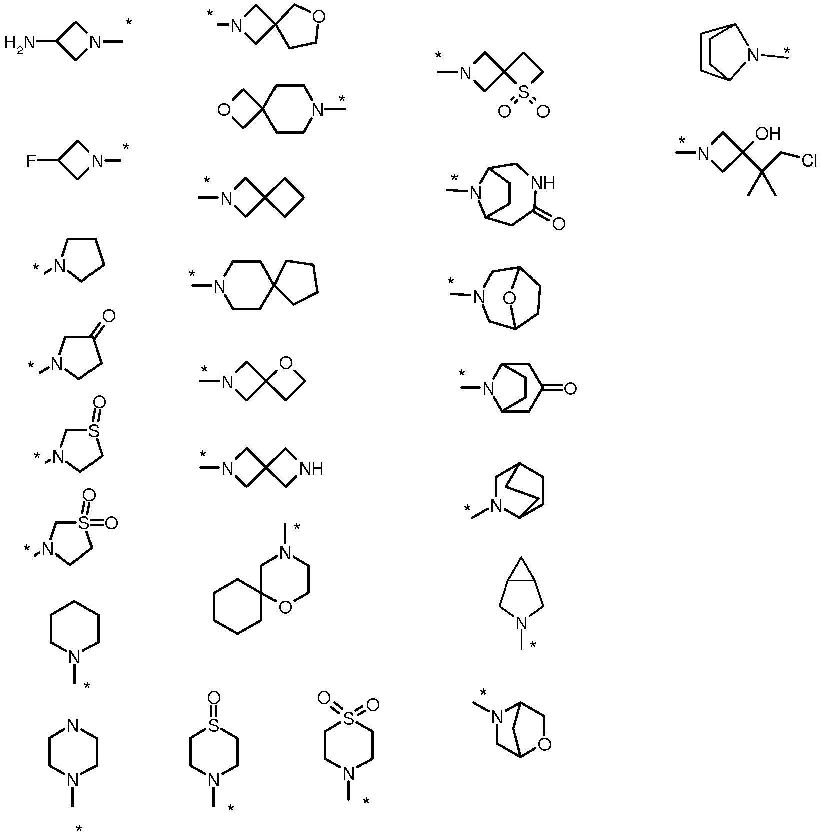 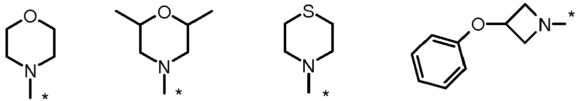
steht,
sowie deren Diastereomere, Razemate, Metaboliten und physiologisch verträglichen Salze.

Besonders interessant sind solche Verbindungen der allgemeinen Formel I, in der
- R¹: für Wasserstoff,
- R²: für Methyl,
- R³: für eine Gruppe -C(O)OR⁸, -C(O)R⁹ oder -C(O)NR⁶R⁷ steht oder für ein 5 gliedriges Ringsystem mit 3 Heteroatomen und einem weiteren Rest R⁵ der Struktur 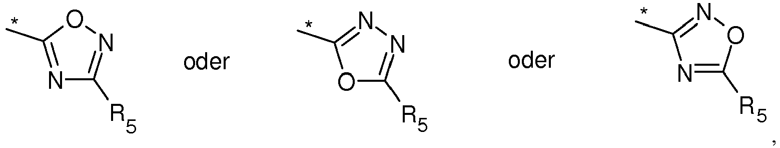 steht
- R⁴: für Chlor steht,
- R⁵: für Methyl, iso-Propyl oder Cylopropyl steht,
- R⁶ und R⁷: unabhängig voneinander für Wasserstoff, Ethyl oder die Gruppe 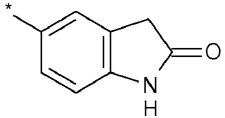 stehen,
- R⁸: für Ethyl oder tert.-Butyl steht,
- R⁹: für die Gruppe 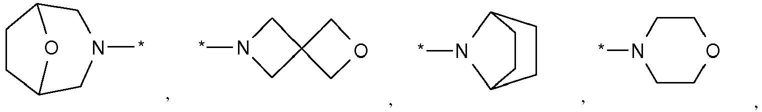 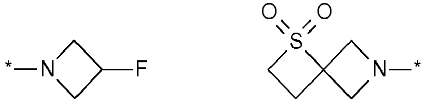 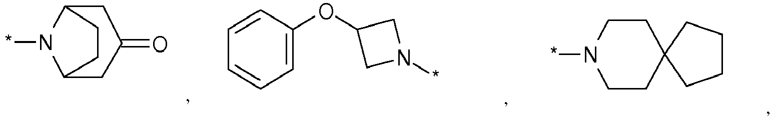 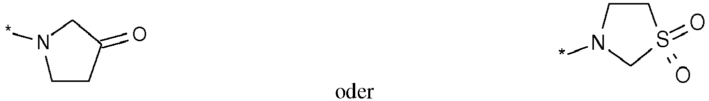
steht sowie deren Diastereomere, Razemate, Metaboliten und physiologisch verträglichen Salze.

In der allgemeinen Formel (I) kann R¹ für Wasserstoff, Hydroxy, C₁-C₆-Alkyl, Halogen-C₁-C₆-Alkyl, C₁-C₆-Alkoxy, Halogen-C₁-C₆-Alkoxy, Halogen oder Cyano stehen,
oder für C₃-C₈-Zykloalkyl, C₃-C₈-Zykloalkoxy, C₃-C₈-Heterozykloalkyl, Aryl oder Heteroaryl mit 5 oder 6 Ringatomen, die gegebenenfalls ein- oder mehrfach, gleich oder verschieden substituiert sein können mit C₁-C₆-Alkyl, Halogen-C₁-C₆-Alkyl, C₁-C₆-Alkoxy, Halogen-C₁-C₆-Alkoxy, Hydroxy, Oxo, Halogen, Cyano, Heteroaryl oder Aryl, oder für -C(=O)-OR⁸, -C(=O)-NR¹²R¹³,-C(=O)-R¹⁴, -S(=O)₂-C₁-C₆-Alkyl, -S(=O)₂-OR⁸ oder -S(=O)₂-NR¹²R¹³.

In der allgemeinen Formel (I) steht R¹ bevorzugt für Wasserstoff, Hydroxy, C₁-C₆-Alkyl, Fluor-C₁-C₆-Alkyl, C₁-C₆-Alkoxy, Fluor-C₁-C₆-Alkoxy, Halogen oder Cyano, oder
für C₃-C₈-Zykloalkyl, C₃-C₈-Zykloalkoxy, C₃-C₈-Heterozykloalkyl, Aryl oder Heteroaryl mit 5 oder 6 Ringatomen, die gegebenenfalls ein- oder mehrfach, gleich oder verschieden substituiert sein können mit C₁-C₆-Alkyl, Fluor-C₁-C₆-Alkyl, C₁-C₆-Alkoxy, Fluor-C₁-C₆-Alkoxy, Hydroxy, Oxo, Halogen, Cyano, Heteroaryl oder Aryl.

In der allgemeinen Formel (I) steht R¹ besonders bevorzugt für Wasserstoff, Hydroxy, C₁-C₃-Alkyl, Fluor-C₁-C₃-Alkyl, C₁-C₃-Alkoxy oder Fluor-C₁-C₃-Alkoxy, oder
für Heteroaryl mit 5 oder 6 Ringatomen, das gegebenenfalls ein- oder mehrfach, gleich oder verschieden substituiert sein kann mit C₁-C₃-Alkyl, Fluor-C₁-C₃-Alkyl, C₁-C₃-Alkoxy, Fluor-C₁-C₃-Alkoxy, Halogen oder Cyano.

In der allgemeinen Formel (I) steht R¹ ganz besonders bevorzugt für Wasserstoff, Hydroxy, C₁-C₃-Alkyl, Fluor-C₁-C₃-Alkyl, C₁-C₃-Alkoxy oder Fluor-C₁-C₃-Alkoxy, oder
für Heteroaryl mit 5 Ringatomen, das gegebenenfalls ein- oder mehrfach, gleich oder verschieden substituiert sein kann mit C₁-C₃-Alkyl oder C₁-C₃-Alkoxy.

In der allgemeinen Formel (I) steht R¹ noch mehr bevorzugt für Wasserstoff, C₁-C₃-Alkoxy oder Fluor-C₁-C₃-Alkoxy, oder für Oxazolyl oder Isoxazolyl, die gegebenenfalls ein- oder mehrfach, gleich oder verschieden substituiert sein können mit C₁-C₃-Alkyl oder C₁-C₃-Alkoxy.

In der allgemeinen Formel (I) steht R¹ überaus bevorzugt für Wasserstoff, Methoxy, Trifluormethoxy oder für 3,5-Dimethylisoxazol-4-yl.

In der allgemeinen Formel (I) steht R¹ ferner überaus bevorzugt für Wasserstoff.

In der allgemeinen Formel (I) steht R¹ ferner überaus bevorzugt für Methoxy.

In der allgemeinen Formel (I) steht R¹ ferner überaus bevorzugt für Trifluormethoxy.

In der allgemeinen Formel (I) steht R¹ ferner überaus bevorzugt für 3,5-Dimethylisoxazol-4-yl.

In der allgemeinen Formel (I) kann R² für Wasserstoff, C₁-C₆-Alkyl oder für -NR⁶R⁷ stehen.

In der allgemeinen Formel (I) steht R² auch für C₁-C₃-Alkyl oder C₁-C₃-Alkyl-Amino-.

In der allgemeinen Formel (I) steht R² auch für C₁-C₃-Alkyl.

In der allgemeinen Formel (I) steht R² bevorzugt für Methyl oder Methylamino-.

In der allgemeinen Formel (I) steht R² besonders bevorzugt für Methyl.

In der allgemeinen Formel (I) kann R³ für Cyano, -C(=O)-OR⁸, -C(=O)-R⁹ oder -C(=O)-NR⁶R⁷ stehen, oder für ein 5- oder 6- gliedriges Ringsystem, enthaltend 0, 1, 2, 3 oder 4 Heteroatome unabhängig voneinander ausgewählt aus der Gruppe O, N oder S, welches wahlweise aromatisch oder auch nicht-aromatisch sein kann und gegebenenfalls ein- oder mehrfach, gleich oder verschieden mit R⁵ substituiert sein kann.

In der allgemeinen Formel (I) steht R³ bevorzugt für Cyano, -C(=O)-OR⁸, -C(=O)-R⁹ oder -C(=O)-NR⁶R⁷, oder für ein 5- oder 6- gliedriges Ringsystem, enthaltend 0, 1, 2 oder 3 Heteroatome unabhängig voneinander ausgewählt aus der Gruppe O, N oder S, welches wahlweise aromatisch oder auch nicht-aromatisch sein kann und gegebenenfalls ein- oder mehrfach, gleich oder verschieden mit R⁵ substituiert sein kann.

In der allgemeinen Formel (I) steht R³ besonders bevorzugt für -C(=O)-OR⁸, -C(=O)-R⁹ oder -C(=O)-NR⁶R⁷ steht, oder für ein 5- gliedriges aromatisches Ringsystem, enthaltend 1, 2 oder 3 Heteroatome unabhängig voneinander ausgewählt aus der Gruppe O, N oder S, welches gegebenenfalls ein- oder mehrfach, gleich oder verschieden mit R⁵ substituiert sein kann.

In der allgemeinen Formel (I) steht R³ ganz besonders bevorzugt für -C(=O)-OR⁸, -C(=O)-R⁹ oder - C(=O)-NR⁶R⁷, oder für eines der folgenden Ringsysteme 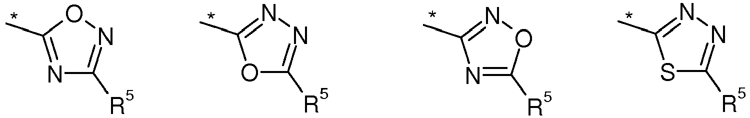 worin "*" den Anknüpfungspunkt an den Rest des Moleküls kennzeichnet.

In der allgemeinen Formel (I) steht R³ ganz besonders bevorzugt für -C(=O)-OR⁸, -C(=O)-R⁹ oder - C(=O)-NR⁶R⁷.

In der allgemeinen Formel (I) steht R³ ganz besonders bevorzugt für -C(=O)-OR⁸.

In der allgemeinen Formel (I) steht R³ ganz besonders bevorzugt für -C(=O)-R⁹.

In der allgemeinen Formel (I) steht R³ ganz besonders bevorzugt für -C(=O)-NR⁶R⁷.

In der allgemeinen Formel (I) steht R³ ganz besonders bevorzugt für eines der folgenden Ringsysteme 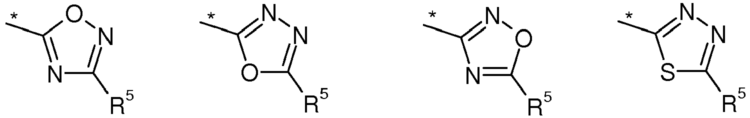 worin "*" den Anknüpfungspunkt an den Rest des Moleküls kennzeichnet.

In der allgemeinen Formel (I) steht R³ ganz besonders bevorzugt für eines der folgenden Ringsysteme 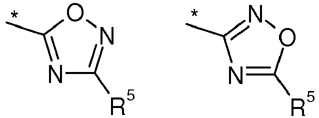 worin "*" den Anknüpfungspunkt an den Rest des Moleküls kennzeichnet.

In der allgemeinen Formel (I) steht R³ ganz besonders bevorzugt für eines der folgenden Ringsysteme 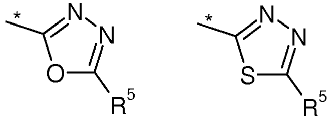 worin "*" den Anknüpfungspunkt an den Rest des Moleküls kennzeichnet.

In der allgemeinen Formel (I) kann R⁴ für Wasserstoff, Fluor, Chlor, Brom oder Cyano stehen.

In der allgemeinen Formel (I) kann R⁴ auch für Wasserstoff, Fluor oder Chlor stehen.

In der allgemeinen Formel (I) steht R⁴ besonders bevorzugt für Chlor.

In der allgemeinen Formel (I) kann R⁵ für Wasserstoff, C₁-C₆-Alkyl-, C₃-C₈-Zykloalkyl, C₃-C₈-Heterozykloalkyl, Halogen-C₁-C₆-Alkyl, Aryl, Heteroaryl, Hydroxy, C₁-C₆-Alkoxy, C₁-C₃-Alkoxy-C₁-C₃-Alkyl-, Aryloxy-C₁-C₃-Alkyl, Aryl-C₁-C₃-Alkoxy-C₁-C₃-Alkyl-, Halogen-C₁-C₆-Alkoxy, Halogen, Cyano oder Oxo stehen.

In der allgemeinen Formel (I) steht R⁵ bevorzugt für Wasserstoff, C₁-C₆-Alkyl-, C₃-C₈-Zykloalkyl, C₃-C₈-Heterozykloalkyl, Fluor-C₁-C₆-Alkyl, Aryl, Heteroaryl, Hydroxy, C₁-C₆-Alkoxy, C₁-C₃-Alkoxy-C₁-C₃-Alkyl-, Aryloxy-C₁-C₃-Alkyl, Aryl-C₁-C₃-Alkoxy-C₁-C₃-Alkyl-, Fluor-C₁-C₆-Alkoxy, Halogen, Cyano oder Oxo.

In der allgemeinen Formel (I) steht R⁵ besonders bevorzugt für Wasserstoff, C₁-C₃-Alkyl-, C₃-C₆-Zykloalkyl, C₃-C₆-Heterozykloalkyl, Fluor-C₁-C₃-Alkyl, Aryl, Heteroaryl, Hydroxy, C₁-C₃-Alkoxy, C₁-C₃-Alkoxy-C₁-C₃-Alkyl-, Aryloxy-C₁-C₃-Alkyl, Aryl-C₁-C₃-Alkoxy-C₁-C₃-Alkyl-, Fluor-C₁-C₃-Alkoxy, Halogen, oder Cyano.

In der allgemeinen Formel (I) steht R⁵ ganz besonders bevorzugt für Wasserstoff, C₁-C₃-Alkyl-, C₃-C₆-Zykloalkyl, C₃-C₆-Heterozykloalkyl, Phenyl, 5- oder 6-gliedriges Heteroaryl, C₁-C₃-Alkoxy-C₁-C₃-Alkyl-, Phenoxy-C₁-C₃-Alkyl- oder Benzyloxy-C₁-C₃-Alkyl-.

In der allgemeinen Formel (I) steht R⁵ noch mehr bevorzugt für Wasserstoff, C₁-C₃-Alkyl-, C₃-C₆-Zykloalkyl, Pyridinyl oder Benzyloxy-C₁-C₃-Alkyl-.

In der allgemeinen Formel (I) steht R⁵ noch mehr bevorzugt für C₁-C₃-Alkyl-.

In der allgemeinen Formel (I) steht R⁵ überaus bevorzugt für Methyl, *iso*-Propyl, Zyklopropyl, Pyridin-3-yl oder Benzyloxymethyl.

In der allgemeinen Formel (I) können R⁶ und R⁷ unabhängig voneinander für Wasserstoff oder für - NH-C(=O)-R¹⁵ stehen, oder für C₁-C₆-Alkyl, C₁-C₆-Alkoxy, C₃-C₈-Heterozykloalkyl, Aryl, Heteroaryl, C₃-C₈-Zykloalkyl, C₆-C₁₂-Bizykloalkyl, C₅-C₁₁-Spirozykloalkyl, C₆-C₁₂-Heterobizycloalkyl oder C₅-C₁₁-Heterospirozykloalkyl, die gegebenenfalls ein- oder mehrfach, gleich oder verschieden substituiert sein können mit C₁-C₆-Alkyl, Halogen-C₁-C₆-Alkyl, C₁-C₆-Alkoxy, Halogen-C₁-C₆-Alkoxy, Phenoxy, Hydroxy, Oxo, Halogen, Cyano, C₃-C₈-Zykloalkyl, C₃-C₈-Heterozykloalkyl, Heteroaryl oder Aryl,
oder für die Gruppe 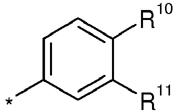 in welcher R¹⁰ und R¹¹ gemeinsam für C₃-C₆-Alkylen oder C₃-C₆-Heteroalkylen stehen, die gegebenenfalls ein- oder mehrfach, gleich oder verschieden substituiert sein können mit Halogen, Oxo, Hydroxy, Cyano, Nitro, C₁-C₃-Alkyl, Halogen-C₁-C₆-Alkyl, C₁-C₆-Alkoxy, Halogen- C₁-C₆-Alkoxy, C₁-C₆-Alkoxy- C₁-C₆-Alkyl, C₁-C₆-Alkylcarbonyl,
und worin "*" den Anknüpfungspunkt an den Rest des Moleküls kennzeichnet.

In der allgemeinen Formel (I) stehen R⁶ und R⁷ bevorzugt und unabhängig voneinander für Wasserstoff oder für -NH-C(=O)-R¹⁵, oder für C₁-C₆-Alkyl, C₁-C₆-Alkoxy, C₃-C₈-Heterozykloalkyl, Aryl, Heteroaryl, C₃-C₈-Zykloalkyl, C₆-C₁₂-Bizykloalkyl, C₅-C₁₁-Spirozykloalkyl, C₆-C₁₂-Heterobizycloalkyl oder C₅-C₁₁-Heterospirozykloalkyl, die gegebenenfalls ein- oder mehrfach, gleich oder verschieden substituiert sein können mit C₁-C₃-Alkyl, Fluor-C₁-C₃-Alkyl, C₁-C₃-Alkoxy, Fluor-C₁-C₃-Alkoxy, Phenoxy, Hydroxy, Oxo, Halogen, Cyano, C₃-C₈-Zykloalkyl, C₃-C₈-Heterozykloalkyl, Heteroaryl oder Aryl,
oder für die Gruppe 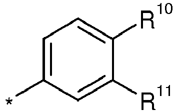 in welcher R¹⁰ und R¹¹ gemeinsam für C₃-C₆-Alkylen oder C₃-C₆-Heteroalkylen stehen, die gegebenenfalls ein- oder mehrfach, gleich oder verschieden substituiert sein können mit Halogen, Oxo, Hydroxy, Cyano, Nitro, C₁-C₃-Alkyl, Fluor-C₁-C₃-Alkyl, C₁-C₃-Alkoxy, Fluor-C₁-C₃-Alkoxy, C₁-C₃-Alkoxy-C₁-C₃-Alkyl, C₁-C₃-Alkylcarbonyl,
und worin "*" den Anknüpfungspunkt an den Rest des Moleküls kennzeichnet.

In der allgemeinen Formel (I) stehen R⁶ und R⁷ ganz besonders bevorzugt und unabhängig voneinander für Wasserstoff oder für -NH-C(=O)-R¹⁵, oder für C₁-C₆-Alkyl, C₃-C₈-Heterozykloalkyl, Phenyl, Heteroaryl mit 5 oder 6 Ringatomen, oder C₃-C₈-Zykloalkyl, die gegebenenfalls ein- oder mehrfach, gleich oder verschieden substituiert sein können mit C₁-C₃-Alkyl, Fluor-C₁-C₃-Alkyl, C₁-C₃-Alkoxy, Phenoxy, Hydroxy, Oxo, Halogen, Cyano, C₃-C₈-Zykloalkyl, C₃-C₈-Heterozykloalkyl, 5- oder 6-gliedriges Heteroaryl oder Phenyl,
oder für die Gruppe 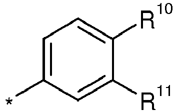 in welcher R¹⁰ und R¹¹ gemeinsam für C₃-C₄-Alkylen oder C₃-C₄-Heteroalkylen stehen, die gegebenenfalls ein- oder mehrfach, gleich oder verschieden substituiert sein können mit Halogen, Oxo, Hydroxy, C₁-C₃-Alkyl, C₁-C₃-Alkoxy, C₁-C₃-Alkylcarbonyl,
und worin "*" den Anknüpfungspunkt an den Rest des Moleküls kennzeichnet.

In der allgemeinen Formel (I) steht ferner R⁷ ganz besonders bevorzugt für Wasserstoff, und R⁶ steht ganz besondes bevorzugt für C₁-C₆-Alkyl, C₃-C₈-Heterozykloalkyl, Phenyl, Heteroaryl mit 5 oder 6 Ringatomen, oder für C₃-C₈-Zykloalkyl, die gegebenenfalls ein- oder mehrfach, gleich oder verschieden substituiert sein können mit C₁-C₃-Alkyl, Fluor-C₁-C₃-Alkyl, C₁-C₃-Alkoxy, Phenoxy, Hydroxy, Oxo, Halogen, Cyano, C₃-C₈-Zykloalkyl, C₃-C₈-Heterozykloalkyl, 5- oder 6-gliedriges Heteroaryl oder Phenyl,
oder für die Gruppe 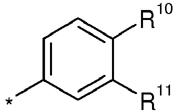 in welcher R¹⁰ und R¹¹ gemeinsam für C₃-C₄-Alkylen oder C₃-C₄-Heteroalkylen stehen, die gegebenenfalls ein- oder mehrfach, gleich oder verschieden substituiert sein können mit Halogen, Oxo, Hydroxy, C₁-C₃-Alkyl, C₁-C₃-Alkoxy, C₁-C₃-Alkylcarbonyl,
und worin "*" den Anknüpfungspunkt an den Rest des Moleküls kennzeichnet.

In der allgemeinen Formel (I) steht ferner R⁷ ganz besonders bevorzugt für Wasserstoff, und R⁶ steht ganz besondes bevorzugt für C₁-C₆-Alkyl, C₃-C₈-Heterozykloalkyl, Phenyl, Heteroaryl mit 5 oder 6 Ringatomen, oder für C₃-C₈-Zykloalkyl, die gegebenenfalls ein- oder mehrfach, gleich oder verschieden substituiert sein können mit C₁-C₃-Alkyl, Fluor-C₁-C₃-Alkyl, C₁-C₃-Alkoxy, Phenoxy, Hydroxy, Oxo, Halogen, Cyano, C₃-C₈-Zykloalkyl, C₃-C₈-Heterozykloalkyl, 5- oder 6-gliedriges Heteroaryl oder Phenyl.

In der allgemeinen Formel (I) steht ferner R⁷ ganz besonders bevorzugt für Wasserstoff, und R⁶ steht ganz besondes bevorzugt für die Gruppe 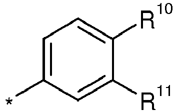 in welcher R¹⁰ und R¹¹ gemeinsam für C₃-C₄-Alkylen oder C₃-C₄-Heteroalkylen stehen, die gegebenenfalls ein- oder mehrfach, gleich oder verschieden substituiert sein können mit Halogen, Oxo, Hydroxy, C₁-C₃-Alkyl, C₁-C₃-Alkoxy, C₁-C₃-Alkylcarbonyl,
und worin "*" den Anknüpfungspunkt an den Rest des Moleküls kennzeichnet.

In der allgemeinen Formel (I) stehen R⁶ und R⁷ noch mehr bevorzugt und unabhängig voneinander für Wasserstoff oder für -NH-C(=O)-R¹⁵, oder für C₁-C₆-Alkyl, das gegebenenfalls ein- oder mehrfach, gleich oder verschieden substituiert sein kann mit C₃-C₈-Heterozykloalkyl,
oder für die Gruppe 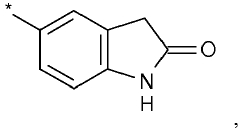 worin "*" den Anknüpfungspunkt an den Rest des Moleküls kennzeichnet.

In der allgemeinen Formel (I) steht R⁷ noch mehr bevorzugt für Wasserstoff und R⁶ steht noch mehr bevorzugt für C₁-C₆-Alkyl, das gegebenenfalls ein- oder mehrfach, gleich oder verschieden substituiert sein kann mit C₃-C₈-Heterozykloalkyl,
oder für die Gruppe 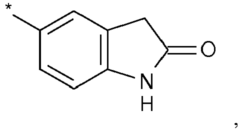 worin "*" den Anknüpfungspunkt an den Rest des Moleküls kennzeichnet.

In der allgemeinen Formel (I) steht R⁷ noch mehr bevorzugt für Wasserstoff und R⁶ steht noch mehr bevorzugt für C₁-C₆-Alkyl, das gegebenenfalls ein- oder mehrfach, gleich oder verschieden substituiert sein kann mit C₃-C₈-Heterozykloalkyl.

In der allgemeinen Formel (I) steht R⁷ noch mehr bevorzugt für Wasserstoff und R⁶ steht noch mehr bevorzugt für die Gruppe 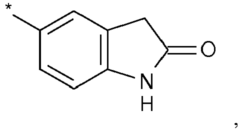 worin "*" den Anknüpfungspunkt an den Rest des Moleküls kennzeichnet.

In der allgemeinen Formel (I) stehen R⁶ und R⁷ überaus bevorzugt und unabhängig voneinander für Wasserstoff oder für -NH-C(=O)-R¹⁵ stehen, oder für Ethyl, das gegebenenfalls einfach substituiert sein kann mit Morpholinyl,
oder für die Gruppe 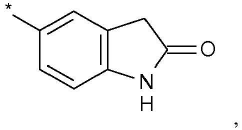 worin "*" den Anknüpfungspunkt an den Rest des Moleküls kennzeichnet.

In der allgemeinen Formel (I) kann R⁸ für Wasserstoff, C₁-C₆-Alkyl, C₃-C₈-Zykloalkyl, C₃-C₈-Heterozykloalkyl, Aryl oder Heteroaryl stehen, die gegebenenfalls ein- oder mehrfach, gleich oder verschieden substituiert sein können mit C₁-C₆-Alkyl, Halogen-C₁-C₆-Alkyl, C₁-C₆-Alkoxy, Halogen-C₁-C₆-Alkoxy, Hydroxy, Oxo, Halogen, Cyano, Nitro, Heteroaryl oder Aryl.

In der allgemeinen Formel (I) steht R⁸ bevorzugt für Wasserstoff, C₁-C₆-Alkyl, C₃-C₈-Zykloalkyl, Aryl oder Heteroaryl, die gegebenenfalls ein- oder mehrfach, gleich oder verschieden substituiert sein können mit C₁-C₆-Alkyl, Fluor-C₁-C₆-Alkyl, C₁-C₆-Alkoxy, Fluor-C₁-C₆-Alkoxy, Hydroxy, Oxo, Halogen, Cyano, Nitro, Heteroaryl oder Aryl.

In der allgemeinen Formel (I) steht R⁸ auch bevorzugt für Wasserstoff oder für C₁-C₆-Alkyl, das gegebenenfalls ein- oder mehrfach, gleich oder verschieden substituiert sein kann C₁-C₆-Alkoxy, Hydroxy, Oxo, Fluor, Cyano, Nitro, Heteroaryl oder Aryl.

In der allgemeinen Formel (I) steht R⁸ besonders bevorzugt für C₁-C₆-Alkyl.

In der allgemeinen Formel (I) steht R⁸ ganz besonders bevorzugt für C₁-C₄-Alkyl.

In der allgemeinen Formel (I) steht R⁸ noch mehr bevorzugt für Ethyl oder *tert*-Butyl.

In der allgemeinen Formel (I) kann R⁹ für C₃-C₈-Heterozykloalkyl, C₅-C₁₁-Spiroheterozykloalkyl, C₆-C₁₂-Heterobizykloalkyl oder für einen überbrückten Heterozyklus, bestehend aus 7 bis 15 Ringatomen, stehen, die gegebenfalls ein- oder mehrfach, gleich oder verschieden substituiert sein können mit C₁-C₄-Alkyl, C₃-C₈-Zykloalkyl, C₃-C₈-Heterozykloalkyl, Halogen-C₁-C₆-Alkyl-, Aryl, Aryloxy, Aryl-C₁-C₂-Alkyl, -C(=O)-O-C₁-C₆-Alkyl, Heteroaryl, Hydroxy, C₁-C₆-Alkoxy, Halogen-C₁-C₆-Alkoxy, Halogen, Cyano oder Oxo.

In der allgemeinen Formel (I) steht R⁹ bevorzugt für C₃-C₈-Heterozykloalkyl, C₅-C₁₁-Spiroheterozykloalkyl, C₆-C₁₂-Heterobizykloalkyl oder für einen überbrückten Heterozyklus, bestehend aus 7 bis 15 Ringatomen, die gegebenfalls ein- oder mehrfach, gleich oder verschieden substituiert sein können mit C₁-C₄-Alkyl, C₃-C₈-Zykloalkyl, C₃-C₈-Heterozykloalkyl, Fluor-C₁-C₆-Alkyl-, Aryl, Aryloxy, Aryl-C₁-C₂-Alkyl, -C(=O)-O-C₁-C₆-Alkyl, Heteroaryl, Hydroxy, C₁-C₆-Alkoxy, Fluor-C₁-C₆-Alkoxy, Halogen, Cyano oder Oxo.

In der allgemeinen Formel (I) steht R⁹ besonders bevorzugt für eine der folgenden Gruppen 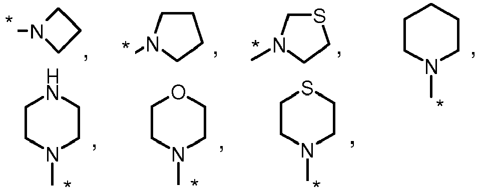 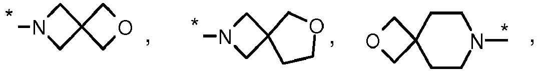 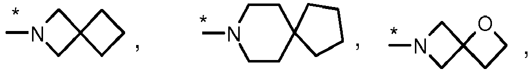 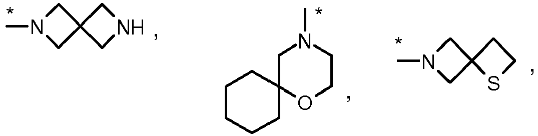 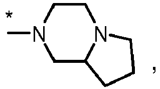 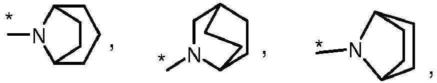 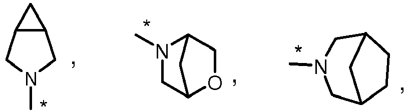 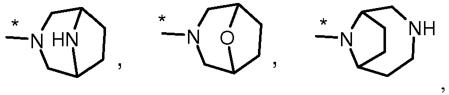 die gegebenfalls ein- oder mehrfach, gleich oder verschieden substituiert sein können mit C₁-C₄-Alkyl, C₃-C₈-Zykloalkyl, C₃-C₈-Heterozykloalkyl, Fluor-C₁-C₆-Alkyl-, Aryl, Aryloxy, Aryl-C₁-C₂-Alkyl, -C(=O)-O-C₁-C₆-Alkyl, Heteroaryl, Hydroxy, C₁-C₆-Alkoxy, Fluor-C₁-C₆-Alkoxy, Halogen, Cyano oder Oxo,
und worin "*" den Anknüpfungspunkt an den Rest des Moleküls kennzeichnet.

In der allgemeinen Formel (I) steht R⁹ ganz besonders bevorzugt für eine der folgenden Gruppen 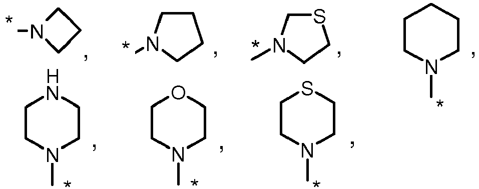 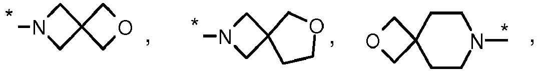 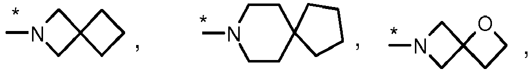 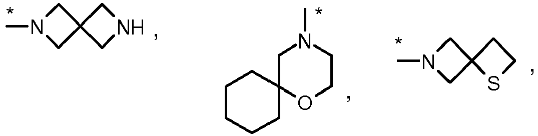 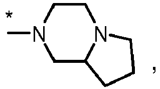 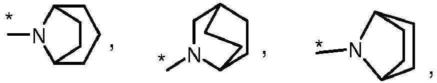 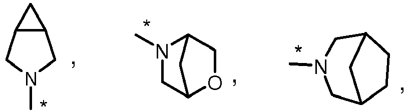 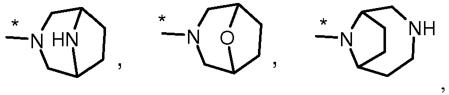 die gegebenfalls ein- oder mehrfach, gleich oder verschieden substituiert sein können mit C₁-C₄-Alkyl, Phenyl, Phenoxy, Benzyl, -C(=O)-O-C₁-C₄-Alkyl, 5- oder 6-gliedriges Heteroaryl, Hydroxy, C₁-C₄-Alkoxy, Fluor, Cyano oder Oxo,
und worin "*" den Anknüpfungspunkt an den Rest des Moleküls kennzeichnet.

In der allgemeinen Formel (I) steht R⁹ noch mehr bevorzugt für eine der folgenden Gruppen 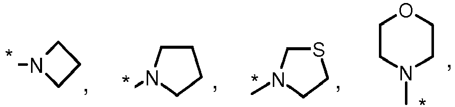 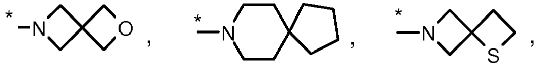 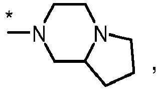 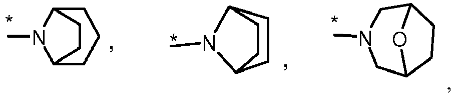 die gegebenfalls ein- oder mehrfach, gleich oder verschieden substituiert sein können mit C₁-C₄-Alkyl, Phenoxy, Benzyl, -C(=O)-O-C₁-C₄-Alkyl, Fluor oder Oxo,
und worin "*" den Anknüpfungspunkt an den Rest des Moleküls kennzeichnet.

In der allgemeinen Formel (I) steht R⁹ überaus bevorzugt für eine der folgenden Gruppen 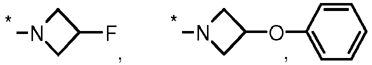 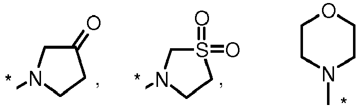 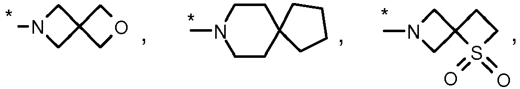 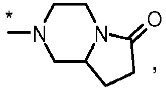 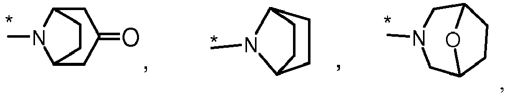 worin "*" den Anknüpfungspunkt an den Rest des Moleküls kennzeichnet.

In der allgemeinen Formel (I) können R¹² und R¹³ unabhängig voneinander stehen für Wasserstoff oder C₁-C₆-Alkyl.

In der allgemeinen Formel (I) können R¹² und R¹³ auch unabhängig voneinander stehen für Wasserstoff oder Methyl.

In der allgemeinen Formel (I) kann R¹⁴ für Wasserstoff, C₁-C₆-Alkyl, Amino, C₁-C₆-Alkoxy, C₃-C₈-Zykloalkyl, C₃-C₈-Heterozykloalkyl, Aryl oder Heteroaryl stehen, die gegebenenfalls ein- oder mehrfach, gleich oder verschieden substituiert sein können mit C₁-C₆-Alkyl, Halogen-C₁-C₆-Alkyl, C₁-C₆-Alkoxy, Halogen-C₁-C₆-Alkoxy, Hydroxy, Oxo, Halogen, Cyano, Nitro, Heteroaryl oder Aryl.

In der allgemeinen Formel (I) kann R¹⁴ auch für C₁-C₆-Alkyl, C₃-C₈-Zykloalkyl, Aryl oder Heteroaryl stehen, die ein- oder mehrfach, gleich oder verschieden substituiert sein können mit C₁-C₃-Alkyl, Fluor-C₁-C₃-Alkyl, C₁-C₃-Alkoxy, Fluor-C₁-C₃-Alkoxy, Hydroxy, Oxo, Halogen, Cyano oder Nitro.

In der allgemeinen Formel (I) kann R¹⁵ für Wasserstoff, C₁-C₆-Alkyl, Halogen-C₁-C₆-Alkyl, Aryl oder Aryl-C₁-C₂-Alkyl stehen, worin das Aryl und das in Aryl-C₁-C₂-Alkyl enthaltene Aryl ihrerseits gegebenenfalls ein- oder mehrfach, gleich oder verschieden substituiert sein können mit Halogen, Cyano, C₁-C₃-Alkyl, C₁-C₃-Alkoxy oder Trifluormethyl.

In der allgemeinen Formel (I) steht R¹⁵ bevorzugt für C₁-C₆-Alkyl, Fluor-C₁-C₃-Alkyl, Phenyl oder Phenyl-C₁-C₂-Alkyl, worin das Phenyl und das in Phenyl-C₁-C₂-Alkyl enthaltene Phenyl ihrerseits gegebenenfalls ein- oder mehrfach, gleich oder verschieden substituiert sein können mit Halogen, Cyano, C₁-C₃-Alkyl, C₁-C₃-Alkoxy oder Trifluormethyl.

In der allgemeinen Formel (I) steht R¹⁵ besonders bevorzugt für C₁-C₃-Alkyl, Trifluormethyl, Phenyl oder Benzyl, worin das Phenyl und das in Benzyl enthaltene Phenyl ihrerseits gegebenenfalls ein- oder mehrfach, gleich oder verschieden substituiert sein können mit Fluor, Chlor, Brom, Methyl oder Methoxy.

In der allgemeinen Formel (I) steht R¹⁵ ganz besonders bevorzugt für C₁-C₃-Alkyl.

In der allgemeinen Formel (I) steht R¹⁵ überaus besonders bevorzugt für Methyl.

Die in den jeweiligen Kombinationen bzw. bevorzugten Kombinationen von Resten im Einzelnen angegebenen Restedefinitionen werden unabhängig von den jeweiligen angegebenen Kombinationen der Reste beliebig auch durch Restedefinitionen anderer Kombination ersetzt.

Ganz besonders bevorzugt sind Kombinationen von zwei oder mehreren der oben genannten Vorzugsbereiche.

Außerordentlich bevorzugt sind die nachfolgenden Verbindungen der allgemeinen Formel (I):
[6-(4-Chlorphenyl)-1-methyl-4H-[1,2,4]triazolo[4,3-a][1]benzazepin-4-yl]essigsäureethylester;
(-)-(4*R*)-[6-(4-Chlorphenyl)-1-methyl-4H-[1,2,4]triazolo[4,3-a][1]benzazepin-4-yl]essigsäureethylester;
2-[6-(4-Chlorphenyl)-1-methyl-4H-[1,2,4]triazolo[4,3-a][1]benzazepin-4-yl]-1-(2-oxa-6-azaspiro[3.3]hept-6-yl)ethanon;
(-)-2-[(4*R*)-6-(4-Chlorphenyl)-1-methyl-4H-[1,2,4]triazolo[4,3-a][1]benzazepin-4-yl]-1-(2-oxa-6-azaspiro[3.3]hept-6-yl)ethanon;
1-(7-Azabicyclo[2.2.1]hept-7-yl)-2-[6-(4-chlorphenyl)-1-methyl-4H-[1,2,4]triazolo[4,3-a][1]benzazepin-4-yl]ethanon;
(-)-1-(7-Azabicyclo[2.2.1]hept-7-yl)-2-[(4*R*)-6-(4-chlorphenyl)-1-methyl-4H-[1,2,4]triazolo[4,3-a][1]benzazepin-4-yl]ethanon;
[6-(3-Chlorphenyl)-1-methyl-4H-[1,2,4]triazolo[4,3-a][1]benzazepin-4-yl]essigsäureethylester;
[6-(4-Chlorphenyl)-1-methyl-4H-[1,2,4]triazolo[4,3-a][1]benzazepin-4-yl]essigsäure-*tert-*butylester;
(-)-[(4*R*)-6-(4-Chlorphenyl)-1-methyl-4H-[1,2,4]triazolo[4,3-a][1]benzazepin-4-yl]essigsäure-*tert-*butylester;
2-[6-(3-Chlorphenyl)-1-methyl-4H-[1,2,4]triazolo[4,3-a][1]benzazepin-4-yl]-1-(2-oxa-6-azaspiro[3.3]hept-6-yl)ethanon;
(-)-2-[(4*R*)-6-(3-Chlorphenyl)-1-methyl-4H-[1,2,4]triazolo[4,3-a][1]benzazepin-4-yl]-1-(2-oxa-6-azaspiro[3.3]hept-6-yl)ethanon;
2-[6-(4-Chlorphenyl)-1-methyl-4H-[1,2,4]triazolo[4,3-a][1]benzazepin-4-yl]-N-ethylacetamid;
(-)-2-[(4*R*)-6-(4-Chlorphenyl)-1-methyl-4H-[1,2,4]triazolo[4,3-a][1]benzazepin-4-yl]-N-ethylacetamid;
2-[6-(4-Chlorphenyl)-1-methyl-4H-[1,2,4]triazolo[4,3-a][1]benzazepin-4-yl]-1-(morpholin-4-yl)ethanon;
(-)-2-[(4*R*)-6-(4-Chlorphenyl)-1-methyl-4H-[1,2,4]triazolo[4,3-a][1]benzazepin-4-yl]-1-(morpholin-4-yl)ethanon;
2-[6-(4-Chlorphenyl)-1-methyl-4H-[1,2,4]triazolo[4,3-a][1]benzazepin-4-yl]-1-(3-fluoroazetidin-1-yl)ethanon;
(-)-2-[(4*R*)-6-(4-Chlorphenyl)-1-methyl-4H-[1,2,4]triazolo[4,3-a][1]benzazepin-4-yl]-1-(3-fluoroazetidin-1-yl)ethanon;
2-[6-(4-Chlorphenyl)-1-methyl-4H-[1,2,4]triazolo[4,3-a][1]benzazepin-4-yl]-1-(1,1-dioxido-1-thia-6-azaspiro[3.3]hept-6-yl)ethanon;
(-)-2-[(4*R*)-6-(4-Chlorphenyl)-1-methyl-4H-[1,2,4]triazolo[4,3-a][1]benzazepin-4-yl]-1-(1,1-dioxido-1-thia-6-azaspiro[3.3]hept-6-yl)ethanon;
2-[6-(4-Chlorphenyl)-1-methyl-4H-[1,2,4]triazolo[4,3-a][1]benzazepin-4-yl]-N-(2-oxo-2,3-dihydro-1H-indol-5-yl)acetamid;
(-)-2-[(4*R*)-6-(4-Chlorphenyl)-1-methyl-4H-[1,2,4]triazolo[4,3-a][1]benzazepin-4-yl]-N-(2-oxo-2,3-dihydro-1H-indol-5-yl)acetamid;
3-{[(-6-(4-Chlorphenyl)-1-methyl-4H-[1,2,4]triazolo[4,3-a][1]benzazepin-4-yl]acetyl}-8-azabicyclo[3.2.1]octan-3-on;
(-)-3-{[(4*R*)-6-(4-Chlorphenyl)-1-methyl-4H-[1,2,4]triazolo[4,3-a][1]benzazepin-4-yl]acetyl}-8-azabicyclo[3.2.1]octan-3-on;
2-[6-(4-Chlorphenyl)-1-methyl-4H-[1,2,4]triazolo[4,3-a][1]benzazepin-4-yl]-1-(3-phenoxyazetidin-1-yl)ethanon;
(-)-2-[(4*R*)-6-(4-Chlorphenyl)-1-methyl-4H-[1,2,4]triazolo[4,3-a][1]benzazepin-4-yl]-1-(3-phenoxyazetidin-1-yl)ethanon;
1-{[6-(4-Chlorphenyl)-1-methyl-4H-[1,2,4]triazolo[4,3-a][1]benzazepin-4-yl]acetyl}pyrrolidin-3-on;
(-)-1-{[(4*R*)-6-(4-Chlorphenyl)-1-methyl-4H-[1,2,4]triazolo[4,3-a][1]benzazepin-4-yl]acetyl}pyrrolidin-3-on;
1-(8-Azaspiro[4.5]dec-8-yl)-2-[6-(4-chlorphenyl)-1-methyl-4H-[1,2,4]triazolo[4,3-a][1]benzazepin-4-yl]ethanon;
(-)-1-(8-Azaspiro[4.5]dec-8-yl)-2-[(*4R*)-6-(4-chlorphenyl)-1-methyl-4H-[1,2,4]triazolo[4,3-a][1]benzazepin-4-yl]ethanon;
2-[6-(4-Chlorphenyl)-1-methyl-4H-[1,2,4]triazolo[4,3-a][1]benzazepin-4-yl]-1-(1,1-dioxido-1,3-thiazolidin-3-yl)ethanon;
(-)-2-[(4*R*)-6-(4-Chlorphenyl)-1-methyl-4H-[1,2,4]triazolo[4,3-a][1]benzazepin-4-yl]-1-(1,1-dioxido-1,3-thiazolidin-3-yl)ethanon;
2-[6-(4-Chlorphenyl)-1-methyl-4H-[1,2,4]triazolo[4,3-a][1]benzazepin-4-yl]-1-(8-oxa-3-azabicyclo[3.2.1]oct-3-yl)ethanon;
(-)-2-[(4*R*)-6-(4-Chlorphenyl)-1-methyl-4H-[1,2,4]triazolo[4,3-a][1]benzazepin-4-yl]-1-(8-oxa-3-azabicyclo[3.2.1]oct-3-yl)ethanon;
6-(4-Chlorphenyl)-1-methyl-4-[(3-methyl-1,2,4-oxadiazol-5-yl)methyl]-4H-[1,2,4]triazolo[4,3-a][1]benzazepin;
(-)-6-[(4*R*)-4-Chlorphenyl]-1-methyl-4-[(3-methyl-1,2,4-oxadiazol-5-yl)methyl]-4H-[1,2,4]triazolo[4,3-a][1]benzazepin;
(-)-(4*R*)-6-(4-Chlorphenyl)-1-methyl-4-{[3-(propan-2-yl)-1,2,4-oxadiazol-5-yl]methyl}-4H-[1,2,4]triazolo[4,3-a][1]benzazepin;
(-)-6-(4*R*)-(4-Chlorphenyl)-4-[(3-cyclopropyl-1,2,4-oxadiazol-5-yl)methyl]-1-methyl-4H-[1,2,4]triazolo[4,3-a][1]benzazepin
(-)-(4*R*)-6-(4-Chlorphenyl)-1-methyl-4-[(5-methyl-1,3,4-oxadiazol-2-yl)methyl]-4H-[1,2,4]triazolo[4,3-a][1]benzazepin;
6-(4-Chlorphenyl)-1-methyl-4-[(5-methyl-1,3,4-oxadiazol-2-yl)methyl]-4H-[1,2,4]triazolo[4,3-a][1]benzazepin;
N'-Acetyl-2-[(4*R*)-6-(4-chlorphenyl)-1-methyl-4H-[1,2,4]triazolo[4,3-a][1]benzazepin-4-yl]acetohydrazid;
6-(4-Chlorphenyl)-1-methyl-4-[(5-methyl-1,3,4-thiadiazol-2-yl)methyl]-4H-[1,2,4]triazolo[4,3-a][1]benzazepin;
(+)-(4*R*)-6-(4-Chlorphenyl)-1-methyl-4-[(5-methyl-1,3,4-thiadiazol-2-yl)methyl]-4H-[1,2,4]triazolo[4,3-a][1]benzazepin;
6-(4-Chlorphenyl)-4-[(5-cyclopropyl-1,3,4-oxadiazol-2-yl)methyl]-1-methyl-4H-[1,2,4]triazolo[4,3-a][1]benzazepin;
6-(4-Chlorphenyl)-1-methyl-4-1[5-(pyridin-3-yl)-1,3,4-oxadiazol-2-yl]methyl}-4H-[1,2,4]triazolo[4,3-a][1]benzazepin;
(-)-(4*R*)-6-(4-Chlorphenyl)-1-methyl-4-{[5-(pyridin-3-yl)-1,3,4-oxadiazol-2-yl]methyl}-4H-[1,2,4]triazolo[4,3-a][1]benzazepin;
6-(4-Chlorphenyl)-1-methyl-4-[(5-methyl-1,2,4-oxadiazol-3-yl)methyl]-4H-[1,2,4]triazolo[4,3-a][1]benzazepin;
6-(4-Chlorphenyl)-8-methoxy-1-methyl-4-[(5-methyl-1,3,4-oxadiazol-2-yl)methyl]-4H-[1,2,4]triazolo[4,3-a][1]benzazepin;
6-(4-Chlorphenyl)-8-(3,5-dimethyl-1,2-oxazol-4-yl)-1-methyl-4-[(5-methyl-1,3,4-oxadiazol-2-yl)methyl]-4H-[1,2,4]triazolo[4,3-a][1]benzazepin;
[6-(4-Chlorphenyl)-1-methyl-8-(trifluormethoxy)-4H-[1,2,4]triazolo[4,3-a][1]benzazepin-4-yl]essigsäure-*tert*-butylester;
(-)-[(4*R*)-6-(4-Chlorphenyl)-1-methyl-8-(trifluoromethoxy)-4H-[1,2,4]triazolo[4,3-a][1]benzazepin-4-yl]essigsäure-*tert*-butylester;
2-[(4*R*)-6-(4-Chlorphenyl)-1-methyl-8-(trifluoromethoxy)-4H-[1,2,4]triazolo[4,3-a][1,4]benzazepin-4-yl]-1-(2-oxa-6-azaspiro[3.3]hept-6-yl)ethanon;
2-[(4*R*)-6-(4-Chlorphenyl)-1-methyl-8-(trifluoromethoxy)-4H-[1,2,4]triazolo[4,3-a][1]benzazepin-4-yl]-N-ethylacetamid;
2-[(4*R*)-6-(4-Chlorphenyl)-1-methyl-8-(trifluoromethoxy)-4H-[1,2,4]triazolo[4,3-a][1]benzazepin-4-yl]-N-[2-(morpholin-4-yl)ethyl]acetamid;
6-(4-Chlorphenyl)-1-methyl-4-[(5-methyl-1,3,4-oxadiazol-2-yl)methyl]-8-(trifluoromethoxy)-4H-[1,2,4]triazolo[4,3-a][1]benzazepin;
(4*R*)-6-(4-Chlorphenyl)-1-methyl-4-[(5-methyl-1,3,4-oxadiazol-2-yl)methyl]-8-(trifluoromethoxy)-4H-[1,2,4]triazolo[4,3-a][1]benzazepin;
2-{[(4*R*)-6-(4-Chlorphenyl)-1-methyl-8-(trifluoromethoxy)-4H-[1,2,4]triazolo[4,3-a][1]benzazepin-4-yl]acetyl}hexahydropyrrolo[1,2-a]pyrazin-6(2H)-on;
(4*R*)-4-({5-[(Benzyloxy)methyl]-1,3,4-oxadiazol-2-yl}methyl)-6-(4-chlorphenyl)-1-methyl-4H-[1,2,4]triazolo[4,3-a][1]benzazepin.

### Definitionen

Der Erfindung liegen folgende Definitionen zugrunde:

Unter C₁-C₆-Alkyl, bzw. einer C₁-C₆-Alkyl-Gruppe ist ein linearer oder verzweigter, gesättigter, monovalenter Kohlenwasserstoffrest zu verstehen, wie z.B. ein Methyl-, Ethyl-, Propyl-, Butyl-, Pentyl-, Hexyl-, *iso*-Propyl-, *iso*-Butyl-, *sec*-Butyl-, *tert*-Butyl-, *iso*-Pentyl-, 2-Methylbutyl-, 1-Methylbutyl-, 1-Ethylpropyl-, 1,2-Dimethylpropyl, *neo*-Pentyl-, 1,1-Dimethylpropyl-, 4-Methylpentyl-, 3-Methylpentyl-, 2-Methylpentyl-, 1-Methylpentyl-, 2-Ethylbutyl-, 1-Ethylbutyl-, 3,3-Dimethylbutyl-, 2,2-Dimethylbutyl-, 1,1-Dimethylbutyl-, 2,3-Dimethylbutyl-, 1,3-Dimethylbutyl- oder 1,2-Dimethylbutyl-Rest.

Vorzugsweise ist unter C₁-C₆-Alkyl bzw. einer C₁-C₆-Alkyl-Gruppe ein Methyl-, Ethyl-, Propyl- oder Isopropyl-Rest zu verstehen.

Unter C₃-C₆-Alkylen, bzw. einer C₃-C₆-Alkylen-Gruppe ist ein linearer oder verzweigter, gesättigter, bivalenter Kohlenwasserstoffrest zu verstehen, wie z.B. ein Propylen-, Butylen-, Pentylen-, Hexylen-, *iso*-Propylen-, *iso*-Butylen-, *sec*-Butylen-, *tert*-Butylen-, *iso*-Pentylen-, 2-Methylbutylen-, 1-Methylbutylen-, 1-Ethylpropylen-, 1,2-Dimethylpropylen, *neo*-Pentylen-, 1,1-Dimethylpropylen-, 4-Methylpentylen-, 3-Methylpentylen-, 2-Methylpentylen-, 1-Methylpentylen-, 2-Ethylbutylen-, 1-Ethylbutylen-, 3,3-Dimethylbutylen-, 2,2-Dimethylbutylen-, 1,1-Dimethylbutylen-, 2,3-Dimethylbutylen-, 1,3-Dimethylbutylen- oder 1,2-Dimethylbutylen-Rest.

Vorzugsweise ist unter C₃-C₆-Alkylen bzw. einer C₃-C₆-Alkylen-Gruppe C₃-C₄-Alkylen, insbesondere ein Propylen- oder Butylen-Rest zu verstehen.

Unter C₃-C₆-Heteroalkylen ist eine C₃-C₆-Alkylen-Gruppe wie voranstehend definiert zu verstehen, in welcher 1, 2 oder 3 Kohlenstoffatome, vorzugsweise 1 oder 2 Kohlenstoffatome, durch Heteroatome, ausgewählt aus Stickstoff, Sauerstoff und Schwefel, ersetzt ist. Bevorzugt ist C₃-C₄-Heteroalkylen.

Unter C₁-C₆-Alkoxy, bzw. einer C₁-C₆-Alkoxy-Gruppe ist ein linearer oder verzweigter, gesättigter Alkyletherrest -0-Alkyl zu verstehen, wie z.B. ein Methoxy-, Ethoxy-, n-Propoxy-, Isopropoxy-, *tert*-Butoxy-, n-Pentoxy- oder n-Hexoxy-Rest.

Vorzugsweise ist unter C₁-C₆-Alkoxy, bzw. einer C₁-C₆-Alkoxy-Gruppe ein Methoxy-, Ethoxy- oder *tert*-Butoxy-Rest zu verstehen.

Unter einem Heteroatom ist ein Sauerstoff-, Stickstoff- oder Schwefel-Atom zu verstehen. Bevorzugt sind ein Sauerstoff- oder ein Stickstoffatom.

Unter Halogen, bzw. Hal ist Fluor, Chlor oder Brom zu verstehen, welches am Phenylring in ortho-, meta- oder para-Stellung stehen kann. Bevorzugt ist Fluor oder Chlor. Die bevorzugte Position ist die meta- oder para-Position.

Unter Oxo, einer Oxogruppe oder einem Oxo-Substituenten ist ein doppelt gebundenes SauerstoffAtom =O zu verstehen. Oxo kann an Atome geeigneter Valenz gebunden sein, beispielsweise an ein gesättigtes Kohlenstoff-Atom oder an Schwefel.

Bevorzugt ist die Bindung an Kohlenstoff unter Bildung einer Carbonyl-Gruppe -C(=O)-. Bevorzugt ist weiterhin die Bindung zweier doppelt gebundenener Sauerstoffatome an ein Schwefelatom unter Bildung einer Sulfonyl-Gruppe -S(=O)₂-.

Unter einem Halogen-C₁-C₆-Alkylrest ist ein C₁-C₆-Alkylrest mit mindestens einem Halogensubstituenten zu verstehen.

Bevorzugt sind Fluoralkylreste wie Fluor-C₁-C₆-Alkyl oder Fluor-C₁-C₃-Alkyl, beispielsweise Difluormethyl, Trifluormethyl, 2,2,2-Trifluorethyl, Pentafluorethyl, 5,5,5,4,4-Pentafluorpentyl oder 5,5,5,4,4,3,3-Heptafluorpentyl.

Besonders bevorzugt sind perfluorierte Alkylreste wie Trifluormethyl oder Pentafluorethyl.

Unter einem Halogen-C₁-C₆-Alkoxyrest ist ein C₁-C₆-Alkoxyrest mit mindestens einem Halogensubstituenten zu verstehen.

Bevorzugt sind Fluoralkoxyreste wie Fluor-C₁-C₆-Alkoxy oder Fluor-C₁-C₃-Alkoxy, beispielsweise Difluorethoxy-, Trifluomethoxy- oder 2,2,2-Trifluorethoxyreste.

Unter einem C₁-C₆-Alkylcarbonylrest ist eine C₁-C₆-(O=)C-Gruppe zu verstehen. Bevorzugt ist hierbei eine C₁-C₄-(O=)C-Gruppe. Besonders bevorzugt ist hier eine C₁-C₃-(O=)C-Gruppe, wie z.B. Acetyl- oder Propanoyl.

Alkyl-Amino- steht für einen Aminorest mit einem oder zwei (unabhängig voneinander gewählten) Alkylsubstituenten mit in der Regel 1 bis 3 Kohlenstoffatomen (C₁-C₃-Alkyl-Amino-).

(C₁-C₃)-Alkylamino- steht beispielsweise für einen Monoalkylaminorest mit 1 bis 3 Kohlenstoffatomen oder für einen Dialkylaminorest mit jeweils 1 bis 3 Kohlenstoffatomen pro Alkylsubstituent.

Beispielhaft seien genannt:
Methylamino-, Ethylamino-, n-Propylamino-, Isopropylamino-, *N*,*N*-Dimethylamino-, *N*,*N-*Diethylamino-, *N*-Ethyl-*N*-methylamino- und *N*-Methyl-N-n-propylamino.

Unter einem C₁-C₆-Alkoxy-C₁-C₆-Alkylrest ist ein mit C₁-C₆-Alkoxy substituierter C₁-C₆-Alkylrest zu verstehen, wie z. B.Methoxymethyl, Methoxyethyl, Ethoxymethyl und Ethoxyethyl. Bevorzugt ist C₁-C₃-Alkoxy-C₁-C₃-Alkyl.

Unter einem Aryloxy-C₁-C₃-Alkylrest ist ein mit Aryloxy wie nachstehend definiert substituierter C₁-C₃-Alkylrest zu verstehen, wie z. B.Phenoxymethyl, Phenoxyethyl und Naphthyloxymethyl. Bevorzugt ist Phenoxy-C₁-C₃-Alkyl.

Unter einem Aryl-C₁-C₃-Alkoxy-C₁-C₃-Alkylrest ist ein mit Aryl wie nachstehend definiert substituierter C₁-C₃-Alkoxy-C₁-C₃-Alkylrest zu verstehen, wie z. B. Benzyloxymethyl, Phenethyloxymethyl oder Benzyloxypropyl. Bevorzugt ist Benzyloxy-C₁-C₃-Alkyl.

Unter einem Aryl-C₁-C₂-Alkylrest ist ein mit Aryl wie nachstehend definiert substituierter C₁-C₂-Alkylrest zu verstehen, wie z. B. Naphthylmethyl, Phenethyl, 1-Phenylethyl oder Benzyl.

Bevorzugt ist Phenyl-C₁-C₂-Alkyl, besonders bevorzugt ist Benzyl.

Unter Aryl ist ein aus Kohlenstoffatomen aufgebautes ungesättigtes vollständig konjugiertes System zu verstehen, welches über 3, 5 oder 7 konjugierte Doppelbindungen verfügt, wie z.B. Phenyl, Naphthyl oder Phenantryl. Bevorzugt ist Phenyl.

Unter Aryloxy ist ein wie voranstehend definierter Alkylrest zu verstehen, der über ein SauerstoffAtom an den Rest des Moleküls gebunden ist, wie z.B. Phenoxy, Naphthyloxy oder Phenantryloxy. Bevorzugt ist Phenoxy.

Unter Heteroaryl sind Ringsysteme zu verstehen, die über ein aromatisch konjugiertes Ringsystem verfügen. Diese können über 5, 6 oder 7 Ringatome verfügen, oder im Fall von kondensierten Ringsystemen auch eine Kombination aus 5 und 6 Ringsystemen, 5 und 5 Ringsystemen oder auch 6 und 6 Ringsystemen verfügen. Ebenso können sie über 1 bis 5 Heteroatomen aus der Gruppe N, O und S verfügen. Als Beispiel seien aufgeführt Ringsysteme wie Pyrrolyl, Pyrazolyl, Imidazolyl, Triazolyl, Tetrazolyl, Furyl, Thienyl, Oxazolyl, Thiazolyl, Isoxazolyl, Oxadiazolyl, Thiadiazolyl, Pyridinyl, Pyrimidinyl, Pyrazinyl, Triazinyl, Oxazinyl, Indolyl, Benzimidazolyl, Indazolyl, Benzotriazolyl, Benzothiazolyl, Benzoxazolyl, Benzofuryl, Benzothienyl, Chinolinyl, Isochinolinyl, Cinnolinyl, Chinazolinyl, Chinoxalinyl, Imidazopyridinyl oder auch Benzoxazinyl. Bevorzugt ist Heteroaryl mit 5 oder 6 Ringatomen, das 1, 2 oder 3 Heteroatome, unabhängig voneinander ausgewählt aus O, N und S, enthält.

Unter C₃-C₈-Zykloalkyl, bzw. einem 5- bis 8-gliedrigen Zykloalkyl ist eine aus Kohlenstoffatomen aufgebautes Ringsystem mit 3-8 Atomen, bzw. 5 bis 8 Atomen wie z.B.Zyklopropyl, Zyklobutyl, Zyklopentyl, Zyklohexyl, Zykloheptyl oder Zyklooktyl zu verstehen.

Unter C₃-C₈-Zykloalkoxy ist eine C₃-C₈-Zykloalkylgruppe gemäss voriger Definition zu verstehen, die über ein Sauerstoffatom an die jeweilig definierte Position angebunden ist. Beispielsweise Zyklopropyloxy, Zyklobutyloxy, Zyklopentyloxy, Zyklohexyloxy, Zykloheptyloxy oder Zyklooktyloxy.

Unter C₃-C₈-Heterozykloalkyl ist ein 3- bis 8-gliedriges, monozyklisches und gesättigtes Ringsystem zu verstehen, in welchem 1, 2 oder 3 Kohlenstoffatome durch Heteroatome aus der Gruppe N, O oder S in beliebiger Kombination ersetzt sind. Beispielhaft zu nennen sind Pyrrolidin, Piperidin, Tetrahydrofuran, Tetrahydropyran, Oxetan, Azetidin, Azepan, Morpholin, Thiomorpholin oder Piperazin.

Unter 5-6 gliedriges Ringsystem ist ein aromatisches oder auch nicht-aromatisches Ringsystem bestehend aus 5-6 Atomen zu verstehen, welches 0-4 Heteroatome aus der Gruppe N, O oder S in jeder möglichen Kombination tragen kann. Bevorzugt sind aromatische Ringsysteme, besonders bevorzugt solche mit 5 Atomen und davon 2-3 Heteroatomen. Besonders bevorzugt sind Ringsysteme wie z.B. 1,2,4-Oxadiazol, 1,3,4-Oxadiazol, 1,3-Thiazol und 1,3-Oxazol. Unter nicht-aromatisches Ringsystem sind sowohl gesättigte als auch ein- oder mehrfach ungesättigte, aber nicht aromatische Ringsysteme zu verstehen.

Unter Heterozykloalkoxy ist eine Heterozykloalkylgruppe gemäss voriger Definition zu verstehen, die über ein Sauerstoffatom an die jeweilig definierte Position angebunden ist.

Unter C₅-C₁₁ -Spirozykloalkyl bzw. Heterospirozykloalkyl mit einem Ersatz von 1-4 Kohlenstoffatomen durch Stickstoff, Sauerstoff und/oder Schwefel, einschließlich seiner beiden oxidierten Formen S(=O) oder S(=O)₂ und seiner als Sulfoximin abgewandelter Derivate, ist eine Fusion aus zwei Ringsystemen zu verstehen, die sich ein gemeinsames Atom teilen. (Beispiele sind Spiro[2.2]pentyl, Spiro[2.3]hexyl, Azaspiro[2.3]hexyl, Spiro[3.3]heptyl, Azaspiro[3.3]heptyl, Oxaazaspiro[3.3]heptyl, Thiaazaspiro[3.3]heptyl, Oxaspiro[3.3]heptyl, Oxazaspiro[5.3]nonyl, Oxazaspiro[4.3]oktyl, Oxazaspiro[5.5]undekyl, Diazaspiro[3.3]heptyl, Thiazaspiro[3.3]heptyl, Thiazaspiro[4.3]oktyl, Azaspiro[5.5]dekyl, sowie die weiteren homologen Spiro[3.4]-, Spiro[4.4]-, Spiro[5.5]-, Spiro[6.6]-, Spiro[2.4]-, Spiro[2.5]-,Spiro[2.6]-, Spiro[3.5]-, Spiro[3.6]-, Spiro[4.5]-, Spiro[4.6]- und Spiro[5.6]-Systeme inklusive der durch Heteroatome modifizierten Varianten gemäss Definition.

Unter C₆-C₁₂-Bizykloalkyl bzw. Heterobizykloalkyl mit einem Ersatz von 1-4 Kohlenstoffatomen durch Stickstoff, Sauerstoff und/oder Schwefel, einschließlich seiner beiden oxidierten Formen S(=O) oder S(=O)₂ und seiner als Sulfoximin abgewandelter Derivate, ist eine Fusion aus zwei Ringsystemen zu verstehen, die sich gemeinsam zwei direkt benachbarte Atome teilen.(Beispiele sind Bizyklo[2.2.0]hexyl, Bizyklo[3.3.0]oktyl, Bizyklo[4.4.0]dekyl, Bizyklo[5.4.0]undekyl, Bizykl[3.2.0]heptyl, Bizyklo[4.2.0]oktyl, Bizyklo[5.2.0]nonyl, Bizyklo[6.2.0]dekyl, Bizyklo[4.3.0]nonyl, Bizyklo[5.3.0]dekyl, Bizyklo[6.3.0]undekyl und Bizyklo[5.4.0]undekyl, inklusive der durch Heteroatome modifizierten Varianten wie z.B. Azabizyklo[3.3.0]oktyl, Azabicyclo[4.3.0]nonyl, Diazabicyclo[4.3.0]nonyl, Oxazabizyklo[4.3.0]nonyl, Thiazabizyklo[4.3.0]nonyl oder Azabizyklo[4.4.0]dekyl sowie die weiteren möglichen Kombinationen gemäss Definition.

Unter einem überbrückten Ringsystem ist eine Fusion aus mindestens zwei Ringen zu verstehen, die sich 2 Atome teilen, die nicht direkt benachbart zueinander sind. Dabei kann sowohl ein überbrückter Zyklus als auch ein überbrückter Heterozyklus mit einem Ersatz von 1-4 Kohlenstoffatomen durch Stickstoff, Sauerstoff und/oder Schwefel, einschließlich seiner beiden oxidierten Formen S(=O) oder S(=O)₂ und seiner als Sulfoximin abgewandelter Derivate, entstehen. Beispiele sind Bizyklo[2.2.1]heptyl, Azabizyklo[2.2.1]heptyl, Oxazabizyklo[2.2.1]heptyl, Thiazabizyklo[2.2.1]heptyl, Diazabizyklo[2.2.1]heptyl, Bizyklo[2.2.2]oktyl, Azabizyklo[2.2.2]oktyl, Diazabizyklo[2.2.2]oktyl, Oxazabizyklo[2.2.2]oktyl, Thiazabizyklo[2.2.2]oktyl, Bizyklo[3.2.1]oktyl, Azabizyklo[3.2.1]oktyl, Diazabizyklo[3.2.1]oktyl, Oxazabizyklo[3.2.1]oktyl, Thiazabizyklo[3.2.1]oktyl, Bizyklo[3.3.1]nonyl, Azabizyklo[3.3.1]nonyl, Diazabizyklo[3.3.1]nonyl, Oxazabizyklo[3.3.1]nonyl, Thiazabizyklo[3.3.1]nonyl, Bizyklo[4.2.1]nonyl, Azabizyklo[4.2.1]nonyl, Diazabizyklo[4.2.1]nonyl, Oxazabizyklo[4.2.1]nonyl, Thiazabizyklo[4.2.1]nonyl, Bizyklo[3.3.2]dekyl, Azabizyklo[3.3.2]dekyl, Diazabizyklo[3.3.2]dekyl, Oxazabizyklo[3.3.2]dekyl, Thiazabizyklo[3.3.2]dekyl oder Azabizyklo[4.2.2]dekyl sowie die weiteren möglichen Kombinationen gemäss Definition.

Unter einer Abgangsgruppe ist ein Atom oder eine Gruppe von Atomen zu verstehen, die in einer chemischen Reaktion als stabile Spezies aus dem Substratmolekül austritt und dabei die Bindungselektronen mit sich nimmt. Beispiele für Abgangsgruppen sind Halogen, Methansulfonyloxy, p-Toluolsulfonyloxy, Trifluormethansulfonyloxy, Nonafluorbutansulfonyloxy, (4-Bromphenyl)sulfonyloxy, (4-Nitrophenyl)sulfonyloxy, (2-Nitrophenyl)-sulfonyloxy, (4-Isopropylphenyl)sulfonyloxy, (2,4,6-Tri-isopropylphenyl)-sulfonyloxy, (2,4,6-Trimethylphenyl)sulfonyloxy, (4-*tert*-Butylphenyl)sulfonyloxy, Phenylsulfonyloxy, und (4-Methoxyphenyl)sulfonyloxy. Bevorzugt ist Halogen, besonders bevorzugt sind Fluor, Chlor, Brom.

Erfindungsgemäße Verbindungen sind die Verbindungen der allgemeinen Formel (I) und deren Salze, Solvate und Solvate der Salze, die von der allgemeinen Formel (I) umfassten Verbindungen der nachfolgend genannten Formeln und deren Salze, Solvate und Solvate der Salze sowie die von der allgemeinen Formel (I) umfassten, nachfolgend als Ausführungsbeispiele genannten Verbindungen und deren Salze, Solvate und Solvate der Salze, soweit es sich bei den von der allgemeinen Formel (I) umfassten, nachfolgend genannten Verbindungen nicht bereits um Salze, Solvate und Solvate der Salze handelt.

Ebenfalls als von der vorliegenden Erfindung als umfasst anzusehen ist die Verwendung der Salze der erfindungsgemäßen Verbindungen.

Als Salze sind im Rahmen der vorliegenden Erfindung physiologisch unbedenkliche Salze der erfindungsgemäßen Verbindungen bevorzugt. Umfasst sind aber auch Salze, die für pharmazeutische Anwendungen selbst nicht geeignet sind aber beispielsweise für die Isolierung oder Reinigung der erfindungsgemäßen Verbindungen verwendet werden können.

Physiologisch unbedenkliche Salze der erfindungsgemäßen Verbindungen umfassen Säureadditionssalze von Mineralsäuren, Carbonsäuren und Sulfonsäuren, z.B. Salze der Chlorwasserstoffsäure, Bromwasserstoffsäure, Schwefelsäure, Phosphorsäure, Methansulfonsäure, Ethansulfonsäure, Toluolsulfonsäure, Benzolsulfonsäure, Naphthalindisulfonsäure, Essigsäure, Trifluoressigsäure, Propionsäure, Milchsäure, Weinsäure, Äpfelsäure, Zitronensäure, Fumarsäure, Maleinsäure und Benzoesäure.

Die vorliegende Erfindung betrifft auch Arzneimittel enthaltend die erfindungsgemäßen Verbindungen zusammen mit mindestens einem oder mehreren weiteren Wirkstoffen, insbesondere zur Prophylaxe und/oder Therapie von Tumorerkrankungen.

Als Solvate werden im Rahmen der Erfindung solche Formen der erfindungsgemäßen Verbindungen bezeichnet, welche in festem oder flüssigem Zustand durch Koordination mit Lösungsmittelmolekülen einen Komplex bilden. Hydrate sind eine spezielle Form der Solvate, bei denen die Koordination mit Wasser erfolgt. Als Solvate sind im Rahmen der vorliegenden Erfindung Hydrate bevorzugt.

Ein weiterer Gegenstand der vorliegenden Erfindung sind alle möglichen kristallinen und polymorphen Formen der erfindungsgemäßen Verbindungen, wobei die Polymorphe entweder als einzelne Polymorphe oder als Gemisch mehrerer Polymorphe in allen Konzentrationsbereichen vorliegen können.

Die erfindungsgemäßen Verbindungen können in Abhängigkeit von ihrer Struktur in unterschiedlichen stereoisomeren Formen existieren, d.h. in Gestalt von Konfigurationsisomeren oder gegebenenfalls auch als Konformationsisomere. Die erfindungsgemäßen Verbindungen weisen an der Position 4 des Benzazepin-Ringes, die jenen Ringkohlenstoff bezeichnet, der über eine Methylengruppe an R³ gebunden ist, ein einheitlich konfiguriertes Asymmetriezentrum auf. Sie können daher als reine Diastereomere oder deren Gemische vorliegen, wenn einer oder mehrere der in der Formel (I) beschriebenen Substituenten ein weiteres Asymmetrieelement enthält, beispielsweise ein chirales Kohlenstoffatom. Die vorliegende Erfindung umfasst deshalb auch Diastereomere und ihre jeweiligen Mischungen. Aus solchen Mischungen lassen sich die reinen Diastereomere stereoisomer in bekannter Weise isolieren; vorzugsweise werden hierfür chromatographische Verfahren verwendet, insbesondere die HPLC-Chromatographie an achiraler bzw. chiraler Phase.

Ein weiterer Gegenstand der vorliegenden Erfindung sind Enantiomerengemische der (4*R*)-konfigurierten erfindungsgemäßen Verbindungen mit ihren (4*S*)-Enantiomeren, insbesondere die entsprechenden Razemate sowie Enantiomerengemische, in denen die (4*R*)-Form überwiegt.

Sofern die erfindungsgemäßen Verbindungen in tautomeren Formen vorkommen können, umfasst die vorliegende Erfindung sämtliche tautomere Formen.

Die vorliegende Erfindung umfasst auch alle geeigneten isotopischen Varianten der erfindungsgemäßen Verbindungen. Unter einer isotopischen Variante einer erfindungsgemäßen Verbindung wird hierbei eine Verbindung verstanden, in welcher mindestens ein Atom innerhalb der erfindungsgemäßen Verbindung gegen ein anderes Atom der gleichen Ordnungszahl, jedoch mit einer anderen Atommasse als der gewöhnlich oder überwiegend in der Natur vorkommenden Atommasse ausgetauscht ist. Beispiele für Isotope, die in eine erfindungsgemäße Verbindung inkorporiert werden können, sind solche von Wasserstoff, Kohlenstoff, Stickstoff, Sauerstoff, Phosphor, Schwefel, Fluor, Chlor, Brom und Iod, wie ²H (Deuterium), ³H (Tritium), ¹³C, ¹⁴C, ¹⁵N, ¹⁷O, ¹⁸O, ³²P, ³³P, ³³S, ³⁴S, ³⁵S, ³⁶S, ¹⁸F, ³⁶Cl, ⁸²Br, ¹²³I, ¹²⁴I, ¹²⁹I und ¹³¹I. Bestimmte isotopische Varianten einer erfindungsgemäßen Verbindung, wie insbesondere solche, bei denen ein oder mehrere radioaktive Isotope inkorporiert sind, können von Nutzen sein beispielsweise für die Untersuchung des Wirkmechanismus oder der Wirkstoff-Verteilung im Körper; aufgrund der vergleichsweise leichten Herstell- und Detektierbarkeit sind hierfür insbesondere mit ³H- oder ¹⁴C-Isotopen markierte Verbindungen geeignet. Darüber hinaus kann der Einbau von Isotopen, wie beispielsweise von Deuterium, zu bestimmten therapeutischen Vorteilen als Folge einer größeren metabolischen Stabilität der Verbindung führen, wie beispielsweise eine Verlängerung der Halbwertszeit im Körper oder eine Reduktion der erforderlichen Wirkdosis; solche Modifikationen der erfindungsgemäßen Verbindungen können daher gegebenenalls auch eine bevorzugte Ausführungsform der vorliegenden Erfindung darstellen. Isotopische Varianten der erfindungsgemäßen Verbindungen können nach den dem Fachmann bekannten Verfahren hergestellt werden, so beispielsweise nach den weiter unten beschriebenen Methoden und den bei den Ausführungsbeispielen wiedergegebenen Vorschriften, indem entsprechende isotopische Modifikationen der jeweiligen Reagentien und/oder Ausgangsverbindungen eingesetzt werden.

Beschrieben werden auch Prodrugs der erfindungsgemäßen Verbindungen. Der Begriff "Prodrugs" umfaßt Verbindungen, welche selbst biologisch aktiv oder inaktiv sein können, jedoch während ihrer Verweilzeit im Körper zu erfindungsgemäßen Verbindungen umgesetzt werden (beispielsweise metabolisch oder hydrolytisch).

Die erfindungsgemäßen Verbindungen können systemisch und/oder lokal wirken. Zu diesem Zweck kann sie auf geeignete Weise appliziert werden, wie z.B. oral, parenteral, pulmonal, nasal, sublingual, lingual, buccal, rectal, dermal, transdermal, conjunctival, otisch oder als Implantat bzw. Stent.

Für diese Applikationswege können die erfindungsgemäßen Verbindungen in geeigneten Applikationsformen verabreicht werden.

Für die orale Applikation eignen sich nach dem Stand der Technik funktionierende schnell und/oder modifiziert die erfindungsgemäßen Verbindungen abgebende Applikationsformen, die die erfindungsgemäßen Verbindungen in kristalliner und/ oder amorphisierter und/oder gelöster Form enthalten, wie z.B. Tabletten (nichtüberzogene oder überzogene Tabletten, beispielsweise mit magensaftresistenten oder sich verzögert auflösenden oder unlöslichen Überzügen, die die Freisetzung der erfindungsgemäßen Verbindung kontrollieren), in der Mundhöhle schnell zerfallende Tabletten oder Filme/Oblaten, Filme/Lyophilisate, Kapseln (beispielsweise Hart- oder Weichgelatinekapseln), Dragees, Granulate, Pellets, Pulver, Emulsionen, Suspensionen, Aerosole oder Lösungen.

Die parenterale Applikation kann unter Umgehung eines Resorptionsschrittes geschehen (z.B. intravenös, intraarteriell, intrakardial, intraspinal oder intralumbal) oder unter Einschaltung einer Resorption (z.B. intramuskulär, subcutan, intracutan, percutan oder intraperitoneal). Für die parenterale Applikation eignen sich als Applikationsformen u.a. Injektions- und Infusionszubereitungen in Form von Lösungen, Suspensionen, Emulsionen, Lyophilisaten oder sterilen Pulvern.

Für die sonstigen Applikationswege eignen sich z.B. Inhalationsarzneiformen (u.a. Pulverinhalatoren, Nebulizer), Nasentropfen, -lösungen, -sprays; lingual, sublingual oder buccal zu applizierende Tabletten, Filme/Oblaten oder Kapseln, Suppositorien, Ohren- oder Augenpräparationen, Vaginalkapseln, wässrige Suspensionen (Lotionen, Schüttelmixturen), lipophile Suspensionen, Salben, Cremes, transdermale therapeutische Systeme (wie beispielsweise Pflaster), Milch, Pasten, Schäume, Streupuder, Implantate oder Stents.

Die erfindungsgemäßen Verbindungen können in die angeführten Applikationsformen überführt werden. Dies kann in an sich bekannter Weise durch Mischen mit inerten, nichttoxischen, pharmazeutisch geeigneten Hilfsstoffen geschehen. Zu diesen Hilfsstoffen zählen u.a. Trägerstoffe (beispielsweise mikrokristalline Cellulose, Laktose, Mannitol), Lösungsmittel (z.B. flüssige Polyethylenglycole), Emulgatoren und Dispergier- oder Netzmittel (beispielsweise Natriumdodecylsulfat, Polyoxysorbitanoleat), Bindemittel (beispielsweise Polyvinylpyrrolidon), synthetische und natürliche Polymere (beispielsweise Albumin), Stabilisatoren (z.B. Antioxidantien wie beispielsweise Ascorbinsäure), Farbstoffe (z.B. anorganische Pigmente wie beispielsweise Eisenoxide) und Geschmacks- und / oder Geruchskorrigentien.

Weiterer Gegenstand der vorliegenden Erfindung sind Arzneimittel, die die erfindungsgemäßen Verbindungen, üblicherweise zusammen mit einem oder mehreren inerten, nichttoxischen, pharmazeutisch geeigneten Hilfsstoffen enthalten, sowie deren Verwendung zu den zuvor genannten Zwecken.

Die Formulierung der erfindungsgemäßen Verbindungen zu pharmazeutischen Präparaten erfolgt in an sich bekannter Weise, indem man den oder die Wirkstoffe mit den in der Galenik gebräuchlichen Hilfsstoffen in die gewünschte Applikationsform überführt.

Als Hilfsstoffe können dabei beispielsweise Trägersubstanzen, Füllstoffe, Sprengmittel, Bindemittel, Feuchthaltemittel, Gleitmittel, Ab- und Adsorptionsmittel, Verdünnungsmittel, Lösungsmittel, Cosolventien, Emulgatoren, Lösungsvermittler, Geschmackskorrigentien, Färbemittel, Konservierungs-, Stabilisierungs-, Netzmittel, Salze zur Veränderung des osmotischen Drucks oder Puffer zum Einsatz kommen. Dabei ist auf Remington's Pharmaceutical Science, 15th ed. Mack Publishing Company, East Pennsylvania (1980) hinzuweisen.

Die pharmazeutischen Formulierungen können in fester Form, zum Beispiel als Tabletten, Dragees, Pillen, Suppositorien, Kapseln, transdermale Systeme oder in halbfester Form , zum Beispiel als Salben, Cremes, Gele, Suppositorien, Emulsionen oder in flüssiger Form, zum Beispiel als Lösungen, Tinkturen, Suspensionen oder Emulsionen vorliegen.

Hilfsstoffe im Sinne der Erfindung können beispielsweise Salze, Saccharide (Mono-, Di-, Tri-, Oligo-, und/oder Polysaccharide), Proteine, Aminosäuren, Peptide, Fette, Wachse, Öle, Kohlenwasserstoffe sowie deren Derivate sein, wobei die Hilfsstoffe natürlichen Ursprungs sein können oder synthetisch bzw. partial synthetisch gewonnen werden können.

Für die orale oder perorale Applikation kommen insbesondere Tabletten, Dragees, Kapseln, Pillen, Pulver, Granulate, Pastillen, Suspensionen, Emulsionen oder Lösungen in Frage.

Für die parenterale Applikation kommen insbesondere Suspensionen, Emulsionen und vor allem Lösungen in Frage.

Die erfindungsgemäßen Verbindungen eignen sich zur Prophylaxe und/oder Therapie von hyperproliferativen Erkrankungen wie beispielsweise Psoriasis, Keloide und andere Hyperplasien, die die Haut betreffen, gutartige Prostathyperplasien (BPH), solide Tumore und hämatologische Tumore.

Als solide Tumore sind erfindungsgemäß beispielsweise Tumore behandelbar der Brust, des Respirationstraktes, des Gehirns , der Fortpflanzungsorgane, des Magen-Darmtraktes, des Urogenitaltraktes, des Auges, der Leber, der Haut, des Kopfes und des Halses, der Schilddrüse, der Nebenschilddrüse, der Knochen sowie des Bindegewebes und Metastasen dieser Tumore.

Als hämatologische Tumore sind beispielsweise behandelbar multiple Myelome, Lymphome oder Leukämien.

Als Brusttumore sind beispielsweise behandelbar Mammakarzinome mit positivem Hormonrezeptorstatus, Mammakarzinome mit negativem Hormonrezeptorstatus, Her-2 positive Mammakarzinome, Hormonrezeptor- und Her-2 negative Mammakarzinome, BRCA -assoziierte Mammakarzinome und entzündliches Mammakarzinom.

Als Tumore des Respirationstraktes sind beispielsweise behandelbar nicht-kleinzellige Bronchialkarzinome und kleinzellige Bronchialkarzinome.

Als Tumore des Gehirns sind beispielsweise behandelbar Gliome, Glioblastome, Astrozytome, Meningiome und Medulloblastome.

Als Tumore der männlichen Fortpflanzungsorgane sind beispielsweise behandelbar Prostatakarzinome, Maligne Nebenhodentumore, Maligne Hodentumore und Peniskarzinome.

Als Tumore der weiblichen Fortpflanzungsorgane sind beispielsweise behandelbar Endometriumkarzinome, Zervixkarzinome, Ovarialkarzinome, Vaginalkarzinome und Vulvarkarzinome.

Als Tumore des Magen- Darm-Traktes sind beispielsweise behandelbar Kolorektale Karzinome, Analkarzinome, Magenkarzinome, Pankreaskarzinome, Ösophagukarzinome, Gallenblasenkarzinome, Dünndarmkarzinome, Speicheldrüsenkarzinome, Neuroendokrine Tumore und Gastrointestinale Stromatumore.

Als Tumore des Urogenital-Traktes sind beispielsweise behandelbar Harnblasenkarzinome, Nierenzellkarzinome, und Karzinome des Nierenbeckens und der ableitenden Harnwege.

Als Tumore des Auges sind beispielsweise behandelbar Retinoblastome und Intraokulare Melanome

Als Tumore der Leber sind beispielsweise behandelbar Hepatozelluläre Karzinome und Cholangiozelluläre Karzinome.

Als Tumore der Haut sind beispielsweise behandelbar Maligne Melanome, Basaliome, Spinaliome, Kaposi-Sarkome und Merkelzellkarzinome.

Als Tumore des Kopfes und Halses sind beispielsweise behandelbar Larynxkarzinome und Karzinome des Pharynx und der Mundhöhle.

Als Sarkome sind beispielsweise behandelbar Weichteilsarkome und Osteosarkome.

Als Lymphome sind beispielsweise behandelbar Non-Hodgkin-Lymphome, Hodgkin-Lymphome, Kutane Lymphome, Lymphome des zentralen Nervensystems und AIDS-assoziierte Lymphome.

Als Leukämien sind beispielsweise behandelbar Akute myeloische Leukämien, Chronische myeloische Leukämien, Akute lymphatische Leukämien, Chronische lymphatische Leukämien und Haarzellleukämien.

Vorteilhaft können die erfindungsgemäßen Verbindungen verwendet werden zur Prophylaxe und/oder Therapie von Leukämien, insbesondere akuten myeolischen Leukämien, Prostatakarzinomen, insbesondere Androgenrezeptor-positiven Prostatakarzinomen, Zervixkarzinomen, Mammakarzinomen, insbesondere von Hormonrezeptor-negativen, Hormonrezeptor-positiven oder BRCA-assoziierten Mammakarzinomen, Pankreaskarzinomen, Nierenzellkarzinomen, Hepatozellulären Karzinomen, Melanomen und anderen Hauttumoren, Nicht-Kleinzelligen Bronchialkarzinomen, Endometriumkarzinomen und Kolorektalen Karzinomen.

Besonders vorteilhaft können die erfindungsgemäßen Verbindungen verwendet werden zur Prophylaxe und/oder Therapie von akuten myeloischen Leukämien, Prostatakarzinomen, insbesondere Androgenrezeptor-positiven Prostatkarzinomen, Zervixkarzinomen, Mammakarzinomen, insbesondere Estrogen-alpha-positiven und Estrogen-alpha-negativen Mammakarzinomen, Prostatkarzinomen oder Melanomen.

Diese Erkrankungen sind gut charakterisiert im Menschen, existieren aber auch bei anderen Säugetieren.

Ein weiterer Gegenstand der vorliegenden Erfindung ist die Verwendung der erfindungsgemäßen Verbindungen zur Herstellung eines Arzneimittels.

Ein weiterer Gegenstand der vorliegenden Erfindung ist die Verwendung der erfindungsgemäßen Verbindungen zur Herstellung eines Arzneimittels zur Prophylaxe und/oder Therapie von Tumorerkrankungen.

Ein weiterer Gegenstand der vorliegenden Erfindung ist die Verwendung der erfindungsgemäßen Verbindungen zur Herstellung eines Arzneimittels zur Prophylaxe und/oder Therapie von Leukämien, insbesondere akuten myeolischen Leukämien, Prostatakarzinomen, insbesondere Androgenrezeptor-positiven Prostatakarzinomen, Zervixkarzinomen, Mammakarzinomen, insbesondere von Hormonrezeptor-negativen, Hormonrezeptor-positiven oder BRCA-assoziierten Mammakarzinomen, Pankreaskarzinomen, Nierenzellkarzinomen, Hepatozellulären Karzinomen, Melanomen und anderen Hauttumoren, Nicht-Kleinzelligen Bronchialkarzinomen, Endometriumkarzinomen und Kolorektalen Karzinomen.

Ein weiterer Gegenstand der vorliegenden Erfindung ist die Verwendung der erfindungsgemäßen Verbindungen zur Herstellung eines Arzneimittels zur Prophylaxe und/oder Therapie akuten myeloischen Leukämien, Prostatakarzinomen, insbesondere Androgenrezeptor-positiven Prostatkarzinomen, Zervixkarzinomen, Mammakarzinomen, insbesondere Estrogen-alphapositiven und Estrogen-alpha-negativen Mammakarzinomen, Prostatkarzinomen oder Melanomen.

Ein weiterer Gegenstand der vorliegenden Erfindung sind pharmazeutische Formulierungen in Form von Tabletten enthaltend eine der erfindungsgemäßen Verbindungen zur Prophylaxe und/oder Therapie von Leukämien, insbesondere akuten myeolischen Leukämien, Prostatakarzinomen, insbesondere Androgenrezeptor-positiven Prostatakarzinomen, Zervixkarzinomen, Mammakarzinomen, insbesondere von Hormonrezeptor-negativen, Hormonrezeptor-positiven oder BRCA-assoziierten Mammakarzinomen, Pankreaskarzinomen, Nierenzellkarzinomen, Hepatozellulären Karzinomen, Melanomen und anderen Hauttumoren, Nicht-Kleinzelligen Bronchialkarzinomen, Endometriumkarzinomen und Kolorektalen Karzinomen.

Ein weiterer Gegenstand der vorliegenden Erfindung sind pharmazeutische Formulierungen in Form von Tabletten enthaltend eine der erfindungsgemäßen Verbindungen zur Prophylaxe und/oder Therapie von akuten myeloischen Leukämien, Prostatakarzinomen, insbesondere Androgenrezeptor-positiven Prostatkarzinomen, Zervixkarzinomen, Mammakarzinomen, insbesondere Estrogen-alpha-positiven und Estrogen-alpha-negativen Mammakarzinomen, Prostatkarzinomen oder Melanomen.

Die erfindungsgemäßen Verbindungen eignen sich auch zur Prophylaxe und/oder Therapie von systemischen inflammatorischen Krankheiten, insbesondere LPS-induzierter endotoxischer Schock und/oder Bakterien-induzierte Sepsis.

Die erfindungsgemäßen Verbindungen eignen sich auch zur Prophylaxe und/oder Therapie von inflammatorischen oder Autoimmunerkrankungen wie zum Beispiel:
- Lungenerkrankungen, die mit entzündlichen, allergischen und/oder proliferativen Prozessen einhergehen: chronisch obstruktive Lungenerkrankungen jeglicher Genese, vor allem Asthma bronchiale; Bronchitis unterschiedlicher Genese; alle Formen der restriktiven Lungenerkrankungen, vor allem allergische Alveolitis; alle Formen des Lungenödems, vor allem toxisches Lungenödem; Sarkoidosen und Granulomatosen, insbesondere Morbus Boeck
- Rheumatische Erkrankungen/Autoimmunerkrankungen/Gelenkerkrankungen, die mit entzündlichen, allergischen und/oder proliferativen Prozessen einhergehen: alle Formen rheumatischer Erkrankungen, insbesondere rheumatoide Arthritis, akutes rheumatisches Fieber, Polymyalgia rheumatica; reaktive Arthritis; entzündliche Weichteilerkrankungen sonstiger Genese; arthritische Symptome bei degenerativen Gelenkerkankungen (Arthrosen); traumatische Arthritiden; Kollagenosen jeglicher Genese, z.B. systemischer Lupus erythematodes, Sklerodermie, Polymyositis, Dermatomyositis, Sjögren-Syndrom, Still-Syndrom, Felty-Syndrom
- Allergien, die mit entzündlichen und/oder proliferativen Prozessen einhergehen: alle Formen allergischer Reaktionen, z.B. Quincke Ödem, Heuschnupfen, Insektenstich, allergische Reaktionen auf Arzneimittel, Blutderivate, Kontrastmittel etc., anaphylaktischer Schock, Urtikaria, Kontaktdermatitis
- Gefäßentzündugen (Vaskulitiden): Panarterilitis nodosa, Arterilitis temporalis, Erythema nodosum
- Dermatologische Erkrankungen, die mit entzündlichen, allergischen und/oder proliferativen Prozessen einhergehen: atopische Dermatitis; Psoriasis; Pityriasis rubra pilaris; erythematöse Erkrankungen, ausgelöst durch unterschiedliche Noxen, z.B. Strahlen, Chemikalien, Verbrennungen etc.; bullöse Dermatosen; Erkrankungen des lichenoiden Formenkreises; Pruritus; Seborrhoisches Ekzem; Rosacea; Pemphigus vulgaris; Erythema exsudativum multiforme; Balanitis; Vulvitis; Haarausfall wie Alopecia areata; kutane T-Zell Lymphome
- Nierenerkrankungen, die mit entzündlichen, allergischen und/oder proliferativen Prozessen einhergehen: nephrotisches Syndrom; alle Nephritiden
- Lebererkrankungen, die mit entzündlichen, allergischen und/oder proliferativen Prozessen einhergehen: akuter Leberzellzerfall; akute Hepatitis unterschiedlicher Genese, z.B. viral, toxisch, arneimittelinduziert; chronisch aggressive und/oder chronisch intermittierende Hepatitis
- Gastrointestinale Erkrankungen, die mit entzündlichen, allergischen und/oder proliferativen Prozessen einhergehen: regionale Enteritis (Morbus Crohn); Colitis ulcerosa; Gastritis; Refluxoesophagitis; Gastroenteritiden anderer Genese, z.B. einheimische Sprue
- Proktologische Erkrankungen, die mit entzündlichen, allergischen und/oder proliferativen Prozessen einhergehen: Analekzem; Fissuren; Hämorrhoiden; idiopatische Proktitis
- Augenerkrankungen, die mit entzündlichen, allergischen und/oder proliferativen Prozessen einhergehen: allergische Keratitis, Uveitis, Iritis; Konjuktivitis; Blepharitis; Neuritis nervi optici; Chlorioditis; Opthalmia sympathica
- Erkrankungen des Hals-Nasen-Ohren-Bereiches, die mit entzündlichen, allergischen und/oder proliferativen Prozessen einhergehen: allergische Rhinitis, Heuschnupfen; Otitis externa, z.B. bedingt durch Kontaktexem, Infektion etc.; Otitis media
- Neurologische Erkrankungen, die mit entzündlichen, allergischen und/oder proliferativen Prozessen einhergehen: Hirnödem, vor allem Tumor-bedingtes Hirnödem; Multiple Sklerose; akute Encephalomyelitis; Meningitis; verschiedene Formen von Krampfanfällen, z.B. BNS-Krämpfe
- Bluterkrankungen, die mit entzündlichen, allergischen und/oder proliferativen Prozessen einhergehen: erworbene hämolytische Anämie; idiopathische Thrombozytopenie
- Tumorerkrankungen, die mit entzündlichen, allergischen und/oder proliferativen Prozessen einhergehen: akute lymphatische Leukämie; maligne Lymphome; Lymphogranulomatosen; Lymphosarkome; ausgedehnte Metastasierungen, vor allem bei Mamma-, Bronchial- und Prostatakarzinom
- Endokrine Erkrankungen, die mit entzündlichen, allergischen und/oder proliferativen Prozessen einhergehen: endokrine Orbitopathie; thyreotoxische Krise; Thyreoditis de Quervain; Hashimoto Thyreoditis; Morbus Basedow
- Organ- und Gewebstransplantationen, Graft-versus-Host disease
- Schwere Schockzuständen, z.B. anaphylaktischer Schock, systemic inflammatory response syndrome (SIRS)
- Substitutionstherapie bei: angeborene primäre Nebenniereninsuffizienz, z.B. kongenitales adrenogenitales Syndrom; erworbene primäre Nebenniereninsuffizienz, z.B. Morbus Addison, autoimmune Adrenalitis, postinfektiös, Tumoren, Metastasen, etc; angeborne sekundäre Nebenniereninsuffizienz, z.B. kongenitaler Hypopituitarismus; erworbene sekundäre Nebenniereninsuffizenz, z.B. postinfektiös, Tumoren, etc
- Emesis, die mit entzündlichen, allergischen und/oder proliferativen Prozessen einhergehen, z.B. in Kombination mit einem 5-HT3-Antagonisten bei Zytostatika-bedingten Erbrechen
- Schmerzen bei entzündlicher Genese, z.B. Lumbago

Die erfindungsgemäßen Verbindungen eignen sich auch für die Behandlung von viralen Erkrankungen, wie zum Beispiel Infektionen die verursacht sind durch Papilloma-Viren, Herpes-Viren, Epstein-Barr-Viren, Hepatitis B- oder C-Viren, und humane Immunschwäche-Viren.

Die erfindungsgemäßen Verbindungen eignen sich auch für die Behandlung von Atherosklerose, Dyslipidemie, Hypercholesterolemie, Hypertriglyceridämie, perifere Gefäßerkrankungen, kardiovaskuläre Erkrankungen, Angina pectoris Ischemie, Schlaganfall, Myokardinfarkt, angioplastische Restenose, Bluthochdruck, Thrombose, Adipositas, Endotoxemie.

Die erfindungsgemäßen Verbindungen eignen sich auch für die Behandlung von neurodegenerativen Krankheiten wie zum Beispiel multiple Sklerose, Alzheimer's Krankheit und Parkinson's Krankheit.

Die erfindungsgemäßen Verbindungen eignen sich auch zur Prophylaxe und/oder Therapie von benignen hyperproliferativen Krankheiten wie zum Beispiel Endometriose, Leiomyom und benigne Prostatahyperplasie.

Die vorliegende Erfindung betrifft somit auch die Verwendung der erfindungsgemäßen Verbindungen zur Herstellung eines Arzneimittels zur Prophylaxe und/ oder Therapie von viralen Infektionen, neurodegenerativen Erkrankungen, inflammatorischen Erkrankungen, atherosklerotischen Erkrankungen und in der männlichen Fertilitätskontrolle.

Die erfindungsgemäßen Verbindungen können allein oder bei Bedarf in Kombination mit einer oder mit mehreren weiteren pharmakologisch wirksamen Substanzen eingesetzt werden, solange diese Kombination nicht zu unerwünschten und inakzeptablen Nebenwirkungen führt. Ein weiterer Gegenstand der vorliegenden Erfindung sind daher Arzneimittel, enthaltend eine der erfindungsgemäßen Verbindungen und einen oder mehrere weitere Wirkstoffe, insbesondere zur Prophylaxe und/oder Therapie der zuvor genannten Erkrankungen.

Beispielsweise können die erfindungsgemäßen Verbindungen mit bekannten anti-hyperproliferativen, zytostatischen oder zytotoxischen Substanzen zur Behandlung von Krebserkrankungen kombiniert werden. Die Kombination der erfindungsgemäßen Verbindungen mit anderen für die Krebstherapie gebräuchlichen Substanzen oder auch mit der Strahlentherapie ist besonders angezeigt.

Als geeignete Kombinationswirkstoffe seien beispielhaft genannt:
Afinitor, Aldesleukin, Alendronsäure, Alfaferon, Alitretinoin, Allopurinol, Aloprim, Aloxi, Altretamin, Aminoglutethimid, Amifostin, Amrubicin, Amsacrin, Anastrozol, Anzmet, Aranesp, Arglabin, Arsentrioxid, Aromasin, 5-Azacytidin, Azathioprin, BCG oder tice-BCG, Bestatin, Betamethason-Acetat, Betamethason-Natriumphosphat, Bexaroten, Bleomycin-Sulfat, Broxuridin, Bortezomib, Busulfan, Calcitonin, Campath, Capecitabin, Carboplatin, Casodex, Cefeson, Celmoleukin, Cerubidin, Chlorambucil, Cisplatin, Cladribin, Clodronsäure, Cyclophosphamid, Cytarabin, Dacarbazin, Dactinomycin, DaunoXome, Decadron, Decadron-Phosphat, Delestrogen, Denileukin Diftitox, Depomedrol, Deslorelin, Dexrazoxan, Diethylstilbestrol, Diflucan, Docetaxel, Doxifluridin, Doxorubicin, Dronabinol, DW-166HC, Eligard, Elitek, Ellence, Emend, Epirubicin, Epoetin-alfa, Epogen, Eptaplatin, Ergamisol, Estrace, Estradiol, Estramustin-Natriumphosphat, Ethinylestradiol, Ethyol, Etidronsäure, Etopophos, Etoposid, Fadrozol, Farston, Filgrastim, Finasterid, Fligrastim, Floxuridin, Fluconazol, Fludarabin, 5-Fluordeoxyuridin-Monophosphat, 5-Fluoruracil (5-FU), Fluoxymesteron, Flutamid, Formestan, Fosteabin, Fotemustin, Fulvestrant, Gammagard, Gemcitabin, Gemtuzumab, Gleevec, Gliadel, Goserelin, Granisetron-Hydrochlorid, Histrelin, Hycamtin, Hydrocorton, erythro-Hydroxynonyladenin, Hydroxyharnstoff, Ibritumomab Tiuxetan, Idarubicin, Ifosfamid, Interferon-alpha, Interferon-alpha-2, Interferon-alpha-2α, Interferon-alpha-2β, Interferon-alpha-n1, Interferon-alpha-n3, Interferon-beta, Interferon-gamma-1α, Interleukin-2, Intron A, Iressa, Irinotecan, Kytril, Lapatinib, Lentinan-Sulfat, Letrozol, Leucovorin, Leuprolid, Leuprolid-Acetat, Levamisol, Levofolinsäure-Calciumsalz, Levothroid, Levoxyl, Lomustin, Lonidamin, Marinol, Mechlorethamin, Mecobalamin, Medroxyprogesteron-Acetat, Megestrol-Acetat, Melphalan, Menest, 6-Mercaptopurin, Mesna, Methotrexat, Metvix, Miltefosin, Minocyclin, Mitomycin C, Mitotan, Mitoxantron, Modrenal, Myocet, Nedaplatin, Neulasta, Neumega, Neupogen, Nilutamid, Nolvadex, NSC-631570, OCT-43, Octreotid, Ondansetron-Hydrochlorid, Orapred, Oxaliplatin, Paclitaxel, Pediapred, Pegaspargase, Pegasys, Pentostatin, Picibanil, Pilocarpin-Hydrochlorid, Pirarubicin, Plicamycin, Porfimer-Natrium, Prednimustin, Prednisolon, Prednison, Premarin, Procarbazin, Procrit, Raltitrexed, RDEA119, Rebif, Regorafenib, Rhenium-186-Etidronat, Rituximab, Roferon-A, Romurtid, Salagen, Sandostatin, Sargramostim, Semustin, Sizofiran, Sobuzoxan, Solu-Medrol, Streptozocin, Strontium-89-chlorid, Synthroid, Tamoxifen, Tamsulosin, Tasonermin, Tastolacton, Taxoter, Teceleukin, Temozolomid, Teniposid, Testosteron-Propionat, Testred, Thioguanin, Thiotepa, Thyrotropin, Tiludronsäure, Topotecan, Toremifen, Tositumomab, Tastuzumab, Teosulfan, Tretinoin, Trexall, Trimethylmelamin, Trimetrexat, Triptorelin-Acetat, Triptorelin-Pamoat, UFT, Uridin, Valrubicin, Vesnarinon, Vinblastin, Vincristin, Vindesin, Vinorelbin, Virulizin, Zinecard, Zinostatin-Stimalamer, Zofran; ABI-007, Acolbifen, Actimmun, Affinitak, Aminopterin, Arzoxifen, Asoprisnil, Atamestan, Atrasentan, BAY 43-9006 (Sorafenib), Avastin, CCI-779, CDC-501, Celebrex, Cetuximab, Crisnatol, Cyproteron-Acetat, Decitabin, DN-101, Doxorubicin-MTC, dSLIM, Dutasterid, Edotecarin, Eflornithin, Exatecan, Fenretinid, Histamin-Dihydrochlorid, Histrelin-Hydrogel-Implant, Holmium-166-DOTMP, Ibandronsäure, Interferon-gamma, Intron-PEG, Ixabepilon, Keyhole Limpet-Hemocyanin, L-651582, Lanreotid, Lasofoxifen, Libra, Lonafarnib, Miproxifen, Minodronat, MS-209, liposomales MTP-PE, MX-6, Nafarelin, Nemorubicin, Neovastat, Nolatrexed, Oblimersen, Onko-TCS, Osidem, Paclitaxel-Polyglutamat, Pamidronat-Dinatrium, PN-401, QS-21, Quazepam, R-1549, Raloxifen, Ranpirnas, 13-*cis*-Retinsäure, Satraplatin, Seocalcitol, T-138067, Tarceva, Taxoprexin, Thymosin-alpha-1, Tiazofurin, Tipifarnib, Tirapazamin, TLK-286, Toremifen, TransMID-107R, Valspodar, Vapreotid, Vatalanib, Verteporfin, Vinflunin, Z-100, Zoledronsäure, sowie Kombinationen hiervon.

In einer bevorzugten Ausführungsform können die erfindungsgemäßen Verbindungen mit antihyperproliferativen Agentien kombiniert werden, welche beispielhaft - ohne dass diese Aufzählung abschließend wäre - sein können:

Aminoglutethimid, L-Asparaginase, Azathioprin, 5-Azacytidin, Bleomycin, Busulfan, Carboplatin, Carmustin, Chlorambucil, Cisplatin, Colaspase, Cyclophosphamid, Cytarabin, Dacarbazin, Dactinomycin, Daunorubicin, Diethylstilbestrol, 2',2'-Difluordeoxycytidin, Docetaxel, Doxorubicin (Adriamycin), Epirubicin, Epothilon und seine Derivate, erythro-Hydroxynonyladenin, Ethinylestradiol, Etoposid, Fludarabin-Phosphat, 5-Fluordeoxyuridin, 5-Fluordeoxyuridin-Monophosphat, 5-Fluoruracil, Fluoxymesteron, Flutamid, Hexamethylmelamin, Hydroxyharnstoff, Hydroxyprogesteron-Caproat, Idarubicin, Ifosfamid, Interferon, Irinotecan, Leucovorin, Lomustin, Mechlorethamin, Medroxyprogesteron-Acetat, Megestrol-Acetat, Melphalan, 6-Mercaptopurin, Mesna, Methotrexat, Mitomycin C, Mitotan, Mitoxantron, Paclitaxel, Pentostatin, N-Phosphonoacetyl-L-aspartat (PALA), Plicamycin, Prednisolon, Prednison, Procarbazin, Raloxifen, Semustin, Streptozocin, Tamoxifen, Teniposid, Testosteron-Propionat, Thioguanin, Thiotepa, Topotecan, Trimethylmelamin, Uridin, Vinblastin, Vincristin, Vindesin und Vinorelbin.

In viel versprechender Weise lassen sich die erfindungsgemäßen Verbindungen auch mit biologischen Therapeutika wie Antikörpern (z.B. Avastin, Rituxan, Erbitux, Herceptin) und rekombinanten Proteinen kombinieren.

Die erfindungsgemäßen Verbindungen können auch in Kombination mit anderen, gegen die Angiogenese gerichteten Therapien positive Effekte erzielen, wie zum Beispiel mit Avastin, Axitinib, Regorafenib, Recentin, Sorafenib oder Sunitinib. Kombinationen mit Inhibitoren des Proteasoms und von mTOR sowie Antihormone und steroidale metabolische Enzyminhibitoren sind wegen ihres günstigen Nebenwirkungsprofils besonders geeignet.

Auch die Kombination der erfindungsgemässen Verbindungen mit einem P-TEFb oder CDK9 Inhibitor ist besonders angezeigt.

Darüber hinaus können die erfindungsgemäßen Verbindungen auch in Verbindung mit einer Strahlentherapie und/oder einer chirurgischen Intervention eingesetzt werden.

### In der vorliegenden Beschreibung bedeuten:

- ACN: Acetonitril
- CDCl₃: Deuterochloroform
- CHAPS: 3-{Dimethyl[3-(4-{5,9,16-trihydroxy-2,15-dimethyltetracyclo [8.7.0.0^{2.7}.0^{11,15}]heptadecan-14-yl}pentanamido)propyl]-azaniumyl}propan-1-sulfonat
- DEA: Diethylamin
- DMF: N,N-Dimethylformamid
- DMSO-d6: deuteriertes Dimethylsulfoxid
- DMSO: Dimethylsulfoxid
- EE: Ethylacetat
- EtOH: Ethanol
- ges.: gesättigt
- HATU: (7-Aza-1H-benzotriazole-1-yl)-1,1,3,3-tetramethyluronium hexafluorophosphat
- HBTU: O-Benzotriazol-N,N,N',N' -tetramethyl-uronium-hexafluor-phosphat
- HPLC: Hochdruckflüssigkeitschromatographie
- KHMDS: Kalium-bis(trimethylsilyl)amid
- KOtBu: Kalium-*tert*-butanolat
- LCMS: Flüssigchromatographie gekoppelt mit Massenspektronmetrie
- LDA: Lithium-di-*iso*-propylamid
- LiHMDS: Lithium-bis(trimethylsilyl)amid
- MeCl: Methylenchlorid, Dichlormethan
- MTBE: Methyl-*tert*-butylether
- PyBOB: (Benzotriazol-1-yl)-oxytripyrrolidinophosphonium hexafluorophosphat)
- RP-HPLC: Reversed Phase Hochdruckflüssigkeitschromatographie
- RT: Raumtemperatur
- sept: Septett
- T3P: 2,4,6-Tripropyl-1,3,5,2,4,6-trioxatriphosphorinan-2,4,6-trioxid
- TFA: Trifluoressigsäure
- THF: Tetrahydrofuran

### Herstellung der erfindungsgemäßen Verbindungen der allgemeinen Formel I

### Allgemeine Beschreibung der Herstellung der erfindungsgemäßen Verbindungen der allgemeinen Formel I

Die in diesem Abschnitt in den Schemata 4, 5, 6, 7a und 7b gezeigten erfindungsgemäßen Verbindungen der Formeln (Ia), (Ib), (Ic), (Id), (Ie), (If), (Ig), (Ih) sowie (Ii) lassen sich herstellen über Synthesewege, die im Folgenden beschrieben werden. Die genannten Formeln stellen verschiedene Teilmengen der allgemeinen Formel (I) dar, in denen R¹, R², R³ und R⁴, sofern im Zusammenhang mit den genannten Schemata nicht anderslautend beschrieben, definiert sind wie für die allgemeine Formel (I).

Zusätzlich zu den nachfolgend besprochenen Synthesequenzen können, entsprechend den allgemeinen Kenntnissen des Fachmannes in der Organischen Chemie, auch weitere Synthesewege für die Synthese von erfindungsgemäßen Verbindungen der allgemeinen Formel (I) beschritten werden. Die Reihenfolge der in den nachfolgenden Schemata gezeigten Syntheseschritte ist nicht bindend, und Syntheseschritte aus verschiedenen der nachfolgend gezeigten Schemata können gegebenenfalls zu neuen Sequenzen kombiniert werden. Zusätzlich können Interkonversionen der Substituenten R¹, R², R³ oder R⁴ vor oder nach den gezeigten Synthesestufen durchgeführt werden. Beispiele für solche Umwandlungen sind die Einführung oder Abspaltung von Schutzgruppen, Reduktion oder Oxidation funktioneller Gruppen, Halogenierung, Metallierung, metallkatalysierte Kupplungsreaktionen, Substitutionsreaktionen oder weitere dem Fachmann bekannte Umsetzungen. Als explizites Beispiel sei an dieser Stelle die Umwandlung von R¹ von Brom in Dimethylisooxazolyl- durch Suzuki-Kupplung genannt, wie sie im Experimentalteil für die Synthese von Beispiel 48 beschrieben ist.

Diese Reaktionen schließen Umsetzungen ein, welche eine funktionelle Gruppe einführen, die weitere Umwandlung von Substituenten ermöglicht. Geeignete Schutzgruppen sowie Methoden zu ihrer Einführung und Abspaltung sind dem Fachmann bekannt (siehe z.B. T.W. Greene und P.G.M. Wuts in: Protective Groups in Organic Synthesis, 3. Auflage, Wiley 1999). Weiterhin ist die Zusammenfassung zweier oder mehrerer Reaktionsschritte ohne zwischenzeitliche Aufarbeitung in einer dem Fachmann bekannten Weise möglich (z.B. in sogenannten "Eintopf "-Reaktionen).

Schema 1 zeigt die Synthese von Zwischenprodukten der Formel (IX) aus Benzazepindionen der Formel (II), in denen R¹ definiert ist wie in der allgemeinen Formel (I). Diese werden mit einem für die Überführung des Lactams in ein Thiolactam geeigneten Reagens, beispielsweise Lawesson's Reagenz (2,4-Bis-(4-methoxyphenyl)-1,3-dithia-2,4-diphosphetan-2,4-disulfid, CAS# 19172-47-5), in Verbindungen der Formel (III) überführt (z.B. US 5,696,111, 1997, Beispiel 9.A, Schritt A).

Diese werden mit Acylhydrazinen der Formel (IV), in denen R² definiert ist wie in der allgemeinen Formel (I), und vorzugsweise für Methyl steht, unter Aufbau eines Triazolringes zu tricyclischen Zwischenprodukten der Formel (V) umgesetzt (Nature 2010, Vol 468, p1067ff, SI, p33-36; Filippakopoulos et al.). Dabei wird intermediär aus der Carbonylgruppe ein Acylhydrazon gebildet, welches durch saure Hydrolyse unter Rückbildung der Carbonylgruppe zersetzt wird. Die Carbonylgruppe in (V) wird nachfolgend in dem Fachmann bekannter Weise (siehe z.B. J. Org. Chem. 58, (1993), S. 600-10, W. Okamura; Org. Lett. 9, (2007), S. 517-20, A. de Meijere, Eur. J. Org. Chem. 8, (1998), S. 1521-34, K. Voigt et al.) durch Umsetzung mit Verbindungen der Formel (VI), in denen LG für eine Abgangsgruppe, bevorzugt Halogen, besonders bevorzugt Fluor, steht und R^{F} für einen perfluorierten C₁-C₆-Alkylrest, bevorzugt Trifluormethyl, 1,1,2,2,2-Pentafluoroethyl oder 1,1,2,2,3,3,4,4,4-Nonafluoro-*n*-butyl- steht, und einer geeigneten Base wie Lithiumhexamethyldisilazid, in ein Enolsulfonat unter Bildung von Verbindungen der Formel (VII) überführt. Dieses wird in einer Suzuki-Kupplung mit Boronsäurederivaten der Formel (VIII) umgesetzt, in denen R⁴ definiert ist wie in der allgemeinen Formel (I), und worin R^{B} für C₁-C₄-Alkyl steht, oder beide R^{B} gemeinsam mit den Sauerstoff-Atomen, an die sie gebunden sind, einen cyclischen Boronsäureester, vorzugsweise ein Pinakolat, bilden. Dabei wird ein PalladiumKatalysator verwendet, beispielsweise Pd(0)-Katalysatoren wie Tetrakis(triphenylphosphin)palladium(0) [Pd(PPh₃)₄], Tris(dibenzylidenaceton)di-palladium(0) [Pd₂(dba)₃], oder Pd(II)-Katalysatoren wie Dichlorbis(triphenylphosphin)-palladium(II) [Pd(PPh₃)₂Cl₂], Palladium(II)-acetat in Kombination mit Triphenylphosphin, oder [1,1'-Bis(diphenylphosphino)ferrocen]palladiumdichlorid [Pd(dppf)Cl₂], und es entstehen die Zwischenprodukte der allgemeinen Formel (IX). Boronsäurederivate der Formel (VIII) sind vielfach käuflich oder können mit dem Fachmann bekannten Methoden hergestellt werden (für eine Übersicht siehe D.G. Hall, Boronic Acids, 2005 WILEY-VCH Verlag GmbH & Co. KGaA, Weinheim, ISBN 3-527-30991-8, und die darin zitierte Literatur). Suzuki-Kupplungen sind dem Fachmann bekannt (für einige Beispiele siehe z. B. Eur. J. Chem 13, 2007, S. 2410-20, H.-U. Reissig et al., Tetrahedron 52, (1996), S. 1529-42, A. Cleve et al.). Die Umsetzung erfolgt bevorzugt unter erhöhter Temperatur, z.B. in der Siedehitze eines entsprechenden Lösungsmittels wie Toluol.

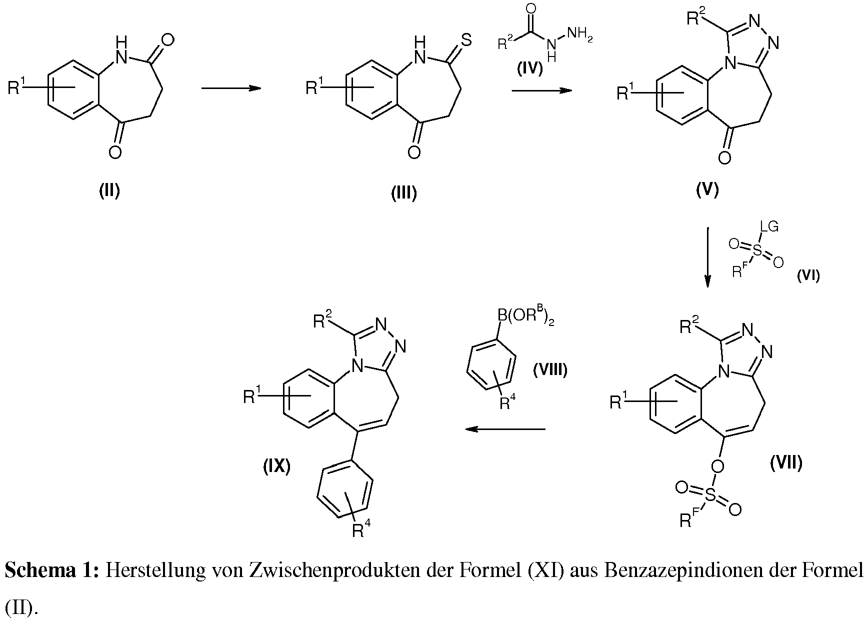

Die als Einsatzstoffe verwendeten Benzazepindione der Formel (II) sind literaturbekannt (siehe beispielsweise Arch. Pharm. 324, (1991), p. 579-81, C. Kunick; Arch. Pharm. 335, (2002), S. 311-317, K. Wiekig et al.,) oder können alternativ auch aus Aminobenzoesäure-Estern der Formel (X), in der R¹ definiert ist wie in der allgemeinen Formel (I), und worin R^{E} für C₁-C₆-Alkyl oder Benzyl steht, hergestellt werden. Dabei werden die Verbindungen (X) mit Succinat-Reagentien der Formel (XI), in denen LG für eine Abgangsgruppe, beispielsweise Halogen und bevorzugt Chlor steht, und worin R^{E} für C₁-C₆-Alkyl oder Benzyl steht, in Gegenwart einer geeigneten Base wie beispielsweise Pyridin umgesetzt, wobei Benzamide der Formel (XII) gebildet werden. Diese können in einer Dieckmann-Zyklisierung in Gegenwart von DMSO und einer geeigneten Base, beispielsweise eines Alkalimetall-Alkoholates wie Kalium-*tert*-butylat, zu Enolestern der Formel (XIII) transformiert werden; Decarboxylierung in DMSO bei erhöhter Temperatur führt dann zu den Benzazepindionen der Formel (II); siehe dazu auch Arch. Pharm. 324, (1991), p. 579-81, C. Kunick; Arch. Pharm. 335, (2002), S. 311-317, K. Wiekig et al.

Die Aminobenzoesäure-Ester der Formel (X) sowie die Succinat-Reagentien der Formel (XI) sind dem Fachmann bekannt und vielfach kommerziell erhältlich.

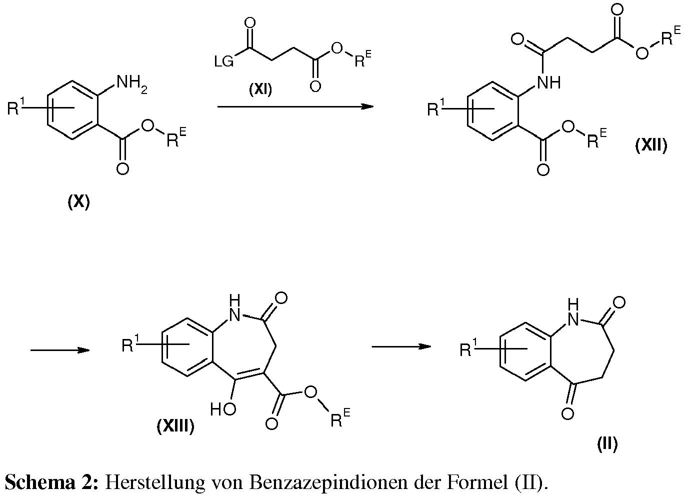

Alternativ können die Zwischenprodukte der Formel (IX) auch, wie in Schema 3 gezeigt, hergestellt werden aus Phenyl-1,3,4,5-tetrahydro-2H-1-benzazepin-2-onen der Formel (XIV), deren Herstellung beispielsweise beschrieben ist in US 5,484,917 sowie in J. Med. Chem. 37, (1994), S. 3789ff, J. Lowe et al., und in denen R¹ und R⁴ definiert sind wie in der allgemeinen Formel (I), die unter Reaktion mit Brom in Anwesenheit einer Lichtquelle, z.B. einer Glühlampe, umgesetzt werden (US4464300A1, 1984, Beispiel 5.f). Dabei entstehen unter nachfolgender Eliminierung von Bromwasserstoff die Verbindungen der Formel (XV). Diese werden wie in Schema 1 für Verbindungen der Formel (II) gezeigt, mit einem Sulfurierungsreagenz, beispielsweise Lawesson's Reagenz, zu Thiolactamen der Formel (XVI) umgesetzt. Abschließend werden diese, in analoger Weise wie in Schema 1 für die Umsetzung von Verbindungen der Formel (III) zu (V) beschrieben, mit Acylhydrazinen der Formel (IV) unter Aufbau des Triazol-Ringes in die Zwischenprodukte der Formel (IX) überführt umgesetzt (Nature 2010, Vol 468, p1067ff, SI, p33-36; Filippakopoulos et al.). Eine saure Hydrolyse intermediärer Acylhydrazone wie oben beschrieben ist in diesem Fall mangels einer Carbonylgruppe im Substrat (XVI) aber entbehrlich.

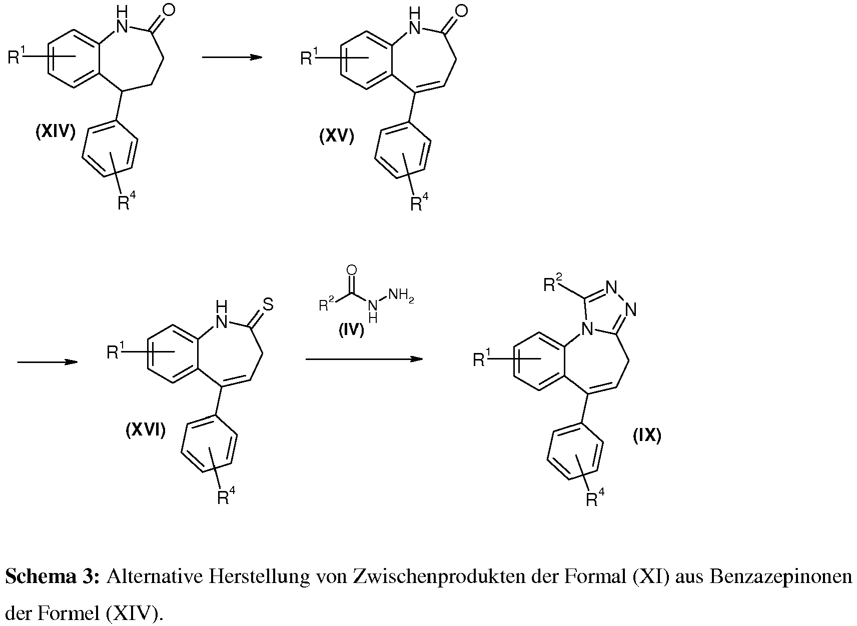

Wie in Schema 4 illustriert, können die Zwischenprodukte der Formel (IX), in denen R¹, R² und R⁴ definiert sind wie in der allgemeinen Formel (I), weiter zu den erfindungsgemäßen Verbindungen der Formeln (Ia), (Ib), (Ic) und (Id), die sämtlich Untergruppen der allgemeinen Formel (I) darstellen, umgesetzt werden. Die Isolierung des (R)-Enantiomers kann dabei auf verschiedenen Stufen mit dem Fachmann bekannten Methoden, beispielsweise chiraler präparativer HPLC, erfolgen. Dazu werden die Zwischenprodukte der Formel (IX) mit Essigsäurederivaten der Formel (XVII), in denen R⁸ definiert wie in der allgemeinen Formel (I), aber von Wasserstoff verschieden ist, und worin LG für eine Abgangsgruppe, beispielsweise Halogen, bevorzugt Chlor oder Brom, steht, in Gegenwart einer geeigneten Base, beispielsweise Lithiumdiisopropylamin, Kalium-*tert-*butylat, Lithiumhexamethyldisilazid, Natriumhexamethyldisilazid oder Natiumhydrid, zu Estern der Formel (Ia) umgesetzt. An in chemischer Hinsicht ähnlichen Systemen ist die Einführung einer kohlenstoffhaltigen Seitenkette in ähnlicher Weise beschrieben, siehe beispielsweise H. Tabata et al., Org. Lett. 2008, 10, S. 4871ff. Die genannten Ester der Formel (Ia) können durch dem Fachmann geläufige Methoden, beispielsweise durch basische Hydrolyse mit beispielsweise mit wäßrigen Alkylihydroxiden oder durch saure Hydrolyse beispielsweise mit Chlorwasserstoff in Dioxan oder Trifluoressigsäure, in die Carbonsäuren (Ib) überführt werden. Diese lassen sich durch Kupplung mit Aminen der Formel (XVIII), in denen R⁶ und R⁷ definiert sind wie in der allgemeinen Formel (I), oder mit cyclischen Aminen der Formel (XIX), in denen R⁹ definiert ist wie in der allgemeinen Formel (I) mit der Maßgabe, dass die Verknüpfung an das in Formel (XIX) gezeichnete Wasserstoffatom über ein Stickstoffatom erfolgt, in Gegenwart eines geeigneten Kupplungsreagenz in die erfindungsgemäßen Carboxamide der Formeln (Ic) und (Id) überführen. Kupplungsreagentien und Methoden für derartige Synthesen von Carboxamiden aus Carbonsäuren und Aminen sind dem Fachmann bekannt. Als Beispiele seien hier genannt die Verwendung von HATU, HBTU, PyBOB oder T3P unter Zusatz einer geeigneten Base. Die Umwandlung der Carbonsäuren in ihre Amide wird allgemein beschrieben in Referenzbüchern wie "Compendium of Organic Synthetic Methods", Band I-VI (Wiley Interscience) oder "The Practice of Peptide Synthesis", Bodansky (Springer Verlag).

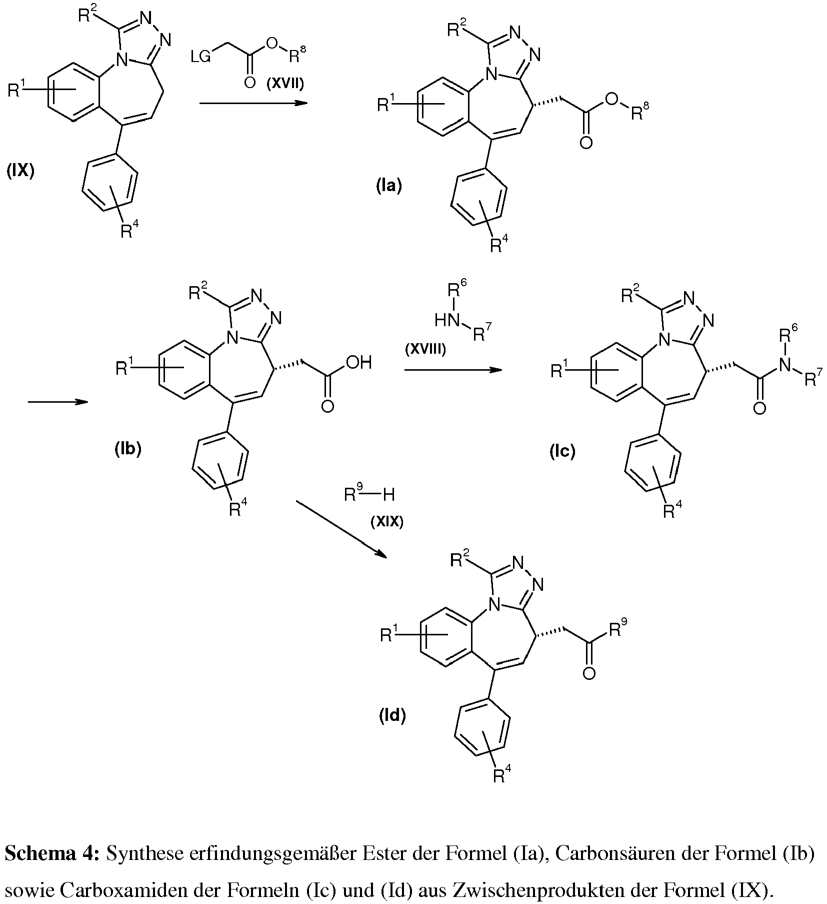

Wie in Schema 5 gezeigt, können die Zwischenprodukte der Formel (IX), in denen R¹, R² und R⁴ definiert sind wie in der allgemeinen Formel (I), weiter zu den erfindungsgemäßen Verbindungen der Formeln (Ie), die eine Untergruppe der allgemeinen Formel (I) darstellen, umgesetzt werden. Dazu werden die Verbindungen der Formel (IX) mit Verbindungen der Formel (XX), in denen LG für eine Abgangsgruppe, beispielsweise Halogen, bevorzugt Chlor oder Brom, und worin "R³-Ring" für ein Ringsystem steht, wie in für R³ in der allgemeinen Formel (I) definiert, in Gegenwart einer geeigneten Base, beispielsweise *sec*-Butyllithium oder Lithiumhexamethyldisilazid, und bei Temperaturen unter 0°C umgesetzt (siehe zu dieser Methode auch beispielsweise H. Tabata et al., Org. Lett. 2008, 10, S. 4871ff.). Aus dem dabei anfallenden racemischen Produkt wird mit einer dem Fachmann bekannten Methode, beispielsweise chiraler präparativer HPLC, das (*R*)-Enantiomer (Ie) erhalten. Verbindungen der Formel (XX) sind dem Fachmann bekannt und in zahlreichen Fällen käuflich. Gegebenenfalls kann die Abgangsgruppe LG aus geeigneten Vorläufern (wie beispielsweise durch Halogenierung einer Hydroxymethylgruppe am R³-Ring) mit dem Fachmann geläufigen Verfahren hergestellt werden.

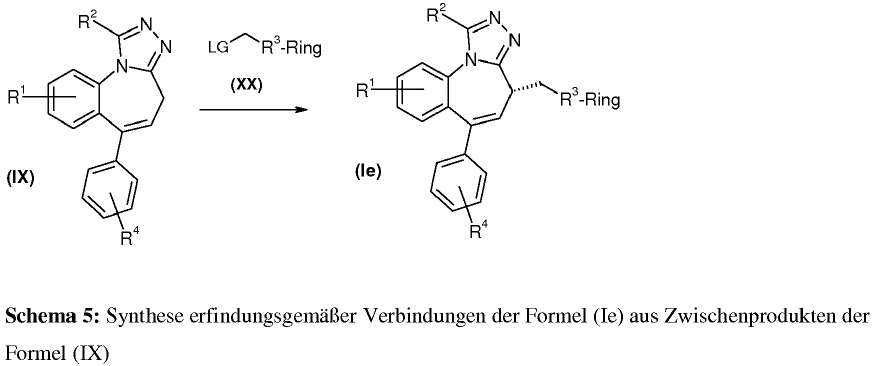

Schema 6 illustriert eine Methode zur Synthese von erfindungsgemäßen Oxadiazol-Derivaten der Formel (If). Hierzu werden die erfindungsgemäßen Verbindungen der Formel (Ia), in denen R¹, R², R⁴ und R⁸ definiert sind wie für die allgemeine Formel (I) mit der Maßgabe, dass R⁸ verschieden von Wasserstoff ist, in Gegenwart eines Alkalimetall-Alkoholates, wie beispielsweise Natriummethylat, mit Imidoximen der Formel (XXI), in denen R^{5a} für C₁-C₆-Alkyl-, C₃-C₈-Zykloalkyl, C₃-C₈-Heterozykloalkyl, Aryl, Heteroaryl, C₁-C₃-Alkoxy-C₁-C₃-Alkyl-, Aryloxy-C₁-C₃-Alkyl oder Aryl-C₁-C₃-Alkoxy-C₁-C₃-Alkyl- steht, direkt zu erfindungsgemäßen Verbindungen der Formel (If) umgesetzt (Tetrahedon Lett. 47, (2006), S.4271ff, W. Du et al.). Die Ester der Formel (Ia) können auch in racemischer Form eingesetzt werden und nachfolgend kann aus dem dabei anfallenden racemischen Produkt mit einer dem Fachmann bekannten Methode, beispielsweise chiraler präparativer HPLC, das (*R*)-Enantiomer (If) erhalten werden. Die einfachen Imidoxime sind üblicherweise kommerziell erhältlich.

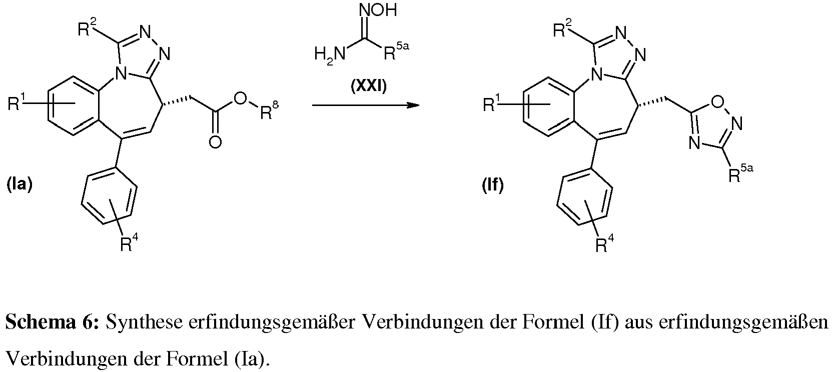

Die Synthese weiterer erfindungsgemäßer Verbindungen der Formeln (Ig), (Ih) und (Ii) wird in Schemata 7a und 7b aufgezeigt. Als Ausgangsmaterial werden erfindungsgemäße Carbonsäuren der Formel (Ib) oder auch deren Racemate eingesetzt. In diesem Fall können aus den dabei anfallenden racemischen Produkten mit einer dem Fachmann bekannten Methode, beispielsweise chiraler präparativer HPLC, die jeweiligen (*R*)-Enantiomere der Formeln (Ig), (Ih) und (Ii) erhalten werden.

Zur Synthese von erfindungsgemäßen Verbindungen der allgemeinen Formel (Ig) werden Carbonsäuren der Formel (Ib), in denen R¹, R² und R⁴ definiert sind wie für die allgemeine Formel (I), mit Acylhydrazinen der Formel (XXII), in denen R^{5b} für C₁-C₆-Alkyl-, C₃-C₈-Zykloalkyl, C₃-C₈-Heterozykloalkyl, Aryl, Heteroaryl, C₁-C₃-Alkoxy-C₁-C₃-Alkyl-, Aryloxy-C₁-C₃-Alkyl oder Aryl-C₁-C₃-Alkoxy-C₁-C₃-Alkyl- steht, in Gegenwart eines geeigneten Amidkupplungs-Reagenz, beispielsweise T3P oder HATU, zu Acylhydraziden der Formel (XXIII) umgesetzt, die nachfolgend in Gegenwart eines geeigneten Reagenz, beispielsweise eines anorganischen Säurechlorides wie Phosphorylchlorid POCl₃, zu den erfindungsgemäßen Verbindungen zyklisiert werden können (Org. Lett. 7, (2005), S.1039ff, J. Balsells et al.). Die Acylhydrazide der Formel (XXIII) können weiterhin in Gegenwart eines geeigneten Sulfurierungsreagenz, beispielsweise Lawesson's Reagens oder P₄S₁₀, zu erfindungsgemäßen Thiadiazol-Derivaten der Formel (Ih) zyklisiert werden (Bioorg. Med. Chem. Lett. 20, (2010), S. 5909ff, G. Le et al.).

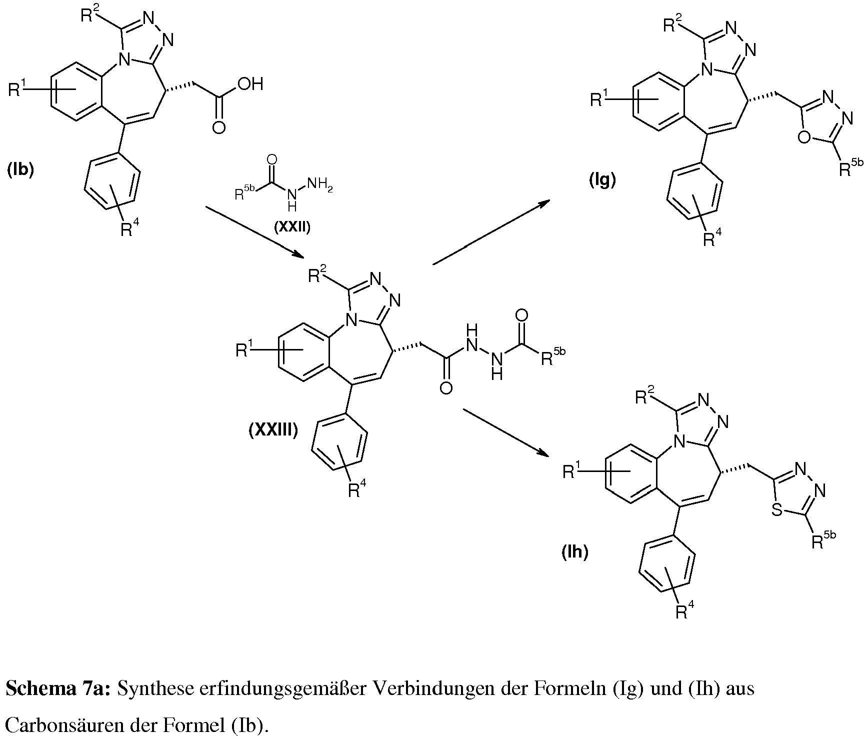

Wie in Schema 7b gezeigt, können in analoger Weise auch die oben genannten Carbonsäuren (Ib), in denen R¹, R² und R⁴ definiert sind wie für die allgemeine Formel (I), mit Acylhydrazinen der Formel (XXIV), in denen R¹⁵ definiert ist wie für die allgemeine Formel (I), unter Bildung erfindungsgemäßer Acylhydrazid-Derivate der allgemeinen Formel (Ii) umgesetzt werden.

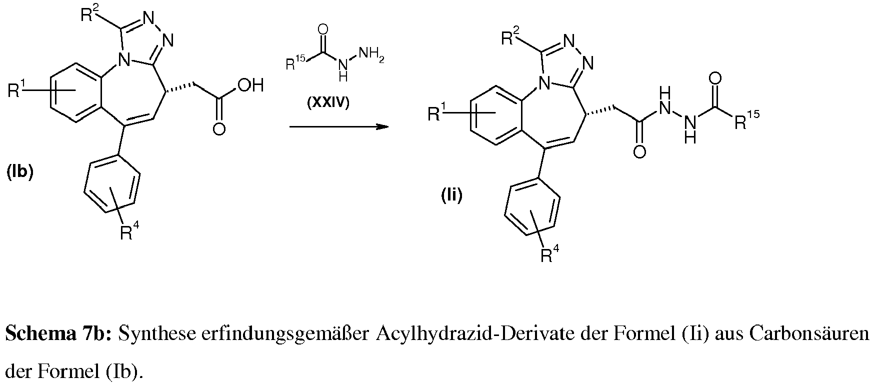

### Ausführungsbeispiele

Die nachfolgenden Beispiele erläutern die Herstellung der erfindungsgemäßen Verbindungen, ohne die Erfindung auf diese Beispiele zu beschränken.

Optische Rotation angegeben in der Konzentration g/L. Multiplizität der NMR Signale s = singulett, d = dublett, t = triplett, q = quartett, qi = quintett, b = breites signal, m = multiplett. NMR-Signale: Verschiebung in ppm. Die Multiplizitäten sind entsprechend der im Spektrum erscheinenden Signalform angegeben, NMR-spektroskopische Effekte höherer Ordnung wurden nicht berücksichtigt.

IUPAC-Namen wurden erstellt mit Hilfe der Nomenklatursoftware ACD Name batch, Version 12.01, von Advanced Chemical Development, Inc., und bei Bedarf angepaßt, beispielsweise an die deutschsprachige Nomenklatur.

### Beispiel 1

### Herstellung von [6-(4-Chlorphenyl)-1-methyl-4H-[1,2,4]triazolo[4,3-a][1]benzazepin-4-yl]essigsäureethylester

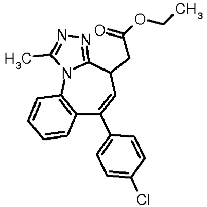

### Beispiel 1A

### Herstellung von 5-(4-Chlorphenyl)-1,3-dihydro-2H-1-benzazepin-2-on

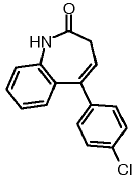

Eine Lösung von 8.1 g 5-(4-Chlorphenyl)-1,3,4,5-tetrahydro-2H-1-benzazepin-2-on (US5484917) in 1.77 L Tetrachlorkohlenstoff wurde mit 5.3 g Brom versetzt und in der Siedehitze mit einer 500W Glühlampe bestrahlt. Nach 10 Stunden wurde der Ansatz vollständig eingeengt, in Ethylacetat aufgenommen und mit gesättigter Natriumhydrogencarbonat Lösung 30 min. gerührt. Die organische Phase wurde abgetrennt, mit Sole gewaschen, über Natriumsulfat getrocknet und eingeengt. Der Rückstand wurde durch Chromatographie (Kieselgel, Hexan / Ethylacetat Gradient 0% - 20%) aufgereinigt.
Ausbeute: 5,6 g 5-(4-Chlorphenyl)-1,3-dihydro-2H-1-benzazepin-2-on.
¹H-NMR (400 MHz, RT, CDCl₃): δ = 3.01 (d, 2H); 6.18 (t, 1H); 7.10-7.18 (m, 3H), 7.20 (d, 2H); 7.33 (d, 2H); 7.38 (dt, 1H); 8.38 (bs, 1H).

### Beispiel 1B

### Herstellung von 5-(4-Chlorphenyl)-1,3-dihydro-2H-1-benzazepin-2-thion

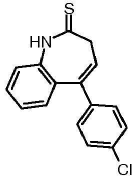

Eine Lösung von 1.1 g 5-(4-Chlorphenyl)-1,3-dihydro-2H-1-benzazepin-2-on (Beispiel 1A) und 0.99 g 2,4-Bis-[4-methoxyphenyl]-1,3-dithia-2,4-diphosphetan-2,4-disulfid (Lawesson's Reagenz) in 27 ml Tetrahydrofuran wurde 2 Stunden auf Siedehitze erwärmt. Dann wurde das Lösungsmittel im Vakuum entfernt und der Rückstand durch Chromatographie (Kieselgel, Hexan / Ethylacetat Gradient 0% - 20%) gereinigt.
Ausbeute: 1.1 g 5-(4-Chlorphenyl)-1,3-dihydro-2H-1-benzazepine-2-thion.
¹H-NMR (300 MHz, RT, CDCl₃): δ = 3.45 (d, 2H); 6.19 (t, 1H); 7.13-7.22 (m, 5H); 7.28-7.41 (m, 3H); 9.74 (bs, 1H).

### Beispiel 1C

### Herstellung von 6-(4-Chlorphenyl)-1-methyl-4H-[1,2,4]triazolo[4,3-a][1]benzazepin

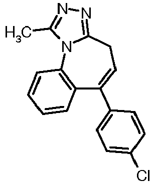

Eine Lösung von 1.1 g 5-(4-Chlorphenyl)-1,3-dihydro-2H-1-benzazepin-2-thion (Beispiel 1B) und 342 mg Acetylhydrazin in 22 ml 1-Butanol wurde 36 Stunden in der Siedehitze gerührt. Dann wurde im Vakuum eingeengt und der Rückstand durch Chromatographie (Kieselgel, Dichlormethan / Methanol Gradient) gereinigt.
Ausbeute: 620 mg 6-(4-Chlorphenyl)-1-methyl-4H-[1,2,4]triazolo[4,3-a][1]benzazepin.
¹H-NMR (300 MHz, RT, CDCl₃): δ = 2.61 (s, 3H); 3.02 (dd, 1H); 3.88 (dd, 1H); 6.35 (dd, 1H); 7.13 (d, 2H); 7.23-7.33 (m, 3H); 7.37 (dt, 1H); 7.40 (d, 1H); 7.50 (dt, 1H).

### Beispiel 1D (Alternativer Zugang zu Beispiel 1C)

### 2-Thioxo-1,2,3,4-tetrahydro-5H-1-benzazepin-5-on

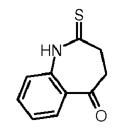

Eine Lösung von 40 g 3,4-Dihydro-1H-1-benzazepin-2,5-dion (Arch. Pharm. 324, (1991), p. 579-81, C. Kunick) und 61.4 g Lawesson's Reagenz in 1.2 L THF wurde 2 Stunden bei 70°C gerührt. Der Ansatz wurde auf 2 L 50%ige Sole gegeben und 3 mal mit Ethylacetat extrahiert. Die vereinten organischen Phasen wurden mit gesättigter Natriumhydrogencarbonat Lösung und Sole gewaschen, über Natriumsulfat getrocknet und das Lösungsmittel im Vakuum entfernt. Der Rückstand wurde mit ca. 400 ml Dichlormethan aufgenommen. Dabei löste sich ein Teil der Substanz nicht auf. Dieser Teil wurde abfiltriert und noch einmal mit 100 ml MTBE in der Siedehitze gerührt und nach Abkühlung abgesaugt. Man erhielt einen ersten Rückstand der Zielverbindung von 24 g. Die Dichlormethan Mutterlauge wurde durch Chromatographie an Kieselgel (Dichlormethan / Methanol-Gradient) gereinigt. Man erhielt weitere 6 g der Titelverbindung.
Ausbeute: 30 g 2-Thioxo-1,2,3,4-tetrahydro-5H-1-benzazepin-5-on
¹H-NMR (300 MHz, RT, CDCl₃): δ = 3.04-3.10 (m, 2H); 3.29-3.36 (m, 2H); 7.05 (d, 1H); 7.33 (dt (1H); 7.58 (dt, 1H); 8.01 (dd, 1H); 9.65 (bs, 1H).

### Beispiel 1E (Alternativer Zugang zu Beispiel 1C)

### 1-Methyl-4,5-dihydro-6H-[1,2,4]triazolo[4,3-a][1]benzazepin-6-on

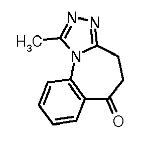

Eine Lösung von 24 g 2-Thioxo-1,2,3,4-tetrahydro-5H-1-benzazepin-5-on (Beispiel 1D) und 27.9 g Acetylhydrazin in 670 ml 1-Butanol wurde zunächst 1 Stunde bei 60°C gerührt. Dann wurde auf 150°C erhitzt und 16 Stunden gerührt. Das Lösungsmittel wurde im Vakuum entfernt und der Rückstand in 670 ml Dioxan aufgenommen. Es wurde 32 ml Wasser zugegeben und anschließend vorsichtig 103.8 ml konzentrierte Salzsäure zugetropft. Der Ansatz wurde 16 Stunden bei RT gerührt. Der entstandene Niederschlag wurde abfiltriert und der Ansatz durch Zugabe von Kaliumcarbonat auf einen basischen pH eingestellt. Der Ansatz wurde erneut filtriert und der Rückstand mit Ethylacetat gewaschen. Die vereinten Phasen wurde getrennt, die organsiche Phase über Natriumsulfat getrocknet und das Lösungsmittel im Vakuum entfernt. Der Rückstand wurde durch Chromatographie an Kieselgel (Dichlormethan / Methanol Gradient) gereinigt.
Ausbeute: 11.7 g 1-Methyl-4,5-dihydro-6H-[1,2,4]triazolo[4,3-a][1]benzazepin-6-on
¹H-NMR (300 MHz, RT, CDCl₃): δ = 2.54 (s, 3H); 3.01-3.09 (m, 2H); 3.20-3.28 (m, 2H); 7.28 (dd, 1H); 7.54 (dt, 1H); 7.71 (dt, 1H); 7.82 (dd, 1H).

### Beispiel 1F (Alternativer Zugang zu Beispiel 1C)

### 1-Methyl-4H-[1,2,4]triazolo[4,3-a][1]benzazepin-6-yl 1,1,2,2,3,3,4,4,4-nonafluorobutan-1-sulfonat

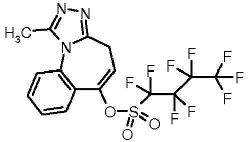

Eine Lösung von 11.7 g 1-Methyl-4,5-dihydro-6H-[1,2,4]triazolo[4,3-a][1]benzazepin-6-on (Beispiel 1E) in 500 ml THF wurde bei -70°C innerhalb von 30 min. mit 78.1 ml Lithiumhexamethyldisilazid-Lösung (1M in Toluol) versetzt. Es wurde weitere 45 min. bei -70°C gerührt. Dazu wurden 23.6 g Nonafluorbutansulfonsäurefluorid (CAS 375-72-4) getropft und 16 Stunden unter Erwärmung auf RT gerührt. Der Ansatz wurde auf halbgesättigte Natriumhydrogensulfat Lösung gegeben und dreimal mit Ethylacetat extrahiert. Die vereinten organischen Phasen wurden mit Sole gewaschen, über Natriumsulfat getrocknet und das Lösungsmittel im Vakuum entfernt. Der Rückstand wurde durch Chromatographie an Kieselgel (Dichlormethan / Methanol Gradient) gereinigt.
Ausbeute: 26.5 g 1-Methyl-4H-[1,2,4]triazolo[4,3-a][1]benzazepin-6-yl 1,1,2,2,3,3,4,4,4-nonafluorobutan-1-sulfonat
¹H-NMR (300 MHz, RT, CDCl₃): δ = 2.52 (s, 3H); 3.00-3.11 (m, 2H); 3.81-3.92 (m, 2H); 6.35 (dd, 1H); 7.43 (dd, 1H); 7.59 (dt, 1H); 7.65 (dt, 1H); 7.79 (dd, 1H).

### Beispiel 1C (Alternativer Zugang)

### 6-(4-Chlorphenyl)-1-methyl-4H-[1,2,4]triazolo[4,3-a][1]benzazepin

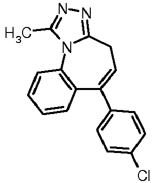

Eine Lösung von 26.5 g 1-Methyl-4H-[1,2,4]triazolo[4,3-a][1]benzazepin-6-yl 1,1,2,2,3,3,4,4,4-nonafluorobutan-1-sulfonat (Beispiel 1F), 10.33 g 4-Chlorphenylboronsäure (CAS 1679-18-1), 4.31 g Lithiumchlorid, 10.77 g Natriumcarbonat und 5.87 g Tetrakis-triphenylphosphinpalladium in 830 ml Toluol und 207 ml Ethanol wurde unter Argonatmosphäre für 3 Stunden bei 95°C gerührt. Der Ansatz wurde auf Wasser gegeben und viermal mit Ethylacetat extrahiert. Die vereinten organischen Phasen wurden mit Sole gewaschen, über Natriumsulfat getrocknet und das Lösungsmittel bis auf ca. 200 ml im Vakuum entfernt. Es wurde über Nacht stehen gelassen. Dabei schied sich die gewünschte Zielverbindung als ein Niederschlag ab, der abfiltriert wurde (8.0 g). Die Mutterlauge wurde weiter eingeengt und der Rückstand durch Chromatographie an Kieselgel (Dichlormethan / Methanol Gradient) gereinigt. Man erhielt weitere 3.5 g der Zielverbindung.
Ausbeute: 11.5 g 6-(4-Chlorphenyl)-1-methyl-4H-[1,2,4]triazolo[4,3-a][1]benzazepin
¹H-NMR (300 MHz, RT, CDCl₃): δ = 2.62 (s, 3H); 3.03 (dd, 1H); 3.89 (dd, 1H); 6.36 (dd, 1H); 7.14 (d, 2H); 7.23-7.34 (m, 3H); 7.34-7.45 (m, 2H); 7.52 (dt, 1H).

### Beispiel 1G

### Herstellung von [6-(4-Chlorphenyl)-1-methyl-4H-[1,2,4]triazolo[4,3-a][1]benzazepin-4-yl]essigsäureethylester

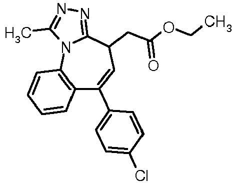

180 mg 6-(4-Chlorphenyl)-1-methyl-4H-[1,2,4]triazolo[4,3-a][1]benzazepin (Beispiel1C) in 5 ml THF wurden bei -78°C mit 0.32 ml einer Lösung von Lithiumdiisopropylamid (2M in THF/Heptan/Ethylbenzol) versetzt. Nach vollständiger Zugabe wurde noch 90 min. bei -78°C gerührt. Dann wurden 0.065 ml Bromessigsäureethylester (CAS 105-36-2) zugegeben und langsam über Nacht auf Raumtemperatur erwärmt. Der Ansatz wurde zwischen verdünnter Salzsäure (0.1M) und Ethylacetat verteilt, die organische Phase abgetrennt, die Wasserphase mit Ethylacetat extrahiert und die vereinigten organischen Phasen mit Wasser und Sole gewaschen, über Natriumsulfat getrocknet und im Vakuum eingeengt. Der Rückstand wurde durch Chromatographie (Kieselgel, Dichlormethan / Methanol Gradient) gereinigt.
Ausbeute: 100 mg [6-(4-Chlorphenyl)-1-methyl-4H-[1,2,4]triazolo[4,3-a][1]benzazepin-4-yl]essigsäureethylester.
¹H-NMR (300 MHz, RT, CDCl₃): δ = 1.30 (t, (3H); 2.59 (s, 3H); 3.16 (dd, 1H); 3.46 (dd, 1H); 3.60-3.69 (m, 1H); 4.21 (q, 2H); 6.06 (dd, 1H); 7.04-7.18 (m, 2H); 7.23-7.33 (m, 3H); 7.34-7.44 (m, 2H); 7.51 (dt, 1H).

### Beispiel 2

### Herstellung von (-)-(4R)- [6-(4-Chlorphenyl)-1-methyl-4H-[1,2,4]triazolo[4,3-a][1]benz-azepin-4-yl]essigsäureethylester

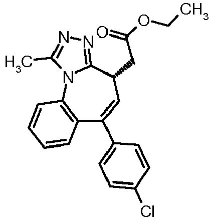

190 mg [6-(4-Chlorphenyl)-1-methyl-4H-[1,2,4]triazolo[4,3-a][1]benz-azepin-4-yl]essigsäureethylester wurden durch chirale HPLC (Chiralpak IC 5µm 250x30 mm, Ethanol / Methanol 50:50 (v/v), 30 mL/min) in die Enantiomere getrennt.
Ausbeute: 22 mg (-)-(4*R*)-[6-(4-Chlorphenyl)-1-methyl-4H-[1,2,4]triazolo[4,3-a][1]benzazepin-4-yl]essigsäureethylester.
Optische Rotation: [α]_{D}²⁰ = -140.3° +/- 0.12° (c=10.3, CHCl₃).

### Beispiel 3

### Herstellung von 2-[6-(4-Chlorphenyl)-1-methyl-4H-[1,2,4]triazolo[4,3-a][1]benzazepin-4-yl]-1-(2-oxa-6-azaspiro[3.3]hept-6-yl)ethanon

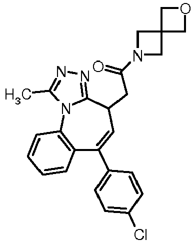

### Beispiel 3A

### Herstellung von [6-(4-Chlorphenyl)-1-methyl-4H-[1,2,4]triazolo[4,3-a][1]benzazepin-4-yl]essigsäure

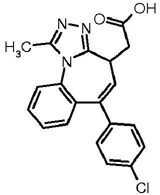

Eine Lösung von 215 mg [6-(4-Chlorphenyl)-1-methyl-4H-[1,2,4]triazolo[4,3-a][1]benzazepin-4-yl]essigsäureethylester und 0.6 ml Natronlauge (1M) in 18 ml Methanol wurden 18 Stunden bei Raumtemperatur gerührt. Die Lösung wurde im Vakuum vollständig eingeengt und der Rückstand wurde durch RP-HPLC (Säule: X-Bridge C18 5µm 100x30mm, Mobile Phase: Acetonitril / Wasser (0.1 Vol% Ameisensäure)-Gradient) gereinigt.
Ausbeute: 200 mg [6-(4-Chlorphenyl)-1-methyl-4H-[1,2,4]triazolo[4,3-a][1]benzazepin-4-yl]essigsäure.
¹H-NMR (400 MHz, RT, CDCl₃): δ = 2.61 (s, 3H); 3.24 (dd, 1H); 3.48 (dd, 1H); 3.65-3.73 (m, 1H); 6.08 (d, 1H); 7.13 (d, 2H); 7.25-7.33 (m, 5H); 7.37-7.46 (m, 2H); 7.52 (dt, 1H).

### Beispiel 3B

### Herstellung von 2-[6-(4-Chlorphenyl)-1-methyl-4H-[1,2,4]triazolo[4,3-a][1]benzazepin-4-yl]-1-(2-oxa-6-azaspiro[3.3]hept-6-yl)ethanon

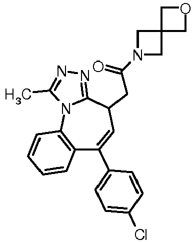

Eine Lösung von 200 mg [6-(4-Chlorphenyl)-1-methyl-4H-[1,2,4]triazolo[4,3-a][1]benzazepin-4-yl]essigsäure (Beispiel 3A), 312 mg HATU, 0.3 ml Triethylamin und 158 mg 2-Oxa-6-azaspiro[3.3]heptan Oxalat (2:1) (CAS 174-78-7 der freien Base) in 10 ml DMF wurden bei Raumtemperatur für 3 Stunden gerührt. Der Ansatz wurde zwischen halbkonzentrierter Sole und Ethylacetat verteilt, die organische Phase abgetrennt und die Wasserphase zweimal mit Ethylacetat extrahiert. Die vereinten organischen Phasen wurden mit Sole gewaschen, über Natriumsulfat getrocknet und im Vakuum eingeengt. Der Rückstand wurde durch Chromatographie (Kieselgel, Dichlormethan / Methanol Gradient) gereinigt.
Ausbeute: 200 mg 2-[6-(4-Chlorphenyl)-1-methyl-4H-[1,2,4]triazolo[4,3-a][1]benzazepin-4-yl]-1-(2-oxa-6-azaspiro[3.3]hept-6-yl)ethanon.
¹H-NMR (400 MHz, RT, DMSO-d6): δ = 2.50 (s, 3H); 2.82 (dd, 1H); 3.05 (dd, 1H); 3.44 (q, 1H); 4.01 (d, 1H); 4.05 (d, 1H); 4.43 (d, 1H); 4.49 (d, 1H); 4.65-4.75 (m, 4H); 6.19 (d, 1H); 7.13-7.29 (m, 3H); 7.41 (d, 2H); 7.47 (dt, 1H); 7.60 (dt. 1H); 7.74 (d, 1H).

### Beispiel 4

### Herstellung von (-)-2-[(4R)-6-(4-Chlorphenyl)-1-methyl-4H-[1,2,4]triazolo[4,3-a][1]benzazepin-4-yl]-1-(2-oxa-6-azaspiro[3.3]hept-6-yl)ethanon

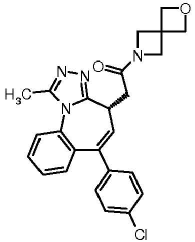

200 mg 2-[6-(4-Chlorphenyl)-1-methyl-4H-[1,2,4]triazolo[4,3-a][1]benzazepin-4-yl]-1-(2-oxa-6-azaspiro[3.3]hept-6-yl)ethanon wurden durch chirale HPLC (Chiralpak IC 5µm 250x30 mm, Ethanol / Methanol 50:50 (v/v), 30 mL/min) in die Enantiomere getrennt.
Ausbeute: 50 mg (-)-2-[6-(4-Chlorphenyl)-1-methyl-4H-[1,2,4]triazolo[4,3-a][1]benzazepin-4-yl]-1-(2-oxa-6-azaspiro[3.3]hept-6-yl)ethanon.
Optische Rotation: [α]_{D}²⁰= -151.2° +/- 0.12° (c=10.6, CHCl₃)].

### Beispiel 5

### Herstellung von 1-(7-Azabicyclo[2.2.1]hept-7-yl)-2-[6-(4-chlorphenyl)-1-methyl-4H-[1,2,4]triazolo[4,3-a][1]benzazepin-4-yl]ethanon

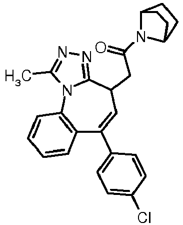

Eine Lösung von 200 mg [6-(4-Chlorphenyl)-1-methyl-4H-[1,2,4]triazolo[4,3-a][1]benzazepin-4-yl]essigsäure (Beispiel 3A), 312 mg HATU, 0.3 ml Triethylamin und 53 mg 7-Azabicyclo [2.2.1]heptan Hydrochlorid (Zerenex ZX-IP016134) in 10 ml DMF wurden bei Raumtemperatur für 3 Stunden gerührt. Der Ansatz wurde zwischen halbkonzentrierter Sole und Ethylacetat verteilt, die organische Phase abgetrennt und die Wasserphase zweimal mit Ethylacetat extrahiert. Die vereinten organischen Phasen wurden mit Sole gewaschen, über Natriumsulfat getrocknet und im Vakuum eingeengt. Der Rückstand wurde durch Chromatographie (Kieselgel, Dichlormethan / Methanol Gradient) gereinigt.
Ausbeute: 210 mg 1-(7-Azabicyclo[2.2.1]hept-7-yl)-2-[6-(4-chlorphenyl)-1-methyl-4H-[1,2,4]triazolo[4,3-a][1]benzazepin-4-yl]ethanon.
¹H-NMR (400 MHz, RT, CDCl₃): δ = 1.40-1.63 (m, 1H); 1.70-2.02 (m, 3H); 2.59 (s, 3H); 3.12 (dd, 1H); 3.41 (dd, 1H); 3.76 (dt, 1H); 4.47 (t, 1H); 4.66 (t, 1H); 6.03 (d, 1H); 7.13 (d, 2H); 7.21-7.31 (m, 3H); 7.31-7.42 (m, 2H); 7.48 (dt, 1H).

### Beispiel 6

### Herstellung von (-)-1 (7-Azabicyclo[2.2.1]hept-7-yl)-2-[(4R)-6-(4-chlorphenyl)-1-methyl-4H-[1,2,4]triazolo[4,3-a][1]benzazepin-4-yl]ethanon

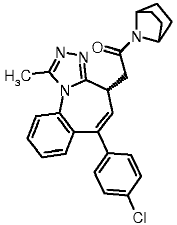

210 mg 1-(7-Azabicyclo[2.2.1]hept-7-yl)-2-[6-(4-chlorphenyl)-1-methyl-4H-[1,2,4]triazolo[4,3-a][1]benzazepin-4-yl]ethanon wurden durch chirale HPLC (Chiralpak IC 5µm 250x30 mm, Ethanol / Methanol 50:50 (v/v), 30 mL/min) in die Enantiomere getrennt.
Ausbeute: 50 mg (-)-1- (7-Azabicyclo[2.2.1]hept-7-yl)-2-[(4*R*)-6-(4-chlorphenyl)-1-methyl-4H-[1,2,4]triazolo[4,3-a][1]benzazepin-4-yl]ethanon.
Optische Rotation: [α]_{D}²⁰= -120.2° +/- 0.14° (c=9.8, CHCl₃).

### Beispiel 7

### Herstellung von [6-(3-Chlorphenyl)-1-methyl-4H-[1,2,4]triazolo[4,3-a][1]benzazepin-4-yl]essigsäureethylester

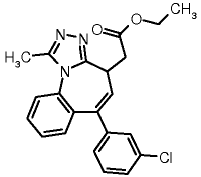

### Beispiel 7A

### 5-(3-Chlorphenyl)-1,3-dihydro-2H-1-benzazepin-2-on

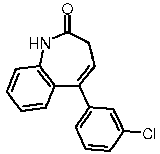

Eine Lösung von 8.1 g 5-(3-Chlorphenyl)-1,3,4,5-tetrahydro-2H-1-benzazepin-2-on (J. Med Chem. 37, (1994), p. 3789ff, J. A. Lowe et al.) in 1.77 L Tetrachlorkohlenstoff wurde mit 5.3 g Brom versetzt und in der Siedehitze mit einer 500W Glühlampe bestrahlt. Nach 10 Stunden wurde der Ansatz vollständig eingeengt, in Ethylacetat aufgenommen und mit gesättigter Natriumhydrogencarbonat Lösung 30 min. gerührt. Die organische Phase wurde abgetrennt, mit Sole gewaschen, über Natriumsulfat getrocknet und eingeengt. Der Rückstand wurde durch Chromatographie (Kieselgel, Hexan / Ethylacetat Gradient 0% - 20%)aufgereinigt.
Ausbeute: Man erhielt 5,6 g 5-(4-Chlorphenyl)-1,3-dihydro-2H-1-benzazepin-2-on.
¹H-NMR (400 MHz, RT, CDCl₃): δ = 3.01 (d, 2H); 6.20 (t, 1H); 7.12-7.24 (m, 4H), 7.27-7.28 (m, 1H); 7.30-7.44 m, 3H); 7.87(bs, 1H).

### Beispiel 7B

### Herstellung von 5-(3-Chlorphenyl)-1,3-dihydro-2H-1-benzazepine-2-thion

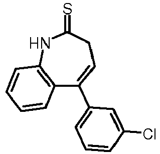

Eine Lösung von 730 mg 5-(3-Chlorphenyl)-1,3-dihydro-2H-1-benzazepin-2-on (Beispiel 7A) und 657 mg 2,4-Bis-[4-methoxyphenyl]-1,3-dithia-2,4-diphosphetan-2,4-disulfid (Lawesson's Reagenz) in 18.1 ml Tetrahydrofuran wurde 2 Stunden auf Siedehitze erwärmt. Dann wurde das Lösungsmittel im Vakuum entfernt und der Rückstand durch Chromatographie (Kieselgel, Hexan / Essigester Gradient 0% - 20%) gereinigt.
Ausbeute: 670 mg 5-(3-Chlorphenyl)-1,3-dihydro-2H-1-benzazepin-2-thion.
¹H-NMR (300 MHz, RT, CDCl₃): δ = 3.46 (d, 2H); 6.22 (t, 1H); 7.11 (dd, 1H); 7.16-7.26 (m, 4H); 7.28 7.42 (m, 3H); 9.64 (bs, 1H).

### Beispiel 7C

### Herstellung von 6-(3-Chlorphenyl)-1-methyl-4H-[1,2,4]triazolo[4,3-a][1]benzazepin

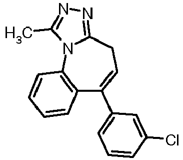

Eine Lösung von 670 mg 5-(3-Chlorphenyl)-1,3-dihydro-2H-1-benzazepin-2-thion (Beispiel 7B) und 208 mg Acetylhydrazin in 13.4 ml 1-Butanol wurde 36 Stunden in der Siedehitze gerührt. Dann wurde im Vakuum eingeengt und der Rückstand durch Chromatographie (Kieselgel, Dichlormethan / Methanol Gradient) gereinigt.
Ausbeute: 440 mg 6-(3-Chlorphenyl)-1-methyl-4H-[1,2,4]triazolo[4,3-a][1]benzazepin.
¹H-NMR (300 MHz, RT, CDCl₃): δ = 2.61 (s, 3H); 3.03 (dd, 1H); 3.89 (dd, 1H); 6.37 (dd, 1H); 7.08 (dd, 1H); 7.18 (bs, 1H); 7.22-7.33 (m, 3H); 7.34-7.44 (m, 2H); 7.51 (dt, 1H).

### Beispiel 7D

### Herstellung von [6-(3-Chlorphenyl)-1-methyl-4H-[1,2,4]triazolo[4,3-a][1]benzazepin-4-yl]essigsäureethylester

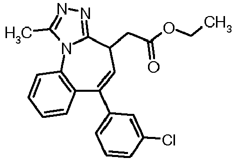

440 mg 6-(3-Chlorphenyl)-1-methyl-4H-[1,2,4]triazolo[4,3-a][1]benzazepin (Beispiel 7C) in 22 ml THF wurden bei -78°C mit 0.79 ml einer Lösung von Lithiumdiisopropylamid (2M in THF/Heptan/Ethylbenzol) versetzt. Nach vollständiger Zugabe wurde noch 90 min. bei -78°C gerührt. Dann wurden 0.16 ml Bromessigsäureethylester zugegeben und langsam über Nacht auf Raumtemperatur erwärmt. Der Ansatz wurde zwischen gesättigter Ammoniumchlorid Lösung und Ethylacetat verteilt, die organische Phase abgetrennt, die Wasserphase mit Ethylacetat extrahiert und die vereinigten organischen Phasen mit Wasser und Sole gewaschen, über Natriumsulfat getrocknet und im Vakuum eingeengt. Der Rückstand wurde durch Chromatographie (Kieselgel, Dichlormethan / Methanol Gradient) gereinigt.
Ausbeute 220 mg [6-(3-Chlorphenyl)-1-methyl-4H-[1,2,4]triazolo[4,3-a][1]benzazepin-4-yl]essigsäureethylester.
¹H-NMR (300 MHz, RT, CDCl₃): δ = 1.30 (t, 3H); 2.60 (s, 3H); 3.16 (dd, 1H); 3.46 (dd, 1H); 3.65 (dt, 1H); 4.21 (q, 2H); 6.07 (d, 1H); 7.06 (d, 1H); 7.21-7.32 (m, 3H); 7.35-7.44 (m, 2H); 7.52 (dt, 1H).

### Beispiel 8

### Herstellung von [6-(4-Chlorphenyl)-1-methyl-4H-[1,2,4]triazolo[4,3-a][1]benzazepin-4-yl]essigsäure-tert-butylester

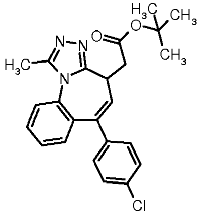

200 mg 6-(4-Chlorphenyl)-1-methyl-4H-[1,2,4]triazolo[4,3-a][1]benzazepin (Beispiel 1C) in 10 ml THF wurden bei -78°C mit 0.36 ml einer Lösung von Lithiumdiisopropylamid (2M in THF/Heptan/Ethylbenzol) versetzt. Nach vollständiger Zugabe wurde noch 90 min. bei -78°C gerührt. Dann wurden 0.095 ml Bromessigsäure-*tert*-butylester zugegeben und langsam über Nacht auf Raumtemperatur erwärmt. Der Ansatz wurde zwischen gesättigter Ammoniumchlorid Lösung und Ethylacetat verteilt, die organische Phase abgetrennt, die Wasserphase mit Ethylacetat extrahiert und die vereinigten organischen Phasen mit Wasser und Sole gewaschen, über Natriumsulfat getrocknet und im Vakuum eingeengt. Der Rückstand wurde durch Chromatographie (Kieselgel, Dichlormethan / Methanol Gradient) gereinigt.
Ausbeute: 110 mg [6-(4-Chlorphenyl)-1-methyl-4H-[1,2,4]triazolo[4,3-a][1]benzazepin-4-yl]essigsäure-*tert*-butylester.
¹H-NMR (300 MHz, RT, CDCl₃): δ = 1.48 (s, 9H); 2.59 (s, 3H); 3.09 (dd, 1H); 3.38 (dd, 1H); 3.59 (dt, 1H); 6.04 (d, 1H); 7.24-7.32 (m, 3H); 7.37 (dt, 1H); 7.40 (dd, 1H); 7.50 (dt, 1H).

### Beispiel 9

### Herstellung von (-)-[(4R)-6-(4-Chlorphenyl)-1-methyl-4H-[1,2,4]triazolo[4,3-a][1]benzazepin-4-yl]essigsäure-tert-butylester

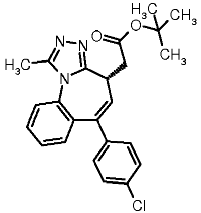

210 mg [6-(4-Chlorphenyl)-1-methyl-4H-[1,2,4]triazolo[4,3-a][1]benzazepin-4-yl]essigsäure-*tert-*butylester wurden durch chirale HPLC (Chiralpak IC 5µm 250x30 mm, Ethanol / Methanol 50:50 (v/v), 30 mL/min) in die Enantiomere getrennt.
Ausbeute: 33 mg (-)[(4*R*)-6-(4-Chlorphenyl)-1-methyl-4H-[1,2,4]triazolo[4,3-a][1]benzazepin-4-yl]essigsäure-*tert*-butylester.
Optische Rotation: [α]_{D}²⁰ = -109.1° +/- 0.14° (c=10.0, CHCl₃).

### Beispiel 10

### Herstellung von 2-[6-(3-Chlorphenyl)-1-methyl-4H-[1,2,4]triazolo[4,3-a][1]benzazepin-4-yl]-1-(2-oxa-6-azaspiro[3.3]hept-6-yl)ethanon

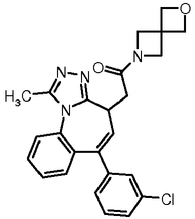

Eine Lösung von 215 mg [6-(3-Chlorphenyl)-1-methyl-4H-[1,2,4]triazolo[4,3-a][1]benzazepin-4-yl]essigsäureethylester (Beispiel 7) und 0.6 ml Natronlauge (1M) in 36 ml THF wurden 18 Stunden bei Raumtemperatur gerührt. Die Lösung wurde im Vakuum vollständig eingeengt. Man erhielt 199 mg [6-(3-Chlorphenyl)-1-methyl-4H-[1,2,4]triazolo[4,3-a][1]benzazepin-4-yl]essigsäure.

Diese wurde in 10 ml DMF gelöst und mit 310 mg HATU, 0.3 ml Triethylamin und 157 mg 2-Oxa-6-azaspiro[3.3]heptan Oxalat (2:1) versetzt und bei Raumtemperatur für 3 Stunden gerührt. Der Ansatz wurde zwischen halbkonzentrierter Sole und Ethylacetat verteilt, die organische Phase abgetrennt und die Wasserphase zweimal mit Ethylacetat extrahiert. Die vereinten organischen Phasen wurden mit Sole gewaschen, über Natriumsulfat getrocknet und im Vakuum eingeengt. Der Rückstand wurde durch Chromatographie (Kieselgel, Dichlormethan / Methanol Gradient) gereinigt.
Ausbeute: 190 mg 2-[6-(3-Chlorphenyl)-1-methyl-4H-[1,2,4]triazolo[4,3-a][1]benzazepin-4-yl]-1-(2-oxa-6-azaspiro[3.3]hept-6-yl)ethanon.
¹H-NMR (400 MHz, RT, CDCl₃): δ = 2.59 (s, 3H); 2.78 (dd, 1H); 3.24 (dd, 1H); 3.75 (dt, 1H); 4.18 (bs, 2H); 4.42 (d, 1H); 4.74-4.89 (m, 5H); 6.01 (d, 1H); 7.06 (d, 1H); 7.18 (bs, 1H); 7.21-7.31 (m, 3H); 7.34-7.42 (m, 2H); 7.50 (dt, 1H).

### Beispiel 11

### Herstellung von (-)-2-[(4R)-6-(3-Chlorphenyl)-1-methyl-4H-[1,2,4]triazolo[4,3-a][1]benzazepin-4-yl]-1-(2-oxa-6-azaspiro[3.3]hept-6-yl)ethanon

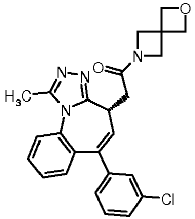

190 mg 2-[6-(3-Chlorphenyl)-1-methyl-4H-[1,2,4]triazolo[4,3-a][1]benzazepin-4-yl]-1-(2-oxa-6-azaspiro[3.3]hept-6-yl)ethanon wurden durch chirale HPLC (Chiralpak IA 5µm 250x20 mm, Methanol / Ethanol 1:1 (v/v) +0,1% DEA, 15 mL/min) in die Enantiomere getrennt.
Ausbeute: 70 mg (-)-2-[(4*R*)-6-(3-Chlorphenyl)-1-methyl-4H-[1,2,4]triazolo[4,3-a][1]benzazepin-4-yl]-1-(2-oxa-6-azaspiro[3.3]hept-6-yl)ethanon.
Optische Rotation: [α]_{D}²⁰ = -129.9° +/- 0.14° (c=9.8, CHCl₃).

### Beispiel 12

### Herstellung von 2-[6-(4-Chlorphenyl)-1-methyl-4H-[1,2,4]triazolo[4,3-a][1]benzazepin-4-yl]-N-ethylacetamid

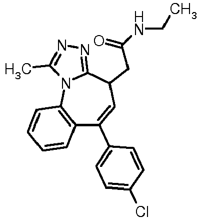

Eine Lösung von 110 mg [6-(4-Chlorphenyl)-1-methyl-4H-[1,2,4]triazolo[4,3-a][1]benzazepin-4-yl]essigsäure (Beispiel 3A), 171 mg HATU, 0.17 ml Triethylamin und 0.165 ml Ethylamin in 3 ml DMF wurden bei Raumtemperatur für 14 Stunden gerührt. Der Ansatz wurde zwischen halbkonzentrierter Sole und Ethylacetat verteilt, die organische Phase abgetrennt und die Wasserphase zweimal mit Ethylacetat extrahiert. Die vereinten organischen Phasen wurden mit Sole gewaschen, über Natriumsulfat getrocknet und im Vakuum eingeengt. Der Rückstand wurde durch Chromatographie (Kieselgel, Dichlormethan / Methanol Gradient) gereinigt.
Ausbeute: 80 mg 2-[6-(4-Chlorphenyl)-1-methyl-4H-[1,2,4]triazolo[4,3-a][1]benzazepin-4-yl]-N-ethylacetamid.
¹H-NMR (400 MHz, RT, CDCl₃): δ = 1.18 (t, 3H); 2.65 (s, 3H); 2.98 (dd, 1H); 3.22-3.43 (m, 3H); 3.72 (dt, 1H); 6.07 (d, 1H); 6.84 (bs, 1H); 7.15 (d, 2H); 7.29-7.35 (m, 3H); 7.39-7.47 (m, 2H); 7.55 (dt, 1H).

### Beispiel 13

### Herstellung von (-)-2-[(4R)-6-(4-Chlorphenyl)-1-methyl-4H-[1,2,4]triazolo[4,3-a][1]benzazepin-4-yl]-N-ethylacetamid

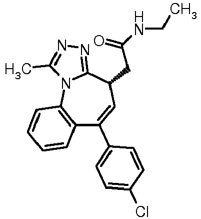

80 mg 2-[6-(4-Chlorphenyl)-1-methyl-4H-[1,2,4]triazolo[4,3-a][1]benzazepin-4-yl]-N-ethylacetamid wurden durch chirale HPLC (Chiralpak IA 5µm 250x20 mm, Hexan/ Ethanol 75:25 (v/v)) in die Enantiomere getrennt.
Ausbeute: 31 mg (-)-2-[(4*R*)-6-(4-Chlorphenyl)-1-methyl-4H-[1,2,4]triazolo[4,3-a][1]benzazepin-4-yl]-N-ethylacetamid.
Optische Rotation: [α]_{D}²⁰ = -153.6° +/- 0.13° (c=10.6, CHCl₃).

### Beispiel 14

### Herstellung von 2-[6-(4-Chlorphenyl)-1-methyl-4H-[1,2,4]triazolo[4,3-a][1] benzazepin-4-yl]-1-(morpholin-4-yl)ethanon

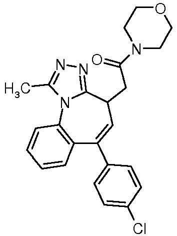

Eine Lösung von 110 mg [6-(4-Chlorphenyl)-1-methyl-4H-[1,2,4]triazolo[4,3-a][1]benzazepin-4-yl]essigsäure Beispiel 3A), 171 mg HATU, 0.17 ml Triethylamin und 0.03 ml Morpholin in 3 ml DMF wurden bei Raumtemperatur für 14 Stunden gerührt. Der Ansatz wurde zwischen halbkonzentrierter Sole und Ethylacetat verteilt, die organische Phase abgetrennt und die Wasserphase zweimal mit Ethylacetat extrahiert. Die vereinten organischen Phasen wurden mit Sole gewaschen, über Natriumsulfat getrocknet und im Vakuum eingeengt. Der Rückstand wurde durch Chromatographie (Kieselgel, Dichlormethan / Methanol Gradient) gereinigt.
Ausbeute: 85 mg 2-[6-(4-Chlorphenyl)-1-methyl-4H-[1,2,4]triazolo[4,3-a][1]benzazepin-4-yl]-1-(morpholin-4-yl)ethanon.
¹H-NMR (300 MHz, RT, CDCl₃): δ = 2.59 (s, 3H); 3.11 (dd, 1H); 3.52 (dd, 1H); 3.58-3.84 (m, 9H); 6.02 (d, 1H); 7.13 (d, 2H); 2.25-7.31 (m, 3H (+CDCl₃)); 7.33-7.42 (m, 2H); 7.49 (dt, 1H).

### Beispiel 15

### Herstellung von (-)-2-[(4R)-6-(4-Chlorphenyl)-1-methyl-4H-[1,2,4]triazolo[4,3-a][1]benzazepin-4-yl]-1-(morpholin-4-yl)ethanon

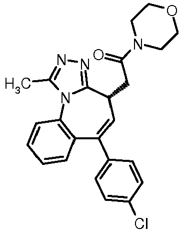

85 mg 2-[6-(4-Chlorphenyl)-1-methyl-4H-[1,2,4]triazolo[4,3-a][1]benzazepin-4-yl]-1-(morpholin-4-yl)ethanon wurden durch chirale HPLC (Chiralpak ID 5µm 250x20 mm, Methanol / Ethanol 1:1 1 (v/v)) in die Enantiomere getrennt.
Ausbeute: 35 mg (-)-2-[(4*R*)-6-(4-Chlorphenyl)-1-methyl-4H-[1,2,4]triazolo[4,3-a][1]benzazepin-4-yl]-1-(morpholin-4-yl)ethanon.
Optische Rotation: [α]_{D}²⁰ = -102.5° +/- 0.13° (c=10.9, Methanol).

### Beispiel 16

### Herstellung von 2-[6-(4-Chlorphenyl)-1-methyl-4H-[1,2,4]triazolo[4,3-a][1]benzazepin-4-yl]-1-(3-fluorazetidin-1-yl)ethanon

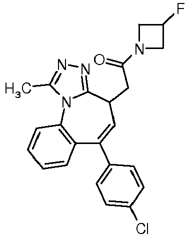

Eine Lösung von 110 mg [6-(4-Chlorphenyl)-1-methyl-4H-[1,2,4]triazolo[4,3-a][1]benzazepin-4-yl]essigsäure (Beispiel 3A), 171 mg HATU, 0.17 ml Triethylamin und 37 mg 3-Fluorazetidin Hydrochlorid (CAS 617718-46-4) in 3 ml DMF wurden bei Raumtemperatur für 14 Stunden gerührt. Der Ansatz wurde zwischen halbkonzentrierter Sole und Ethylacetat verteilt, die organische Phase abgetrennt und die Wasserphase zweimal mit Ethylacetat extrahiert. Die vereinten organischen Phasen wurden mit Sole gewaschen, über Natriumsulfat getrocknet und im Vakuum eingeengt. Der Rückstand wurde durch Chromatographie (Kieselgel, Dichlormethan / Methanol Gradient) gereinigt.
Ausbeute: 80 mg 2-[6-(4-Chlorphenyl)-1-methyl-4H-[1,2,4]triazolo[4,3-a][1]benzazepin-4-yl]-1-(3-fluorazetidin-1-yl)ethanon.
¹H-NMR (300 MHz, RT, CDCl₃, Spektrum beinhaltet Signale diastereotoper Protonen): δ = 2.59 + 2.59 (2s, 3H); 2.79 + 2.88 (2dd, 1H); 3.19-3.11 (m, 1H); 3.67-3.82 (m, 1H); 4.04-4.23 (m, 1H); 4.24-4.70 (2H); 4.89-5.05 (m, 1/2 H); 5.35 (dm, 1H); 6.00 + 6.03 (2d, 1H); 7.12 (d, 2H); 7.22-7.32 (m+CDCl₃, 3H); 7.33-7.42 (m, 2H); 7.49 (dt, 1H).

### Beispiel 17

### Herstellung von (-)-2-[(4R)-6-(4-Chlorphenyl)-1-methyl-4H-[1,2,4]triazolo[4,3-a][1]benzazepin-4-yl]-1-(3-fluorazetidin-1-yl)ethanon

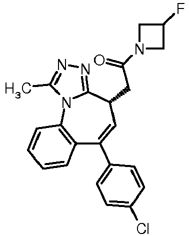

80 mg 2-[6-(4-Chlorphenyl)-1-methyl-4H-[1,2,4]triazolo[4,3-a][1]benzazepin-4-yl]-1-(3-fluoroazetidin-1-yl)ethanon wurden durch chirale HPLC (Chiralpak ID 5µm 250x20 mm, Methanol / Ethanol 1:1 (v/v), 22 mL/min) in die Enantiomere getrennt.
Ausbeute: 25 mg (-)-2-[(4*R*)-6-(4-Chlorphenyl)-1-methyl-4H-[1,2,4]triazolo[4,3-a][1]benzazepin-4-yl]-1-(3-fluorazetidin-1-yl)ethanon.
Optische Rotation: [α]_{D}²⁰ = -87.8° +/- 0.21° (c=10.0, Methanol).

### Beispiel 18

### Herstellung von 2-[6-(4-Chlorphenyl)-1-methyl-4H-[1,2,4]triazolo[4,3-a][1]benzazepin-4-yl]-1-(1,1-dioxido-1-thia-6-azaspiro[3.3]hept-6-yl)ethanon

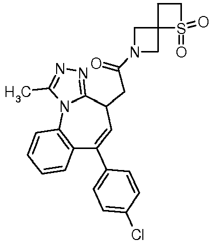

Eine Lösung von 110 mg [6-(4-Chlorphenyl)-1-methyl-4H-[1,2,4]triazolo[4,3-a][1]benzazepin-4-yl]essigsäure (Beispiel 3A), 171 mg HATU, 0.17 ml Triethylamin und 48.7 mg 1,1-Dioxido-1-thia-6-azaspiro[3.3]heptan TFA Salz (CAS 1352546-75-8 der greien Base) in 3 ml DMF wurden bei

Raumtemperatur für 14 Stunden gerührt. Der Ansatz wurde zwischen halbkonzentrierter Sole und Ethylacetat verteilt, die organische Phase abgetrennt und die Wasserphase zweimal mit Ethylacetat extrahiert. Die vereinten organischen Phasen wurden mit Sole gewaschen, über Natriumsulfat getrocknet und im Vakuum eingeengt. Der Rückstand wurde durch Chromatographie (Kieselgel, Dichlormethan / Methanol Gradient) gereinigt.
Ausbeute: 90 mg 2-[6-(4-Chlorphenyl)-1-methyl-4H-[1,2,4]triazolo[4,3-a][1]benzazepin-4-yl]-1-(1,1-dioxido-1-thia-6-azaspiro[3.3]hept-6-yl)ethanon.
¹H-NMR (300 MHz, RT, DMSO-d6, charakteristische Signale): δ = 2.83-2.99 (m, 1H); 3.04-3.18 (m, 1H); 3.46 (q, 1H); 4.08-4.19 (m, 3H); 4.24 (t, 1H); 4.60 (dd, 1H); 4.73 (t, 1H); 6.22 (d, 1H); 7.17-7.26 (m, 3H); 7.42 (d, 2H); 7.47 (t, 1H); 7.60 (dt, 1H); 7.75 (dd, 1H).

### Beispiel 19

### Herstellung von (-)-2-[(4R)-6-(4-Chlorphenyl)-1-methyl-4H-[1,2,4]triazolo[4,3-a][1]benzazepin-4-yl]-1-(1,1-dioxido-1-thia-6-azaspiro[3.3]hept-6-yl)ethanon

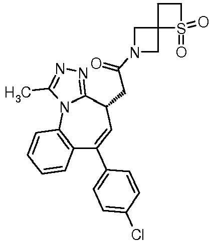

90 mg 2-[6-(4-Chlorphenyl)-1-methyl-4H-[1,2,4]triazolo[4,3-a][1]benzazepin-4-yl]-1-(1,1-dioxido-1-thia-6-azaspiro[3.3]hept-6-yl)ethanon wurden durch chirale HPLC (Chiralpak IA 5µm 250x20 mm, CO₂ / 2-Propanol (v/v) +0,2% DEA 60:40, 80 mL/min, 150bar, 40°C) in die Enantiomere getrennt.
Ausbeute: 32 mg (-)-2-[(4*R*)-6-(4-Chlorphenyl)-1-methyl-4H-[1,2,4]triazolo[4,3-a][1]benzazepin-4-yl]-1-(1,1-dioxido-1-thia-6-azaspiro[3.3]hept-6-yl)ethanon.
Optische Rotation: [α]_{D}²⁰ = -85.7° +/- 0.13° (c=10.4, Methanol).

### Beispiel 20

### Herstellung von 2-[6-(4-Chlorphenyl)-1-methyl-4H-[1,2,4]triazolo[4,3-a][1]benzazepin-4-yl]-N-(2-oxo-2,3-dihydro-1H-indol-5-yl)acetamid

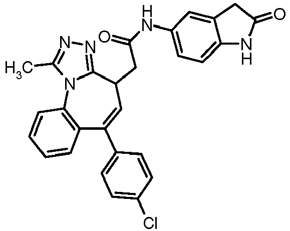

Eine Lösung von 110 mg [6-(4-Chlorphenyl)-1-methyl-4H-[1,2,4]triazolo[4,3-a][1]benzazepin-4-yl]essigsäure (Beispiel 3A), 171 mg HATU, 0.17 ml Triethylamin und 49 mg 5-Amino-indol-2-on in 2 ml DMF wurden bei Raumtemperatur für 14 Stunden gerührt. Der Ansatz wurde zwischen halbkonzentrierter Sole und Ethylacetat verteilt, die organische Phase abgetrennt und die Wasserphase zweimal mit Ethylacetat extrahiert. Die vereinten organischen Phasen wurden mit Sole gewaschen, über Natriumsulfat getrocknet und im Vakuum eingeengt. Der Rückstand wurde durch Chromatographie (Kieselgel, Dichlormethan / Methanol Gradient) gereinigt.
Ausbeute: 95 mg 2-[6-(4-Chlorphenyl)-1-methyl-4H-[1,2,4]triazolo[4,3-a][1]benzazepin-4-yl]-N-(2-oxo-2,3-dihydro-1H-indol-5-yl)acetamid.
¹H-NMR (300 MHz, CDCl₃): δ = 2.62 (s, 3H); 3.16 (dd, 1H); 3.46 (s, 2H); 3.49 (dd, 1H); 3.67-3.77 (m, 1H); 6.08 (d, 1H); 6.74 (d, 1H); 7.12 (d, 2H); 7.23-7.39 (m, 4H+CDCD₃); 7.41 (d, 2H); 7.48-7.57 (m, 2H), 7.74 (bs, 1H); 9.63 (bs, 1H).

### Beispiel 21

### Herstellung von (-)-2-[(4R)-6-(4-Chlorphenyl)-1-methyl-4H-[1,2,4]triazolo[4,3-a][1]benzazepin-4-yl]-N-(2-oxo-2,3-dihydro-1H-indol-5-yl)acetamid

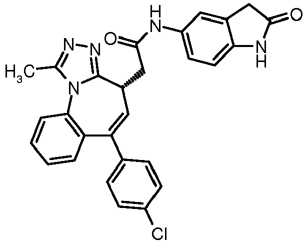

90 mg 2-[6-(4-Chlorphenyl)-1-methyl-4H-[1,2,4]triazolo[4,3-a][1]benzazepin-4-yl]-N-(2-oxo-2,3-dihydro-1H-indol-5-yl)acetamid wurden durch chirale HPLC (Chiralpak IA 5µm 250x20 mm, CO₂ / 2-Propanol (v/v) +0,2% DEA 60:40, 80 mL/min, 150bar, 40°C) in die Enantiomere getrennt.
Ausbeute: 30 mg (-)-2-[(4*R*)-6-(4-Chlorphenyl)-1-methyl-4H-[1,2,4]triazolo[4,3-a][1]benzazepin-4-yl]-N-(2-oxo-2,3-dihydro-1H-indol-5-yl)acetamid.
Optische Rotation: [α]_{D}²⁰ = -128.3° +/- 0.18° (c=10.0, Methanol).

### Beispiel 22

### Herstellung von 3-{[(-6-(4-Chlorphenyl)-1-methyl-4H-[1,2,4]triazolo[4,3-a][1]benzazepin-4-yl]acetyl}-8-azabicyclo[3.2.1]octan-3-on

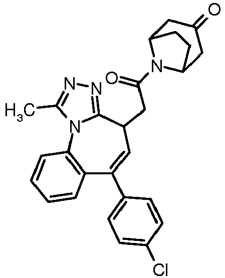

Eine Lösung von 110 mg [6-(4-Chlorphenyl)-1-methyl-4H-[1,2,4]triazolo[4,3-a][1]benzazepin-4-yl]essigsäure (Beispiel 3A), 171 mg HATU, 0.17 ml Triethylamin und 53 mg Nortropinon Hydrochlorid (CAS 25602-68-0) in 3 ml DMF wurden bei Raumtemperatur für 14 Stunden gerührt.

Der Ansatz wurde zwischen halbkonzentrierter Sole und Ethylacetat verteilt, die organische Phase abgetrennt und die Wasserphase zweimal mit Ethylacetat extrahiert. Die vereinten organischen Phasen wurden mit Sole gewaschen, über Natriumsulfat getrocknet und im Vakuum eingeengt. Der Rückstand wurde durch Chromatographie (Kieselgel, Dichlormethan / Methanol Gradient) gereinigt.
Ausbeute: 95 mg 3-{[(-6-(4-Chlorphenyl)-1-methyl-4H-[1,2,4]triazolo[4,3-a][1]benzazepin-4-yl]acetyl}-3-azabicyclo[3.2.1]octan-8-on.
¹H-NMR (300 MHz, CDCl₃, Signale diastereotoper Protonen enthalten): δ = 1.66-1.78 (m, 1H); 1.80-1.91 (m, 2H); 2.02-2.16 (m, 1H); 2.32-2.49 (m, 2H); 2.59-2.70 (m+s, 4H); 2.73-2.84 (m, 1H); 2.99 + 3.07 (2dd, 1H); 3.45 + 3.61 (2dd, 1H); 3.85 (q, 1H); 3.89-3.98 (m, 2H); 4.0-4.1 (m, 1H); 4.35-4.45 (m, 1H); 6.04 + 6.05 (2d, 1H); 7.13 (d, 2H); 7.25-7.33 (m, 3H+CDCl₃); 7.36-7.44 (m, 2H); 7.51 (dt, 1H).

### Beispiel 23

### Herstellung von (-)-3-{[(4R)-6-(4-Chlorphenyl)-1-methyl-4H-[1,2,4]triazolo[4,3-a][1]benzazepin-4-yl]acetyl}-3-azabicyclo[3.2.1]octan-8-on

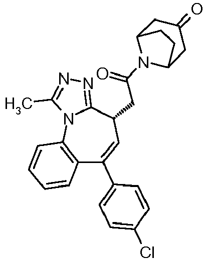

90 mg 3-{[6-(4-Chlorphenyl)-1-methyl-4H-[1,2,4]triazolo[4,3-a][1]benzazepin-4-yl]acetyl}-8-azabicyclo[3.2.1]octan-3-on wurden durch chirale HPLC (Chiralpak IA 5µm 250x20 mm, Ethanol / Methanol (v/v) 50:50, 20 mL/min, RT°C) in die Enantiomere getrennt.
Ausbeute: 38 mg (-)-3-{[(4*R*)-6-(4-Chlorphenyl)-1-methyl-4H-[1,2,4]triazolo[4,3-a][1]benzazepin-4-yl]acetyl}-3-azabicyclo[3.2.1]octan-8-on.
Optische Rotation: [α]_{D}²⁰ = -98.7° +/- 0.09° (c=10.0, Methanol).

### Beispiel 24

### Herstellung von 2-[6-(4-Chlorphenyl)-1-methyl-4H-[1,2,4]triazolo[4,3-a][1]benzazepin-4-yl]-1-(3-phenoxyazetidin-1-yl)ethanon

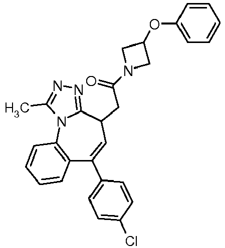

Eine Lösung von 110 mg [6-(4-Chlorphenyl)-1-methyl-4H-[1,2,4]triazolo[4,3-a][1]benzazepin-4-yl]essigsäure (Beispiel 3A), 171 mg HATU, 0.17 ml Triethylamin und 61.4 mg 3-Phenoxyazetidin Hydrochlorid in 3 ml DMF wurden bei Raumtemperatur für 14 Stunden gerührt. Der Ansatz wurde zwischen halbkonzentrierter Sole und Ethylacetat verteilt, die organische Phase abgetrennt und die Wasserphase 2 Mal mit Ethylacetat extrahiert. Die vereinten organischen Phasen wurden mit Sole gewaschen, über Natriumsulfat getrocknet und im Vakuum eingeengt. Der Rückstand wurde durch Chromatographie (Kieselgel, Dichlormethan / Methanol Gradient) gereinigt.
Ausbeute: 100 mg 2-[6-(4-Chlorphenyl)-1-methyl-4H-[1,2,4]triazolo[4,3-a][1]benzazepin-4-yl]-1-(3-phenoxyazetidin-1-yl)ethanon.
¹H-NMR (300 MHz, DMSO-d6): δ = ca. 2.50 (s, 3H, Signal durch DMSO verdeckt); 2.90 (ddd, 1H); 3.14 (ddd, 1H); 3.48 (dq, 1H); 3.80 (dt, 1H); 4.23-4.33 (m, 2H); 4.77 (ddd, 1H); 5.04-5.13 (m, 1H); 6.21 (d, 1H); 6.88 (d, 2H); 7.0 (t, 1H); 7.17-7.24 (m, 3H); 7.33 (dd, 2H); 7.42 (dd, 2H); 7.47 (t, 1H); 7.60 (t, 1H); 7.75 (d, 1H).

### Beispiel 25

### Herstellung von (-)-2-[(4R)-6-(4-Chlorphenyl)-1-methyl-4H-[1,2,4]triazolo[4,3-a][1]benzazepin-4-yl]-1-(3-phenoxyazetidin-1-yl)ethanon

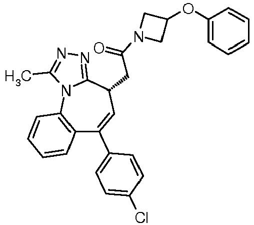

95 mg 2-[6-(4-Chlorphenyl)-1-methyl-4H-[1,2,4]triazolo[4,3-a][1]benzazepin-4-yl]-1-(3-phenoxyazetidin-1-yl)ethanon wurden durch chirale HPLC (Chiralpak IB 5µm 250x20 mm, CO₂ / Methanol (v/v) 70:30, 150 bar, 80 mL/min, 40°C) in die Enantiomere getrennt.
Ausbeute: 39 mg (-)-2-[(4*R*)-6-(4-Chlorphenyl)-1-methyl-4H-[1,2,4]triazolo[4,3-a][1]benzazepin-4-yl]-1-(3-phenoxyazetidin-1-yl)ethanon.
Optische Rotation: [α]_{D}²⁰ = -100.7° +/- 0.14° (c=10.0, Methanol).

### Beispiel 26

### Herstellung von 1-1[6-(4-Chlorphenyl)-1-methyl-4H-[1,2,4]triazolo[4,3-a][1]benzazepin-4-yl]acetyl}pyrrolidin-3-on

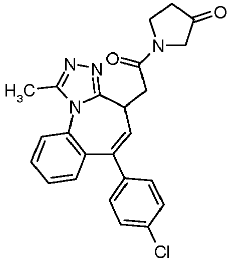

Eine Lösung von 110 mg [6-(4-Chlorphenyl)-1-methyl-4H-[1,2,4]triazolo[4,3-a][1]benzazepin-4-yl]essigsäure (Beispiel 3A), 171 mg HATU, 0.17 ml Triethylamin und 40.2 mg Pyrrolidin-3-on Hydrochlorid in 3 ml DMF wurden bei Raumtemperatur für 14 Stunden gerührt. Der Ansatz wurde zwischen halbkonzentrierter Sole und Ethylacetat verteilt, die organische Phase abgetrennt und die Wasserphase zweimal mit Ethylacetat extrahiert. Die vereinten organischen Phasen wurden mit Sole gewaschen, über Natriumsulfat getrocknet und im Vakuum eingeengt. Der Rückstand wurde durch Chromatographie (Kieselgel, Dichlormethan / Methanol Gradient) gereinigt.
Ausbeute: 60 mg 1-{[6-(4-Chlorphenyl)-1-methyl-4H-[1,2,4]triazolo[4,3-a][1]benzazepin-4-yl]acetyl}pyrrolidin-3-on.
¹H-NMR (300 MHz, CDCl₃, Signale diastereotoper Protonen enthalten): δ = 2.61+2.62 (2s, 3H); 2.61-2.69 (m, 1H); 2.75-2.84 (m, 1H); 3.00+ 3.07 (2dd, 1H); 3.46+3.62 (2dd, 1H); 3.86 (q, 1H); 3.90-3.98 (m, 2H); 4.01-4.11 (m, 1H); 4.36-4.47 (m, 1H); 6.05+6.06 (2d, 1H); 7.14 (d, 2H); 7.26-7.33 (m, 3H); 7.37-7.45 (m, 2H); 7.52 (dt, 1H).

### Beispiel 27

### Herstellung von (-)-1-{[(4R)-6-(4-Chlorphenyl)-1-methyl-4H-[1,2,4]triazolo[4,3-a][1]benzazepin-4-yl]acetyl}pyrrolidin-3-on

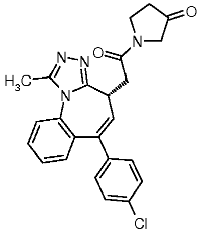

60 mg 1-{[6-(4-Chlorphenyl)-1-methyl-4H-[1,2,4]triazolo[4,3-a][1]benzazepin-4-yl]acetyl}pyrrolidin-3-on wurden durch chirale HPLC (Chiralpak IB 5µm 250x20 mm, CO₂ / Ethanol (v/v) 70:30, 150 bar, 60 mL/min, 40°C) in die Enantiomere getrennt.
Ausbeute: 19 mg (-)-1-{[(4*R*)-6-(4-Chlorphenyl)-1-methyl-4H-[1,2,4]triazolo[4,3-a][1]benzazepin-4-yl]acetyl}pyrrolidin-3-on.
Optische Rotation: [α]_{D}²⁰ = -82.9° +/- 0.29° (c=10.0, Methanol).

### Beispiel 28

### Herstellung von 1-(8-Azaspiro[4.5]dec-8-yl)-2-[6-(4-chlorphenyl)-1-methyl-4H-[1,2,4]triazolo[4,3-a][1]benzazepin-4-yl]ethanon

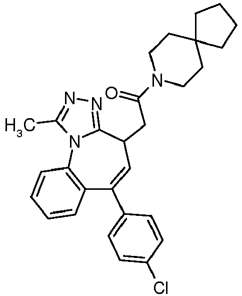

Eine Lösung von 110 mg [6-(4-Chlorphenyl)-1-methyl-4H-[1,2,4]triazolo[4,3-a][1]benzazepin-4-yl]essigsäure (Beispiel 3A), 171 mg HATU, 0.17 ml Triethylamin und 46.1 mg 8-Azaspiro[4.5]decan (CAS 176-64-7) in 3 ml DMF wurden bei Raumtemperatur für 14 Stunden gerührt. Der Ansatz wurde zwischen halbkonzentrierter Sole und Ethylacetat verteilt, die organische Phase abgetrennt und die Wasserphase zweimal mit Ethylacetat extrahiert. Die vereinten organischen Phasen wurden mit Sole gewaschen, über Natriumsulfat getrocknet und im Vakuum eingeengt. Der Rückstand wurde durch Chromatographie (Kieselgel, Dichlormethan / Methanol Gradient) gereinigt.
Ausbeute: 55 mg 1-(8-Azaspiro[4.5]dec-8-yl)-2-[6-(4-chlorphenyl)-1-methyl-4H-[1,2,4]triazolo[4,3-a][1]benzazepin-4-yl]ethanon.
¹H-NMR (500 MHz, DMSO-d6): δ = 1.33 (t, 2H); 1.42-1.47 (m, 4H); 1.49 (q, 2H); 1.58-1.62 (m, 4H); 3.08 (dd, 1H); 3.38-3.47 (m, 3H); 3.25-3.55 (m, 3H); 6.20 (d, 1H); 7.21 (dd, 1H); 7.22 (d, 2H); 7.41 (d, 2H); 7.47 (dt, 1H); 7.60 (dt, 1H); 7.75 (dd, 1H).
¹H-NMR (300 MHz, CDCl₃): δ = 1.43-1.56 (m, 6H); 1.61-1.72 (m, 6H); 2.61 (s, 3H); 3.20 (dd, 1H); 3.48 (dd, 1H); 3.56-3.65 (m, 4H); 3.76-3.83 (m, 1H); 6.04 (d, 1H); 7.15 (d, 2H); 7.25-7.33 (m, 3H+CHCl₃); 7.34-7.43 (m, 2H); 7.49 (dt, 1H).

### Beispiel 29

### Herstellung von (-)-1-(8-Azaspiro[4.5]dec-8-yl)-2-[(4R)-6-(4-chlorphenyl)-1-methyl-4H-[1,2,4]triazolo[4,3-a][1]benzazepin-4-yl]ethanon

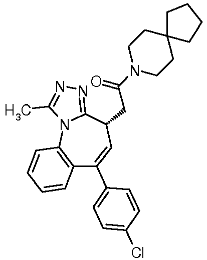

55 mg 1-(8-Azaspiro[4.5]dec-8-yl)-2-[6-(4-chlorphenyl)-1-methyl-4H-[1,2,4]triazolo[4,3-a][1]benzazepin-4-yl]ethanon wurden durch chirale HPLC (Chiralpak IA 5µm 250x20 mm, CO₂ / 2-Propanol (v/v) 60:40, 150 bar, 60 mL/min, 40°C) in die Enantiomere getrennt.
Ausbeute: 32 mg (-)-1-(8-Azaspiro[4.5]dec-8-yl)-2-[(*4R*)-6-(4-chlorphenyl)-1-methyl-4H-[1,2,4]triazolo[4,3-a][1]benzazepin-4-yl]ethanon.
Optische Rotation: [α]_{D}²⁰ = -97.3° +/- 0.16° (c=10.0, Methanol).

### Beispiel 30

### Herstellung von 2-[6-(4-Chlorphenyl)-1-methyl-4H-[1,2,4]triazolo[4,3-a][1]benzazepin-4-yl]-1-(1,1-dioxido-1,3-thiazolidin-3-yl)ethanon

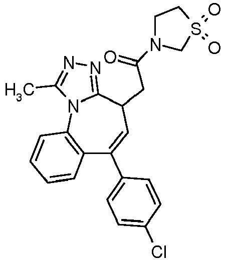

Eine Lösung von 110 mg [6-(4-Chlorphenyl)-1-methyl-4H-[1,2,4]triazolo[4,3-a][1]benzazepin-4-yl]essigsäure (Beispiel 3A), 171 mg HATU, 0.17 ml Triethylamin und 40.1 mg 1,1-Dioxido-1,3-thiazolidin in 3 ml DMF wurden bei Raumtemperatur für 14 Stunden gerührt. Der Ansatz wurde zwischen halbkonzentrierter Sole und Ethylacetat verteilt, die organische Phase abgetrennt und die Wasserphase zweimal mit Ethylacetat extrahiert. Die vereinten organischen Phasen wurden mit Sole gewaschen, über Natriumsulfat getrocknet und im Vakuum eingeengt. Der Rückstand wurde durch Chromatographie (Kieselgel, Dichlormethan / Methanol Gradient) gereinigt.
Ausbeute: 65 mg 2-[6-(4-Chlorphenyl)-1-methyl-4H-[1,2,4]triazolo[4,3-a][1]benzazepin-4-yl]-1-(1,1-dioxido-1,3-thiazolidin-3-yl)ethanon.
¹H-NMR (300 MHz, DMSO-d6, Signale diastereotoper Protonen enthalten, ausgewählte Signale): δ = ca. 2.50 (s, 3H, Signal durch DMSO verdeckt); 3.1-3.24 (m, 1H); 3.45 (t, 1H); 3.53 (dd, 1H); 3.60 (t, 1H); 3.75-3.95 (m, 1H); 4.16-4.25 (m, 1H); 4.43-4.55 (m, 1H); 4.89 (s, 1H); 6.18+6.22 (2d, 1H); 7.18-7.27 (m, 3H); 7.42 (d, 1H); 7.48 (dt, 1H); 7.61 (dt, 1H); 7.76 (dd, 1H).

### Beispiel 31

### Herstellung von (-)-2-[(4R)-6-(4-Chlorphenyl)-1-methyl-4H-[1,2,4]triazolo[4,3-a][1]benzazepin-4-yl]-1-(1,1-dioxido-1,3-thiazolidin-3-yl)ethanon

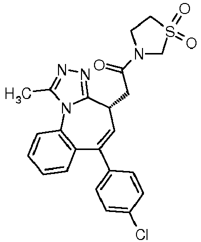

65 mg 2-[6-(4-Chlorphenyl)-1-methyl-4H-[1,2,4]triazolo[4,3-a][1]benzazepin-4-yl]-1-(1,1-dioxido-1,3-thiazolidin-3-yl)ethanon wurden durch chirale HPLC (Chiralpak IA 5µm 250x20 mm, CO₂ / Methanol (v/v) 60:40, 150 bar, 80 mL/min, 40°C) in die Enantiomere getrennt.
Ausbeute: 23 mg (-)-2-[(4*R*)-6-(4-Chlorphenyl)-1-methyl-4H-[1,2,4]triazolo[4,3-a][1]benzazepin-4-yl]-1-(1,1-dioxido-1,3-thiazolidin-3-yl)ethanon.
Optische Rotation: [α]_{D}²⁰ = -97.8° +/- 0.20° (c=10.0, Methanol).

### Beispiel 32

### Herstellung von 2-[6-(4-Chlorphenyl)-1-methyl-4H-[1,2,4]triazolo[4,3-a][1]benzazepin-4-yl]-1-(8-oxa-3-azabicyclo[3.2.1]oct-3-yl)ethanon

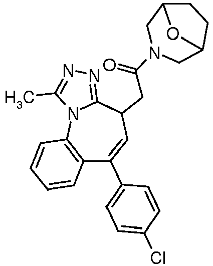

Eine Lösung von 110 mg [6-(4-Chlorphenyl)-1-methyl-4H-[1,2,4]triazolo[4,3-a][1]benzazepin-4-yl]essigsäure (Beispiel 3A), 171 mg HATU, 0.17 ml Triethylamin und 49.5 mg 8-Oxa-3-azabicyclo[3.2.1]octan Hydrochlorid (CAS 54745-74-3) in 3 ml DMF wurden bei Raumtemperatur für 14 Stunden gerührt. Der Ansatz wurde zwischen halbkonzentrierter Sole und Ethylacetat verteilt, die organische Phase abgetrennt und die Wasserphase zweimal mit Ethylacetat extrahiert. Die vereinten organischen Phasen wurden mit Sole gewaschen, über Natriumsulfat getrocknet und im Vakuum eingeengt. Der Rückstand wurde durch Chromatographie (Kieselgel, Dichlormethan / Methanol Gradient) gereinigt.
Ausbeute: 95 mg 2-[6-(4-Chlorphenyl)-1-methyl-4H-[1,2,4]triazolo[4,3-a][1]benzazepin-4-yl]-1-(8-oxa-3-azabicyclo[3.2.1]oct-3-yl)ethanon.
¹H-NMR (300 MHz, DMSO-d6, ausgewählte Signale): δ = 1.46-1.60 (m, 1H); 1.73-2.03 2m, 3H); ca. 2.50 (s, 3H, Signal durch DMSO verdeckt); 2.72-2.85 (m, 1H); 2.88-3.00 (m, 1/2H); 3.14-3.41 (m+Wasser); 3.48 (t, 1/2H); 3.52-3.59 (m, 1H); 3.75 (dd, 1H); 3.95 (dd, 1H); 3.28-3.39 (m, 2H); 6.17-6.24 (m, 1H); 7.17-7.26 (m, 3H); 7.38-7.46 (m, 2H); 7.47 dt, 1H); 7.60 (dt, 1H); 7.75 (bd, 1H).

### Beispiel 33

### Herstellung von (-)-2-[(4R)-6-(4-Chlorphenyl)-1-methyl-4H-[1,2,4]triazolo[4,3-a][1]benzazepin-4-yl]-1-(8-oxa-3-azabicyclo[3.2.1]oct-3-yl)ethanon

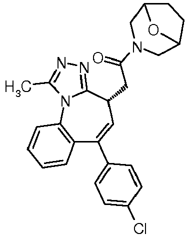

65 mg 2-[6-(4-Chlorphenyl)-1-methyl-4H-[1,2,4]triazolo[4,3-a][1]benzazepin-4-yl]-1-(8-oxa-3-azabicyclo[3.2.1]oct-3-yl)ethanon wurden durch chirale HPLC (Chiralpak ID 5µm 250x20 mm, CO₂ / Ethanol (v/v) 60:40, 150 bar, 80 mL/min, 40°C) in die Enantiomere getrennt.
Ausbeute: 33 mg (-)-2-[(4*R*)-6-(4-Chlorphenyl)-1-methyl-4H-[1,2,4]triazolo[4,3-a][1]benzazepin-4-yl]-1-(8-oxa-3-azabicyclo[3.2.1]oct-3-yl)ethanon.
Optische Rotation: [α]_{D}²⁰ = -93.° +/- 0.10° (c=10.0, Methanol).

### Beispiel 34

### Herstellung von 6-(4-Chlorphenyl)-1-methyl-4-[(3-methyl-1,2,4-oxadiazol-5-yl)methyl]-4H-[1,2,4]triazolo[4,3-a][1]benzazepin

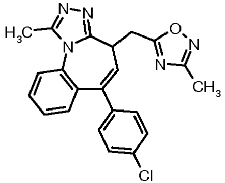

108 mg Acetamidoxim (CAS 22059-22-9) in 0.46 ml 1-Methyl-2-pyrrolidon wurde bei Raumtemperatur gerührt, bis sich eine homogene Lösung bildete. Dazu wurde eine Lösung von 130 mg [6-(4-Chlorphenyl)-1-methyl-4H-[1,2,4]triazolo[4,3-a][1]benzazepin-4-yl]essigsäure-tert-butylester (Beispiel 8) in 0.46 ml 1-Methyl-2-pyrrolidon gegeben und langsam 63 mg Natriummethylat zugegeben. Es wurde 3 Stunden bei 70°C gerührt. Der Ansatz wurde auf Wasser gegeben und dreimal mit Ethylacetat extrahiert. Die vereinten organischen Phasen wurden mit Sole gewaschen, über Natriumsulfat getrocknet und im Vakuum vollständig eingeengt. Der Rückstand wurde durch Chromatographie an Kieselgel (Dichlormethan / Methanol Gradient) gereinigt.
Ausbeute: 36 mg 6-(4-Chlorphenyl)-1-methyl-4-[(3-methyl-1,2,4-oxadiazol-5-yl)methyl]-4H-[1,2,4]triazolo[4,3-a][1]benzazepin.
¹H-NMR (400 MHz, DMSO-d6): δ = 2.29 (s, 3H); ca. 2.50 (s, 3H, Signal durch DMSO verdeckt); 3.69-3.77 (m, 2H); 3.86 (dd, 1H); 6.35 (d, 1H); 7.20-7.28 (m, 3H); 7.43 (d, 2H); 7.49 (dt, 1H); 7.62 (dt, 1H); 7.79 (d, 1H).

### Beispiel 35

### Herstellung von (-)-6-[(4R)-4-Chlorphenyl]-1-methyl-4-[(3-methyl-1,2,4-oxadiazol-5-yl)methyl]-4H-[1,2,4]triazolo[4,3-a][1]benzazepin

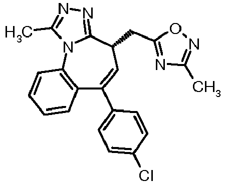

27 mg 6-(4-Chlorphenyl)-1-methyl-4-[(3-methyl-1,2,4-oxadiazol-5-yl)methyl]-4H-[1,2,4]triazolo[4,3-a][1]benzazepin wurden durch chirale HPLC (Chiralpak IA 5µm 250x20 mm, CO₂ / 2-Propanol (v/v) 75:25, 150 bar, 80 mL/min, 40°C) in die Enantiomere getrennt.
Ausbeute: 4 mg (-)-6-[(4*R*)-4-Chlorphenyl]-1-methyl-4-[(3-methyl-1,2,4-oxadiazol-5-yl)methyl]-4H-[1,2,4]triazolo[4,3-a][1]benzazepin.
Optische Rotation: [α]_{D}²⁰ = -141.7° (c=10.0, Methanol).

### Beispiel 36

### Herstellung von (-)-(4R)-6-(4-Chlorphenyl)-1-methyl-4-{[3-(propan-2-yl)-1,2,4-oxadiazol-5-yl]methyl}-4H-[1,2,4]triazolo[4,3-a][1]benzazepin

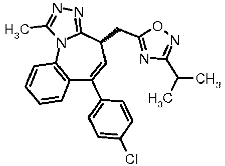

344 mg 2-Methylpropylimidoxim (CAS 35613-84-4) in 0.53 ml 1-Methyl-2-pyrrolidon wurde bei Raumtemperatur gerührt, bis sich eine homogene Lösung bildete. Dazu wurde eine Lösung von 150 mg [6-(4-Chlorphenyl)-1-methyl-4H-[1,2,4]triazolo[4,3-a][1]benzazepin-4-yl]essigsäure-*tert-*butylester (Beispiel 8) in 0.53 ml 1-Methyl-2-pyrrolidon gegeben und langsam 73 mg Natriummethylat zugegeben. Es wurde 3 Stunden bei 70°C gerührt. Es wurden weitere 73 mg Natriummethylat zugegeben und 10 Stunden bei 90°C gerührt. Der Ansatz wurde auf Wasser gegeben und dreimal mit Ethylacetat extrahiert. Die vereinten organischen Phasen wurden mit Sole gewaschen, über Natriumsulfat getrocknet und im Vakuum vollständig eingeengt. Der Rückstand wurde durch Chromatographie an Kieselgel (Dichlormethan / Methanol Gradient) gereinigt. Es wurden 74 mg des Racemates erhalten, welche durch chirale HPLC (Chiralpak IA 5µm 250x20 mm, CO₂ / 2-Propanol+0.4 % Diethylamin (v/v) 78:22, 150 bar, 80 mL/min, 40°C) in die Enantiomere getrennt wurden.
Ausbeute: 6 mg (-)-(4*R*)-6-(4-Chlorphenyl)-1-methyl-4-{[3-(propan-2-yl)-1,2,4-oxadiazol-5-yl]methyl}-4H-[1,2,4]triazolo[4,3-a][1]benzazepin.
¹H-NMR (300 MHz, DMSO-d6, z.T. diastereotope Signalsätze): δ = 1.22 und 1.24 (2d, 6H); ca. 2.50 (s, 3H, Signal durch DMSO verdeckt); 3.02 (sept, 1H); 3.70-3.89 (m, 3H); 6.33 (d, 1H); 7.19-7.27 (m, 3H); 7.43 (d, 2H); 7.49 (dt, 1H); 7.62 (dt, 1H); 7.78 (d, 1H).
Optische Rotation: [α]_{D}²⁰ = -48.0° (c=10.0, Methanol).

### Beispiel 37

### Herstellung von (-)-(4R)-6-(4-Chlorphenyl)-4-[(3-cyclopropyl-1,2,4-oxadiazol-5-yl)methyl]-1-methyl-4H-[1,2,4]triazolo[4,3-a][1]benzazepin

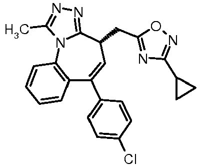

338 mg N'-Hydroxycyclopropancarboximidamid (CAS 51285-13-3) in 0.53 ml 1-Methyl-2-pyrrolidon wurde bei Raumtemperatur gerührt, bis sich eine homogene Lösung bildete. Dazu wurde eine Lösung von 150 mg [6-(4-Chlorphenyl)-1-methyl-4H-[1,2,4]triazolo[4,3-a][1]benzazepin-4-yl]essigsäure-*tert*-butylester (Beispiel 8) in 0.53 ml 1-Methyl-2-pyrrolidon gegeben und langsam 73 mg Natriummethylat zugegeben. Es wurde 3 Stunden bei 70°C gerührt. Es wurden weitere 73 mg Natriummethylat zugegeben und 3 Stunden bei 90°C gerührt. Der Ansatz wurde auf Wasser gegeben und 3-mal mit Ethylacetat extrahiert. Die vereinten organischen Phasen wurden mit Sole gewaschen, über Natriumsulfat getrocknet und im Vakuum vollständig eingeengt. Der Rückstand wurde durch Chromatographie an Kieselgel (Dichlormethan / Methanol Gradient) gereinigt. Es wurden 66 mg des Racemates erhalten, welche durch chirale HPLC (Chiralpak IA 5µm 250x20 mm, CO₂ / 2-Propanol+0.2 % Diethylamin (v/v) 75:25, 150 bar, 80 mL/min, 40°C) in die Enantiomere getrennt wurden.
Ausbeute: 11 mg (-)-(4*R*)-6-(4-Chlorphenyl)-4-[(3-cyclopropyl-1,2,4-oxadiazol-5-yl)methyl]-1-methyl-4H-[1,2,4]triazolo[4,3-a][1]benzazepin.
¹H-NMR (300 MHz, DMSO-d6): δ = 0.80-0-87 (m, 2H); 0.99-1.07 (m, 2H); 2.02-2.12 (m, 1H); ca. 2.50 (s, 3H, Signal durch DMSO verdeckt); 3.65-3.87 (m, 3H); 6.32 (d, 1H); 7.19-7.26 (m, 3H); 7.43 (d, 2H); 7.48 (dt, 1H); 7.62 (dt, 1H); 7.78 (d, 1H).
Optische Rotation: [α]_{D}²⁰ = -75.9° (c=10.0, Methanol).

### Beispiel 38

### Herstellung von (-)-(4R)-6-(4-Chlorphenyl)-1-methyl-4-[(5-methyl-1,3,4-oxadiazol-2-yl)methyl]-4H-[1,2,4]triazolo[4,3-a][1]benzazepin

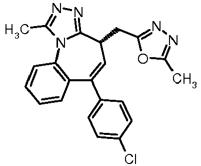

Eine Lösung von 150 mg [6-(4-Chlorphenyl)-1-methyl-4H-[1,2,4]triazolo[4,3-a][1]benzazepin-4-yl]essigsäure (Beispiel 3A), 57 mg Acetylhydrazin, 0.27 ml Triethylamin und 1.24 g 1-Propanphosphonsäure cyclisches Anhydrid (T3P, CAS 68957-94-8) in 1.2 ml Ethylacetat wurde 4 Stunden bei 90°C gerührt. Der Ansatz wurde auf Sole gegeben und dreimal mit Ethylacetat extrahiert. Die vereinten organischen Phasen wurden über Natriumsulfat getrocknet und im Vakuum vollständig eingeengt. Der Rückstand wurde in 1 ml Acetonitril gelöst und es wurde 0.06 ml Phosphoroxychlorid bei Eisbadkühlung zugegeben. Es wurde auf 80°C erhitzt und 3 Stunden gerührt. Der Ansatz wurde auf Sole gegeben und dreimal mit Ethylacetat extrahiert. Die vereinten organischen Phasen wurden über Natriumsulfat getrocknet und das Lösungsmittel im Vakuum entfernt. Der Rückstand wurde durch Chromatographie an Kieselgel (Dichlormethan / Methanol Gradient) gereinigt. Es wurden 32 mg des Racemates erhalten, welche durch chirale HPLC (Chiralpak IA 5µm 250x20 mm, CO₂ / 2-Propanol+0.2 % Diethylamin (v/v) 75:25, 150 bar, 80 mL/min, 40°C) in die Enantiomere getrennt wurden.
Ausbeute: 10 mg (-)-(4*R*)-6-(4-Chlorphenyl)-1-methyl-4-[(5-methyl-1,3,4-oxadiazol-2-yl)methyl]-4H-[1,2,4]triazolo[4,3-a][1]benzazepin.
¹H-NMR (300 MHz, DMSO-d6): δ = 2.47 (s, 3H); ca. 2.50 (s, 3H, Signal durch DMSO verdeckt); 3.65-3.80 (m, 3H); 6.32 (d, 1H); 7.19-7.27 (m, 3H); 7.42 (d, 2H); 7.49 (dt, 1H); 7.62 (dt, 1H); 7.77 (d, 1H).
Optische Rotation: [α]_{D}²⁰ = -95.7° (c=10.0, Methanol).

Die absolute Stereochemie des Kohlenstoffatoms C4 wurde durch Röntgenstrukturanalyse in der (*R*)-Konfiguration bestätigt.

### Beispiel 39

### Herstellung von 6-(4-Chlorphenyl)-1-methyl-4-[(5-methyl-1,3,4-oxadiazol-2-yl)methyl]-4H-[1,2,4]triazolo[4,3-a][1]benzazepin

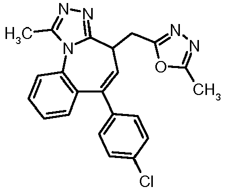

Eine Lösung von 10.1 g 6-(4-Chlorphenyl)-1-methyl-4H-[1,2,4]triazolo[4,3-a][1]benzazepin (Beispiel 1C) in 1 L THF wurde bei -70°C langsam mit 36 ml Lithiumhexamethyldisilazid-Lösung (1M in Toluol) versetzt. Es wurde 90 min. bei -70°C gerührt und dann eine Lösung von 4.35 g 2-(Chlormethyl)-5-methyl-1,3,4-oxadiazol (CAS 3914-42-9) in 15 ml THF zugetropft. Unter Erwärmung auf RT wurde 16 Stunden gerührt. Der Ansatz wurde auf gesättigte Ammoniumchlorid Lösung gegeben und dreimal mit Dichlormethan extrahiert. Die vereinten organischen Phasen wurden mit 50%iger Sole gewaschen, über Natriumsulfat getrocknet und das Lösungsmittel im Vakuum entfernt. Der Rückstand wurde durch Chromatographie an Kieselgel (Dichlormethan / Methanol Gradient) gereinigt.
Ausbeute: 9.2 g 6-(4-Chlorphenyl)-1-methyl-4-[(5-methyl-1,3,4-oxadiazol-2-yl)methyl]-4H-[1,2,4]triazolo[4,3-a][1]benzazepin.
¹H-NMR (300 MHz, DMSO-d6): δ = 2.47 (s, 3H); ca. 2.50 (s, 3H, Signal durch DMSO verdeckt);3.65-3.80 (m, 3H); 6.32 (d, 1H); 7.19-7.27 (m, 3H); 7.42 (d, 2H); 7.49 (dt, 1H); 7.62 (dt, 1H); 7.77 (d, 1H).

### Beispiel 40

### Herstellung von N'-Acetyl-2-[(4R)-6-(4-chlorphenyl)-1-methyl-4H-[1,2,4]triazolo[4,3-a][1]benzazepin-4-yl]acetohydrazid

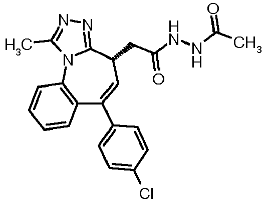

Eine Lösung von 1 g [6-(4-Chlorphenyl)-1-methyl-4H-[1,2,4]triazolo[4,3-a][1]benzazepin-4-yl]essigsäure (Beispiel 3A), 148 mg HATU, 1.5 ml Triethylamin und 212 mg Acetylhydrazin in 14 ml DMF wurden bei Raumtemperatur für 14 Stunden gerührt. Der Ansatz wurde auf ges. Natriumhydrogencarbonat Lösung gegeben und 3 Mal mit Dichlormethan extrahiert. Die vereinten organischen Phasen wurden mit Sole gewaschen, über Natriumsulfat getrocknet und im Vakuum eingeengt. Der Rückstand wurde durch Chromatographie (Kieselgel, Dichlormethan / Methanol Gradient) gereinigt. Das erhaltene Racemat (210 mg) der Titelverbindung wurde durch chirale HPLC (Chiralpak IA 5µm 250x20 mm, CO₂ / Ethanol+0.4 % Diethylamin (v/v) 60:40, 150 bar, 80 mL/min, 40°C) in die Enantiomere getrennt.
Ausbeute: 20 mg N'-Acetyl-2-[(4*R*)_6-(4-chlorphenyl)-1-methyl-4H-[1,2,4]triazolo[4,3-a][1]benzazepin-4-yl]acetohydrazid.
¹H-NMR (300 MHz, DMSO-d6): δ = 1.86 (s, 3H); ca. 2.50 (s, 3H, Signal durch DMSO verdeckt);3.02 (d, 2H); 3.45 (q, 1H); 6.22 (d, 1H); 7.19 (dd, 1H); 7.28 (d, 2H); 7.40 (d, 2H); 7.46 (dt, 1H); 7.59 (dt, 1H); 7.73 (dd, 1H); 9.79 (bs, 1H); 10.03 (bs, 1H).

### Beispiel 41

### Herstellung von 6-(4-Chlorphenyl)-1-methyl-4-[(5-methyl-1,3,4-thiadiazol-2-yl)methyl]-4H-[1,2,4]triazolo[4,3-a][1]benzazepin

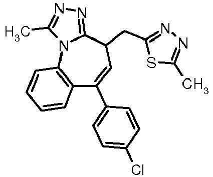

Eine Lösung von 50 mg N'-Acetyl-2-[6-(4-chlorphenyl)-1-methyl-4H-[1,2,4]triazolo[4,3-a][1]benzazepin-4-yl]acetohydrazid (Beispiel 40, Racemat) und 45 mg Lawesson's Reagenz in 2 ml THF wurde 2 Stunden bei 65°C gerührt. Es wurde auf Natronlauge (1N) gegeben und dreimal mit Dichlormethan extrahiert. Die vereinten organischen Phasen wurden mit Sole gewaschen, über Natriumsulfat getrocknet und das Lösungsmittel vollständig im Vakuum entfernt. Der Rückstand wurde durch Chromatographie an Kieselgel (Dichlormethan / Methanol Gradient) gereinigt.
Ausbeute: 40 mg 6-(4-Chlorphenyl)-1-methyl-4-[(5-methyl-1,3,4-thiadiazol-2-yl)methyl]-4H-[1,2,4]triazolo[4,3-a][1]benzazepin.
¹H-NMR (300 MHz, DMSO-d6): δ = ca. 2.50 (s, 3H, Signal durch DMSO verdeckt); 2.67 (s, 3H); 3.65 (dd, 1H); 3.85 (dd, 1H); 3.99 (dd, 1H); 6.29 (d, 1H); 7.17-7.25 (m, 3H); 7.42 (d, 2H); 7.47)dt, 1H); 7.61 (dt, 1H); 7.76 (dd, 1H).

### Beispiel 42

### Herstellung von (+)-(4R)-6-(4-Chlorphenyl)-1-methyl-4-[(5-methyl-1,3,4-thiadiazol-2-yl)methyl]-4H-[1,2,4]triazolo[4,3-a][1]benzazepin

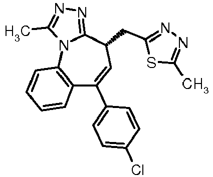

80 mg 6-(4-Chlorphenyl)-1-methyl-4-[(5-methyl-1,3,4-thiadiazol-2-yl)methyl]-4H-[1,2,4]triazolo[4,3-a][1]benzazepin wurden durch chirale HPLC (Chiralpak IC 5µm 250x20 mm, Acetonitril / Ethanol 90:10, 31 mL/min, RT) in die Enantiomere getrennt.
Ausbeute: 30 mg (+)-(4*R*)-6-(4-Chlorphenyl)-1-methyl-4-[(5-methyl-1,3,4-thiadiazol-2-yl)methyl]-4H-[1,2,4]triazolo[4,3-a][1]benzazepin.
¹H-NMR (300 MHz, DMSO-d6): δ = ca. 2.50 (s, 3H, Signal durch DMSO verdeckt); 2.67 (s, 3H); 3.65 (dd, 1H); 3.85 (dd, 1H); 3.99 (dd, 1H); 6.29 (d, 1H); 7.17-7.25 (m, 3H); 7.42 (d, 2H); 7.47)dt, 1H); 7.61 (dt, 1H); 7.76 (dd, 1H).
Optische Rotation: [α]_{D}²⁰ = +105.3° (c=10.0, Methanol).

### Beispiel 43

### Herstellung von 6-(4-Chlorphenyl)-4-[(5-cyclopropyl-1,3,4-oxadiazol-2-yl)methyl]-1-methyl-4H-[1,2,4]triazolo[4,3-a][1]benzazepin

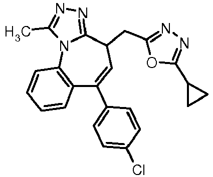

### Beispiel 43A

### Herstellung von N'-{2-[6-(4-Chlorphenyl)-1-methyl-4H-[1,2,4]triazolo[4,3-a][1]benzazepin-4-yl]acetyl}cyclopropanecarbohydrazid

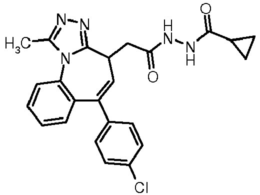

Eine Lösung von 50 mg [6-(4-Chlorphenyl)-1-methyl-4H-[1,2,4]triazolo[4,3-a][1]benzazepin-4-yl]essigsäure (Beispiel 3A), 23 mg 1-(3-Dimethylaminopropyl)-3-ethylcarbodiimid Hydrochlorid, 18 mg 1-Hydroxy-1H-benzotriazol Hydrat und 12 mg Cyclopropancarbonsäurehydrazid (CAS 6952-93-8) in 0.6 ml THF wurden bei Raumtemperatur für 14 Stunden gerührt. Der Ansatz wurde auf ges. Natriumhydrogencarbonat Lösung gegeben und 3 Mal mit Dichlormethan extrahiert. Die vereinten organischen Phasen wurden mit Sole gewaschen, über Natriumsulfat getrocknet und im Vakuum eingeengt. Der Rückstand wurde durch Chromatographie (Kieselgel, Dichlormethan / Methanol Gradient) gereinigt. Man erhielt 29 mg N'-{2-[6-(4-Chlorphenyl)-1-methyl-4H-[1,2,4]triazolo[4,3-a][1]benzazepin-4-yl]acetyl}cyclopropanecarbohydrazid.
¹H-NMR (300 MHz, DMSO-d6): δ = 0.67-0-79 (m, 4H); 1.56-1.68 (m, 1H); ca. 2.50 (s, 3H, Signal durch DMSO verdeckt); 2.97-3.06 (m, 2H); 3.40-3.52 (m, 1H); 6.22 (d, 1H); 7.19 (d, 1H); 7.26 (d, 2H); 7.40 (d, 2H); 7.46 (t, 1H); 7.60 (t, 1H); 7.73 (d, 1H); 10.02 (s, 1H); 10.04 (s, 1H).

### Beispiel 43B

### Herstellung von 6-(4-Chlorphenyl)-4-[(5-cyclopropyl-1,3,4-oxadiazol-2-yl)methyl]-1-methyl-4H-[1,2,4]triazolo[4,3-a][1]benzazepin

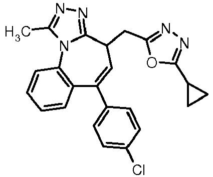

Eine Lösung von 24 mg N'-{2-[6-(4-Chlorphenyl)-1-methyl-4H-[1,2,4]triazolo[4,3-a][1]benzazepin-4-yl]acetyl}cyclopropanecarbohydrazid (Beispiel 43A) in 0.25 ml Acetonitril wurde bei Eisbadkühlung mit 0.028 ml Phosphoroxychlorid versetzt. Es wurde auf 95°C erhitzt und 3 Stunden gerührt. Der Ansatz wurde auf Sole gegeben und dreimal mit Ethylacetat extrahiert. Die vereinten organischen Phasen wurden über Natriumsulfat getrocknet und das Lösungsmittel im Vakuum entfernt. Der Rückstand wurde durch Chromatographie an Kieselgel (Dichlormethan / Methanol Gradient) gereinigt.
Ausbeute: 13 mg 6-(4-Chlorphenyl)-4-[(5-cyclopropyl-1,3,4-oxadiazol-2-yl)methyl]-1-methyl-4H-[1,2,4]triazolo[4,3-a][1]benzazepin.
¹H-NMR (300 MHz, DMSO-d6): δ = 0.94-1.00 (m, 2H); 1.07-1.16 (m, 2H); 2.16-2.25 (m, 1H); ca. 2.50 (s, 3H, Signal durch DMSO verdeckt); 3.56-3.76 (m, 3H); 6.32 (d, 2H); 7.19-7.27 (m, 3H); 7.43 (d, 2H); 7.48 (dt, 1H); 7.62 (dt, 1H); 7.77 (dd, 1H).

### Beispiel 44

### Herstellung von 6-(4-Chlorphenyl)-1-methyl-4-{[5-(pyridin-3-yl)-1,3,4-oxadiazol-2-yl]methyl}-4H-[1,2,4]triazolo[4,3-a][1]benzazepin

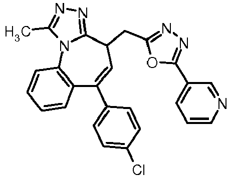

In Analogie zur Herstellung von Beispiel 39 wurde ausgehend von 280 mg 6-(4-Chlorphenyl)-1-methyl-4H-[1,2,4]triazolo[4,3-a][1]benzazepin (Beispiel 1C) und 169 mg 3-[5-(Chlormethyl)-1,3,4-oxadiazol-2-yl]pyridin (CAS 677347-79-4) 145 mg 6-(4-Chlorphenyl)-1-methyl-4-{[5-(pyridin-3-yl)-1,3,4-oxadiazol-2-yl]methyl}-4H-[1,2,4]triazolo[4,3-a][1]benzazepin erhalten.
Ausbeute: 145 mg 6-(4-Chlorphenyl)-1-methyl-4-{[5-(pyridin-3-yl)-1,3,4-oxadiazol-2-yl]methyl}-4H-[1,2,4]triazolo[4,3-a][1]benzazepin.
¹H-NMR (300 MHz, DMSO-d6): δ = 2.52 (s, 3H); 3.75-3.98 (m, 3H); 6.41 (d, 1H); 7.20-7.30 (m, 3H); 7.43 (d, 2H); 7.49 (dt, 1H); 7.57-7.69 (m, 2H); 7.79 (dd, 1H); 8.36 (dt, 1H); 8.80 (dd, 1H); 9.16 (dd, 1H).

### Beispiel 45

### Herstellung von (-)-(4R)-6-(4-Chlorphenyl)-1-methyl-4-{[5-(pyridin-3-yl)-1,3,4-oxadiazol-2-yl]methyl}-4H-[1,2,4]triazolo[4,3-a][1]benzazepin

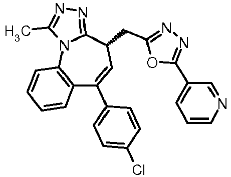

145 mg 6-(4-Chlorphenyl)-1-methyl-4-{[5-(pyridin-3-yl)-1,3,4-oxadiazol-2-yl]methyl}-4H-[1,2,4]triazolo[4,3-a][1]benzazepin wurden durch chirale HPLC (Chiralpak IC 5µm 250x20 mm, Acetonitril / Ethanol 90:10, 50 mL/min, RT) in die Enantiomere getrennt.
Ausbeute: 46 mg (-)-(4*R*)-6-(4-Chlorphenyl)-1-methyl-4-{[5-(pyridin-3-yl)-1,3,4-oxadiazol-2-yl]methyl}-4H-[1,2,4]triazolo[4,3-a][1]benzazepin.
¹H-NMR (300 MHz, DMSO-d6): δ = 2.52 (s, 3H); 3.75-3.98 (m, 3H); 6.41 (d, 1H); 7.20-7.30 (m, 3H); 7.43 (d, 2H); 7.49 (dt, 1H); 7.57-7.69 (m, 2H); 7.79 (dd, 1H); 8.36 (dt, 1H); 8.80 (dd, 1H); 9.16 (dd, 1H).
Optische Rotation: [α]_{D}²⁰ = -152.1° (c=8.0, Methanol).

### Beispiel 46

### Herstellung von 6-(4-Chlorphenyl)-1-methyl-4-[(5-methyl-1,2,4-oxadiazol-3-yl)methyl]-4H-[1,2,4]triazolo[4,3-a][1]benzazepin

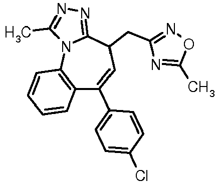

In Analogie zur Herstellung von Beispiel 39 wurde ausgehend von 150 mg 6-(4-Chlorphenyl)-1-methyl-4H-[1,2,4]triazolo[4,3-a][1]benzazepin (Beispiel 1C) und 61 mg 3-(Chlormethyl)-5-methyl-1,2,4-oxadiazol (CAS 1192-80-9) 12 mg 6-(4-Chlorphenyl)-1-methyl-4-[(5-methyl-1,2,4-oxadiazol-3-yl)methyl]-4H-[1,2,4]triazolo[4,3-a][1]benzazepin erhalten.
Ausbeute: 12 mg 6-(4-Chlorphenyl)-1-methyl-4-[(5-methyl-1,2,4-oxadiazol-3-yl)methyl]-4H-[1,2,4]triazolo[4,3-a][1]benzazepin.
¹H-NMR (300 MHz, DMSO-d6): δ = ca. 2.50 (s, 3H, Signal durch DMSO verdeckt); 2.55 (s, 3H); 3.51 (dd, 1H); 3.57-3.69 (m, 2H); 6.27 (d, 1H); 7.18-7.25 (m, 3H); 7.42 (d, 2H); 7.47 (dt, 1H); 7.61 (dt, 1H); 7.77 (dd, 1H).

### Beispiel 47

### Herstellung von 6-(4-Chlorphenyl)-8-methoxy-1-methyl-4-[(5-methyl-1,3,4-oxadiazol-2-yl)methyl]-4H-[1,2,4]triazolo[4,3-a][1]benzazepin

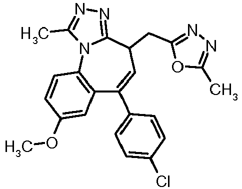

### Beispiel 47A

### Herstellung von 7-Methoxy-2-thioxo-1,2,3,4-tetrahydro-5H-1-benzazepin-5-on

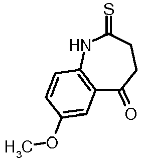

In Analogie zur Herstellung von 2-Thioxo-1,2,3,4-tetrahydro-5H-1-benzazepin-5-on (Beispiel 1D) wurden ausgehend von 50 g 7-Methoxy-3,4-dihydro-1H-1-benzazepine-2,5-dion (Arch. Pharm. 2002, 335, S. 311-17, K. Wieking et al.) und 72.9 g Lawesson's Reagenz 40.9 g 7-Methoxy-2-thioxo-1,2,3,4-tetrahydro-5H-1-benzazepin-5-on erhalten.
Ausbeute: 40.9 g 7-Methoxy-2-thioxo-1,2,3,4-tetrahydro-5H-1-benzazepin-5-on.
¹H-NMR (300 MHz, DMSO-d6): δ = 2.91-2.98 (m, 2H); 3.08-3.17 (m, 2H); 3.80 (s, 3H); 7.20-7.29 (m, 3H); 11.98 (bs, 1H).

### Beispiel 47B

### Herstellung von 8-Methoxy-1-methyl-4,5-dihydro-6H-[1,2,4]triazolo[4,3-a][1]benzazepin-6-on

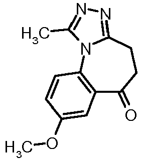

In Analogie zur Herstellung von 1-Methyl-4,5-dihydro-6H-[1,2,4]triazolo[4,3-a][1]benzazepin-6-on (Beispiel 1E) wurden ausgehend von 20 g 7-Methoxy-2-thioxo-1,2,3,4-tetrahydro-5H-1-benzazepin-5-on und 20.1 g Acetylhydrazin 5.8 g 8-Methoxy-1-methyl-4,5-dihydro-6H-[1,2,4]triazolo[4,3-a][1]benzazepin-6-on erhalten.
Ausbeute: 5.8 g 8-Methoxy-1-methyl-4,5-dihydro-6H-[1,2,4]triazolo[4,3-a][1]benzazepin-6-on.
¹H-NMR (300 MHz, CDCl₃): δ = 2.50 (s, 3H); 2.99-3.07 (m, 2H); 3.17-3.25 (m, 2H); 3.89 (s, 3H); 7.19 (bs, 2H); 7.26 (bs, 1H).

### Beispiel 47C

### Herstellung von 8-Methoxy-1-methyl-4H-[1,2,4]triazolo[4,3-a][1]benzazepin-6-yl 1,1,2,2,3,3,4,4,4-nonafluorobutan-1-sulfonat

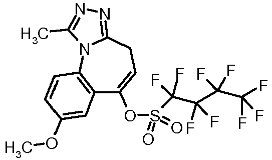

In Analogie zur Herstellung von 1-Methyl-4H-[1,2,4]triazolo[4,3-a][1]benzazepin-6-yl 1,1,2,2,3,3,4,4,4-nonafluorobutan-1-sulfonat (Beispiel 1F) wurden ausgehend von 2 g 8-Methoxy-1-methyl-4,5-dihydro-6H-[1,2,4]triazolo[4,3-a][1]benzazepin-6-on und 1.96 g Nonafluorbutansulfonsäurefluorid 140 mg 8-Methoxy-1-methyl-4H-[1,2,4]triazolo[4,3-a][1]benzazepin-6-yl 1,1,2,2,3,3,4,4,4-nonafluorobutan-1-sulfonat erhalten.
Ausbeute: 140 mg 8-Methoxy-1-methyl-4H-[1,2,4]triazolo[4,3-a][1]benzazepin-6-yl 1,1,2,2,3,3,4,4,4-nonafluorobutan-1-sulfonat.
¹H-NMR (300 MHz, CDCl₃): δ = 2.63 (s, 3H); 3.04-3.13 (m, 1H); 3.85-3.96 (m+s, 4H); 6.32-6.39 (m, 1H); 7.16 (dd, 1H); 7.24 (d, 1H); 7.35 (d, 1H).

### Beispiel 47 D

### Herstellung von 6-(4-Chlorphenyl)-8-methoxy-1-methyl-4H-[1,2,4]triazolo[4,3-a][1]benzazepin

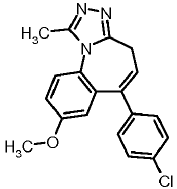

In Analogie zur Herstellung von 6-(4-Chlorphenyl)-1-methyl-4H-[1,2,4]triazolo[4,3-a][1]benzazepin (Beispiel 1C, Alternativer Zugang) wurde ausgehend von 125 mg 8-Methoxy-1-methyl-4H-[1,2,4]triazolo[4,3-a][1]benzazepin-6-yl 1,1,2,2,3,3,4,4,4-nonafluorobutan-1-sulfonat (Beispiel 47C) und 46 mg 4-Chlorphenylboronsäure die Titelverbindung erhalten.
Ausbeute: 60 mg 6-(4-Chlorphenyl)-8-methoxy-1-methyl-4H-[1,2,4]triazolo[4,3-a][1]benzazepin.
¹H-NMR (300 MHz, CDCl₃): δ = 2.59 (s, 3H); 3.04 (dd, 1H); 3.76 (s, 3H); 3.87 (dd, 1H); 6.34 (dd, 1H); 6.70 (d, 1H); 7.04 (dd, 1H); 7.16 (d, 2H); 7.30 (d, 2H); 7.33 (d, 1H).

### Beispiel 47E

### Herstellung von 6-(4-Chlorphenyl)-8-methoxy-1-methyl-4-[(5-methyl-1,3,4-oxadiazol-2-yl)methyl]-4H-[1,2,4]triazolo[4,3-a][1]benzazepin

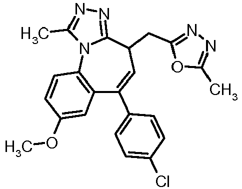

In Analogie zur Herstellung von Beispiel 39 wurde ausgehend von 50 mg 6-(4-Chlorphenyl)-8-methoxy-1-methyl-4H-[1,2,4]triazolo[4,3-a][1]benzazepin (Beispiel 47D) und 19 mg 3-(Chlormethyl)-5-methyl-1,3,4-oxadiazol (CAS 3914-42-9) 6 mg 6-(4-Chlorphenyl)-8-methoxy-1-methyl-4-[(5-methyl-1,3,4-oxadiazol-2-yl)methyl]-4H-[1,2,4]triazolo[4,3-a][1]benzazepin erhalten.
Ausbeute: 6 mg 6-(4-Chlorphenyl)-8-methoxy-1-methyl-4-[(5-methyl-1,3,4-oxadiazol-2-yl)methyl]-4H-[1,2,4]triazolo[4,3-a][1]benzazepin.
¹H-NMR (300 MHz, DMSO-d6): δ = ca. 2.50 (2s, 6H, Signale durch DMSO verdeckt);3.61-3.80 (m+s, 6H); 6.31 (d, 1H); 6.64 (d, 1H); 7.20 (dd, 1H); 7.24 (d, 2H); 7.42 (d, 2H); 7.72 (d, 1H).

### Beispiel 48

### Herstellung von 6-(4-Chlorphenyl)-8-(3,5-dimethyl-1,2-oxazol-4-yl)-1-methyl-4-[(5-methyl-1,3,4-oxadiazol-2-yl)methyl]-4H-[1,2,4]triazolo[4,3-a][1]benzazepin

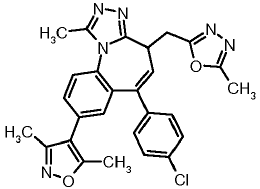

### Beispiel 48A

### Herstellung von 7-Brom-2-thioxo-1,2,3,4-tetrahydro-5H-1-benzazepin-5-on

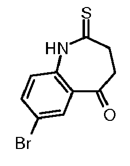

23.1 g 7-Brom-3,4-dihydro-1H-1-benzazepin-2,5-dion (CAS 137046-58-3) wurden zusammen mit 27.2 g Lawesson's Reagens (CAS 19172-47-5) in 471 ml Tetrahydrofuran 1 h bei 60 °C gerührt. Die Mischung wurde eingeengt, der Rückstand mit Methanol verrieben und das Produkt abgesaugt. Nach Trocknen im Vakuum bei 40 °C wurden 18.7 g erhalten.
¹H-NMR (600MHz, DMSO-d₆): δ = 2.93 - 2.98 (m, 2H), 3.16 - 3.21 (m, 2H), 7.27 (d, 1H), 7.80 (dd, 1H), 7.86 (d, 1H).

### Beispiel 48B

### Herstellung von N'-(8-Brom-1-methyl-4,5-dihydro-6H-[1,2,4]triazolo[4,3-a][1]benzazepin-6-yliden)acetohydrazid

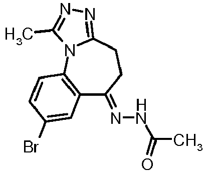

15.3 g 7-Brom-2-thioxo-1,2,3,4-tetrahydro-5H-1-benzazepin-5-on und 12.6 g Acetylhydrazin (CAS# 1068-57-1) wurden in 1-Butanol 1 h bei 60 °C und 16 h bei 125 °C gerührt. Die Mischung wurde eingeengt und direkt weiter umgesetzt. Man erhielt 30.2 g Rohprodukt.

### Beispiel 48C

### Herstellung von 8-Brom-1-methyl-4,5-dihydro-6H-[1,2,4]triazolo[4,3-a][1]benzazepin-6-on

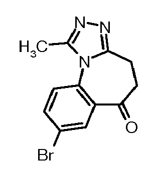

6.04 g N'-(8-Brom-1-methyl-4,5-dihydro-6H-[1,2,4]triazolo[4,3-a][1]benzazepin-6-yliden)acetohydrazid Rohprodukt wurdenmit 14.4 ml konz. Salzsäure in 93 ml Dioxan und 4 ml Wasser über Nacht bei Raumtemperatur gerührt. Der Feststoff wurde abgesaugt und mit Ethylacetat gewaschen. Das Filtrat wurde mit Natriumhydrogencarbonat-Lösung auf pH 8 eingestellt. Die wässrige Phase wurde mit Ethylacetat und Ethylacetat/Ethanol 99/1 extrahiert. Der Extrakt wurde über Natriumsulfat getrocknet und eingeengt. Das Produkt wurde durch Säulenchromatographie an 50 g Kieselgel mit MeCl/EtOH 100/0 - 90/10 gereinigt. Es wurden 2.82 g Produkt erhalten.
¹H-NMR (300MHz, DMSO-d₆): δ = 2.42 (s, 3H), 2.92 - 2.99 (m, 2H), 3.08 - 3.16 (m, 2H), 7.59 (d, 1H), 7.80 (d, 1H), 7.96 (dd, 1H).

### Beispiel 48D

### Herstellung von 8-(3,5-Dimethyl-1,2-oxazol-4-yl)-1-methyl-4,5-dihydro-6H-[1,2,4]triazolo[4,3-a][1]benzazepin-6-on

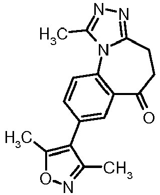

7.5 g 8-Brom-1-methyl-4,5-dihydro-6H-[1,2,4]triazolo[4,3-a][1]benzazepin-6-on, 5.0 g 3,5-Dimethylisoxazol-4-boronsäure (CAS 16114-47-9), 2.1 g [1,1'-Bis-(diphenylphosphino)-ferrocen]-dichloropalladium (II), Komplex mit Dichlormethan (CAS 95464-05-4) und 5.0 g Natriumhydrogencarbonat wurden mit 73 ml Wasser und 300 ml Dioxan versetzt. Die Mischung wurde im Ultraschall entgast und unter Argon 1 h bei 80 °C gerührt und anschließend eingeengt. Der Rückstand wurde mit 100 ml gesättigter Natriumhydrogencarbonat-Lösung versetzt und mit Dichlormethan extrahiert. Der Extrakt wurde über Natriumsulfat getrocknet und eingeengt. Es wurden 10 g Rohprodukt erhalten, die durch Säulenchromatographie an 340 g Kieselgel mit MeCl/EtOH 100/0 - 90/10 gereinigt wurden. Es wurden 7.85 g Produkt erhalten.
¹H-NMR (300MHz, CDCl₃): δ = 2.34 (s, 3H), 2.49 (s, 3H), 2.59 (s, 3H), 3.07 - 3.14 (m, 2H), 3.26 - 3.35 (m, 2H), 7.38 (d, 1H), 7.61 (dd, 1H), 7.72 (d, 1H).

### Beispiel 48E

### Herstellung von 6-(4-Chlorphenyl)-8-(3,5-dimethyl-1,2-oxazol-4-yl)-1-methyl-4H-[1,2,4]triazolo[4,3-a][1]benzazepin

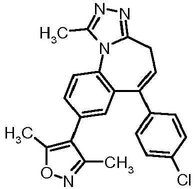

1) 1.16 g 8-(3,5-Dimethyl-1,2-oxazol-4-yl)-1-methyl-4,5-dihydro-6H-[1,2,4]triazolo[4,3-a][1]benzazepin-6-on wurden in 145 ml THF suspendiert und mit 18.8 ml 1.0 M 4-Chlorphenylmagnesiumbromid-Lösung versetzt.Die Mischung wurde unter Stickstoff 17 h bei 60 °C und 18 h bei 70 °C gerührt und anschließend auf circa 50% des Volumens eingeengt. Der Rückstand wurde mit Eis und 1N HCl versetzt (pH 1.5) und mit 250 ml Dichlormethan/i-Propanol (4:1) extrahiert. Der Extrakt wurde mit 30 ml Wasser gewaschen und eingeengt. Es wurden 2.97 g erhalten.
2) 1.58 g des Rückstandes wurden mit 134 ml Toluol und 1.94 g p-Toluolsulfonsäure versetzt und 2 h bei 110 °C gerührt. Die Lösung wurde mit Natriumhydrogencarbonat-Lösung gewaschen und die wässrige Phase mit Ethylacetat extrahiert. Die organischen Phasen wurden vereinigt, über Natriumsulfat getrocknet und eingeengt. Der Rückstand wurde über 100 g Kieselgel mit MeCl/EtOH 100/0 - 95/5 chromatographiert. Es wurden 190 mg Produkt erhalten.
¹H-NMR (300MHz, CDCl₃): δ = 2.20 (s, 3H), 2.34 (s, 3H), 2.66 (s, 3H), 3.10 (dd, 1H), 3.94 (dd, 1H), 6.42 (dd, 1H), 7.12 (d, 1H), 7.17 (d, 2H), 7.32 (d, 2H), 7.40 (dd, 1H), 7.50 (d, 1H).

### Beispiel 48F

### Herstellung von 6-(4-Chlorphenyl)-8-(3,5-dimethyl-1,2-oxazol-4-yl)-1-methyl-4-[(5-methyl-1,3,4-oxadiazol-2-yl)methyl]-4H-[1,2,4]triazolo[4,3-a][1]benzazepin

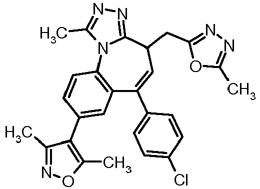

75 mg 6-(4-Chlorphenyl)-8-(3,5-dimethyl-1,2-oxazol-4-yl)-1-methyl-4H-[1,2,4]triazolo[4,3-a][1]benzazepin wurden in 5.7 ml THF vorgelegt. Die Lösung wurde auf -60 °C gekühlt und unter Argon langsam mit 0.40 ml 1.4 M sec.-Butyllithium-Lösung versetzt (Zutropfzeit 1 min). Die Lösung wurde dunkel und 1 h bei -67 - -60 °C nachgerührt. 74 mg 2-(Chlormethyl)-5-methyl-1,3,4-oxadiazol (CAS 3914-42-9) in 1.3 ml THF wurden bei -65 °C zugetropft. Das Kältebad wurde entfernt und die Mischung über Nacht bei Raumtemperatur gerührt und mit 3 ml Ammoniumchlorid-Lösung versetzt. Die Mischung wurde dreimal mit Ethylacetat extrahiert. Der Extrakt wurde über Natriumsulfat getrocknet und eingeengt. Es wurden 110 mg Rohprodukt erhalten, die durch Säulenchromatographie an 10 g Kieselgel mit MeCl/EtOH 100/0 - 95/5 gereinigt wurden. Es wurden 16 mg Produkt erhalten, die durch RP-HPLC (Säule: X-Bridge C18 5µm 100x30mm, Mobile Phase: Acetonitril / Wasser (0.1 Vol% Ameisensäure)-Gradient, Fluss 50ml/min, RT) aufgereinigt wurden. Es wurden 2.4 mg 6-(4-Chlorphenyl)-8-(3,5-dimethyl-1,2-oxazol-4-yl)-1-methyl-4-[(5-methyl-1,3,4-oxadiazol-2-yl)methyl]-4H-[1,2,4]triazolo[4,3-a][1]benzazepin erhalten.
¹H-NMR (400MHz, CDCl₃): δ = 2.21 (s, 3H), 2.36 (s, 3H), 2.56 (s, 3H), 2.66 (s, 3H), 3.64 - 3.77 (m, 1H), 3.86 - 3.99 (m, 2H), 6.19 (d, 1H), 7.13 - 7.17 (m, 3H), 7.31 (d, 2H), 7.43 (dd, 1H), 7.52 (d, 1H).

### Beispiel 49

### Herstellung von [6-(4-Chlorphenyl)-1-methyl-8-(trifluormethoxy)-4H-[1,2,4]triazolo[4,3-a][1]benzazepin-4-yl]essigsäure-tert-butylester

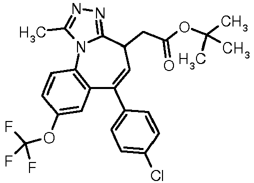

### Beispiel 49A

### Herstellung von 2-[(4-Ethoxy-4-oxobutanoyl)amino]-5-(trifluormethoxy) benzoesäureethylester

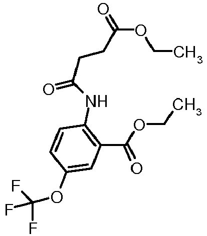

25.8 g Ethylsuccinylchlorid wurden langsam bei RT zu einer Lösung von 23.6 g 2-Amino-5-(trifluormethoxy)benzoesäureethylester (CAS 220107-20-0) in 7.6 ml Pyridin und 450 ml THF getropft. Man ließ 16 Stunden bei RT rühren. Der Ansatz wurde auf Wasser gegeben und mit Ethylacetat extrahiert. Die organische Phase wurde mit 2M Salzsäure, gesättigter Natriumhydrogencarbonat Lösung und Sole gewaschen, über Natriumsulfat getrocknet und das Lösungsmittel im Vakuum entfernt.
Ausbeute: 25.7 g 2-[(4-Ethoxy-4-oxobutanoyl)amino]-5-(trifluormethoxy) benzoesäureethylester.
¹H-NMR (400MHz, CDCl₃): δ = 1.27 (t, 3H); 1.44 (t, 3H); 2.71-2.81 (m, 4H); 4.17 (q, 2H); 4.42 (q, 2H); 7.39 (dd, 1H); 7.88 (d, 1H); 8.77 (d, 1H); 11.15 (bs, 1H).

### Beispiel 49B

### Herstellung von 5-Hydroxy-2-oxo-7-(trifluormethoxy)-2,3-dihydro-1H-1-benzazepin-4-carbonsäureethylester

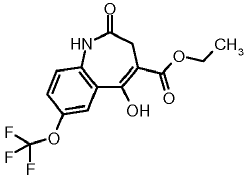

Zu einer Lösung von 40.5 g Kalium-*tert*-butylat in 477 ml DMF wurden bei RT 35.8 g 2-[(4-Ethoxy-4-oxobutanoyl)amino]-5-(trifluormethoxy) benzoesäureethylester (Beispiel 49A) gegeben. Wenige Minuten später wurden 176 ml DMSO zugegeben und es wurde 90 min. bei RT gerührt. Der Ansatz wurde auf 2 L Eiswasser gegeben und der pH mit Salzsäure (1N) auf kleiner 4 eingestellt. Dabei fiel ein Feststoff aus. Die Suspension wurde eine Stunde gerührt und danach wurde der Feststoff abgesaugt. Dieser wurde im Trockenschrank unter Vakuum bei 50°C getrocknet.
Ausbeute: 31.6 g 5-Hydroxy-2-oxo-7-(trifluormethoxy)-2,3-dihydro-1H-1-benzazepin-4-carbonsäureethylester.
¹H-NMR (400MHz, CDCl₃): δ = 1.40 (t, 3H); 3.14 (s, 2H); 4.36 (q, 2H); 7.13 (d, 1H); 7.35 (dd, 1H); 7.78 (d, 1H); 8.65 (bs, 1H); 12.74 (bs, 1H).

### Beispiel 49C

### Herstellung von 7-(Trifluormethoxy)-3,4-dihydro-1H-1-benzazepine-2,5-dion

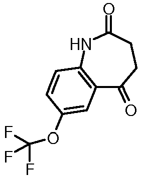

Eine Lösung von 31.6 g 5-Hydroxy-2-oxo-7-(trifluormethoxy)-2,3-dihydro-1H-1-benzazepin-4-carbonsäureethylester (Beispiel 49B) in 125 ml DMSO wurde 9 Stunden bei 150°C gerührt. Der Ansatz wurde nach Abkühlen auf 1.2 L Eiswasser gegeben und gerührt. Es wurde mit Ethylacetat extrahiert, die organische Phase mit Wasser und Sole gewaschen, über Natriumsulfat getrocknet und das Lösungsmittel im Vakuum entfernt. Der Rückstand wurde durch Chromatographie an Kieselgel (Hexan / Ethylacetat Gradient) gereinigt.
Ausbeute: 11.1 g 7-(Trifluormethoxy)-3,4-dihydro-1H-1-benzazepine-2,5-dion.
¹H-NMR (400MHz, DMSO-d6): δ = 2.66-2.75 (m, 2H); 2.90-2.99 (m, 2H); 7.28 (d, 1H); 7.61 (dd, 1H); 7.69 (d, 1H); 10.28 (bs, 1H).

### Beispiel 49D

### Herstellung von 2-Thioxo-7-(trifluormethoxy)-1,2,3,4-tetrahydro-5H-1-benzazepin-5-on

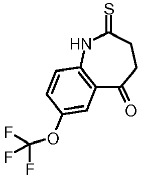

In Analogie zur Herstellung von 2-Thioxo-1,2,3,4-tetrahydro-5H-1-benzazepin-5-on (Beispiel 1D) wurden ausgehend von 11.1 g 7-(Trifluormethoxy)-3,4-dihydro-1H-1-benzazepine-2,5-dion (Beispiel 49C) und 11.5 g Lawesson's Reagenz 8.5 g 2-Thioxo-7-(trifluormethoxy)-1,2,3,4-tetrahydro-5H-1-benzazepin-5-on erhalten.
Ausbeute: 8.5 g 2-Thioxo-7-(trifluormethoxy)-1,2,3,4-tetrahydro-5H-1-benzazepin-5-on.
¹H-NMR (400MHz, CDCl₃): δ = 3.04-3.14 (m, 2H); 3.29-3.40 (m, 2H); 7.09 (d, 1H); 7.43 (dd, 1H); 7.87 (d, 1H); 9.55 (bs, 1H).

### Beispiel 49E

### Herstellung von 1-Methyl-8-(trifluormethoxy)-4,5-dihydro-6H-[1,2,4]triazolo[4,3-a][1]benzazepin-6-on

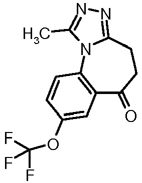

In Analogie zur Herstellung von 1-Methyl-4,5-dihydro-6H-[1,2,4]triazolo[4,3-a][1]benzazepin-6-on (Beispiel 1E) wurden ausgehend von 8.5 g 2-Thioxo-7-(trifluoromethoxy)-1,2,3,4-tetrahydro-5H-1-benzazepin-5-on (Beispiel 49D) und 6.8 g Acetylhydrazin 3.6 g 1-Methyl-8-(trifluormethoxy)-4,5-dihydro-6H-[1,2,4]triazolo[4,3-a][1]benzazepin-6-on erhalten.
Ausbeute: 3.6 g 1-Methyl-8-(trifluormethoxy)-4,5-dihydro-6H-[1,2,4]triazolo[4,3-a][1]benzazepin-6-on.
¹H-NMR (400MHz, CDCl₃): δ = 2.56 (s, 3H); 3.05-3.12 (m, 2H); 3.23-3.31 (m, 2H); 7.35 (d, 1H); 7.55 (dd, 1H); 7.68 (d, 1H).

### Beispiel 49F

### Herstellung von 1-Methyl-8-(trifluormethoxy)-4H-[1,2,4]triazolo[4,3-a][1]benzazepin-6-yl 1,1,2,2,3,3,4,4,4-nonafluorobutan-1-sulfonat

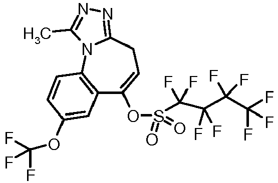

In Analogie zur Herstellung von 1-Methyl-4H-[1,2,4]triazolo[4,3-a][1]benzazepin-6-yl 1,1,2,2,3,3,4,4,4-nonafluorbutan-1-sulfonat (Beispiel 1F) wurden ausgehend von 3.6 g 1-Methyl-8-(trifluormethoxy)-4,5-dihydro-6H-[1,2,4]triazolo[4,3-a][1]benzazepin-6-on (Beispiel 49E) und 5.21 g Nonafluorbutansulfonsäurefluorid 4.5 mg 1-Methyl-8-(trifluormethoxy)-4H-[1,2,4]triazolo[4,3-a][1]benzazepin-6-yl 1,1,2,2,3, 3,4,4,4-nonafluorobutane-1-sulfonat erhalten.
Ausbeute: 4.5 g 1-Methyl-8-(trifluormethoxy)-4H-[1,2,4]triazolo[4,3-a][1]benzazepin-6-yl 1,1,2,2,3,3,4,4,4-nonafluorbutan-1-sulfonat.
¹H-NMR (400MHz, CDCl₃): δ = 2.56 (s, 3H); 3.05-3.19 (m, 1H); 3.88-4.03 (m, 1H); 6.46 (dd, 1H); 7.50 (bs, 2H); 7.64 (bs, 1H).

### Beispiel 49G

### Herstellung von 6-(4-Chlorphenyl)-1-methyl-8-(trifluormethoxy)-4H-[1,2,4]triazolo[4,3-a][1]benzazepin

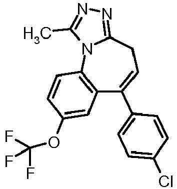

In Analogie zur Herstellung von 6-(4-Chlorphenyl)-1-methyl-4H-[1,2,4]triazolo[4,3-a][1]benzazepin (Beispiel 1C, Alternativer Zugang) wurde ausgehend von 4.5 g 1-Methyl-8-(trifluormethoxy)-4H-[1,2,4]triazolo[4,3-a][1]benzazepin-6-yl 1,1,2,2,3,3,4,4,4-nonafluorbutan-1-sulfonat (Beispiel 49F) und 1.18 g 4-Chlorphenylboronsäure die Titelverbindung erhalten.
Ausbeute: 1.5 g 6-(4-Chlorphenyl)-1-methyl-8-(trifluormethoxy)-4H-[1,2,4]triazolo[4,3-a][1]benzazepin.
¹H-NMR (400MHz, CDCl₃): δ = 2.62 (s, 3H); 3.05 (dd, 1H); 3.93 (dd, 1H); 6.42 (dd, 1H); 7.09-7.17 (m, 3H); 7.33 (d, 2H); 7.38 (dd, 1H); 7.46 (d, 1H).

### Beispiel 49H

### Herstellung von [6-(4-Chlorphenyl)-1-methyl-8-(trifluormethoxy)-4H-[1,2,4]triazolo[4,3-a][1]benzazepin-4-yl]essigsäure-tert-butylester

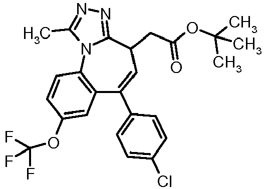

In Analogie zur Herstellung von Beispiel 39 wurde ausgehend von 1 g 6-(4-Chlorphenyl)-1-methyl-8-(trifluormethoxy)-4H-[1,2,4]triazolo[4,3-a][1]benzazepin (Beispiel 49G) und 483 mg Bromessigsäure-*tert*-butylester 690 mg [6-(4-Chlorphenyl)-1-methyl-8-(trifluormethoxy)-4H-[1,2,4]triazolo[4,3-a][1]benzazepin-4-yl]essigsäure-*tert*-butylester erhalten.
Ausbeute: 690 mg [6-(4-Chlorphenyl)-1-methyl-8-(trifluormethoxy)-4H-[1,2,4]triazolo[4,3-a][1]benzazepin-4-yl]essigsäure-*tert*-butylester.
¹H-NMR (400MHz, CDCl₃): δ = 1.50 (s, 9H); 2.60 (s, 3H); 3.10 (dd, 1H); 3.41 (dd, 1H); 3.57-3.66 (m, 1H); 6.11 (d, 1H); 7.08-7.15 (m, 3H); 7.32 (d, 2H); 7.37 (dd, 1H); 7.46 (d, 1H).

### Beispiel 50

### Herstellung von (-)-[(4R)-6-(4-Chlorphenyl)-1-methyl-8-(trifluoromethoxy)-4H-[1,2,4]triazolo[4,3-a][1]benzazepin-4-yl]essigsäure-tert-butylester

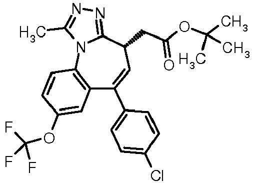

680 mg [6-(4-Chlorphenyl)-1-methyl-8-(trifluormethoxy)-4H-[1,2,4]triazolo[4,3-a][1]benzazepin-4-yl]essigsäure-*tert*-butylester (Beispiel 49H) wurden durch chirale HPLC (Chiralpak IC 5µm 250x20 mm, Acetonitril / Ethanol 90:10, 20 mL/min, RT) in die Enantiomere getrennt.
Ausbeute: 280 mg (-)-[(4*R*)-6-(4-Chlorphenyl)-1-methyl-8-(trifluoromethoxy)-4H-[1,2,4]triazolo[4,3-a][1]benzazepin-4-yl]essigsäure-*tert*-butylester.
¹H-NMR (400MHz, CDCl₃): δ = 1.50 (s, 9H); 2.60 (s, 3H); 3.10 (dd, 1H); 3.41 (dd, 1H); 3.57-3.66 (m, 1H); 6.11 (d, 1H); 7.08-7.15 (m, 3H); 7.32 (d, 2H); 7.37 (dd, 1H); 7.46 (d, 1H).
Optische Rotation: [α]_{D}²⁰ = -87.7° (c=10.7, Methanol).

### Beispiel 51

### Herstellung von 2-[(4R)-6-(4-Chlorphenyl)-1-methyl-8-(trifluoromethoxy)-4H-[1,2,4]triazolo[4,3-a][1,4]benzodiazepin-4-yl]-1-(2-oxa-6-azaspiro[3.3]hept-6-yl)ethanon

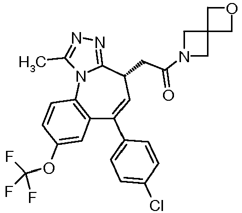

### Beispiel 51A

### Herstellung von [(4R)-6-(4-Chlorphenyl)-1-methyl-8-(trifluormethoxy)-4H-[1,2,4]triazolo[4,3-a][1]benzazepin-4-yl]essigsäure

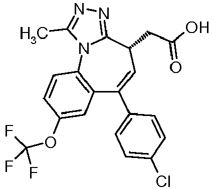

Eine Lösung von 240 mg [(4*R*)-6-(4-Chlorphenyl)-1-methyl-8-(trifluoromethoxy)-4H-[1,2,4]triazolo[4,3-a][1]benzazepin-4-yl]essigsäure-*ter*t-butylester (Beispiel 50) in 2.25 ml HCl in Dioxan (4M) wurde 4 Stunden bei RT gerührt. Das Lösungsmittel wurden anschließend im Vakuum entfernt.
Ausbeute: 240 mg [(4*R*)-6-(4-Chlorphenyl)-1-methyl-8-(trifluormethoxy)-4H-[1,2,4]triazolo[4,3-a][1]benzazepin-4-yl]essigsäure.
Analytik: UPLC-MS: Waters Acquity UPLC-MS SQD; Column: Acquity UPLC BEH C18 1.7 50x2.1mm; Eluent A: Wasser + 0.1% vol. Ameisensäure (99%), Eluent B: Acetonitril; Gradient: 0-1.6 min 1-99% B, 1.6-2.0 min 99% B; flow 0.8 ml/min; T: 60 °C; Injektion: 2 µl; DAD scan: 210-400 nm
Rt = 1.23 min.

### Beispiel 51B

### Herstellung von 2-[(4R)-6-(4-Chlorphenyl)-1-methyl-8-(trifluoromethoxy)-4H-[1,2,4]triazolo[4,3-a][1,4]benzazepin-4-yl]-1-(2-oxa-6-azaspiro[3.3]hept-6-yl)ethanon

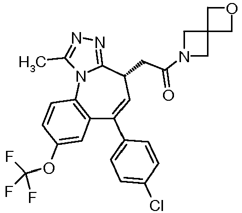

In Analogie zur Herstellung von Beispiel 3B wurde ausgehend von 50 mg [(4*R*)-6-(4-Chlorphenyl)-1-methyl-8-(trifluormethoxy)-4H-[1,2,4]triazolo[4,3-a][1]benzazepin-4-yl]essigsäure (Beispiel 51A) und 32 mg 2-Oxa-6-azaspiro[3.3]heptan Oxalat(2:1) die Titelverbindung erhalten.
Ausbeute: 35 mg 2-[(4*R*)-6-(4-Chlorphenyl)-1-methyl-8-(trifluoromethoxy)-4H-[1,2,4]triazolo[4,3-a][1,4]benzazepin-4-yl]-1-(2-oxa-6-azaspiro[3.3]hept-6-yl)ethanon.
¹H-NMR (400MHz, CDCl₃): δ = 2.60 (s, 3H); 2.80 (dd, 1H); 3.26 (dd, 1H); 3.71-3.80 (m, 1H); 4.19 (bs, 2H); 4.43 (d, 1H); 4.73-4.91 (m, 5H); 6.09 (d, 1H); 7.08-7.17 (m, 3H); 7.27-7.35 (m, 2H); 7.35-7.42 (m, 1H); 7.45 (d, 1H).

### Beispiel 52

### Herstellung von 2-[(4R)-6-(4-Chlorphenyl)-1-methyl-8-(trifluoromethoxy)-4H-[1,2,4]triazolo[4,3-a][1]benzazepin-4-yl]-N-ethylacetamid

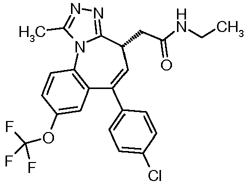

In Analogie zur Herstellung von Beispiel 3B wurde ausgehend von 50 mg [(4R)-6-(4-Chlorphenyl)-1-methyl-8-(trifluormethoxy)-4H-[1,2,4]triazolo[4,3-a][1]benzazepin-4-yl]essigsäure (Beispiel 51A) und 6 mg Ethylamin die Titelverbindung erhalten.
Ausbeute: 40 mg 2-[(4*R*)-6-(4-Chlorphenyl)-1-methyl-8-(trifluoromethoxy)-4H-[1,2,4]triazolo[4,3-a][1]benzazepin-4-yl]-N-ethylacetamid.
¹H-NMR (400MHz, CDCl₃): δ = 1.18 (t, 3H); 2.61 (s, 3H); 3.06 (dd, 1H); 3.22-3.44 (m, 3H); 3.73 (q, 1H); 6.13 (d, 1H); 7.08-7.18 (m, 4H); 7.26-7.35 (m, 2H); 7.38 (bd, 1H); 7.47 (d, 1H).

### Beispiel 53

### Herstellung von 2-[(4R)-6-(4-Chlorphenyl)-1-methyl-8-(trilluoromethoxy)-4H-[1,2,4]triazolo[4,3-a][1]benzazepin-4-yl]-N-[2-(morpholin-4-yl)ethyl]acetamid

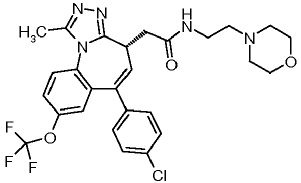

In Analogie zur Herstellung von Beispiel 3B wurde ausgehend von 50 mg [(4R)-6-(4-Chlorphenyl)-1-methyl-8-(trifluormethoxy)-4H-[1,2,4]triazolo[4,3-a][1]benzazepin-4-yl]essigsäure (Beispiel 51A) und 16 mg 4-(2-Aminoethyl)-morpholin die Titelverbindung erhalten.
Ausbeute: 50 mg 2-[(4*R*)-6-(4-Chlorphenyl)-1-methyl-8-(trifluoromethoxy)-4H-[1,2,4]triazolo[4,3-a][1]benzazepin-4-yl]-N-[2-(morpholin-4-yl)ethyl]acetamid.
¹H-NMR (400MHz, CDCl₃): δ = 2.42-2.56 (m, 6H); 2.61 (s, 3H); 3.04 (dd, 1H); 3.30-3.45 (m, 3H); 3.67-3.79 (m, 5H); 6.14 (d, 1H); 6.90 (bs, 1H); 7.10-7.19 (m, 3H); 7.32 (d, 2H); 7.38)bd, 1H); 7.47 (d, 1H).

### Beispiel 54

### Herstellung von 6-(4-Chlorphenyl)-1-methyl-4-[(5-methyl-1,3,4-oxadiazol-2-yl)methyl]-8-(trifluoromethoxy)-4H-[1,2,4]triazolo[4,3-a][1]benzazepin

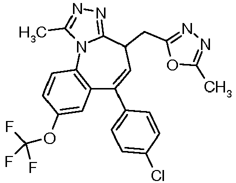

In Analogie zur Herstellung von Beispiel 39 wurde ausgehend von 100 mg 6-(4-Chlorphenyl)-1-methyl-8-(trifluormethoxy)-4H-[1,2,4]triazolo[4,3-a][1]benzazepin (Beispiel 49G) und 33.8 mg 3-(Chlormethyl)-5-methyl-1,3,4-oxadiazol (CAS 3914-42-9) 30 mg 6-(4-Chlorphenyl)-1-methyl-4-[(5-methyl-1,3,4-oxadiazol-2-yl)methyl]-8-(trifluoromethoxy)-4H-[1,2,4]triazolo[4,3-a][1]benzazepin erhalten.
Ausbeute: 30 mg 6-(4-Chlorphenyl)-1-methyl-4-[(5-methyl-1,3,4-oxadiazol-2-yl)methyl]-8-(trifluoromethoxy)-4H-[1,2,4]triazolo[4,3-a][1]benzazepin.
¹H-NMR (400MHz, CDCl₃): δ = 2.56 (s, 3H); 2.62 (s, 3H); 3.71 (dd, 1H); 3.79-3.87 (m, 1H); 3.91 (dd, 1H); 6.21 (d, 1H); 7.08-7.15 (m, 3H); 7.32 (d, 2H); 7.40 (dd, 1H); 7.49 (d, 1H).

### Beispiel 55

### Herstellung von (4R)-6-(4-Chlorphenyl)-1-methyl-4-[(5-methyl-1,3,4-oxadiazol-2-yl)methyl]-8-(trifluoromethoxy)-4H-[1,2,4]triazolo[4,3-a][1]benzazepin

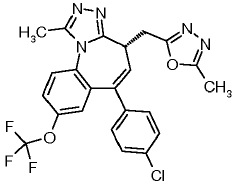

30 mg 6-(4-Chlorphenyl)-1-methyl-4-[(5-methyl-1,3,4-oxadiazol-2-yl)methyl]-8-(trifluoromethoxy)-4H-[1,2,4]triazolo[4,3-a][1]benzazepin (Beispiel 54) wurden durch chirale HPLC (Chiralpak IB 5µm 250x20 mm, Hexan / Ethanol / Diethylamin 70:30:0.1, 20 mL/min, RT) in die Enantiomere getrennt.
Ausbeute: 8 mg (4*R*)-6-(4-Chlorphenyl)-1-methyl-4-[(5-methyl-1,3,4-oxadiazol-2-yl)methyl]-8-(trifluoromethoxy)-4H-[1,2,4]triazolo[4,3-a][1]benzazepin.
¹H-NMR (400MHz, CDCl₃): δ = 2.56 (s, 3H); 2.62 (s, 3H); 3.71 (dd, 1H); 3.79-3.87 (m, 1H); 3.91 (dd, 1H); 6.21 (d, 1H); 7.08-7.15 (m, 3H); 7.32 (d, 2H); 7.40 (dd, 1H); 7.49 (d, 1H).

### Beispiel 56

### Herstellung von 2-{[(4R)-6-(4-Chlorphenyl)-1-methyl-8-(trilluoromethoxy)-4H-[1,2,4]triazolo[4,3-a][1]benzazepin-4-yl]acetyl}hexahydropyrrolo[1,2-a]pyrazin-6(2H)-on

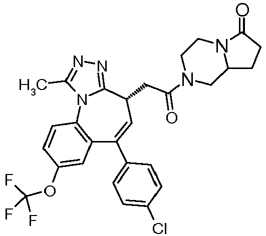

In Analogie zur Herstellung von Beispiel 3B wurde ausgehend von 50 mg [(4R)-6-(4-Chlorphenyl)-1-methyl-8-(trifluormethoxy)-4H-[1,2,4]triazolo[4,3-a][1]benzazepin-4-yl]essigsäure (Beispiel 51A) und 17 mg (rac)-Hexahydropyrrolo[1,2-a]pyrazin-6-on (CAS 117810-52-3) die Titelverbindung erhalten.
Ausbeute: 50 mg 2-{[(4*R*)-6-(4-Chlorphenyl)-1-methyl-8-(trifluoromethoxy)-4H-[1,2,4]triazolo[4,3-a][1]benzazepin-4-yl]acetyl}hexahydropyrrolo[1,2-a]pyrazin-6(2H)-on.
¹H-NMR (400MHz, CDCl₃, ausgewählte Signale): δ = 1.57-1.80 (m); 2.15-2.54 (m, 4H); 2.61 (s, 3H); 3.02-3.30 (m, 2H); 3.48-3.73 (m, 2H); 4.76 (dd, 1H); 6.02-6.18 (m, 1H); 7.07-7.20 (m, 3H); 7.32 (d, 2H); 7.38 (bd, 1H); 7.47 (d, 1H).

### Beispiel 57

### Herstellung von (4R)-4-({5-[(Benzyloxy)methyl]-1,3,4-oxadiazol-2-yl}methyl)-6-(4-chlorphenyl)-1-methyl-4H-[1,2,4]triazolo[4,3-a][1]benzazepin

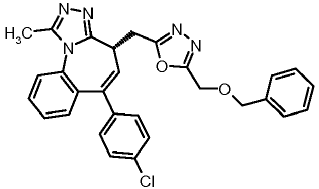

### Beispiel 57A

### Herstellung von (4R)-[6-(4-Chlorphenyl)-1-methyl-4H-[1,2,4]triazolo[4,3-a][1]benzazepin-4-yl]essigsäure

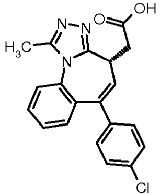

Eine Lösung von 500 mg (-)-[(4*R*)-6-(4-Chlorphenyl)-1-methyl-4H-[1,2,4]triazolo[4,3-a][1]benzazepin-4-yl]essigsäure-*tert*-butylester (Beispiel 9) in 6 ml Salzsäure in Dioxan Lösung (4M) wurde 3 Stunden bei Raumtemperatur gerührt. Die Lösung wurde im Vakuum vollständig eingeengt.
Ausbeute: 420 mg (4*R*)-[6-(4-Chlorphenyl)-1-methyl-4H-[1,2,4]triazolo[4,3-a][1]benzazepin-4-yl]essigsäure.
¹H-NMR (400 MHz, RT, DMSO-d6): δ = 2.67 (s, 3H); 3.14 (dd, 1H); 3.23 (dd, 1H); 3.52 (dd, 1H); 6.26 (d, 1H); 7.22-7.31 (n, 3H); 7.44 (d, 2H); 7.56 (dt, 1H); 7.67 (dt, 1H); 7.87 (dd, 1H).

### Beispiel 57B

### Herstellung von 2-(Benzyloxy)-N'-{[(4R)-6-(4-chlorphenyl)-1-methyl-4H-[1,2,4]triazolo[4,3-a][1]benzazepin-4-yl]acetyl}acetohydrazid

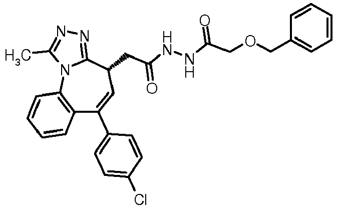

In Analogie zur Herstellung von Beispiel 3 B wurde ausgehend von 420 mg (4R)- [6-(4-Chlorphenyl)-1-methyl-4H-[1,2,4]triazolo[4,3-a][1]benzazepin-4-yl]essigsäure (Beispiel 57A) und 228 mg 2-(Benzyloxy)-acetohydrazid (CAS 39256-35-4) die Titelverbindung erhalten.
Ausbeute: 520 mg 2-(Benzyloxy)-N'-{[(4*R*)-6-(4-chlorphenyl)-1-methyl-4H-[1,2,4]triazolo[4,3-a][1]benzazepin-4-yl]acetyl}acetohydrazid.
¹H-NMR (300 MHz, CDCl₃): δ = 2.56 (s, 3H); 3.14 (dd, 1H); 3.43 (dd, 1H); 3.66-3.77 (m, 1H); 4.08 (s, 2H); 4.56 (s, 2H); 6.08 (d, 1H); 7.13 (d, 2H); 7.22-7.37 (m, 8H); 7.39 (d, 2H); 7.50 (dt, 1H); 8.67 (s, 1H); 9.78 (bs, 1H).

### Beispiel 57C

### Herstellung von (4R)-4-({5-[(Benzyloxy)methyl]-1,3,4-oxadiazol-2-yl}methyl)-6-(4-chlorphenyl)-1-methyl-4H-[1,2,4]triazolo[4,3-a][1]benzazepin

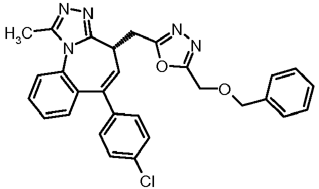

In Analogie zur Herstellung von Beispiel 43 B wurde ausgehend von 380 mg 2-(Benzyloxy)-N'-{[(4*R*)-6-(4-chlorphenyl)-1-methyl-4H-[1,2,4]triazolo[4,3-a][1]benzazepin-4-yl]acetyl}acetohydrazid und 1.05 g Phosphorylchlorid die Titelverbindung erhalten.
Ausbeute: 140 mg (4*R*)-4-({5-[(Benzyloxy)methyl]-1,3,4-oxadiazol-2-yl}methyl)-6-(4-chlorphenyl)-1-methyl-4H-[1,2,4]triazolo[4,3-a][1]benzazepin.
¹H-NMR (300 MHz, CDCl₃): δ = 2.62 (s, 3H); 3.70-3.85 (m, 2H); 3.86-4.01 (m, 1H); 4.66 (s, 2H); 4.73 (s, 2H); 6.14 (d, 1H); 7.10 (d, 2H); 7.23-7.46 (m, 10H); 7.54 (dt, 1H).

### Biologische Wirksamkeit der erfindungsgemäßen Verbindungen

### 1. BRD4-Bindungsstärke

Zur Beurteilung der BRD4-Bindungsstärke der erfindungsgemäßen Verbindungen wurde deren Fähigkeit quantifiziert, die Wechselwirkung zwischen BRD4 und acetyliertem Histon H4 dosisabhängig zu hemmen.

Zu diesem Zweck wurde ein Zeit-aufgelöster Fluoreszenz-Resonanz-Energie-Transfer (TR-FRET) Assay verwendet, der die Bindung zwischen N-terminal His₆-getaggtem BRD4(1) (Aminosäuren 44-168) und einem synthetischen acetylierten Histon H4 (Ac-H4) Peptid mit Sequenz GRGK(Ac)GGK(Ac)GLGK(Ac)GGAK(Ac)RHGSGSK-Biotin misst. Das nach Filippakopoulos et al., Cell, 2012, 149:214-231 im Haus produzierte- rekombinante BRD4 Protein wurde in *E. coli* exprimiert und mittels (Ni-NTA) Affinitäts- und (Sephadex G-75) Größenausschlusschromatografie gereinigt. Das Ac-H4 Peptid kann von z.B. Biosyntan (Berlin, Deutschland) gekauft werden.

Im Assay wurden typischerweise 11 verschiedene Konzentrationen von jeder Substanz (0,1 nM, 0,33 nM, 1,1 nM, 3,8 nM, 13 nM, 44 nM, 0,15 µM, 0,51 µM, 1,7 µM, 5,9 µM and 20 µM) als Duplikate auf derselben Mikrotiter-Platte gemessen. Dafür wurden 100-fach konzentrierte Lösungen in DMSO vorbereitet durch serielle Verdünnungen (1:3,4) einer 2 mM Stammlösung in eine klare, 384-Well Mikrotiter-Platte (Greiner Bio-One, Frickenhausen, Germany). Daraus wurden 50 nl in eine schwarze Testplatte (Greiner Bio-One, Frickenhausen, Germany) überführt. Der Test wurde gestartet durch die Zufuhr von 2 µl einer 2,5-fach konzentrierten BRD4-Lösung (üblicherweise 10 bis 50 nM Endkonzentration in den 5 µl des Reaktionsvolums) in wässrigem Assaypuffer [50 mM HEPES pH 7.5, 50 mM Natriumchlorid (NaCl), 0,25 mM CHAPS und 0,05% Rinderserumalbumin (BSA)] zu den Substanzen in der Testplatte. Darauf folgte ein 10-minütiger Inkubationsschritt bei 22°C für die Voräquilibrierung von putativen Komplexen zwischen BRD4 und den Substanzen. Anschließend wurden 3 µl einer 1,67-fach konzentrierten Lösung (im Assaypuffer) bestehend aus Ac-H4 Peptid (83.5 nM) und TR-FRET Detektionsreagenzien [16,7 nM Anti-6His-XL665 und 3,34 nM Streptavidin-Kryptate (beide von Cisbio Bioassays, Codolet, France), so wie 668 mM Kaliumfluorid (KF)] zugegeben.

Die Mischung wurde dann im Dunkeln für eine Stunde bei 22°C und anschließend über Nacht bei 4°C inkubiert. Die Bildung von BRD4 / Ac-H4 Komplexen wurde bestimmt durch die Messung des Resonanzenergietransfers von dem Streptavidin-Eu-Kryptat zum anti-6His-XL665 Antikörper der sich in der Reaktion befindet. Dafür wurden die Fluorescenzemission bei 620 nm und 665 nm nach Anregung bei 330-350 nm in einem TR-FRET Messgerät, z.B. ein Rubystar oder Pherastar (beide von BMG Lab Technologies, Offenburg, Germany) oder ein Viewlux (Perkin-Elmer) gemessen. Das Verhältnis der Emission bei 665 nm und bei 622 nm (Ratio) wurde als Indikator für die Menge der gebildeten BRD4/Ac-H4 Komplexe genommen.

Die erhaltenen Daten (Ratio) wurden normalisiert, wobei 0% Inhibition dem Mittelwert aus den Messwerten eines Satzes von Kontrollen (üblicherweise 32 Datenpunkte) entsprach, bei denen alle Reagenzien enthalten waren. Dabei wurden anstatt von Testsubstanzen 50 nl DMSO (100%) eingesetzt. Inhibition von 100% entsprach dem Mittelwert aus den Messwerten eines Satzes von Kontrollen (üblicherweise 32 Datenpunkte), bei denen alle Reagenzien außer BRD4 enthalten waren. Die Bestimmung des IC₅₀ Wertes erfolgte durch Regressionsanalyse auf Basis einer 4-Parameter Gleichung (Minimum, Maximum, IC₅₀, Hill; Y = Max + (Min - Max) / (1 + (X/IC50)^{Hill})) mit Hilfe einer geeigneten Analysesoftware.

Die Ergebnisse sind in der Tabelle 1 aufgeführt

**Tabelle 1**

| **Verbindung Beispiel Nr**. | **HTRF IC₅₀ (nmol/L)** |
|---|---|
| 1 | 180 |
| 2 | 30 |
| 3 | 220 |
| 4 | 130 |
| 5 | 1400 |
| 6 | 600 |
| 7 | 230 |
| 8 | 350 |
| 9 | 200 |
| 10 | 730 |
| 11 | 300 |
| 12 | 130 |
| 13 | 30 |
| 14 | 310 |
| 15 | 40 |
| 16 | 220 |
| 17 | 90 |
| 18 | 200 |
| 19 | 50 |
| 20 | 240 |
| 21 | 80 |
| 22 | 340 |
| 23 | 450 |
| 24 | 220 |
| 25 | 1400 |
| 26 | 40 |
| 27 | 110 |
| 28 | 1400 |
| 29 | 1800 |
| 30 | 80 |
| 31 | 180 |
| 32 | 390 |
| 33 | 450 |
| 34 | 90 |
| 35 | 30 |
| 36 | 90 |
| 37 | 50 |
| 38 | 20 |
| 39 | 40 |
| 40 | 40 |
| 41 | 100 |
| 42 | 50 |
| 43 | 130 |
| 44 | 90 |
| 45 | 50 |
| 46 | 80 |
| 47 | 50 |
| 48 | 340 |
| 50 | 790 |
| 51 | 220 |
| 52 | 190 |
| 53 | 80 |
| 54 | 130 |
| 55 | 90 |
| 56 | 180 |
| 57 | 270 |

### 2. Zellproliferation

Es wurde die Fähigkeit der erfindungsgemäßen Verbindungen, die Proliferation von verschiedenen Zelllinien zu hemmen, bestimmt.

Die Zellviabilität wurde mittels des alamarBlue® Reagenz (Invitrogen) bestimmt. Die Zellen wurden in unterschiedlichen Dichten (MOLM-13, LAPC-4, MOLP-8 und MDA-MB-231: 4000 Zellen/Well; B16F10: 400 Zellen/Well) in 100µl Wachstumsmedium auf 96-well Microtiterplatten ausgesät. Nach einer Übernachtinkubation bei 37°C, wurden die Fluoreszenzwerte bestimmt (CI Werte). Dann wurden die Platten mit verschiedenen Substanzverdünnungen behandelt und während 96 Stunden (MOLM-13, MDA-MB-231 und B16F10 Zellen), 120 Stunden (MOLP-8 Zellen) bzw. 168 Stunden (LAPC-4 Zellen) bei 37°C inkubiert. Anschließend wurden die Fluoreszenzwerte bestimmt (CO Werte). Für die Datenanalyse wurden die CI Werte von den CO Werten abgezogen und die Ergebnisse verglichen zwischen Zellen, die mit verschiedenen Verdünnungen der Substanz oder nur mit Pufferlösung behandelt wurden. Die IC₅₀-Werte (Substanzkonzentration die für eine 50%ige Hemmung der Zellproliferation notwendig ist) wurden daraus berechnet.

Die erfindungsgemäßen Verbindungen wurden in den Zelllinien der Tabelle 2 untersucht, die beispielhaft die angegebenen Indikationen vertreten:

**Tabelle 2**

| **Zelllinie** | **Quelle** | **Indikation** |
|---|---|---|
| MOLM-13 | ATCC | Akute myeloische Leukämie |
| LAPC-4 | ATCC | Prostatakarzinom (Androgenrezeptor-positiv) |
| MOLP-8 | ATCC | Multiples Myelom |
| MDA-MB-231 | ATCC | Mammakarzinom |
| B16F10 | ATCC | Melanom |

Die Ergebnisse der Tests zur Hemmung der Zellproliferation sind in den folgenden Tabellen 3a und 3 b aufgeführt. Die mit den verschiedenen Zelllinien korrespondierenden Indikationen können Tabelle 2 entnommen werden.

**Tabelle 3a**

| **Verbindung Beispiel Nr**. | **MOLM-13 IC₅₀ (nmol/L)** | **LAPC-4 IC₅₀ (nmol/L)** | **B16F10 IC₅₀ (nmol/L)** | **MOLP-8 IC₅₀ (nmol/L)** |
|---|---|---|---|---|
| 1 | 330 | 260 | 300 | |
| 2 | 120 | 80 | 100 | |
| 3 | 630 | 470 | 740 | |
| 4 | 360 | 660 | 550 | 740 |
| 5 | 1120 | 620 | 870 | |
| 6 | 670 | 1060 | 1020 | |
| 7 | 720 | 750 | 780 | |
| 8 | 3910 | 820 | 1000 | 4800 |
| 9 | 430 | 310 | 430 | 350 |
| 10 | 2630 | 1610 | 3030 | 1290 |
| 11 | 4190 | 7890 | 9470 | |
| 12 | 270 | 80 | 230 | |
| 13 | 310 | 60 | 150 | |
| 14 | 460 | 210 | 410 | |
| 15 | 230 | 60 | 130 | |
| 16 | 530 | 260 | 460 | |
| 17 | 340 | 140 | 280 | |
| 18 | 330 | 200 | 360 | |
| 19 | 150 | 170 | 160 | 190 |
| 20 | 540 | 140 | 350 | |
| 21 | 170 | 80 | 190 | |
| 22 | 1730 | 210 | 840 | |
| 23 | 360 | 170 | 440 | |
| 24 | 2940 | 550 | 1050 | |
| 25 | 1130 | 170 | 490 | 590 |
| 26 | 830 | 310 | 610 | 380 |
| 27 | 250 | 180 | 340 | |
| 28 | >10000 | 1200 | 7050 | 6300 |
| 29 | 2960 | 670 | 2120 | 1670 |
| 30 | 850 | 190 | 490 | |
| 31 | 190 | 80 | 220 | |
| 32 | 1810 | 250 | 800 | |
| 33 | 330 | 150 | 400 | |
| 34 | 360 | | | |
| 35 | 160 | 80 | 150 | 130 |
| 36 | 400 | 160 | 240 | |
| 37 | 330 | 310 | 220 | |
| 38 | 120 | 40 | 60 | 45 |
| 39 | 320 | | | |
| 40 | 170 | | | 80 |
| 41 | 720 | | | 410 |
| 42 | 170 | | 140 | 110 |
| 43 | 370 | | | 210 |
| 44 | 420 | | | 220 |
| 45 | 100 | | 60 | |
| 46 | 270 | | | 140 |
| 47 | 150 | | | 90 |
| 48 | 1380 | | | 1620 |
| 49 | 1690 | | 2200 | 1890 |
| 50 | 880 | | 1170 | 1050 |
| 51 | 390 | | 580 | 360 |
| 52 | 310 | | 420 | 260 |
| 53 | 150 | | 220 | 110 |
| 54 | 300 | | 500 | 360 |
| 55 | 180 | | 270 | 170 |
| 56 | 150 | | 220 | 120 |
| 57 | 410 | | 570 | 420 |

**Tabelle 3b**

| **Verbindung Beispiel Nr**. | **MDA-MB-231 IC₅₀** (**nmol**/**L**) |
|---|---|
| 4 | 2370 |
| 12 | 480 |
| 13 | 320 |
| 14 | 1010 |
| 15 | 350 |
| 16 | 1040 |
| 17 | 570 |
| 18 | 800 |
| 19 | 380 |
| 20 | 980 |
| 21 | 480 |
| 22 | 2900 |
| 23 | 1350 |
| 24 | 2530 |
| 25 | 1330 |
| 26 | 1360 |
| 27 | 710 |
| 28 | >10000 |
| 29 | 4730 |
| 30 | 1130 |
| 31 | 560 |
| 32 | 2040 |
| 33 | 1290 |
| 35 | 210 |
| 36 | 410 |
| 37 | 380 |
| 38 | 110 |

## Patentansprüche

1. Verbindungen der allgemeinen Formel (I) 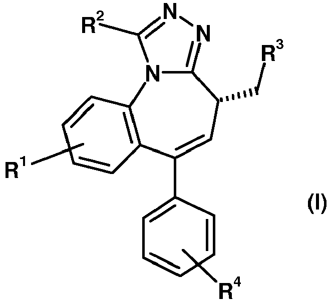 in der
R¹ für Wasserstoff, Hydroxy, C₁-C₆-Alkyl, Halogen-C₁-C₆-Alkyl, C₁-C₆-Alkoxy, Halogen-C₁-C₆-Alkoxy, Halogen oder Cyano steht,
oder
für C₃-C₈-Zykloalkyl, C₃-C₈-Zykloalkoxy, C₃-C₈-Heterozykloalkyl, Aryl oder Heteroaryl mit 5 oder 6 Ringatomen steht, die gegebenenfalls ein- oder mehrfach, gleich oder verschieden substituiert sein können mit C₁-C₆-Alkyl, Halogen-C₁-C₆-Alkyl, C₁-C₆-Alkoxy, Halogen-C₁-C₆-Alkoxy, Hydroxy, Oxo, Halogen, Cyano, Heteroaryl oder Aryl, oder für -C(=O)-OR⁸, -C(=O)-NR¹²R¹³, -C(=O)-R¹⁴, -S(=O)₂-C₁-C₆-Alkyl, -S(=O)₂-OR⁸ oder-S(=O)₂-NR¹²R¹³ steht,
R² für Wasserstoff, C₁-C₆-Alkyl oder für -NR⁶R⁷ steht,
R³ für Cyano, -C(=O)-OR⁸, -C(=O)-R⁹ oder -C(=O)-NR⁶R⁷ steht,
oder
für ein 5- oder 6- gliedriges Ringsystem steht, enthaltend 0, 1, 2, 3 oder 4 Heteroatome unabhängig voneinander ausgewählt aus der Gruppe O, N oder S, welches wahlweise aromatisch oder auch nicht-aromatisch sein kann und gegebenenfalls ein- oder mehrfach, gleich oder verschieden mit R⁵ substituiert sein kann,
R⁴ für Wasserstoff, Fluor, Chlor, Brom oder Cyano steht,
R⁵ für Wasserstoff, C₁-C₆-Alkyl-, C₃-C₈-Zykloalkyl, C₃-C₈-Heterozykloalkyl, Halogen-C₁-C₆-Alkyl, Aryl, Heteroaryl, Hydroxy, C₁-C₆-Alkoxy, C₁-C₃-Alkoxy-C₁-C₃-Alkyl-, Aryloxy-C₁-C₃-Alkyl, Aryl-C₁-C₃-Alkoxy-C₁-C₃-Alkyl-, Halogen-C₁-C₆-Alkoxy, Halogen, Cyano oder Oxo steht,
R⁶ und R⁷ unabhängig voneinander für Wasserstoff oder für -NH-C(=O)-R¹⁵ stehen,
oder
für C₁-C₆-Alkyl, C₁-C₆-Alkoxy, C₃-C₈-Heterozykloalkyl, Aryl, Heteroaryl, C₃-C₈-Zykloalkyl, C₆-C₁₂-Bizykloalkyl, C₅-C₁₁-Spirozykloalkyl, C₆-C₁₂-Heterobizycloalkyl oder C₅-C₁₁-Heterospirozykloalkyl stehen, die gegebenenfalls ein- oder mehrfach, gleich oder verschieden substituiert sein können mit C₁-C₆-Alkyl, Halogen-C₁-C₆-Alkyl, C₁-C₆-Alkoxy, Halogen-C₁-C₆-Alkoxy, Phenoxy, Hydroxy, Oxo, Halogen, Cyano, C₃-C₈-Zykloalkyl, C₃-C₈-Heterozykloalkyl, Heteroaryl oder Aryl, oder für die Gruppe 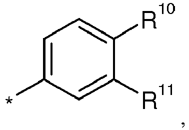 stehen, in welcher
R¹⁰ und R¹¹ gemeinsam für C₃-C₆-Alkylen oder C₃-C₆-Heteroalkylen stehen, die gegebenenfalls ein- oder mehrfach, gleich oder verschieden substituiert sein können mit Halogen, Oxo, Hydroxy, Cyano, Nitro, C₁-C₃-Alkyl, Halogen-C₁-C₆-Alkyl, C₁-C₆-Alkoxy, Halogen- C₁-C₆-Alkoxy, C₁-C₆-Alkoxy- C₁-C₆-Alkyl, C₁-C₆-Alkylcarbonyl, und worin "*" den Anknüpfungspunkt an den Rest des Moleküls kennzeichnet,
R⁸ für Wasserstoff, C₁-C₆-Alkyl, C₃-C₈-Zykloalkyl, C₃-C₈-Heterozykloalkyl, Aryl oder Heteroaryl steht, die gegebenenfalls ein- oder mehrfach, gleich oder verschieden substituiert sein können mit C₁-C₆-Alkyl, Halogen-C₁-C₆-Alkyl, C₁-C₆-Alkoxy, Halogen-C₁-C₆-Alkoxy, Hydroxy, Oxo, Halogen, Cyano, Nitro, Heteroaryl oder Aryl,
R⁹ für C₃-C₈-Heterozykloalkyl, C₅-C₁₁-Spiroheterozykloalkyl, C₆-C₁₂-Heterobizykloalkyl oder für einen überbrückten Heterozyklus, bestehend aus 7 bis 15 Ringatomen, steht, die gegebenfalls ein- oder mehrfach, gleich oder verschieden substituiert sein können mit C₁-C₄-Alkyl, C₃-C₈-Zykloalkyl, C₃-C₈-Heterozykloalkyl, Halogen-C₁-C₆-Alkyl-, Aryl, Aryloxy, Aryl-C₁-C₂-Alkyl, -C(=O)-O-C₁-C₆-Alkyl, Heteroaryl, Hydroxy, C₁-C₆-Alkoxy, Halogen-C₁-C₆-Alkoxy, Halogen, Cyano oder Oxo,
R¹² und R¹³ unabhängig voneinander für Wasserstoff, C₁-C₆-Alkyl stehen,
R¹⁴ für Wasserstoff, C₁-C₆-Alkyl, Amino, C₁-C₆-Alkoxy, C₃-C₈-Zykloalkyl, C₃-C₈-Heterozykloalkyl, Aryl oder Heteroaryl steht, die gegebenenfalls ein- oder mehrfach, gleich oder verschieden substituiert sein können mit C₁-C₆-Alkyl, Halogen-C₁-C₆-Alkyl, C₁-C₆-Alkoxy, Halogen-C₁-C₆-Alkoxy, Hydroxy, Oxo, Halogen, Cyano, Nitro, Heteroaryl oder Aryl, und
R¹⁵ für Wasserstoff, C₁-C₆-Alkyl, Halogen-C₁-C₆-Alkyl, Aryl oder ryl-C₁-C₂-Alkyl steht, worin das Aryl und das in Aryl-C₁-C₂-Alkyl enthaltene Aryl ihrerseits gegebenenfalls ein- oder mehrfach, gleich oder verschieden substituiert sein können mit Halogen, Cyano, C₁-C₃-Alkyl, C₁-C₃-Alkoxy oder Trifluormethyl,
sowie deren Diastereomere, Razemate, Tautomere, Polymorphe, Solvate und physiologisch verträglichen Salze.

2. Verbindungen der allgemeinen Formel I, gemäß Anspruch 1, in der
R¹ für Wasserstoff, Hydroxy, C₁-C₆-Alkyl, Fluor-C₁-C₆-Alkyl, C₁-C₆-Alkoxy, Fluor-C₁-C₆-Alkoxy, Halogen oder Cyano steht,
oder
für C₃-C₈-Zykloalkyl, C₃-C₈-Zykloalkoxy, C₃-C₈-Heterozykloalkyl, Aryl oder Heteroaryl mit 5 oder 6 Ringatomen steht, die gegebenenfalls ein- oder mehrfach, gleich oder verschieden substituiert sein können mit C₁-C₆-Alkyl, Fluor-C₁-C₆-Alkyl, C₁-C₆-Alkoxy, Fluor-C₁-C₆-Alkoxy, Hydroxy, Oxo, Halogen, Cyano, Heteroaryl oder Aryl,
R² für Methyl oder Methylamino- steht,
R³ für Cyano, -C(=O)-OR⁸, -C(=O)-R⁹ oder -C(=O)-NR⁶R⁷ steht,
oder
für ein 5- oder 6- gliedriges Ringsystem steht, enthaltend 0, 1, 2 oder 3 Heteroatome unabhängig voneinander ausgewählt aus der Gruppe O, N oder S, welches wahlweise aromatisch oder auch nicht-aromatisch sein kann und gegebenenfalls ein- oder mehrfach, gleich oder verschieden mit R⁵ substituiert sein kann,
R⁴ für Wasserstoff, Fluor, Chlor, Brom oder Cyano steht,
R⁵ für Wasserstoff, C₁-C₆-Alkyl-, C₃-C₈-Zykloalkyl, C₃-C₈-Heterozykloalkyl, Fluor-C₁-C₆-Alkyl, Aryl, Heteroaryl, Hydroxy, C₁-C₆-Alkoxy, C₁-C₃-Alkoxy-C₁-C₃-Alkyl-, Aryloxy-C₁-C₃-Alkyl, Aryl-C₁-C₃-Alkoxy-C₁-C₃-Alkyl-, Fluor-C₁-C₆-Alkoxy, Halogen, Cyano oder Oxo steht,
R⁶ und R⁷ unabhängig voneinander für Wasserstoff oder für -NH-C(=O)-R¹⁵ stehen,
oder
für C₁-C₆-Alkyl, C₁-C₆-Alkoxy, C₃-C₈-Heterozykloalkyl, Aryl, Heteroaryl, C₃-C₈-Zykloalkyl, C₆-C₁₂-Bizykloalkyl, C₅-C₁₁-Spirozykloalkyl, C₆-C₁₂-Heterobizycloalkyl oder C₅-C₁₁-Heterospirozykloalkyl stehen, die gegebenenfalls ein- oder mehrfach, gleich oder verschieden substituiert sein können mit C₁-C₃-Alkyl, Fluor-C₁-C₃-Alkyl, C₁-C₃-Alkoxy, Fluor-C₁-C₃-Alkoxy, Phenoxy, Hydroxy, Oxo, Halogen, Cyano, C₃-C₈-Zykloalkyl, C₃-C₈-Heterozykloalkyl, Heteroaryl oder Aryl, oder für die Gruppe 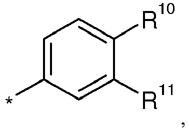 stehen, in welcher
R¹⁰ und R¹¹ gemeinsam für C₃-C₆-Alkylen oder C₃-C₆-Heteroalkylen stehen, die gegebenenfalls ein- oder mehrfach, gleich oder verschieden substituiert sein können mit Halogen, Oxo, Hydroxy, Cyano, Nitro, C₁-C₃-Alkyl, Fluor-C₁-C₃-Alkyl, C₁-C₃-Alkoxy, Fluor-C₁-C₃-Alkoxy, C₁-C₃-Alkoxy-C₁-C₃-Alkyl, C₁-C₃-Alkylcarbonyl, und worin "*" den Anknüpfungspunkt an den Rest des Moleküls kennzeichnet,
R⁸ für Wasserstoff, C₁-C₆-Alkyl, C₃-C₈-Zykloalkyl, Aryl oder Heteroaryl steht, die gegebenenfalls ein- oder mehrfach, gleich oder verschieden substituiert sein können mit C₁-C₆-Alkyl, Fluor-C₁-C₆-Alkyl, C₁-C₆-Alkoxy, Fluor-C₁-C₆-Alkoxy, Hydroxy, Oxo, Halogen, Cyano, Nitro, Heteroaryl oder Aryl,
R⁹ für C₃-C₈-Heterozykloalkyl, C₅-C₁₁-Spiroheterozykloalkyl, C₆-C₁₂-Heterobizykloalkyl oder für einen überbrückten Heterozyklus, bestehend aus 7 bis 15 Ringatomen, steht, die gegebenfalls ein- oder mehrfach, gleich oder verschieden substituiert sein können mit C₁-C₄-Alkyl, C₃-C₈-Zykloalkyl, C₃-C₈-Heterozykloalkyl, Fluor-C₁-C₆-Alkyl-, Aryl, Aryloxy, Aryl-C₁-C₂-Alkyl, -C(=O)-O-C₁-C₆-Alkyl, Heteroaryl, Hydroxy, C₁-C₆-Alkoxy, Fluor-C₁-C₆-Alkoxy, Halogen, Cyano oder Oxo, und
R¹⁵ für C₁-C₆-Alkyl, Fluor-C₁-C₃-Alkyl, Phenyl oder Phenyl-C₁-C₂-Alkyl steht, worin das Phenyl und das in Phenyl-C₁-C₂-Alkyl enthaltene Phenyl ihrerseits gegebenenfalls ein- oder mehrfach, gleich oder verschieden substituiert sein können mit Halogen, Cyano, C₁-C₃-Alkyl, C₁-C₃-Alkoxy oder Trifluormethyl,
sowie deren Diastereomere, Razemate, Tautomere, Polymorphe, Solvate und physiologisch verträglichen Salze.

3. Verbindungen der allgemeinen Formel I, gemäß den Ansprüchen 1 und 2, in der
R¹ für Wasserstoff, Hydroxy, C₁-C₃-Alkyl, Fluor-C₁-C₃-Alkyl, C₁-C₃-Alkoxy oder Fluor-C₁-C₃-Alkoxy steht,
oder
für Heteroaryl mit 5 oder 6 Ringatomen steht, das gegebenenfalls ein- oder mehrfach, gleich oder verschieden substituiert sein kann mit C₁-C₃-Alkyl, Fluor-C₁-C₃-Alkyl, C₁-C₃-Alkoxy, Fluor-C₁-C₃-Alkoxy, Halogen oder Cyano,
R² für Methyl steht,
R³ für -C(=O)-OR⁸, -C(=O)-R⁹ oder -C(=O)-NR⁶R⁷ steht,
oder
für ein 5- gliedriges aromatisches Ringsystem steht, enthaltend 1, 2 oder 3 Heteroatome unabhängig voneinander ausgewählt aus der Gruppe O, N oder S, welches gegebenenfalls ein- oder mehrfach, gleich oder verschieden mit R⁵ substituiert sein kann,
R⁴ für Chlor steht,
R⁵ für Wasserstoff, C₁-C₃-Alkyl-, C₃-C₆-Zykloalkyl, C₃-C₆-Heterozykloalkyl, Fluor-C₁-C₃-Alkyl, Aryl, Heteroaryl, Hydroxy, C₁-C₃-Alkoxy, C₁-C₃-Alkoxy-C₁-C₃-Alkyl-, Aryloxy-C₁-C₃-Alkyl, Aryl-C₁-C₃-Alkoxy-C₁-C₃-Alkyl-, Fluor-C₁-C₃-Alkoxy, Halogen, oder Cyano steht,
R⁶ und R⁷ unabhängig voneinander für Wasserstoff oder für -NH-C(=O)-R¹⁵ stehen,
oder
für C₁-C₆-Alkyl, C₁-C₆-Alkoxy, C₃-C₈-Heterozykloalkyl, Aryl, Heteroaryl, C₃-C₈-Zykloalkyl, C₆-C₁₂-Bizykloalkyl, C₅-C₁₁-Spirozykloalkyl, C₆-C₁₂-Heterobizycloalkyl oder C₅-C₁₁-Heterospirozykloalkyl stehen, die gegebenenfalls ein- oder mehrfach, gleich oder verschieden substituiert sein können mit C₁-C₃-Alkyl, Fluor-C₁-C₃-Alkyl, C₁-C₃-Alkoxy, Fluor-C₁-C₃-Alkoxy, Phenoxy, Hydroxy, Oxo, Halogen, Cyano, C₃-C₈-Zykloalkyl, C₃-C₈-Heterozykloalkyl, Heteroaryl oder Aryl, oder für die Gruppe 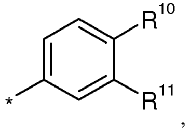 stehen, in welcher
R¹⁰ und R¹¹ gemeinsam für C₃-C₆-Alkylen oder C₃-C₆-Heteroalkylen stehen, die gegebenenfalls ein- oder mehrfach, gleich oder verschieden substituiert sein können mit Halogen, Oxo, Hydroxy, Cyano, Nitro, C₁-C₃-Alkyl, Fluor-C₁-C₃-Alkyl, C₁-C₃-Alkoxy, Fluor-C₁-C₃-Alkoxy, C₁-C₃-Alkoxy-C₁-C₃-Alkyl, C₁-C₃-Alkylcarbonyl, und worin "*" den Anknüpfungspunkt an den Rest des Moleküls kennzeichnet,
R⁸ für C₁-C₆-Alkyl steht,
R⁹ für eine der folgenden Gruppen 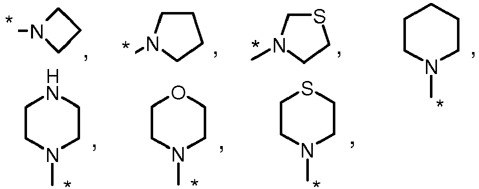 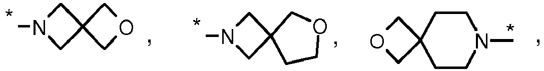 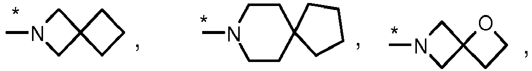 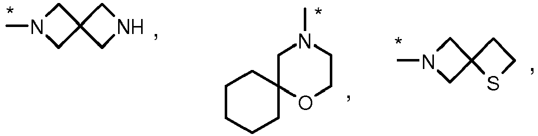 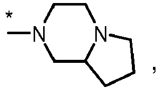 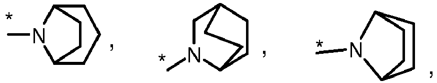 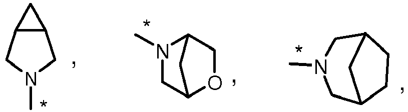 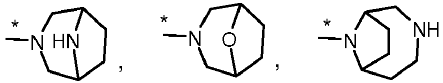 steht, die gegebenfalls ein- oder mehrfach, gleich oder verschieden substituiert sein können mit C₁-C₄-Alkyl, C₃-C₈-Zykloalkyl, C₃-C₈-Heterozykloalkyl, Fluor-C₁-C₆-Alkyl-, Aryl, Aryloxy, Aryl-C₁-C₂-Alkyl, -C(=O)-O-C₁-C₆-Alkyl, Heteroaryl, Hydroxy, C₁-C₆-Alkoxy, Fluor-C₁-C₆-Alkoxy, Halogen, Cyano oder Oxo, und worin "*" den Anknüpfungspunkt an den Rest des Moleküls kennzeichnet, und
R¹⁵ für C₁-C₃-Alkyl, Trifluormethyl, Phenyl oder Benzyl steht, worin das Phenyl und das in Benzyl enthaltene Phenyl ihrerseits gegebenenfalls ein- oder mehrfach, gleich oder verschieden substituiert sein können mit Fluor, Chlor, Brom, Methyl oder Methoxy,
sowie deren Diastereomere, Razemate, Tautomere, Polymorphe,Solvate und physiologisch verträglichen Salze.

4. Verbindungen der allgemeinen Formel I, gemäß den Ansprüchen 1 bis 3, in der
R¹ für Wasserstoff, Hydroxy, C₁-C₃-Alkyl, Fluor-C₁-C₃-Alkyl, C₁-C₃-Alkoxy oder Fluor-C₁-C₃-Alkoxy steht, oder
für Heteroaryl mit 5 Ringatomen steht, das gegebenenfalls ein- oder mehrfach, gleich oder verschieden substituiert sein kann mit C₁-C₃-Alkyl oder C₁-C₃-Alkoxy,
R² für Methyl steht,
R³ für -C(=O)-OR⁸, -C(=O)-R⁹ oder -C(=O)-NR⁶R⁷ steht, oder
für eines der folgenden Ringsysteme 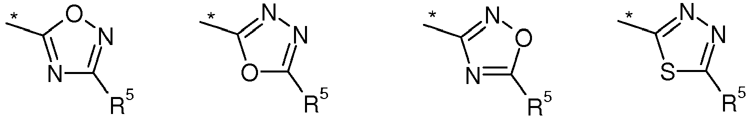 worin "*" den Anknüpfungspunkt an den Rest des Moleküls kennzeichnet, steht,
R⁴ für Chlor steht,
R⁵ für Wasserstoff, C₁-C₃-Alkyl-, C₃-C₆-Zykloalkyl, C₃-C₆-Heterozykloalkyl, Phenyl, 5- oder 6-gliedriges Heteroaryl, C₁-C₃-Alkoxy-C₁-C₃-Alkyl-, Phenoxy-C₁-C₃-Alkyl- oder Benzyloxy-C₁-C₃-Alkyl- steht,
R⁶ und R⁷ unabhängig voneinander für Wasserstoff oder für -NH-C(=O)-R¹⁵ stehen,
oder
für C₁-C₆-Alkyl, C₃-C₈-Heterozykloalkyl, Phenyl, Heteroaryl mit 5 oder 6 Ringatomen, oder C₃-C₈-Zykloalkyl stehen, die gegebenenfalls ein- oder mehrfach, gleich oder verschieden substituiert sein können mit C₁-C₃-Alkyl, Fluor-C₁-C₃-Alkyl, C₁-C₃-Alkoxy, Phenoxy, Hydroxy, Oxo, Halogen, Cyano, C₃-C₈-Zykloalkyl, C₃-C₈-Heterozykloalkyl, 5- oder 6-gliedriges Heteroaryl oder Phenyl, oder für die Gruppe 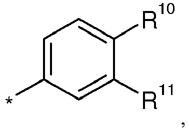 stehen, in welcher
R¹⁰ und R¹¹ gemeinsam für C₃-C₄-Alkylen oder C₃-C₄-Heteroalkylen stehen, die gegebenenfalls ein- oder mehrfach, gleich oder verschieden substituiert sein können mit Halogen, Oxo, Hydroxy, C₁-C₃-Alkyl, C₁-C₃-Alkoxy, C₁-C₃-Alkylcarbonyl, und worin "*" den Anknüpfungspunkt an den Rest des Moleküls kennzeichnet,
R⁸ für C₁-C₄-Alkyl steht,
R⁹ für eine der folgenden Gruppen 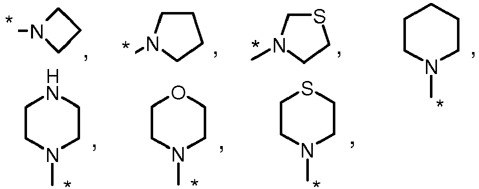 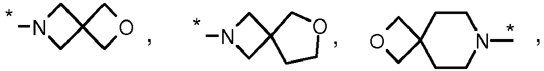 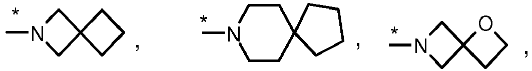 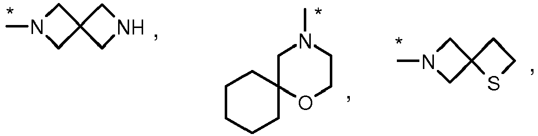 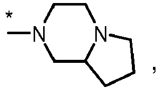 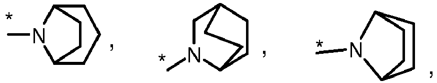 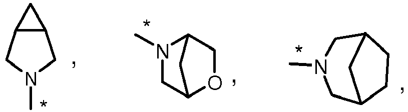 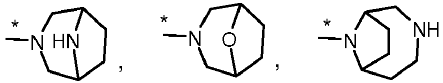 steht, die gegebenfalls ein- oder mehrfach, gleich oder verschieden substituiert sein können mit C₁-C₄-Alkyl, Phenyl, Phenoxy, Benzyl, -C(=O)-O-C₁-C₄-Alkyl, 5- oder 6-gliedriges Heteroaryl, Hydroxy, C₁-C₄-Alkoxy, Fluor, Cyano oder Oxo, und worin "*" den Anknüpfungspunkt an den Rest des Moleküls kennzeichnet, und
R¹⁵ für C₁-C₃-Alkyl steht,
sowie deren Diastereomere, Razemate, Tautomere, Polymorphe, Solvate und physiologisch verträglichen Salze.

5. Verbindungen der allgemeinen Formel I, gemäß den Ansprüchen 1 bis 4, in der
R¹ für Wasserstoff, C₁-C₃-Alkoxy oder Fluor-C₁-C₃-Alkoxy steht,
oder
für Oxazolyl oder Isoxazolyl steht, die gegebenenfalls ein- oder mehrfach, gleich oder verschieden substituiert sein können mit C₁-C₃-Alkyl oder C₁-C₃-Alkoxy,
R² für Methyl steht,
R³ für -C(=O)-OR⁸, -C(=O)-R⁹ oder -C(=O)-NR⁶R⁷ steht, oder
für eines der folgenden Ringsysteme 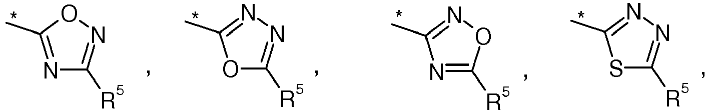 worin "*" den Anknüpfungspunkt an den Rest des Moleküls kennzeichnet, steht,
R⁴ für Chlor steht,
R⁵ für Wasserstoff, C₁-C₃-Alkyl-, C₃-C₆-Zykloalkyl, Pyridinyl oder Benzyloxy-C₁-C₃-Alkyl- steht,
R⁶ und R⁷ unabhängig voneinander für Wasserstoff oder für -NH-C(=O)-R¹⁵ stehen,
oder
für C₁-C₆-Alkyl stehen, das gegebenenfalls ein- oder mehrfach, gleich oder verschieden substituiert sein kann mit C₃-C₈-Heterozykloalkyl, oder für die Gruppe 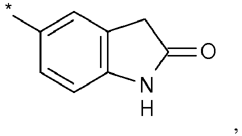 stehen, worin "*" den Anknüpfungspunkt an den Rest des Moleküls kennzeichnet,
R⁸ für Ethyl oder *tert*-Butyl steht,
R⁹ für eine der folgenden Gruppen 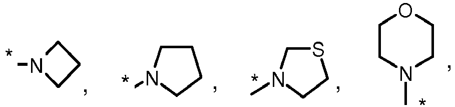 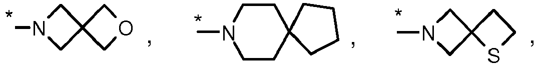 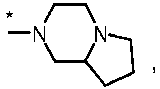 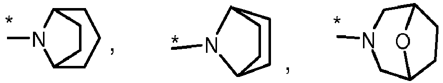 steht, die gegebenfalls ein- oder mehrfach, gleich oder verschieden substituiert sein können mit C₁-C₄-Alkyl, Phenoxy, Benzyl,-C(=O)-O-C₁-C₄-Alkyl, Fluor oder Oxo, und worin "*" den Anknüpfungspunkt an den Rest des Moleküls kennzeichnet, und
R¹⁵ für C₁-C₃-Alkyl steht,
sowie deren Diastereomere, Razemate, Tautomere, Polymorphe, Solvate und physiologisch verträglichen Salze.

6. Verbindungen der allgemeinen Formel I, gemäß den Ansprüchen 1 bis 5 in der
R¹ für Wasserstoff, Methoxy, Trifluormethoxy oder für 3,5-Dimethylisoxazol-4-yl steht,
R² für Methyl steht,
R³ für -C(=O)-OR⁸, -C(=O)-R⁹ oder -C(=O)-NR⁶R⁷ steht, oder für eines der folgenden Ringsysteme 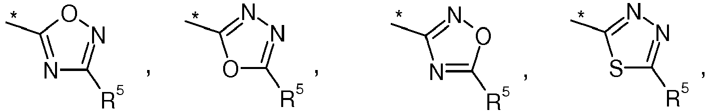 worin "*" den Anknüpfungspunkt an den Rest des Moleküls kennzeichnet, steht,
R⁴ für Chlor steht,
R⁵ für Methyl, *iso*-Propyl, Zyklopropyl, Pyridin-3-yl oder Benzyloxymethyl steht,
R⁶ und R⁷ unabhängig voneinander für Wasserstoff oder für -NH-C(=O)-R¹⁵ stehen,
oder
für Ethyl stehen das gegebenenfalls einfach substituiert sein kann mit Morpholinyl, oder für die Gruppe 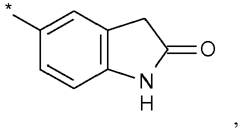 stehen, worin "*" den Anknüpfungspunkt an den Rest des Moleküls kennzeichnet,
R⁸ für Ethyl oder *tert*-Butyl steht,
R⁹ für eine der folgenden Gruppen 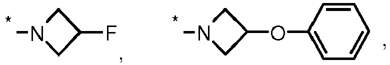 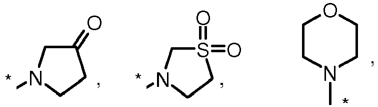 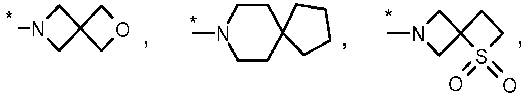 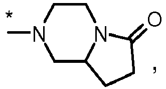 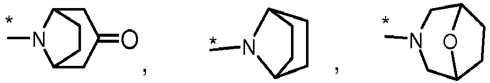 steht,
worin "*" den Anknüpfungspunkt an den Rest des Moleküls kennzeichnet, und
R¹⁵ für Methyl steht,
sowie deren Diastereomere, Razemate, Tautomere, Polymorphe, Solvate und physiologisch verträglichen Salze.

7. Verbindungen der allgemeinen Formel I gemäß den Ansprüchen 1 bis 6:
[6-(4-Chlorphenyl)-1-methyl-4H-[1,2,4]triazolo[4,3-a][1]benzazepin-4-yl]essigsäureethylester;
(-)-(4*R*)-[6-(4-Chlorphenyl)-1-methyl-4H-[1,2,4]triazolo[4,3-a][1]benzazepin-4-yl]essigsäureethylester;
2-[6-(4-Chlorphenyl)-1-methyl-4H-[1,2,4]triazolo[4,3-a][1]benzazepin-4-yl]-1-(2-oxa-6-azaspiro[3.3]hept-6-yl)ethanon;
(-)-2-[(4*R*)-6-(4-Chlorphenyl)-1-methyl-4H-[1,2,4]triazolo[4,3-a][1]benzazepin-4-yl]-1-(2-oxa-6-azaspiro[3.3]hept-6-yl)ethanon;
1-(7-Azabicyclo[2.2.1]hept-7-yl)-2-[6-(4-chlorphenyl)-1-methyl-4H-[1,2,4]triazolo[4,3-a][1]benzazepin-4-yl]ethanon;
(-)-1-(7-Azabicyclo[2.2.1]hept-7-yl)-2-[(4*R*)-6-(4-chlorphenyl)-1-methyl-4H-[1,2,4]triazolo[4,3-a][1]benzazepin-4-yl]ethanon;
[6-(3-Chlorphenyl)-1-methyl-4H-[1,2,4]triazolo[4,3-a][1]benzazepin-4-yl]essigsäureethylester;
[6-(4-Chlorphenyl)-1-methyl-4H-[1,2,4]triazolo[4,3-a][1]benzazepin-4-yl]essigsäure-*tert-*butylester;
(-)-[(4*R*)-6-(4-Chlorphenyl)-1-methyl-4H-[1,2,4]triazolo[4,3-a][1]benzazepin-4-yl]essigsäure-*tert*-butylester;
2-[6-(3-Chlorphenyl)-1-methyl-4H-[1,2,4]triazolo[4,3-a][1]benzazepin-4-yl]-1-(2-oxa-6-azaspiro[3.3]hept-6-yl)ethanon;
(-)-2-[(4*R*)-6-(3-Chlorphenyl)-1-methyl-4H-[1,2,4]triazolo[4,3-a][1]benzazepin-4-yl]-1-(2-oxa-6-azaspiro[3.3]hept-6-yl)ethanon;
2-[6-(4-Chlorphenyl)-1-methyl-4H-[1,2,4]triazolo[4,3-a][1]benzazepin-4-yl] -N-ethylacetamid;
(-)-2-[(4*R*)-6-(4-Chlorphenyl)-1-methyl-4H-[1,2,4]triazolo[4,3-a][1]benzazepin-4-yl]-N-ethylacetamid;
2-[6-(4-Chlorphenyl)-1-methyl-4H-[1,2,4]triazolo[4,3-a][1]benzazepin-4-yl]-1-(morpholin-4-yl)ethanon;
(-)-2-[(4*R*)-6-(4-Chlorphenyl)-1-methyl-4H-[1,2,4]triazolo[4,3-a][1]benzazepin-4-yl]-1-(morpholin-4-yl)ethanon;
2-[6-(4-Chlorphenyl)-1-methyl-4H-[1,2,4]triazolo[4,3-a][1]benzazepin-4-yl]-1-(3-fluoroazetidin-1-yl)ethanon;
(-)-2-[(4*R*)-6-(4-Chlorphenyl)-1-methyl-4H-[1,2,4]triazolo[4,3-a][1]benzazepin-4-yl]-1-(3-fluoroazetidin-1-yl)ethanon;
2-[6-(4-Chlorphenyl)-1-methyl-4H-[1,2,4]triazolo[4,3-a][1]benzazepin-4-yl]-1-(1,1-dioxido-1-thia-6-azaspiro[3.3]hept-6-yl)ethanon;
(-)-2-[(4*R*)-6-(4-Chlorphenyl)-1-methyl-4H-[1,2,4]triazolo[4,3-a][1]benzazepin-4-yl]-1-(1,1-dioxido-1-thia-6-azaspiro[3.3]hept-6-yl)ethanon;
2-[6-(4-Chlorphenyl)-1-methyl-4H-[1,2,4]triazolo[4,3-a][1]benzazepin-4-yl]-N-(2-oxo-2,3-dihydro-1H-indol-5-yl)acetamid;
(-)-2-[(4*R*)-6-(4-Chlorphenyl)-1-methyl-4H-[1,2,4]triazolo[4,3-a][1]benzazepin-4-yl]-N-(2-oxo-2,3-dihydro-1H-indol-5-yl)acetamid;
3-{[(-6-(4-Chlorphenyl)-1-methyl-4H-[1,2,4]triazolo[4,3-a][1]benzazepin-4-yl]acetyl}-8-azabicyclo[3.2.1]octan-3-on;
(-)-3-{[(4R)-6-(4-Chlorphenyl)-1-methyl-4H-[1,2,4]triazolo[4,3-a][1]benzazepin-4-yl]acetyl}-8-azabicyclo[3.2.1]octan-3-on;
2-[6-(4-Chlorphenyl)-1-methyl-4H-[1,2,4]triazolo[4,3-a][1]benzazepin-4-yl]-1-(3-phenoxyazetidin-1-yl)ethanon;
(-)-2-[(4R)-6-(4-Chlorphenyl)-1-methyl-4H-[1,2,4]triazolo[4,3-a][1]benzazepin-4-yl]-1-(3-phenoxyazetidin-1-yl)ethanon;
1-{[6-(4-Chlorphenyl)-1-methyl-4H-[1,2,4]triazolo[4,3-a][1]benzazepin-4-yl]acetyl}pyrrolidin-3-on;
(-)-1-{[(4*R*)-6-(4-Chlorphenyl)-1-methyl-4H-[1,2,4]triazolo[4,3-a][1]benzazepin-4-yl]acetyl}pyrrolidin-3-on;
1-(8-Azaspiro[4.5]dec-8-yl)-2-[6-(4-chlorphenyl)-1-methyl-4H-[1,2,4]triazolo[4,3-a][1]benzazepin-4-yl]ethanon;
(-)-1-(8-Azaspiro[4.5]dec-8-yl)-2-[(4*R*)-6-(4-chlorphenyl)-1-methyl-4H-[1,2,4]triazolo[4,3-a][1]benzazepin-4-yl]ethanon;
2-[6-(4-Chlorphenyl)-1-methyl-4H-[1,2,4]triazolo[4,3-a][1]benzazepin-4-yl]-1-(1,1-dioxido-1,3-thiazolidin-3-yl)ethanon;
(-)-2-[(4*R*)-6-(4-Chlorphenyl)-1-methyl-4H-[1,2,4]triazolo[4,3-a] [1]benzazepin-4-yl]-1-(1,1-dioxido-1,3-thiazolidin-3-yl)ethanon;
2-[6-(4-Chlorphenyl)-1-methyl-4H-[1,2,4]triazolo[4,3-a][1]benzazepin-4-yl]-1-(8-oxa-3-azabicyclo[3.2.1]oct-3-yl)ethanon;
(-)-2-[(4*R*)-6-(4-Chlorphenyl)-1-methyl-4H-[1,2,4]triazolo[4,3-a][1]benzazepin-4-yl]-1-(8-oxa-3-azabicyclo[3.2.1]oct-3-yl)ethanon;
6-(4-Chlorphenyl)-1-methyl-4-[(3-methyl-1,2,4-oxadiazol-5-yl)methyl]-4H-[1,2,4]triazolo[4,3-a][1]benzazepin;
(-)-6-[(4*R*)-4-Chlorphenyl]-1-methyl-4-[(3-methyl-1,2,4-oxadiazol-5-yl)methyl]-4H-[1,2,4]triazolo[4,3-a][1]benzazepin;
(-)-(4*R*)-6-(4-Chlorphenyl)-1-methyl-4-{[3-(propan-2-yl)-1,2,4-oxadiazol-5-yl]methyl}-4H-[1,2,4]triazolo[4,3-a][1]benzazepin;
(-)-6-(4*R*)-(4-Chlorphenyl)-4-[(3-cyclopropyl-1,2,4-oxadiazol-5-yl)methyl]-1-methyl-4H-[1,2,4]triazolo[4,3-a][1]benzazepin
(-)-(4R)-6-(4-Chlorphenyl)-1-methyl-4-[(5-methyl-1,3,4-oxadiazol-2-yl)methyl]-4H-[1,2,4]triazolo[4,3-a][1]benzazepin;
6-(4-Chlorphenyl)-1-methyl-4-[(5-methyl-1,3,4-oxadiazol-2-yl)methyl]-4H-[1,2,4]triazolo[4,3-a][1]benzazepin;
N'-Acetyl-2-[(4*R*)-6-(4-chlorphenyl)-1-methyl-4H-[1,2,4]triazolo[4,3-a][1]benzazepin-4-yl]acetohydrazid;
6-(4-Chlorphenyl)-1-methyl-4-[(5-methyl-1,3,4-thiadiazol-2-yl)methyl]-4H-[1,2,4]triazolo[4,3-a][1]benzazepin;
(+)-(4*R*)-6-(4-Chlorphenyl)-1-methyl-4-[(5-methyl-1,3,4-thiadiazol-2-yl)methyl]-4H-[1,2,4]triazolo[4,3-a][1]benzazepin;
6-(4-Chlorphenyl)-4-[(5-cyclopropyl-1,3,4-oxadiazol-2-yl)methyl]-1-methyl-4H-[1,2,4]triazolo[4,3-a][1]benzazepin;
6-(4-Chlorphenyl)-1-methyl-4-{[5-(pyridin-3-yl)-1,3,4-oxadiazol-2-yl]methyl1-4H-[1,2,4]triazolo[4,3-a][1]benzazepin;
(-)-(4*R*)-6-(4-Chlorphenyl)-1-methyl-4-{[5-(pyridin-3-yl)-1,3,4-oxadiazol-2-yl]methyl}-4H-[1,2,4]triazolo[4,3-a][1]benzazepin;
6-(4-Chlorphenyl)-1-methyl-4-[(5-methyl-1,2,4-oxadiazol-3-yl)methyl]-4H-[1,2,4]triazolo[4,3-a][1]benzazepin;
6-(4-Chlorphenyl)-8-methoxy-1-methyl-4-[(5-methyl-1,3,4-oxadiazol-2-yl)methyl]-4H-[1,2,4]triazolo[4,3-a][1]benzazepin;
6-(4-Chlorphenyl)-8-(3,5-dimethyl-1,2-oxazol-4-yl)-1-methyl-4-[(5-methyl-1,3,4-oxadiazol-2-yl)methyl]-4H-[1,2,4]triazolo[4,3-a][1]benzazepin;
[6-(4-Chlorphenyl)-1-methyl-8-(trifluormethoxy)-4H-[1,2,4]triazolo[4,3-a][1]benzazepin-4-yl]essigsäure-*tert*-butylester;
(-)-[(4*R*)-6-(4-Chlorphenyl)-1-methyl-8-(trifluoromethoxy)-4H-[1,2,4]triazolo[4,3-a][1]benzazepin-4-yl]essigsäure-*tert*-butylester;
2-[(4*R*)-6-(4-Chlorphenyl)-1-methyl-8-(trifluoromethoxy)-4H-[1,2,4]triazolo[4,3-a][1,4]benzazepin-4-yl]-1-(2-oxa-6-azaspiro[3.3]hept-6-yl)ethanon;
2-[(4*R*)-6-(4-Chlorphenyl)-1-methyl-8-(trifluoromethoxy)-4H-[1,2,4]triazolo[4,3-a][1]benzazepin-4-yl]-N-ethylacetamid;
2-[(4*R*)-6-(4-Chlorphenyl)-1-methyl-8-(trifluoromethoxy)-4H-[1,2,4]triazolo[4,3-a][1]benzazepin-4-yl]-N-[2-(morpholin-4-yl)ethyl]acetamid;
6-(4-Chlorphenyl)-1-methyl-4-[(5-methyl-1,3,4-oxadiazol-2-yl)methyl]-8-(trifluoromethoxy)-4H-[1,2,4]triazolo[4,3-a][1]benzazepin;
(4*R*-6-(4-Chlorphenyl)-1-methyl-4-[(5-methyl-1,3,4-oxadiazol-2-yl)methyl]-8-(trifluoromethoxy)-4H-[1,2,4]triazolo[4,3-a][1]benzazepin;
2-{[(4*R*)-6-(4-Chlorphenyl)-1-methyl-8-(trifluoromethoxy)-4H-[1,2,4]triazolo[4,3-a][1]benzazepin-4-yl]acetyl}hexahydropyrrolo[1,2-a]pyrazin-6(2H)-on;
(4*R*)-4-({5-[(Benzyloxy)methyl]-1,3,4-oxadiazol-2-yl}methyl)-6-(4-chlorphenyl)-1-methyl-4H-[1,2,4]triazolo[4,3-a][1]benzazepin.

8. Verwendung der Verbindungen gemäß den Ansprüchen 1 bis 7, zur Herstellung eines Arzneimittels.

9. Verwendung der Verbindungen gemäß den Ansprüchen 1 bis 7 zur Herstellung eines Arzneimittels zur Prophylaxe und/oder Therapie von Tumorerkrankungen.

10. Verwendung der Verbindungen gemäß den Ansprüchen 1 bis 7 zur Herstellung eines Arzneimittels zur Prophylaxe und/ oder Therapie von viralen Infektionen, neurodegenerativen Erkrankungen, inflammatorischen Erkrankungen, atherosklerotischen Erkrankungen und in der männlichen Fertilitätskontrolle.

11. Verbindungen gemäß den Ansprüchen 1 bis 7 in Kombination mit ein oder mehreren weiteren pharmakologisch wirksamen Substanzen.

12. Verbindungen in Kombination gemäß Anspruch 11, zur Prophylaxe und/oder Therapie von hyperproliferativen Erkrankungen.

13. Verbindungen in Kombination gemäß Anspruch 11, zur Prophylaxe und/oder Therapie von Tumorerkrankungen.

14. Verbindungen in Kombination gemäß Anspruch 11 zur Prophylaxe und/ oder Therapie von viralen Infektionen, neurodegenerativen Erkrankungen, inflammatorischen Erkrankungen, atherosklerotischen Erkrankungen und in der männlichen Fertilitätskontrolle.

## Claims

1. Compounds of the general formula (I) 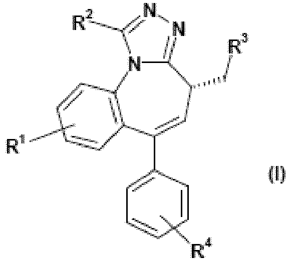 in which
R¹ represents hydrogen, hydroxy, C₁-C₆-alkyl, halo-C₁-C₆-alkyl, C₁-C₆-alkoxy, halo-C₁-C₆-alkoxy, halogen or cyano,
or
represents C₃-C₈-cycloalkyl, C₃-C₈-cycloalkoxy, C₃-C₈-heterocycloalkyl, aryl or heteroaryl having 5 or 6 ring atoms which may optionally be mono- or polysubstituted by identical or different substituents from the group consisting of C₁-C₆-alkyl, halo-C₁-C₆-alkyl, C₁-C₆-alkoxy, halo-C₁-C₆-alkoxy, hydroxy, oxo, halogen, cyano, heteroaryl and aryl, or represents -C(=O)-OR⁸, -C(=O)-NR¹²R¹³, -C(=O)-R¹⁴, -S(=O)₂-C₁-C₆-alkyl, -S(=O)₂-OR⁸ or -S(=O)₂-NR¹²R¹³,
R² represents hydrogen, C₁-C₆-alkyl or -NR⁶R⁷,
R³ represents cyano, -C(=O)-OR⁸, -C(=O)-R⁹ or -C(=O)-NR⁶R⁷,
or
represents a 5- or 6-membered ring system which contains 0, 1, 2, 3 or 4 heteroatoms independently of one another selected from the group consisting of O, N and S, which may optionally be aromatic or else non-aromatic and which may optionally be mono- or polysubstituted by identical or different radicals R⁵,
R⁴ represents hydrogen, fluorine, chlorine, bromine or cyano,
R⁵ represents hydrogen, C₁-C₆-alkyl, C₃-C₈-cycloalkyl, C₃-C₈-heterocycloalkyl, halo-C₁-C₆-alkyl, aryl, heteroaryl, hydroxy, C₁-C₆-alkoxy, C₁-C₃-alkoxy-C₁-C₃-alkyl, aryloxy-C₁-C₃-alkyl, aryl-C₁-C₃-alkoxy-C₁-C₃-alkyl, halo-C₁-C₆-alkoxy, halogen, cyano or oxo,
R⁶ and R⁷ independently of one another represent hydrogen or -NH-C(=O)-R¹⁵,
or r
epresent C₁-C₆-alkyl, C₁-C₆-alkoxy, C₃-C₈-heterocycloalkyl, aryl, heteroaryl, C₃-C₈-cycloalkyl, C₆-C₁₂-bicycloalkyl, C₅-C₁₁-spirocycloalkyl, C₆-C₁₂-heterobicycloalkyl or C₅-C₁₁-heterospirocycloalkyl which may optionally be mono- or polysubstituted by identical or different substituents from the group consisting of C₁-C₆-alkyl, halo-C₁-C₆-alkyl, C₁-C₆-alkoxy, halo-C₁-C₆-alkoxy, phenoxy, hydroxy, oxo, halogen, cyano, C₃-C₈-cycloalkyl, C₃-C₈-heterocycloalkyl, heteroaryl and aryl, or represent the group 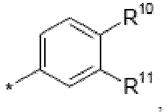 in which
R¹⁰ and R¹¹ together represent C₃-C₆-alkylene or C₃-C₆-heteroalkylene which may optionally be mono- or polysubstituted by identical or different substituents from the group consisting of halogen, oxo, hydroxy, cyano, nitro, C₁-C₃-alkyl, halo-C₁-C₆-alkyl, C₁-C₆-alkoxy, halo-C₁-C₆-alkoxy, C₁-C₆-alkoxy-C₁-C₆-alkyl, C₁-C₆-alkylcarbonyl,
and in which "*" denotes the point of attachment to the remainder of the molecule,
R⁸ represents hydrogen, C₁-C₆-alkyl, C₃-C₈-cycloalkyl, C₃-C₈-heterocycloalkyl, aryl or heteroaryl which may optionally be mono- or polysubstituted by identical or different substituents from the group consisting of C₁-C₆-alkyl, halo-C₁-C₆-alkyl, C₁-C₆-alkoxy, halo-C₁-C₆-alkoxy, hydroxy, oxo, halogen, cyano, nitro, heteroaryl and aryl,
R⁹ represents C₃-C₈-heterocycloalkyl, C₅-C₁₁-spiroheterocycloalkyl, C₆-C₁₂-heterobicycloalkyl or represents a bridged heterocycle consisting of 7 to 15 ring atoms which may optionally be mono- or polysubstituted by identical or different substituents from the group consisting of C₁-C₄-alkyl, C₃-C₈-cycloalkyl, C₃-C₈-heterocycloalkyl, halo-C₁-C₆-alkyl, aryl, aryloxy, aryl-C₁-C₂-alkyl,-C(=O)-O-C₁-C₆-alkyl, heteroaryl, hydroxy, C₁-C₆-alkoxy, halo-C₁-C₆-alkoxy, halogen, cyano and oxo,
R¹² and R¹³ independently of one another represent hydrogen, C₁-C₆-alkyl,
R¹⁴ represents hydrogen, C₁-C₆-alkyl, amino, C₁-C₆-alkoxy, C₃-C₈-cycloalkyl, C₃-C₈-heterocycloalkyl, aryl or heteroaryl which may optionally be mono- or polysubstituted by identical or different substituents from the group consisting of C₁-C₆-alkyl, halo-C₁-C₆-alkyl, C₁-C₆-alkoxy, halo-C₁-C₆-alkoxy, hydroxy, oxo, halogen, cyano, nitro, heteroaryl and aryl, and
R¹⁵ represents hydrogen, C₁-C₆-alkyl, halo-C₁-C₆-alkyl, aryl or aryl-C₁-C₂-alkyl,
where the aryl and the aryl present in aryl-C₁-C₂-alkyl for their part may optionally be mono- or polysubstituted by identical or different substituents from the group consisting of halogen, cyano, C₁-C₃-alkyl, C₁-C₃-alkoxy and trifluoromethyl,
and the diastereomers, racemates, tautomers, polymorphs, solvates and physiologically acceptable salts thereof.

2. Compounds of the general formula (I) according to Claim 1 in which
R¹ represents hydrogen, hydroxy, C₁-C₆-alkyl, fluoro-C₁-C₆-alkyl, C₁-C₆-alkoxy, fluoro-C₁-C₆-alkoxy, halogen or cyano,
or
represents C₃-C₈-cycloalkyl, C₃-C₈-cycloalkoxy, C₃-C₈-heterocycloalkyl, aryl or heteroaryl having 5 or 6 ring atoms which may optionally be mono- or polysubstituted by identical or different substituents from the group consisting of C₁-C₆-alkyl, fluoro-C₁-C₆-alkyl, C₁-C₆-alkoxy, fluoro-C₁-C₆-alkoxy, hydroxy, oxo, halogen, cyano, heteroaryl and aryl,
R² represents methyl or methylamino,
R³ represents cyano, -C(=O)-OR⁸,-C(=O)-R⁹ or -C (=O)-NR⁶R⁷,
or
represents a 5- or 6-membered ring system which contains 0, 1, 2 or 3 heteroatoms independently of one another selected from the group consisting of O, N and S, which may optionally be aromatic or else non-aromatic and which may optionally be mono- or polysubstituted by identical or different radicals R⁵,
R⁴ represents hydrogen, fluorine, chlorine, bromine or cyano,
R⁵ represents hydrogen, C₁-C₆-alkyl, C₃-C₈-cycloalkyl, C₃-C₈-heterocycloalkyl, fluoro-C₁-C₆-alkyl, aryl, heteroaryl, hydroxy, C₁-C₆-alkoxy, C₁-C₃-alkoxy-C₁-C₃-alkyl, aryloxy-C₁-C₃-alkyl, aryl-C₁-C₃-alkoxy-C₁-C₃-alkyl, fluoro-C₁-C₆-alkoxy, halogen, cyano or oxo,
R⁶ and R⁷ independently of one another represent hydrogen or -NH-C(=O)-R¹⁵,
or
represent C₁-C₆-alkyl, C₁-C₆-alkoxy, C₃-C₈-heterocycloalkyl, aryl, heteroaryl, C₃-C₈-cycloalkyl, C₆-C₁₂-bicycloalkyl, C₅-C₁₁-spirocycloalkyl, C₆-C₁₂-heterobicycloalkyl or C₅-C₁₁-heterospirocycloalkyl which may optionally be mono- or polysubstituted by identical or different substituents from the group consisting of C₁-C₃-alkyl, fluoro-C₁-C₃-alkyl, C₁-C₃-alkoxy, fluoro-C₁-C₃-alkoxy, phenoxy, hydroxy, oxo, halogen, cyano, C₃-C₈-cycloalkyl, C₃-C₈-heterocycloalkyl, heteroaryl and aryl, or represent the group 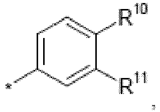 in which
R¹⁰ and R¹¹ together represent C₃-C₆-alkylene or C₃-C₆-heteroalkylene which may optionally be mono- or polysubstituted by identical or different substituents from the group consisting of halogen, oxo, hydroxy, cyano, nitro, C₁-C₃-alkyl, fluoro-C₁-C₃-alkyl, C₁-C₃-alkoxy, fluoro-C₁-C₃-alkoxy, C₁-C₃-alkoxy-C₁-C₃-alkyl, C₁-C₃-alkylcarbonyl, and in which "*" denotes the point of attachment to the remainder of the molecule,
R⁸ represents hydrogen, C₁-C₆-alkyl, C₃-C₈-cycloalkyl, aryl or heteroaryl which may optionally be mono- or polysubstituted by identical or different substituents from the group consisting of C₁-C₆-alkyl, fluoro-C₁-C₆-alkyl, C₁-C₆-alkoxy, fluoro-C₁-C₆-alkoxy, hydroxy, oxo, halogen, cyano, nitro, heteroaryl and aryl,
R⁹ represents C₃-C₈-heterocycloalkyl, C₅-C₁₁-spiroheterocycloalkyl, C₆-C₁₂-heterobicycloalkyl or represents a bridged heterocycle consisting of 7 to 15 ring atoms which may optionally be mono- or polysubstituted by identical or different substituents from the group consisting of C₁-C₄-alkyl, C₃-C₈-cycloalkyl, C₃-C₈-heterocycloalkyl, fluoro-C₁-C₆-alkyl, aryl, aryloxy, aryl-C₁-C₂-alkyl, -C(=O)-O-C₁-C₆-alkyl, heteroaryl, hydroxy, C₁-C₆-alkoxy, fluoro-C₁-C₆-alkoxy, halogen, cyano and oxo, and
R¹⁵ represents C₁-C₆-alkyl, fluoro-C₁-C₃-alkyl, phenyl or phenyl-C₁-C₂-alkyl, in which the phenyl and the phenyl present in phenyl-C₁-C₂-alkyl for their part may optionally be mono-or polysubstituted by identical or different substituents from the group consisting of halogen, cyano, C₁-C₃-alkyl, C₁-C₃-alkoxy and trifluoromethyl,
and the diastereomers, racemates, tautomers, polymorphs, solvates and physiologically acceptable salts thereof.

3. Compounds of the general formula (I) according to Claim 1 or 2 in which
R¹ represents hydrogen, hydroxy, C₁-C₃-alkyl, fluoro-C₁-C₃-alkyl, C₁-C₃-alkoxy or fluoro-C₁-C₃-alkoxy,
or
represents heteroaryl having 5 or 6 ring atoms which may optionally be mono- or polysubstituted by identical or different substituents from the group consisting of C₁-C₃-alkyl, fluoro-C₁-C₃-alkyl, C₁-C₃-alkoxy, fluoro-C₁-C₃-alkoxy, halogen and cyano,
R² represents methyl,
R³ represents -C(=O)-OR⁸, -C(=O)-R⁹ or -C(=O)-NR⁶R⁷,
or
represents a 5-membered aromatic ring system which contains 1, 2 or 3 heteroatoms independently of one another selected from the group consisting of O, N and S, which may optionally be mono- or polysubstituted by identical or different radicals R⁵,
R⁴ represents chlorine,
R⁵ represents hydrogen, C₁-C₃-alkyl, C₃-C₆-cycloalkyl, C₃-C₆-heterocycloalkyl, fluoro-C₁-C₃-alkyl, aryl, heteroaryl, hydroxy, C₁-C₃-alkoxy, C₁-C₃-alkoxy-C₁-C₃-alkyl, aryloxy-C₁-C₃-alkyl, aryl-C₁-C₃-alkoxy-C₁-C₃-alkyl, fluoro-C₁-C₃-alkoxy, halogen or cyano,
R⁶ and R⁷ independently of one another represent hydrogen or NH-C(=O)-R¹⁵,
or
represent C₁-C₆-alkyl, C₁-C₆-alkoxy, C₃-C₈-heterocycloalkyl, aryl, heteroaryl, C₃-C₈-cycloalkyl, C₆-C₁₂-bicycloalkyl, C₅-C₁₁-spirocycloalkyl, C₆-C₁₂-heterobicycloalkyl or C₅-C₁₁-heterospirocycloalkyl which may optionally be mono- or polysubstituted by identical or different substituents from the group consisting of C₁-C₃-alkyl, fluoro-C₁-C₃-alkyl, C₁-C₃-alkoxy, fluoro-C₁-C₃-alkoxy, phenoxy, hydroxy, oxo, halogen, cyano, C₃-C₈-cycloalkyl, C₃-C₈-heterocycloalkyl, heteroaryl and aryl, or represent the group 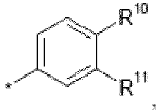 in which
R¹⁰ and R¹¹ together represent C₃-C₆-alkylene or C₃-C₆-heteroalkylene which may optionally be mono- or polysubstituted by identical or different substituents from the group consisting of halogen, oxo, hydroxy, cyano, nitro, C₁-C₃-alkyl, fluoro-C₁-C₃-alkyl, C₁-C₃-alkoxy, fluoro-C₁-C₃-alkoxy, C₁-C₃-alkoxy-C₁-C₃-alkyl, C₁-C₃-alkylcarbonyl, and in which "*" denotes the point of attachment to the remainder of the molecule,
R⁸ represents C₁-C₆-alkyl,
R⁹ represents one of the groups below 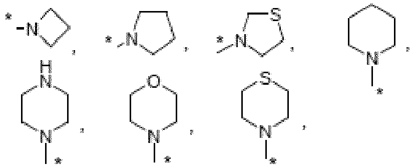 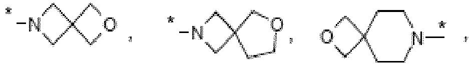 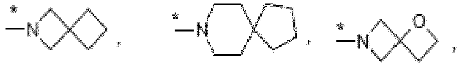 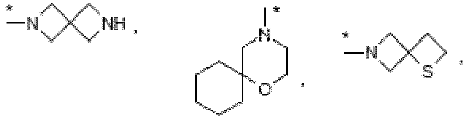 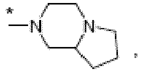 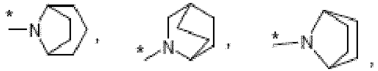 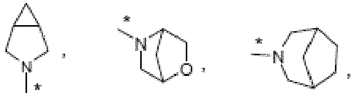 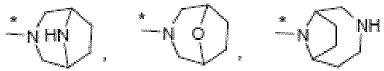 which may optionally be mono- or polysubstituted by identical or different substituents from the group consisting of C₁-C₄-alkyl, C₃-C₈-cycloalkyl, C₃-C₈-heterocycloalkyl, fluoro-C₁-C₆-alkyl, aryl, aryloxy, aryl-C₁-C₂-alkyl, -C(=O)-O-C₁-C₆-alkyl, heteroaryl, hydroxy, C₁-C₆-alkoxy, fluoro-C₁-C₆-alkoxy, halogen, cyano and oxo,
and in which "*" denotes the point of attachment to the remainder of the molecule, and
R¹⁵ represents C₁-C₃-alkyl, trifluoromethyl, phenyl or benzyl, in which the phenyl and the phenyl present in benzyl for their part may optionally be mono- or polysubstituted by identical or different substituents from the group consisting of fluorine, chlorine, bromine, methyl and methoxy,
and the diastereomers, racemates, tautomers, polymorphs, solvates and physiologically acceptable salts thereof.

4. Compounds of the general formula (I) according to any of Claims 1 to 3 in which
R¹ represents hydrogen, hydroxy, C₁-C₃-alkyl, fluoro-C₁-C₃-alkyl, C₁-C₃-alkoxy or fluoro-C₁-C₃-alkoxy,
or
represents heteroaryl having 5 ring atoms which may optionally be mono- or polysubstituted by identical or different substituents from the group consisting of C₁-C₃-alkyl and C₁-C₃-alkoxy,
R² represents methyl,
R³ represents -C(=O)-OR⁸, -C(=O)-R⁹ or -C(=O)-NR⁶R⁷,
or
represents one of the ring systems below 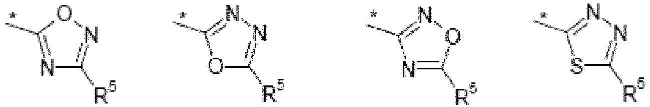 in which "*" denotes the point of attachment to the remainder of the molecule,
R⁴ represents chlorine,
R⁵ represents hydrogen, C₁-C₃-alkyl, C₃-C₆-cycloalkyl, C₃-C₆-heterocycloalkyl, phenyl, 5- or 6-membered heteroaryl, C₁-C₃-alkoxy-C₁-C₃-alkyl, phenoxy-C₁-C₃-alkyl or benzyloxy-C₁-C₃-alkyl,
R⁶ and R⁷ independently of one another represent hydrogen or -NH-C(=O)-R¹⁵,
or
represent C₁-C₆-alkyl, C₃-C₈-heterocycloalkyl, phenyl, heteroaryl having 5 or 6 ring atoms or C₃-C₈-cycloalkyl which may optionally be mono- or polysubstituted by identical or different substituents from the group consisting of C₁-C₃-alkyl, fluoro-C₁-C₃-alkyl, C₁-C₃-alkoxy, phenoxy, hydroxy, oxo, halogen, cyano, C₃-C₈-cycloalkyl, C₃-C₈-heterocycloalkyl, 5- or 6-membered heteroaryl and phenyl, or represent the group 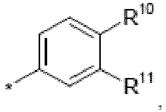 in which
R¹⁰ and R¹¹ together represent C₃-C₄-alkylene or C₃-C₄-heteroalkylene which may optionally be mono- or polysubstituted by identical or different substituents from the group consisting of halogen, oxo, hydroxy, C₁-C₃-alkyl, C₁-C₃-alkoxy, C₁-C₃-alkylcarbonyl,
and in which "*" denotes the point of attachment to the remainder of the molecule,
R⁸ represents C₁-C₄-alkyl,
R⁹ represents one of the groups below 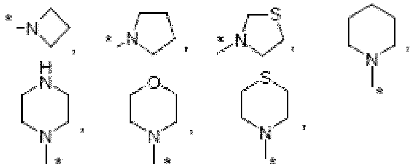 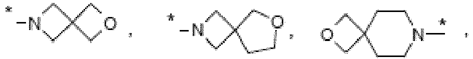 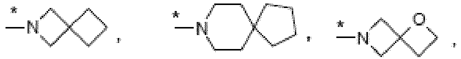 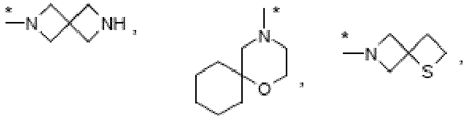 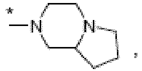 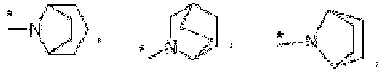 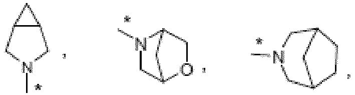 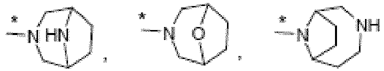 which may optionally be mono- or polysubstituted by identical or different substituents from the group consisting of C₁-C₄-alkyl, phenyl, phenoxy, benzyl, -C(=O)-O-C₁-C₄-alkyl, 5- or 6-membered heteroaryl, hydroxy, C₁-C₄-alkoxy, fluorine, cyano and oxo, and in which "*" denotes the point of attachment to the remainder of the molecule, and
R¹⁵ represents C₁-C₃-alkyl,
and the diastereomers, racemates, tautomers, polymorphs, solvates and physiologically acceptable salts thereof.

5. Compounds of the general formula (I) according to any of Claims 1 to 4 in which
R¹ represents hydrogen, C₁-C₃-alkoxy or fluoro-C₁-C₃-alkoxy,
or
represents oxazolyl or isoxazolyl which may optionally be mono- or polysubstituted by identical or different substituents from the group consisting of C₁-C₃-alkyl and C1-C₃-alkoxy,
R² represents methyl,
R³ represents -C(=O)-OR⁸, -C(=O)-R⁹ or -C(=O)-NR⁶R⁷,
or
represents one of the ring systems below 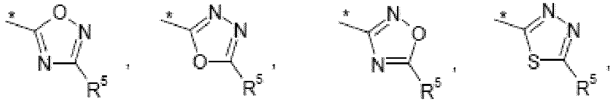 in which "*" denotes the point of attachment to the remainder of the molecule,
R⁴ represents chlorine,
R⁵ represents hydrogen, C₁-C₃-alkyl-, C₃-C₆-cycloalkyl, pyridinyl or benzyloxy-C₁-C₃-alkyl,
R⁶ and R⁷ independently of one another represent hydrogen or NH-C(=O)-R¹⁵,
or
represent C₁-C₆-alkyl which may optionally be mono- or polysubstituted by identical or different substituents from the group consisting of C₃-C₈-heterocycloalkyl, or represent the group 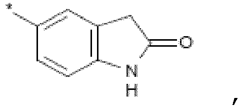 in which "*" denotes the point of attachment to the remainder of the molecule,
R⁸ represents ethyl or *tert*-butyl,
R⁹ represents one of the groups below 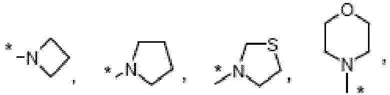 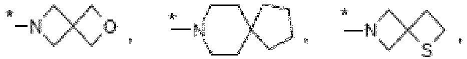 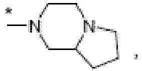 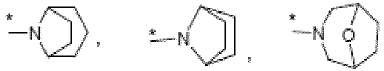 which may optionally be mono- or polysubstituted by identical or different substituents from the group consisting of C₁-C₄-alkyl, phenoxy, benzyl, -C(=O)-O-C₁-C₄-alkyl, fluorine and oxo, and in which "*" denotes the point of attachment to the remainder of the molecule, and
R¹⁵ represents C₁-C₃-alkyl,
and the diastereomers, racemates, tautomers, polymorphs, solvates and physiologically acceptable salts thereof.

6. Compounds of the general formula (I) according to any of Claims 1 to 5 in which
R¹ represents hydrogen, methoxy, trifluoromethoxy or represents 3,5-dimethylisoxazol-4-yl,
R² represents methyl,
R³ represents -C(=O)-OR⁸, -C(=O)-R⁹ or -C(=O)-NR⁶R⁷,
or
represents one of the ring systems below 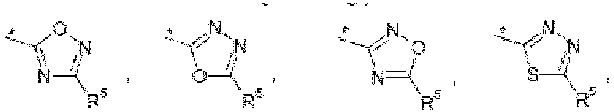 in which "*" denotes the point of attachment to the remainder of the molecule,
R⁴ represents chlorine,
R⁵ represents methyl, isopropyl, cyclopropyl, pyridin-3-yl or benzyloxymethyl,
R⁶ and R⁷ independently of one another represent hydrogen or -NH-C(=O)-R¹⁵,
or
represent ethyl which may optionally be monosubstituted by morpholinyl, or represent the group 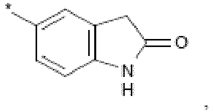 in which "*" denotes the point of attachment to the remainder of the molecule,
R⁸ represents ethyl or *tert*-butyl,
R⁹ represents one of the groups below 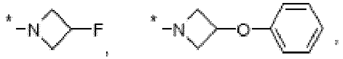 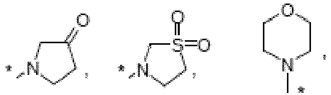 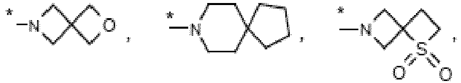 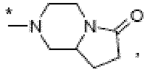 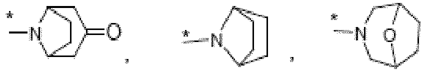 in which "*" denotes the point of attachment to the remainder of the molecule, and
R¹⁵ represents methyl,
and the diastereomers, racemates, tautomers, polymorphs, solvates and physiologically acceptable salts thereof.

7. Compounds of the general formula (I) according to any of Claims 1 to 6:
ethyl [6-(4-chlorophenyl)-1-methyl-4H-[1,2,4]triazolo[4,3-a][1]benzazepin-4-yl]acetate;
(-)-ethyl (4*R*)-[6-(4-chlorophenyl)-1-methyl-4H-[1,2,4]triazolo[4,3-a][1]benzazepin-4-yl]acetate;
2-[6-(4-chlorophenyl)-1-methyl-4H-[1,2,4]triazolo[4,3-a][1]benzazepin-4-yl]-1-(2-oxa-6-azaspiro[3.3]hept-6-yl)ethanone;
(-)-2-[(4*R*)-6-(4-chlorophenyl)-1-methyl-4H-[1,2,4]triazolo[4,3-a][1]benzazepin-4-yl]-1-(2-oxa-6-azaspiro[3.3]hept-6-yl)ethanone;
1-(7-azabicyclo[2.2.1]hept-7-yl)-2-[6-(4-chlorophenyl)-1-methyl-4H-[1,2,4]triazolo[4,3-a][1]benzazepin-4-yl]ethanone;
(-)-1-(7-azabicyclo[2.2.1]hept-7-yl)-2-[(4*R*)-6-(4-chlorophenyl)-1-methyl-4H-[1,2,4]triazolo[4,3-a][1]benzazepin-4-yl]ethanone;
ethyl [6-(3-chlorophenyl)-1-methyl-4H-[1,2,4]triazolo[4,3-a][1]benzazepin-4-yl]acetate;
*tert*-butyl [6-(4-chlorophenyl)-1-methyl-4H-[1,2,4]triazolo[4,3-a][1]benzazepin-4-yl]acetate;
(-)-*tert*-butyl [(4*R*)-6-(4-chlorophenyl)-1-methyl-4H-[1,2,4]triazolo[4,3-a][1]benzazepin-4-yl]acetate;
2-[6-(3-chlorophenyl)-1-methyl-4H-[1,2,4]triazolo[4,3-a][1]benzazepin-4-yl]-1-(2-oxa-6-azaspiro[3.3]hept-6-yl)ethanone;
(-)-2-[(4*R*)-6-(3-chlorophenyl)-1-methyl-4H-[1,2,4]triazolo[4,3-a][1]benzazepin-4-yl]-1-(2-oxa-6-azaspiro[3.3]hept-6-yl)ethanone;
2-[6-(4-chlorophenyl)-1-methyl-4H-[1,2,4]triazolo[4,3-a][1]benzazepin-4-yl]-N-ethylacetamide;
(-)-2-[(4*R*)-6-(4-chlorophenyl)-1-methyl-4H-[1,2,4]triazolo[4,3-a][1]benzazepin-4-yl]-N-ethylacetamide;
2-[6-(4-chlorophenyl)-1-methyl-4H-[1,2,4]triazolo[4,3-a][1]benzazepin-4-yl]-1-(morpholin-4-yl)ethanone;
(-)-2-[(4*R*)-6-(4-chlorophenyl)-1-methyl-4H-[1,2,4]triazolo[4,3-a][1]benzazepin-4-yl]-1-(morpholin-4-yl)ethanone;
2-[6-(4-chlorophenyl)-1-methyl-4H-[1,2,4]triazolo[4,3-a][1]benzazepin-4-yl]-1-(3-fluoroazetidin-1-yl)ethanone;
(-)-2-[(4*R*)-6-(4-chlorophenyl)-1-methyl-4H-[1,2,4]triazolo[4,3-a][1]benzazepin-4-yl]-1-(3-fluoroazetidin-1-yl)ethanone;
2-[6-(4-chlorophenyl)-1-methyl-4H-[1,2,4]triazolo[4,3-a][1]benzazepin-4-yl]-1-(1,1-dioxido-1-thia-6-azaspiro[3.3]hept-6-yl)ethanone;
(-)-2-[(4*R*)-6-(4-chlorophenyl)-1-methyl-4H-[1,2,4]triazolo[4,3-a][1]benzazepin-4-yl]-1-(1,1-dioxido-1-thia-6-azaspiro[3.3]hept-6-yl)ethanone;
2-[6-(4-chlorophenyl)-1-methyl-4H-[1,2,4]triazolo[4,3-a][1]benzazepin-4-yl]-N-(2-oxo-2,3-dihydro-1H-indol-5-yl)acetamide;
(-)-2-[(4*R*)-6-(4-chlorophenyl)-1-methyl-4H-[1,2,4]triazolo[4,3-a][1]benzazepin-4-yl]-N-(2-oxo-2,3-dihydro-1H-indol-5-yl)acetamide;
3-{[(6-(4-chlorophenyl)-1-methyl-4H-[1,2,4]triazolo[4,3-a][1]benzazepin-4-yl]acetyl}-8-azabicyclo[3.2.1]octan-3-one;
(-)-3-{[(4*R*)-6-(4-chlorophenyl)-1-methyl-4H-[1,2,4]triazolo[4,3-a][1]benzazepin-4-yl]acetyl}-8-azabicyclo[3.2.1]octan-3-one;
2-[6-(4-chlorophenyl)-1-methyl-4H-[1,2,4]triazolo[4,3-a][1]benzazepin-4-yl]-1-(3-phenoxyazetidin-1-yl)ethanone;
(-)-2-[(4*R*)-6-(4-chlorophenyl)-1-methyl-4H-[1,2,4]triazolo[4,3-a][1]benzazepin-4-yl]-1-(3-phenoxyazetidin-l-yl)ethanone;
1-{[6-(4-chlorophenyl)-1-methyl-4H-[1,2,4]triazolo[4,3-a][1]benzazepin-4-yl]acetyl}pyrrolidin-3-one;
(-)-1-{[(4*R*)-6-(4-chlorophenyl)-1-methyl-4H-[1,2,4]triazolo[4,3-a][1]benzazepin-4-yl]acetyl}pyrrolidin-3-one;
1-(8-azaspiro[4.5]dec-8-yl)-2-[6-(4-chlorophenyl)-1-methyl-4H-[1,2,4]triazolo[4,3-a][1]benzazepin-4-yl]ethanone;
(-)-1-(8-azaspiro[4.5]dec-8-yl)-2-[(4R)-6-(4-chlorophenyl)-1-methyl-4H-[1,2,4]triazolo[4,3-a][1]benzazepin-4-yl]ethanone;
2-[6-(4-chlorophenyl)-1-methyl-4H-[1,2,4]triazolo[4,3-a][1]benzazepin-4-yl]-1-(1,1-dioxido-1,3-thiazolidin-3-yl)ethanone;
(-)-2-[(4*R*)-6-(4-chlorophenyl)-1-methyl-4H-[1,2,4]triazolo[4,3-a][1]benzazepin-4-yl]-1-(1,1-dioxido-1,3-thiazolidin-3-yl)ethanone;
2-[6-(4-chlorophenyl)-1-methyl-4H-[1,2,4]triazolo[4,3-a][1]benzazepin-4-yl]-1-(8-oxa-3-azabicyclo[3.2.1]oct-3-yl)ethanone;
(-)-2-[(4*R*)-6-(4-chlorophenyl)-1-methyl-4H-[1,2,4]triazolo[4,3-a][1]benzazepin-4-yl]-1-(8-oxa-3-azabicyclo[3.2.1]oct-3-yl)ethanone;
6-(4-chlorophenyl)-1-methyl-4-[(3-methyl-1,2,4-oxadiazol-5-yl)methyl]-4H-[1,2,4]triazolo[4,3-a][1]benzazepine;
(-)-6-[(4*R*)-4-chlorophenyl]-1-methyl-4-[(3-methyl-1,2,4-oxadiazol-5-yl)methyl]-4H-[1,2,4]triazolo[4,3-a][1]benzazepine;
(-)-(4*R*)-6-(4-chlorophenyl)-1-methyl-4-{[3-(propan-2-yl)-1,2,4-oxadiazol-5-yl]methyl}-4H-[1,2,4]triazolo[4,3-a][1]benzazepine;
(-)-6-(4*R*)-(4-chlorophenyl)-4-[(3-cyclopropyl-1,2,4-oxadiazol-5-yl)methyl]-1-methyl-4H-[1,2,4]triazolo[4,3-a][1]benzazepine;
(-)-(4*R*)-6-(4-chlorophenyl)-1-methyl-4-[(5-methyl-1,3,4-oxadiazol-2-yl)methyl]-4H-[1,2,4]triazolo[4,3-a][1]benzazepine;
6-(4-chlorophenyl)-1-methyl-4-[(5-methyl-1,3,4-oxadiazol-2-yl)methyl]-4H-[1,2,4]triazolo[4,3-a][1]benzazepine;
N'-acetyl-2-[(4*R*)-6-(4-chlorophenyl)-1-methyl-4H-[1,2,4]triazolo[4,3-a][1]benzazepin-4-yl]acetohydrazide;
6-(4-chlorophenyl)-1-methyl-4-[(5-methyl-1,3,4-thiadiazol-2-yl)methyl]-4H-[1,2,4]triazolo[4,3-a][1]benzazepine;
(+)-(4*R*)-6-(4-chlorophenyl)-1-methyl-4-[(5-methyl-1,3,4-thiadiazol-2-yl)methyl]-4H-[1,2,4]triazolo[4,3-a][1]benzazepine;
6-(4-chlorophenyl)-4-[(5-cyclopropyl-1,3,4-oxadiazol-2-yl)methyl]-1-methyl-4H-[1,2,4]triazolo[4,3-a][1]benzazepine;
6-(4-chlorophenyl)-1-methyl-4-{[5-(pyridin-3-yl)-1,3,4-oxadiazol-2-yl]methyl}-4H-[1,2,4]triazolo[4,3-a][1]benzazepine;
(-)-(4*R*)-6-(4-chlorophenyl)-1-methyl-4-{[5-(pyridin-3-yl)-1,3,4-oxadiazol-2-yl]methyl}-4H-[1,2,4]triazolo[4,3-a][1]benzazepine;
6-(4-chlorophenyl)-1-methyl-4-[(5-methyl-1,2,4-oxadiazol-3-yl)methyl]-4H-[1,2,4]triazolo[4,3-a][1]benzazepine;
6-(4-chlorophenyl)-8-methoxy-1-methyl-4-[(5-methyl-1,3,4-oxadiazol-2-yl)methyl]-4H-[1,2,4]triazolo[4,3-a][1]benzazepine;
6-(4-chlorophenyl)-8-(3,5-dimethyl-1,2-oxazol-4-yl)-1-methyl-4-[(5-methyl-1,3,4-oxadiazol-2-yl)methyl]-4H-[1,2,4]triazolo[4,3-a][1]benzazepine;
*tert*-butyl [6-(4-chlorophenyl)-1-methyl-8-(trifluoromethoxy)-4H-[1,2,4]triazolo[4,3-a][1]benzazepin-4-yl]acetate;
(-)-*tert*-butyl [(4*R*)-6-(4-chlorophenyl)-1-methyl-8-(trifluormethoxy)-4H-[1,2,4]triazolo[4,3-a][1]benzazepin-4-yl]acetate;
2-[(4*R*)-6-(4-chlorophenyl)-1-methyl-8-(trifluoromethoxy)-4H-[1,2,4]triazolo[4,3-a][1,4]benzazepin-4-yl]-1-(2-oxa-6-azaspiro[3.3]hept-6-yl)ethanone;
2-[(4*R*)-6-(4-chlorophenyl)-1-methyl-8-(trifluoromethoxy)-4H-[1,2,4]triazolo[4,3-a][1]benzazepin-4-yl]-N-ethylacetamide;
2-[(4*R*)-6-(4-chlorophenyl)-1-methyl-8-(trifluoromethoxy)-4H-[1,2,4]triazolo[4,3-a][1]benzazepin-4-yl]-N-[2-(morpholin-4-yl)ethyl]acetamide;
6-(4-chlorophenyl)-1-methyl-4-[(5-methyl-1,3,4-oxadiazol-2-yl)methyl]-8-(trifluoromethoxy)-4H-[1,2,4]triazolo[4,3-a][1]benzazepine;
(4*R*)-6-(4-chlorophenyl)-1-methyl-4-[(5-methyl-1,3,4-oxadiazol-2-yl)methyl]-8-(trifluoromethoxy)-4H-[1,2,4]triazolo[4,3-a][1]benzazepine;
2-{[(4*R*)-6-(4-chlorophenyl)-1-methyl-8-(trifluoromethoxy)-4H-[1,2,4]triazolo[4,3-a][1]benzazepin-4-yl]acetyl}hexahydropyrrolo[1,2-a]pyrazin-6(2H)one;
(4*R*)-4-({5-[(benzyloxy)methyl]-1,3,4-oxadiazol-2-yl}methyl)-6-(4-chlorophenyl)-1-methyl-4H-[1,2,4]triazolo[4,3-a][1]benzazepine.

8. Use of the compounds according to any of Claims 1 to 7 for preparing a medicament.

9. Use of the compounds according to any of Claims 1 to 7 for preparing a medicament for the prophylaxis and/or therapy of tumour diseases.

10. Use of the compounds according to any of Claims 1 to 7 for preparing a medicament for the prophylaxis and/or therapy of viral infections, neurodegenerative disorders, inflammatory disorders, atherosclerotic disorders and in male fertility control.

11. Compounds according to any of Claims 1 to 7 in combination with one or more further pharmacologically active substances.

12. Compounds in combination according to Claim 11 for the prophylaxis and/or therapy of hyperproliferative disorders.

13. Compounds in combination according to Claim 11 for the prophylaxis and/or therapy of tumour diseases.

14. Compounds in combination according to Claim 11 for the prophylaxis and/or therapy of viral infections, neurodegenerative disorders, inflammatory disorders, atherosclerotic disorders and in male fertility control.

## Revendications

1. Composés de formule générale (I) 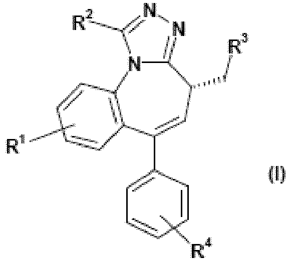 dans laquelle
R¹ représente un atome d'hydrogène, un groupe hydroxy, alkyle en C₁-C₆, halogéno-alkyle (C₁-C₆), alcoxy en C₁-C₆, halogéno-alcoxy(C₁-C₆), un atome d'halogène ou le groupe cyano,
ou
représente un groupe cycloalkyle en C₃-C₈, cycloalcoxy en C₃-C₈, hétérocycloalkyle en C₃-C₈, aryle ou hétéroaryle ayant 5 ou 6 atomes formant le cycle, qui peuvent éventuellement porter un ou plusieurs substituants identiques ou différents alkyle en C₁-C₆, halogénoalkyle (C₁-C₆), alcoxy en C₁-C₆, halogénoalcoxy (C₁-C₆), hydroxy, oxo, halogéno, cyano, hétéroaryle ou aryle, ou représente un groupe -C(=O)-OR⁸, -C(=O)-NR¹²R¹³, -C(=O)-R¹⁴, -S(=O)₂-alkyle(C₁-C₆), -S(=O)₂-OR⁸ ou -S(=O)₂-NR¹²R¹³,
R² représente un atome d'hydrogène, un groupe alkyle en C₁-C₆ ou -NR⁶R⁷,
R³ représente un groupe cyano, -C(=O)-OR⁸, -C(=O)-R⁹ ou -C(=O)-NR⁶R⁷,
ou
représente un système cyclique à 5 ou 6 chaînons, contenant 0, 1, 2, 3 ou 4 hétéroatomes choisis indépendamment les uns des autres dans le groupe constitué par les atomes d'oxygène, d'azote et de soufre, qui peut au choix être aromatique ou bien non aromatique et peut éventuellement porter un ou plusieurs substituants identiques ou différents R⁵,
R⁴ représente un atome d'hydrogène, de fluor, de chlore, de brome ou le groupe cyano,
R⁵ représente un atome d'hydrogène, un groupe alkyle en C₁-C₆, cycloalkyle en C₃-C₈, hétérocycloalkyle en C₃-c₈, halogéno-alkyle(C₁-C₆), aryle, hétéroaryle, hydroxy, alcoxy en C₁-C₆, alcoxy (C₁-C₃) -alkyle (C₁-C₃), aryloxyalkyle(C₁-C₃), aryl-alcoxy(C₁-C₃)-alkyle(C₁-C₃), halogéno-alcoxy (C₁-C₆), un atome d'halogène, le groupe cyano ou oxo,
R⁶ et R⁷ représentent indépendamment l'un de l'autre un atome d'hydrogène ou un groupe -NH-C(=O)-R¹⁵,
ou
représentent un groupe alkyle en C₁-C₆, alcoxy en C₁-C₆, hétérocycloalkyle en C₃-C₈, aryle, hétéroaryle, cycloalkyle en C₃-C₈, bicycloalkyle en C₆-C₁₂, spirocycloalkyle en C₅-C₁₁, hétérobicycloalkyle en C₆-C₁₂ ou hétérospirocycloalkyle en C₅-C₁₁, qui peuvent éventuellement porter un ou plusieurs substituants identiques ou différents alkyle en C₁-C₆, halogénoalkyle (C₁-C₆), alcoxy en C₁-C₆, halogénoalcoxy (C₁-C₆), phénoxy, hydroxy, oxo, halogéno, cyano, cycloalkyle en C₃-C₈, hétérocycloalkyle en C₃-C₈, hétéroaryle ou aryle, ou représentent le groupe 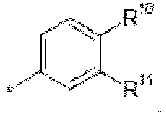 dans lequel
R¹⁰ et R¹¹ représentent ensemble un groupe alkylène en C₃-C₆ ou hétéroalkylène en C₃-C₆, qui peuvent éventuellement porter un ou plusieurs substituants identiques ou différents halogéno, oxo, hydroxy, cyano, nitro, alkyle en C₁-C₃, halogénoalkyle(C₁-C₆), alcoxy en C₁-C₆, halogénoalcoxy (C₁-C₆), alcoxy(C₁-C₆)-alkyle(C₁-C₆), alkyl (C₁-C₆) carbonyle, et dans lequel « * » désigne le point d'attachement au reste de la molécule,
R⁸ représente un atome d'hydrogène, un groupe alkyle en C₁-C₆, cycloalkyle en C₃-C₈, hétérocycloalkyle en C₃-C₈, aryle ou hétéroaryle, qui peuvent éventuellement porter un ou plusieurs substituants identiques ou différents alkyle en C₁-C₆, halogénoalkyle (C₁-C₆), alcoxy en C₁-C₆, halogénoalcoxy(C₁-C₆), hydroxy, oxo, halogéno, cyano, nitro, hétéroaryle ou aryle,
R⁹ représente un groupe hétérocycloalkyle en C₃-C₈, spirohétérocyloalkyle en C₅-C₁₁, hétérobicycloalkyle en C₆-C₁₂ ou représente un hétérocycle ponté, constitué de 7 à 15 atomes formant le cycle, qui peuvent éventuellement porter un ou plusieurs substituants identiques ou différents alkyle en C₁-C₄, cycloalkyle en C₃-C₈, hétérocycloalkyle en C₃-C₈, halogéno-alkyle(C₁-C₆), aryle, aryloxy, aryl-alkyle(C₁-C₂), -C(=O)-O-alkyle (C₁-C₆), hétéroaryle, hydroxy, alcoxy en C₁-C₆, halogénoalcoxy(C₁-C₆), halogéno, cyano ou oxo,
R¹² et R¹³ représentent indépendamment l'un de l'autre un atome d'hydrogène, un groupe alkyle en C₁-C₆,
R¹⁴ représente un atome d'hydrogène, un groupe alkyle en C₁-C₆, amino, alcoxy en C₁-C₆, cycloalkyle en C₃-C₈, hétérocycloalkyle en C₃-C₈, aryle ou hétéroaryle, qui peuvent éventuellement porter un ou plusieurs substituants identiques ou différents alkyle en C₁-C₆, halogénoalkyle(C₁-C₆), alcoxy en C₁-C₆, halogénoalcoxy (C₁-C₆), hydroxy, oxo, halogéno, cyano, nitro, hétéroaryle ou aryle, et
R¹⁵ représente un atome d'hydrogène, un groupe alkyle en C₁-C₆, halogénoalkyle (C₁-C₆), aryle ou aryl-alkyle (C₁-C₂), le groupe aryle et le fragment aryle contenu dans le groupe aryl-alkyle(C₁-C₂) pouvant pour leur part éventuellement porter un ou plusieurs substituants identiques ou différents halogéno, cyano, alkyle en C₁-C₃, alcoxy en C₁-C₃ ou trifluorométhyle,
ainsi que leurs diastéréoisomères, racémates, tautomères, polymorphes, produits de solvatation et sels physiologiquement acceptables.

2. Composés de formule générale (I), selon la revendication 1, dans lesquels
R¹ représente un atome d'hydrogène, un groupe hydroxy, alkyle en C₁-C₆, fluoroalkyle(C₁-C₆), alcoxy en C₁-C₆, fluoroalcoxy(C₁-C₆), un atome d'halogène ou le groupe cyano,
ou
représente un groupe cycloalkyle en C₃-C₈, cycloalcoxy en C₃-C₈, hétérocycloalkyle en C₃-C₈, aryle ou hétéroaryle ayant 5 ou 6 atomes formant le cycle, qui peuvent éventuellement porter un ou plusieurs substituants identiques ou différents alkyle en C₁-C₆, fluoroalkyle (C₁-C₆), alcoxy en C₁-C₆, fluoroalcoxy (C₁-C₆), hydroxy, oxo, halogéno, cyano, hétéroaryle ou aryle,
R² représente le groupe méthyle ou méthylamino,
R³ représente un groupe cyano, -C(=O)-OR⁸, -C(=O)-R⁹ ou -C(=O)-NR⁶R⁷,
ou
représente un système cyclique à 5 ou 6 chaînons, contenant 0, 1, 2 ou 3 hétéroatomes choisis chacun indépendamment dans le groupe constitué par les atomes d'oxygène, d'azote et de soufre, qui peut au choix être aromatique ou bien non aromatique et peut éventuellement porter un ou plusieurs substituants identiques ou différents R⁵,
R⁴ représente un atome d'hydrogène, de fluor, de chlore, de brome ou le groupe cyano,
R⁵ représente un atome d'hydrogène, un groupe alkyle en C₁-C₆, cycloalkyle en C₃-C₈, hétérocycloalkyle en C₃-C₈, fluoroalkyle(C₁-C₆), aryle, hétéroaryle, hydroxy, alcoxy en C₁-C₆, alcoxy (C₁-C₃)-alkyle (C₁-C₃), aryloxy-alkyle(C₁-C₃), aryl-alcoxy(C₁-C₃)-alkyle(C₁-C₃), fluoro-alcoxy (C₁-C₆), un atome d'halogène, le groupe cyano ou oxo,
R⁶ et R⁷ représentent indépendamment l'un de l'autre un atome d'hydrogène ou un groupe -NH-C(=O)-R¹⁵,
ou
représentent un groupe alkyle en C₁-C₆, alcoxy en C₁-C₆, hétérocycloalkyle en C₃-C₈, aryle, hétéroaryle, cycloalkyle en C₃-C₈, bicycloalkyle en C₆-C₁₂, spirocycloalkyle en C₅-C₁₁, hétérobicycloalkyle en C₆-C₁₂ ou hétérospirocycloalkyle en C₅-C₁₁, qui peuvent éventuellement porter un ou plusieurs substituants identiques ou différents alkyle en C₁-C₃, fluoroalkyle (C₁-C₃), alcoxy en C₁-C₃, fluoroalcoxy(C₁-C₃), phénoxy, hydroxy, oxo, halogéno, cyano, cycloalkyle en C₃-C₈, hétérocycloalkyle en C₃-C₈, hétéroaryle ou aryle,
ou représentent le groupe 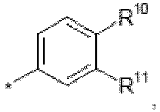 dans lequel
R¹⁰ et R¹¹ représentent ensemble un groupe alkylène en C₃-C₆ ou hétéroalkylène en C₃-C₆, qui peuvent éventuellement porter un ou plusieurs substituants identiques ou différents halogéno, oxo, hydroxy, cyano, nitro, alkyle en C₁-C₃, fluoroalkyle (C₁-C₃), alcoxy en C₁-C₃, fluoroalcoxy (C₁-C₃), alcoxy (C₁-C₃)-alkyle(C₁-C₃), alkyl(C₁-C₃)carbonyle, et dans lequel « * » désigne le point d'attachement au reste de la molécule,
R⁸ représente un atome d'hydrogène, un groupe alkyle en C₁-C₆, cycloalkyle en C₃-C₈, aryle ou hétéroaryle, qui peuvent éventuellement porter un ou plusieurs substituants identiques ou différents alkyle en C₁-C₆, fluoroalkyle (C₁-C₆), alcoxy en C₁-C₆, fluoroalcoxy(C₁-C₆), hydroxy, oxo, halogéno, cyano, nitro, hétéroaryle ou aryle,
R⁹ représente un groupe hétérocycloalkyle en C₃-C₈, spirohétérocyloalkyle en C₅-C₁₁, hétérobicycloalkyle en C₆-C₁₂ ou représente un hétérocycle ponté, constitué de 7 à 15 atomes formant le cycle, qui peuvent éventuellement porter un ou plusieurs substituants identiques ou différents alkyle en C₁-C₄, cycloalkyle en C₃-C₈, hétérocycloalkyle en C₃-C₈, fluoro-alkyle(C₁-C₆), aryle, aryloxy, aryl-alkyle(C₁-C₂), -C(=O)-O-alkyle (C₁-C₆), hétéroaryle, hydroxy, alcoxy en C₁-C₆, fluoroalcoxy(C₁-C₆), halogéno, cyano ou oxo,
R¹⁵ représente un groupe alkyle en C₁-C₆, fluoroalkyle(C₁-C₃), phényle ou phényl-alkyle(C₁-C₂), le groupe phényle et le fragment phényle contenu dans le groupe phényl-alkyle(C₁-C₂) pouvant pour leur part éventuellement porter un ou plusieurs substituants identiques ou différents halogéno, cyano, alkyle en C₁-C₃, alcoxy en C₁-C₃ ou trifluorométhyle,
ainsi que leurs diastéréoisomères, racémates, tautomères, polymorphes, produits de solvatation et sels physiologiquement acceptables.

3. Composés de formule générale (I), selon les revendications 1 et 2, dans lesquels
R¹ représente un atome d'hydrogène, un groupe hydroxy, alkyle en C₁-C₃, fluoroalkyle(C₁-C₃), alcoxy en C₁-C₃ ou fluoroalcoxy(C₁-C₃),
ou
représente un groupe hétéroaryle ayant 5 ou 6 atomes formant le cycle, qui peut éventuellement porter un ou plusieurs substituants identiques ou différents alkyle en C₁-C₃, fluoroalkyle (C₁-C₃), alcoxy en C₁-C₃, fluoroalcoxy(C₁-C₃), halogéno ou cyano,
R² représente le groupe méthyle,
R³ représente un groupe -C(=O)-OR⁸, -C(=O)-R⁹ ou -C(=O)-NR⁶R⁷,
ou
représente un système cyclique aromatique à 5 chaînons, contenant 1, 2 ou 3 hétéroatomes choisis chacun indépendamment dans le groupe constitué par les atomes d'oxygène, d'azote et de soufre, qui peut éventuellement porter un ou plusieurs substituants identiques ou différents R⁵,
R⁴ représente un atome de chlore,
R⁵ représente un atome d'hydrogène, un groupe alkyle en C₁-C₃, cycloalkyle en C₃-C₆, hétérocycloalkyle en C₃-C₆, fluoro-alkyle(C₁-C₃), aryle, hétéroaryle, hydroxy, alcoxy en C₁-C₃, alcoxy(C₁-C₃) -alkyle(C₁-C₃), aryloxyalkyle(C₁-C₃), aryl-alcoxy(C₁-C₃)-alkyle(C₁-C₃), fluoro-alcoxy(C₁-C₃), un atome d'halogène ou le groupe cyano,
R⁶ et R⁷ représentent indépendamment l'un de l'autre un atome d'hydrogène ou un groupe -NH-C(=O)-R¹⁵,
ou
représentent un groupe alkyle en C₁-C₆, alcoxy en C₁-C₆, hétérocycloalkyle en C₃-C₈, aryle, hétéroaryle, cycloalkyle en C₃-C₈, bicycloalkyle en C₆-C₁₂, spirocycloalkyle en C₅-C₁₁, hétérobicycloalkyle en C₆-C₁₂ ou hétérospirocycloalkyle en C₅-C₁₁, qui peuvent éventuellement porter un ou plusieurs substituants identiques ou différents alkyle en C₁-C₃, fluoroalkyle (C₁-C₃), alcoxy en C₁-C₃, fluoroalcoxy(C₁-C₃), phénoxy, hydroxy, oxo, halogéno, cyano, cycloalkyle en C₃-C₈, hétérocycloalkyle en C₃-C₈, hétéroaryle ou aryle, ou représentent le groupe 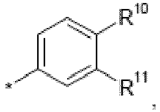 dans lequel
R¹⁰ et R¹¹ représentent ensemble un groupe alkylène en C₃-C₆ ou hétéroalkylène en C₃-C₆, qui peuvent éventuellement porter un ou plusieurs substituants identiques ou différents halogéno, oxo, hydroxy, cyano, nitro, alkyle en C₁-C₃, fluoroalkyle(C₁-C₃), alcoxy en C₁-C₃, fluoroalcoxy(C₁-C₃), alcoxy(C₁-C₃)-alkyle(C₁-C₃), alkyl(C₁-C₃)carbonyle, et dans lequel « * » désigne le point d'attachement au reste de la molécule,
R⁸ représente un groupe alkyle en C₁-C₆,
R⁹ représente l'un des groupes suivants 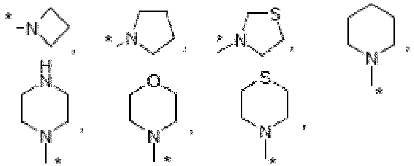 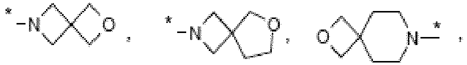 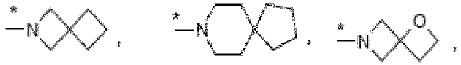 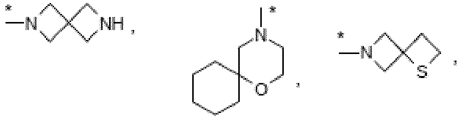 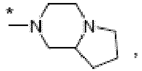 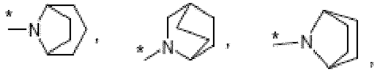 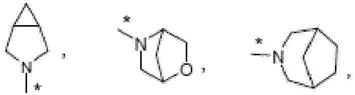 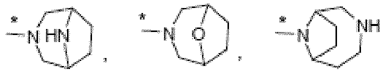 qui peuvent éventuellement porter un ou plusieurs substituants identiques ou différents alkyle en C₁-C₄, cycloalkyle en C₃-C₈, hétérocycloalkyle en C₃-C₈, fluoro-alkyle(C₁-C₆), aryle, aryloxy, aryl-alkyle(C₁-C₂), -C(=O)-O-alkyle (C₁-C₆), hétéroaryle, hydroxy, alcoxy en C₁-C₆, fluoroalcoxy(C₁-C₆), halogéno, cyano ou oxo, et dans lesquels « * » désigne le point d'attachement au reste de la molécule, et
R¹⁵ représente un groupe alkyle en C₁-C₃, trifluorométhyle, phényle ou benzyle, le groupe phényle et le fragment phényle contenu dans le groupe benzyle pouvant pour leur part éventuellement porter un ou plusieurs substituants identiques ou différents fluoro, chloro, bromo, méthyle ou méthoxy,
ainsi que leurs diastéréoisomères, racémates, tautomères, polymorphes, produits de solvatation et sels physiologiquement acceptables.

4. Composés de formule générale (I), selon les revendications 1 à 3, dans lesquels
R¹ représente un atome d'hydrogène, un groupe hydroxy, alkyle en C₁-C₃, fluoroalkyle(C₁-C₃), alcoxy en C₁-C₃ ou fluoroalcoxy(C₁-C₃),
ou
représente un groupe hétéroaryle ayant 5 atomes formant le cycle, qui peut éventuellement porter un ou plusieurs substituants identiques ou différents alkyle en C₁-C₃ ou alcoxy en C₁-C₃,
R² représente le groupe méthyle,
R³ représente un groupe -C(=O)-OR⁸, -C(=O)-R⁹ ou -C(=O)-NR⁶R⁷,
ou
représente l'un des systèmes cycliques suivants 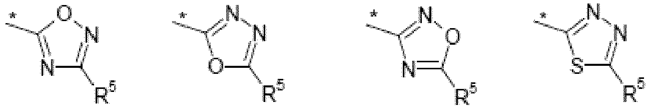 dans lesquels « * » désigne le point d'attachement au reste de la molécule,
R⁴ représente un atome de chlore,
R⁵ représente un atome d'hydrogène, un groupe alkyle en C₁-C₃, cycloalkyle en C₃-C₆, hétérocycloalkyle en C₃-C₆, phényle, hétéroaryle à 5 ou 6 chaînons, alcoxy(C₁-C₃)-alkyle(C₁-C₃), phénoxyalkyle (C₁-C₃) ou benzyloxy-alkyle(C₁-C₃),
R⁶ et R⁷ représentent indépendamment l'un de l'autre un atome d'hydrogène ou un groupe -NH-C(=O)-R¹⁵,
ou
représentent un groupe alkyle en C₁-C₆, hétérocycloalkyle en C₃-C₈, phényle, hétéroaryle ayant 5 ou 6 chaînons formant le cycle ou cycloalkyle en C₃-C₈, qui peuvent éventuellement porter un ou plusieurs substituants identiques ou différents alkyle en C₁-C₃, fluoroalkyle (C₁-C₃), alcoxy en C₁-C₃, phénoxy, hydroxy, oxo, halogéno, cyano, cycloalkyle en C₃-C₈, hétérocycloalkyle en C₃-C₈, hétéroaryle à 5 ou 6 chaînons ou phényle,
ou représentent le groupe 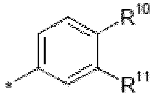 dans lequel
R¹⁰ et R¹¹ représentent ensemble un groupe alkylène en C₃-C₄ ou hétéroalkylène en C₃-C₄, qui peuvent éventuellement porter un ou plusieurs substituants identiques ou différents halogéno, oxo, hydroxy, alkyle en C₁-C₃, alcoxy en C₁-C₃, alkyl(C₁-C₃)carbonyle, et dans lequel « * » désigne le point d'attachement au reste de la molécule,
R⁸ représente un groupe alkyle en C₁-C₄,
R⁹ représente l'un des groupes suivants 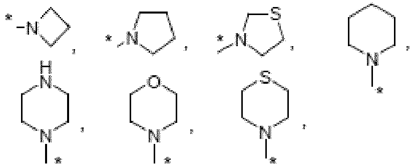 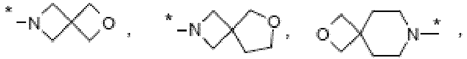 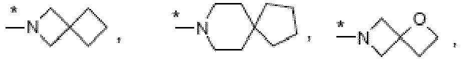 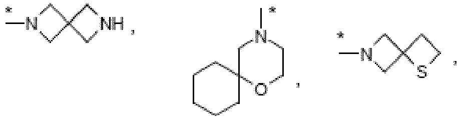 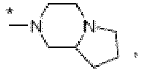 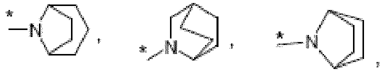 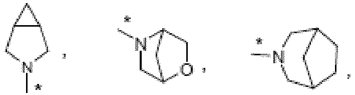 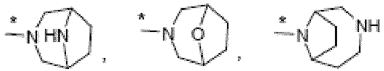 qui peuvent éventuellement porter un ou plusieurs substituants identiques ou différents alkyle en C₁-C₄, phényle, phénoxy, benzyle, -C(=O)-O-alkyle(C₁-C₄), hétéroaryle à 5 ou 6 chaînons, hydroxy, alcoxy en C₁-C₄, fluoro, cyano ou oxo, et dans lesquels « * » désigne le point d'attachement au reste de la molécule, et
R¹⁵ représente un groupe alkyle en C₁-C₃,
ainsi que leurs diastéréoisomères, racémates, tautomères, polymorphes, produits de solvatation et sels physiologiquement acceptables.

5. Composés de formule générale (I), selon les revendications 1 à 4, dans lesquels
R¹ représente un atome d'hydrogène, un groupe alcoxy en C₁-C₃ ou fluoroalcoxy(C₁-C₃),
ou
représente un groupe oxazolyle ou isoxazolyle, qui peuvent éventuellement porter un ou plusieurs substituants identiques ou différents alkyle en C₁-C₃ ou alcoxy en C₁-C₃,
R² représente le groupe méthyle,
R³ représente un groupe -C(=O)-OR⁸, -C(=O)-R⁹ ou -C(=O)-NR⁶R⁷,
ou
représente l'un des systèmes cycliques suivants 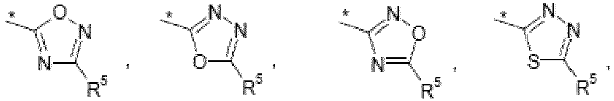 dans lesquels « * » désigne le point d'attachement au reste de la molécule,
R⁴ représente un atome de chlore,
R⁵ représente un atome d'hydrogène, un groupe alkyle en C₁-C₃, cycloalkyle en C₃-C₆, pyridinyle ou benzyloxy-alkyle(C₁-C₃),
R⁶ et R⁷ représentent indépendamment l'un de l'autre un atome d'hydrogène ou un groupe -NH-C(=O)-R¹⁵,
ou
représentent un groupe alkyle en C₁-C₆, qui peut éventuellement porter un ou plusieurs substituants identiques ou différents hétérocycloalkyle en C₃-C₈, ou représentent le groupe 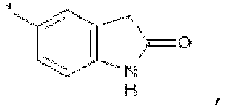 dans lequel « * » désigne le point d'attachement au reste de la molécule,
R⁸ représente le groupe éthyle ou *tert*-butyle,
R⁹ représente l'un des groupes suivants 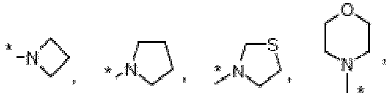 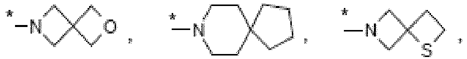 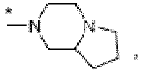 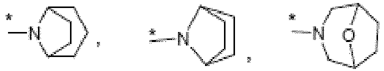 qui peuvent éventuellement porter un ou plusieurs substituants identiques ou différents alkyle en C₁-C₄, phénoxy, benzyle, -C(=O)-O-alkyle(C₁-C₄), fluoro ou oxo, et dans lesquels « * » désigne le point d'attachement au reste de la molécule, et
R¹⁵ représente un groupe alkyle en C₁-C₃,
ainsi que leurs diastéréoisomères, racémates, tautomères, polymorphes, produits de solvatation et sels physiologiquement acceptables.

6. Composés de formule générale (I), selon les revendications 1 à 5, dans lesquels
R¹ représente un atome d'hydrogène, le groupe méthoxy, trifluorométhoxy ou 3,5-diméthylisoxazol-4-yle,
R² représente le groupe méthyle,
R³ représente un groupe -C(=O)-OR⁸, -C(=O)-R⁹ ou -C(=O)-NR⁶R⁷,
ou
représente l'un des systèmes cycliques suivants 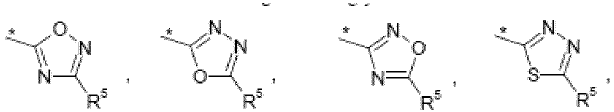 dans lesquels « * » désigne le point d'attachement au reste de la molécule,
R⁴ représente un atome de chlore,
R⁵ représente le groupe méthyle, iso-propyle, cyclopropyle, pyridin-3-yle ou benzyloxy-méthyle,
R⁶ et R⁷ représentent indépendamment l'un de l'autre un atome d'hydrogène ou un groupe -NH-C(=O)-R¹⁵,
ou
représentent un groupe éthyle qui peut éventuellement être une fois substitué par morpholinyle, ou représentent le groupe 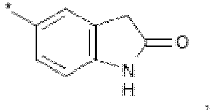 dans lequel « * » désigne le point d'attachement au reste de la molécule,
R⁸ représente le groupe éthyle ou *tert*-butyle,
R⁹ représente l'un des groupes suivants 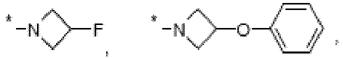 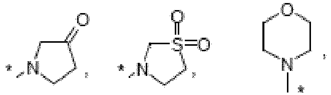 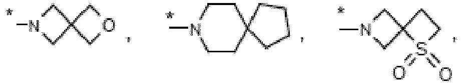 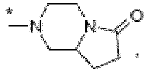 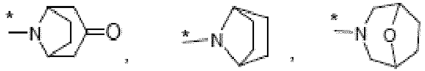 dans lesquels « * » désigne le point d'attachement au reste de la molécule, et
R¹⁵ représente le groupe méthyle,
ainsi que leurs diastéréoisomères, racémates, tautomères, polymorphes, produits de solvatation et sels physiologiquement acceptables.

7. Composés de formule générale (I), selon les revendications 1 à 6 :
[6-(4-chlorophényl)-1-méthyl-4H-[1,2,4]triazolo[4,3-a]-[1]benzazépin-4-yl]acétate d'éthyle ;
(-)-(4*R*)-[6-(4-chlorophényl)-1-méthyl-4H-[1,2,4]-triazolo[4,3-a][1]benzazépin-4-yl]acétate d'éthyle ;
2-[6-(4-chlorophényl)-1-méthyl-4H-[1,2,4]triazolo[4,3-a][1]benzazépin-4-yl]-1-(2-oxa-6-azaspiro[3.3]hept-6-yl)éthanone ;
(-)-2-[(4*R*)-6-(4-chlorophényl)-1-méthyl-4H-[1,2,4]-triazolo[4,3-a][1]benzazépin-4-yl]-1-(2-oxa-6-azaspiro[3.3]hept-6-yl)éthanone ;
1-(7-azabicyclo[2.2.1]hept-7-yl)-2-[6-(4-chlorophényl)-1-méthyl-4H-[1,2,4]triazolo[4,3-a][1]benzazépin-4-yl]-éthanone ;
(-)-1-(7-azabicyclo[2.2.1]hept-7-yl)-2-[(4*R*)-6-(4-chlorophényl)-1-méthyl-4H-[1,2,4]triazolo[4,3-a][1]-benzazépin-4-yl]éthanone ;
[6-(3-chlorophényl)-1-méthyl-4H-[1,2,4]triazolo[4,3-a][1]benzazépin-4-yl]acétate d'éthyle ;
[6-(4-chlorophényl)-1-méthyl-4H-[1,2,4]triazolo[4,3-a][1]benzazépin-4-yl]acétate de *tert*-butyle ;
(-)-[(4*R*)-6-(4-chlorophényl)-1-méthyl-4H-[1,2,4]-triazolo[4,3-a][1]benzazépin-4-yl]acétate de tert-butyle ;
2-[6-(3-chlorophényl)-1-méthyl-4H-[1,2,4]triazolo[4,3-a][1]benzazépin-4-yl]-1-(2-oxa-6-azaspiro[3.3]hept-6-yl)éthanone ;
(-)-2-[(4*R*)-6-(3-chlorophényl)-1-méthyl-4H-[1,2,4]-triazolo[4,3-a][1]benzazépin-4-yl]-1-(2-oxa-6-azaspiro[3.3]hept-6-yl)éthanone ;
2-[6-(4-chlorophényl)-1-méthyl-4H-[1,2,4]triazolo[4,3-a][1]benzazépin-4-yl]-N-éthylacétamide ;
(-)-2-[(4*R*)-6-(4-chlorophényl)-1-méthyl-4H-[1,2,4]-triazolo[4,3-a][1]benzazépin-4-yl]-N-éthylacetamide ;
2-[6-(4-chlorophényl)-l-méthyl-4H-[1,2,4]triazolo[4,3-a][1]benzazépin-4-yl]-1-(morpholin-4-yl)éthanone ;
(-)-2-[(4R)-6-(4-Chlorophényl)-1-méthyl-4H-[1,2,4]-triazolo[4,3-a][1]benzazépin-4-yl]-1-(morpholin-4-yl)-éthanone ;
2-[6-(4-chlorophényl)-1-méthyl-4H-[1,2,4]triazolo[4,3-a][1]benzazépin-4-yl]-1-(3-fluoroazetidin-1-yl)-éthanone ;
(-)-2-[(4*R*)-6-(4-chlorophényl)-1-méthyl-4H-[1,2,4]-triazolo[4,3-a][1]benzazépin-4-yl]-1-(3-fluoroazétidin-1-yl)éthanone ;
2-[6-(4-chlorophényl)-1-méthyl-4H-[1,2,4]triazolo[4,3-a][1]benzazépin-4-yl]-1-(1,1-dioxydo-1-thia-6-azaspiro-[3.3]hept-6-yl)éthanone ;
(-)-2-[(4*R*)-6-(4-chlorophényl)-1-méthyl-4H-[1,2,4]-triazolo[4,3-a][1]benzazépin-4-yl]-1-(1,1-dioxydo-1-thia-6-azaspiro[3.3]hept-6-yl)éthanone ;
2-[6-(4-chlorophényl)-l-méthyl-4H-[1,2,4]triazolo[4,3-a][1]benzazépin-4-yl]-N-(2-oxo-2,3-dihydro-1H-indol-5-yl)acétamide ;
(-)-2-[(4*R*)-6-(4-chlorophényl)-1-méthyl-4H-[1,2,4]-triazolo[4,3-a][1]benzazépin-4-yl]-N-(2-oxo-2,3-dihydro-1H-indol-5-yl)acétamide ;
3-{[(-6-(4-chlorophényl)-1-méthyl-4H-[1,2,4]triazolo-[4,3-a][1]benzazépin-4-yl]acétyl}-8-azabicyclo[3.2.1]-octan-3-one ;
(-)-3-{[(4R)-6-(4-chlorophényl)-1-méthyl-4H-[1,2,4]-triazolo[4,3-a][1]benzazépin-4-yl]acétyl}-8-azabicyclo-[3.2.1]octan-3-one ;
2-[6-(4-chlorophényl)-1-méthyl-4H-[1,2,4]triazolo[4,3-a][1]benzazépin-4-yl]-1-(3-phénoxyazetidin-1-yl)-éthanone ;
(-)-2-[(4R)-6-(4-chlorophényl)-1-méthyl-4H-[1,2,4]-triazolo[4,3-a][1]benzazépin-4-yl]-1-(3-phénoxy-azétidin-1-yl)éthanone ;
1-{[6-(4-chlorophényl)-1-méthyl-4H-[1,2,4]triazolo[4,3-a][1]benzazépin-4-yl]acétyl}pyrrolidin-3-one ;
(-)-1-{[(4R)-6-(4-chlorophényl)-1-méthyl-4H-[1,2,4]-triazolo[4,3-a][1]benzazépin-4-yl]acétyl}pyrrolidin-3-one ;
1-(8-azaspiro[4.5]déc-8-yl)-2-[6-(4-chlorophényl)-1-méthyl-4H-[1,2,4]triazolo[4,3-a][1]benzazépin-4-yl]-éthanone ;
(-)-1-(8-azaspiro[4.5]déc-8-yl)-2-[(4R)-6-(4-chloro-phényl)-1-méthyl-4H-[1,2,4]triazolo[4,3-a][1]-benzazépin-4-yl]éthanone ;
2-[6-(4-chlorophényl)-1-méthyl-4H-[1,2,4]triazolo[4,3-a][1]benzazépin-4-yl]-1-(1,1-dioxydo-1,3-thiazolidin-3-yl)éthanone ;
(-)-2-[(4R)-6-(4-chlorophényl)-1-méthyl-4H-[1,2,4]-triazolo[4,3-a][1]benzazépin-4-yl]-1-(1,1-dioxydo-1,3-thiazolidin-3-yl)éthanone ;
2-[6-(4-chlorophényl)-1-méthyl-4H-[1,2,4]triazolo[4,3-a][1]benzazépin-4-yl]-1-(8-oxa-3-azabicyclo[3.2.1]oct-3-yl)éthanone ;
(-)-2-[(4R)-6-(4-chlorophényl)-1-méthyl-4H-[1,2,4]-triazolo[4,3-a][1]benzazépin-4-yl]-1-(8-oxa-3-azabicyclo[3.2.1]oct-3-yl)éthanone ;
6-(4-chlorophényl)-1-méthyl-4-[(3-méthyl-1,2,4-oxadiazol-5-yl)méthyl]-4H-[1,2,4]triazolo[4,3-a][1]-benzazépine ;
(-)-6-[(4R)-4-chlorophényl]-1-méthyl-4-[(3-méthyl-1,2,4-oxadiazol-5-yl)méthyl]-4H-[1,2,4]triazolo[4,3-a]-[1]benzazépine ;
(-)-(4R)-6-(4-chlorophényl)-1-méthyl-4-{[3-(propan-2-yl)-1,2,4-oxadiazol-5-yl]méthyl}-4H-[1,2,4]triazolo-[4,3-a][1]benzazépine ;
(-)-6-(4R)-(4-chlorophényl)-4-[(3-cyclopropyl-1,2,4-oxadiazol-5-yl)méthyl]-1-méthyl-4H-[1,2,4]triazolo[4,3-a][1]benzazépine ;
(-)-(4*R*)-6-(4-chlorophényl)-1-méthyl-4-[(5-méthyl-1,3,4-oxadiazol-2-yl)méthyl]-4H-[1,2,4]triazolo[4,3-a]-[1]benzazépine ;
6-(4-chlorophényl)-1-méthyl-4-[(5-méthyl-1,3,4-oxadiazol-2-yl)méthyl]-4H-[1,2,4]triazolo[4,3-a][1]-benzazépine ;
N'-acétyl-2-[(4*R*)-6-(4-chlorophényl)-1-méthyl-4H-[1,2,4]triazolo[4,3-a][1]benzazépin-4-yl]acéto-hydrazide ;
6-(4-chlorophényl)-1-méthyl-4-[(5-méthyl-1,3,4-thiadiazol-2-yl)méthyl]-4H-[1,2,4]triazolo[4,3-a][1]-benzazépine ;
(+)-(4*R*)-6-(4-chlorophényl)-1-méthyl-4-[(5-méthyl-1,3,4-thiadiazol-2-yl)méthyl]-4H-[1,2,4]triazolo[4,3-a][1]benzazépine ;
6-(4-chlorophényl)-4-[(5-cyclopropyl-1,3,4-oxadiazol-2-yl)méthyl]-1-méthyl-4H-[1,2,4]triazolo[4,3-a][1]-benzazépine ;
6-(4-chlorophényl)-1-méthyl-4-{[5-(pyridin-3-yl)-1,3,4-oxadiazol-2-yl]méthyl}-4H-[1,2,4]triazolo[4,3-a][1]-benzazépine ;
(-)-(4*R*)-6-(4-chlorophényl)-1-méthyl-4-{[5-(pyridin-3-yl)-1,3,4-oxadiazol-2-yl]méthyl}-4H-[1,2,4]triazolo-[4,3-a][1]benzazépine ;
6-(4-chlorophényl)-1-méthyl-4-[(5-méthyl-1,2,4-oxadiazol-3-yl)méthyl]-4H-[1,2,4]triazolo[4,3-a][1]-benzazépine ;
6-(4-chlorophényl)-8-méthoxy-1-méthyl-4-[(5-méthyl-1,3,4-oxadiazol-2-yl)méthyl]-4H-[1,2,4]triazolo[4,3-a][1]benzazépine ;
6-(4-chlorophényl)-8-(3,5-diméthyl-1,2-oxazol-4-yl)-1-méthyl-4-[(5-méthyl-1,3,4-oxadiazol-2-yl)méthyl]-4H-[1,2,4]triazolo[4,3-a][1]benzazépine ;
[6-(4-chlorophényl)-1-méthyl-8-(trifluorméthoxy)-4H-[1,2,4]triazolo[4,3-a][1]benzazépin-4-yl]acétate de *tert*-butyle ;
(-)-[(4*R*)-6-(4-chlorophényl)-1-méthyl-8-(trifluoro-méthoxy)-4H-[1,2,4]triazolo[4,3-a][1]benzazépin-4-yl]-acétate de *tert*-butyle ;
2-[(4*R*)-6-(4-chlorophényl)-1-méthyl-8-(trifluoro-méthoxy)-4H-[1,2,4]triazolo[4,3-a][1,4]benzazépin-4-yl]-1-(2-oxa-6-azaspiro[3.3]hept-6-yl)éthanone ;
2-[(4*R*)-6-(4-chlorophényl)-1-méthyl-8-(trifluoro-méthoxy)-4H-[1,2,4]triazolo[4,3-a][1]benzazépin-4-yl]-N-éthylacétamide ;
2-[(4*R*)-6-(4-chlorophényl)-1-méthyl-8-(trifluoro-méthoxy)-4H-[1,2,4]triazolo[4,3-a][1]benzazépin-4-yl]-N-[2-(morpholin-4-yl)éthyl]acétamide ;
6-(4-chlorophényl)-1-méthyl-4-[(5-méthyl-1,3,4-oxadiazol-2-yl)méthyl]-8-(trifluorométhoxy)-4H-[1,2,4]-triazolo[4,3-a][1]benzazépine ;
(4*R*)-6-(4-chlorophényl)-1-méthyl-4-[(5-méthyl-1,3,4-oxadiazol-2-yl)méthyl]-8-(trifluorométhoxy)-4H-[1,2,4]-triazolo[4,3-a][1]benzazépine ;
2-{[(4*R*)-6-(4-chlorophényl)-1-méthyl-8-(trifluoro-méthoxy)-4H-[1,2,4]triazolo[4,3-a][1]benzazépin-4-yl]acétyl}hexahydropyrrolo[1,2-a]pyrazin-6(2H)-one ;
(4*R*)-4-({5-[(benzyloxy)méthyl]-1,3,4-oxadiazol-2-yl}-méthyl)-6-(4-chlorophényl)-1-méthyl-4H-[1,2,4]triazolo-[4,3-a][1]benzazépine.

8. Utilisation des composés selon les revendications 1 à 7, pour la fabrication d'un médicament.

9. Utilisation des composés selon les revendications 1 à 7, pour la fabrication d'un médicament destiné à la prophylaxie et/ou à la thérapie de maladies tumorales.

10. Utilisation des composés selon les revendications 1 à 7, pour la fabrication d'un médicament destiné à la prophylaxie et/ou la thérapie d'infections virales, de maladies neurodégénératives, de maladies inflammatoires, de maladies athéro-scléreuses et dans la régulation de la fertilité masculine.

11. Composés selon les revendications 1 à 7, en association avec une ou plusieurs autres substances pharmacologiquement actives.

12. Composés en association selon la revendication 11, destinés à la prophylaxie et/ou la thérapie de maladies hyperprolifératives.

13. Composés en association selon la revendication 11, destinés à la prophylaxie et/ou la thérapie de maladies tumorales.

14. Composés en association selon la revendication 11, destinés à la prophylaxie et/ou la thérapie d'infections virales, de maladies neurodégénératives, de maladies inflammatoires, de maladies athéroscléreuses et dans la régulation de la fertilité masculine.
